# EUROPEAN PATENT SPECIFICATION

(11) **EP 4 294 804 B1**
(45) Date of publication and mention of the grant of the patent: **08.04.2026**
(21) Application number: 22707995.1
(22) Date of filing: 18.02.2022
(51) Int. Cl.: C07D 471/04, C07D 519/00, A61P 35/00, A61K 31/4375, A61K 31/519

(54) **PYRIDONE COMPOUNDS AND METHODS OF USE**
PYRIDONVERBINDUNGEN UND VERFAHREN ZUR VERWENDUNG
COMPOSÉS DE PYRIDONE ET LEURS PROCÉDÉS D'UTILISATION

(30) Priority: 19.02.2021 US 202163151334 P; 31.03.2021 US 202163168968 P
(43) Date of publication of application: 27.12.2023
(73) Proprietor: Exelixis, Inc., Alameda, CA 94502 (US)
(72) Inventor: BANNEN, Lynne, Alameda, California 94502 (US); XU, Wei, Alameda, California 94502 (US); RAUB, Andrew, Alameda, California 94502 (US); JIANG, Faming, Alameda, California 94502 (US); JEONG, Joon Won, Alameda, California 94502 (US); TSO, Kin, Alameda, California 94502 (US); SALVANT, Justin, Alameda, California 94502 (US)
(74) Representative: J A Kemp LLP
(86) International application number: PCT/US2022/016915
(87) International publication number: WO 2022/178205

(56) References cited:
- EP-A1- 3 026 045
- EP-A1- 3 239 147
- WO-A1-2007/033196
- WO-A1-2013/074633
- WO-A1-2013/180949
- WO-A1-2021/062245
- WO-A1-2021/173591
- CN-A- 104 072 480
- CN-A- 108 530 426
- WEI ZHANG ET AL: "Discovery of novel type II c-Met inhibitors based on BMS-777607", EUROPEAN JOURNAL OF MEDICINAL CHEMISTRY, vol. 80, 1 June 2014 (2014-06-01), AMSTERDAM, NL, pages 254 - 266, XP055394473, ISSN: 0223-5234, DOI: 10.1016/j.ejmech.2014.04.056
- LI SAI ET AL: "Design, synthesis and antitumour activity of bisquinoline derivatives connected by 4-oxy-3-fluoroaniline moiety", EUROPEAN JOURNAL OF MEDICINAL CHEMISTRY, ELSEVIER, AMSTERDAM, NL, vol. 64, 15 April 2013 (2013-04-15), pages 62 - 73, XP028566334, ISSN: 0223-5234, DOI: 10.1016/J.EJMECH.2013.04.001
- QIDONG TANG ET AL: "Synthesis and antiproliferative activity of 6,7-disubstituted-4-phenoxyquinoline derivatives bearing the 1,8-naphthyridin-2-one moiety", EUROPEAN JOURNAL OF MEDICINAL CHEMISTRY, vol. 158, 1 October 2018 (2018-10-01), AMSTERDAM, NL, pages 201 - 213, XP055748184, ISSN: 0223-5234, DOI: 10.1016/j.ejmech.2018.08.066
- WEI ZHANG ET AL: "Discovery of Novel c-Met Inhibitors Bearing a 3-Carboxyl Piperidin-2-one Scaffold", MOLECULES, vol. 19, no. 2, 24 February 2014 (2014-02-24), pages 2655 - 2673, XP055460997, DOI: 10.3390/molecules19022655
- LIU JU ET AL: "Design, synthesis and biological evaluation of novel 4-phenoxyquinoline derivatives containing 3-oxo-3,4-dihydroquinoxaline moiety as c-Met kinase inhibitors", BIOORGANIC, ELSEVIER, AMSTERDAM, NL, vol. 25, no. 16, 27 June 2017 (2017-06-27), pages 4475 - 4486, XP085133591, ISSN: 0968-0896, DOI: 10.1016/J.BMC.2017.06.037
- TANG QIDONG ET AL: "Design, synthesis, and structure-activity relationships of novel 6,7-disubstituted-4-phenoxyquinoline derivatives as potential antitumor ag", EUROPEAN JOURNAL OF MEDICINAL CHEMISTRY, ELSEVIER, AMSTERDAM, NL, vol. 69, 19 August 2013 (2013-08-19), pages 77 - 89, XP028762794, ISSN: 0223-5234, DOI: 10.1016/J.EJMECH.2013.08.019

## Description

### FIELD

Provided herein are compounds and pharmaceutical compositions suitable as modulators of protein kinases, and their use in methods of treating disorders mediated, at least in part by, protein kinases.

### BACKGROUND

Human Axl belongs to the TAM subfamily of receptor tyrosine kinases that includes Mer. TAM kinases are characterized by an extracellular ligand binding domain consisting of two immunoglobulin-like domains and two fibronectin type III domains. Axl is overexpressed in a number of tumor cell types and was initially cloned from patients with chronic myelogenous leukemia. When overexpressed, Axl exhibits transforming potential. Axl signaling is believed to cause tumor growth through activation of proliferative and anti-apoptotic signaling pathways. Axl has been associated with cancers including, but not limiting to lung cancer, myeloid leukemia, uterine cancer, ovarian cancer, gliomas, melanoma, thyroid cancer, renal cell carcinoma, osteosarcoma, gastric cancer, prostate cancer, and breast cancer. The over-expression of Axl results in a poor prognosis for patients with the indicated cancers.

Activation of Mer, like Axl, conveys downstream signaling pathways that cause tumor growth and activation. Mer binds ligands such as the soluble protein Gas-6. Gas-6 binding to Mer induces autophosphorylation of Mer on its intracellular domain, resulting in downstream signal activation. Over-expression of Mer in cancer cells leads to increased metastasis, most likely by generation of soluble Mer extracellular domain protein as a decoy receptor. Tumor cells secrete a soluble form of the extracellular Mer receptor which reduces the ability of soluble Gas-6 ligand to activate Mer on endothelial cells, leading to cancer progression.

c-Met, is the prototypic member of a subfamily of heterodimeric receptor tyrosine kinases (RTKs) which include Met, Ron and Sea. Expression of c-Met occurs in a wide variety of cell types including epithelial, endothelial and mesenchymal cells where activation of the receptor induces cell migration, invasion, proliferation and other biological activities associated with invasive cell growth. Signal transduction through c-Met receptor activation is responsible for many of the characteristics of tumor cells.

KDR is a tyrosine kinase receptor that binds vascular endothelial growth factor (VEGF). The binding of VEGF to the KDR receptor leads to angiogenesis. High levels of VEGF are found in various cancers causing tumor angiogenesis and permitting the rapid growth of cancerous cells.

Therefore, a need exists for new compounds that modulate Axl, Mer, c-Met, and/or KDR kinases for the treatment of cancers.

Wei Zhang et al: EJMC, vol. 80, 1 June 2014, pages 254-266 discusses type II c-Met inhibitors based on BMS-777607. WO 2013/180949 discusses substituted quinoline compounds, pharmaceutical acceptable salts and formulations thereof useful in modulating the protein tyrosine kinase activity. CN 108530426 discusses quinoxalinone-containing 4-phenoxy substituted quinoline derivatives with c-Met inhibitory activity. CN 104072480 discusses quinoline derivatives with c-Met inhibitory activity. EP 3239147 discusses quinoline derivatives with Axl inhibitory activity. WO 2007/033196 discusses compounds that are useful for treating cancer. Li Sai et al: EJMC, vol. 64, 15 April 2013, pages 62-73 disucsses the design, synthesis and antitumour activity of bisquinoline derivatives connected by 4-oxy-3-fluoroaniline moiety. Qidong Tang et al: EJMC vol. 158, 1 October 2018, pages 201 -213, discusses synthesis and antiproliferative activity of 6,7-disubstituted-4-phenoxyquinoline derivatives bearing the 1,8-naphthyridin-2-one moiety. Wei Zhang et al: Molecules, vol. 19, no. 2, 24 February 2014, pages 2655-2673 discusses c-Met inhibitors bearing a 3-carboxyl piperidin-2-one scaffold. Liu Ju et al: Bioorganic, vol. 25, no. 16, 27 June 2017, pages 4475-4486 discusses th design, synthesis and biological evaluation of 4-phenoxyquinoline derivatives containing 3-oxo-3,4-dihydroquinoxaline moiety as c-Met kinase inhibitors. EP 3 026 045 discusses compounds with Axl inhibitory activity. Tang Qidong et al: , EJMC, vol. 69, 19 August 2013 (2013-08-19), pages 77-89 discusses design, synthesis, and structure-activity relationships of 6,7-disubstituted-4-phenoxyquinoline derivatives as potential antitumor agents. WO 2013/074633 discusses uracil derivatives as Axl and c-Met inhibitors.

### SUMMARY

Provided herein are compounds that inhibit c-Met, Axl, Mer and/or KDR. In certain embodiments, the compounds are of Formula (Ia), Formula (Ib), or Formula (Ic) as described in the detailed description section: 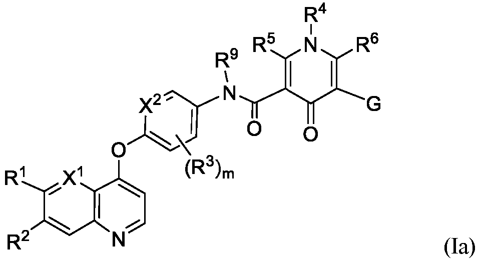 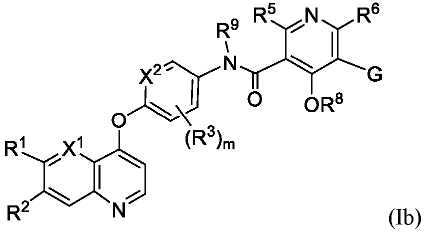 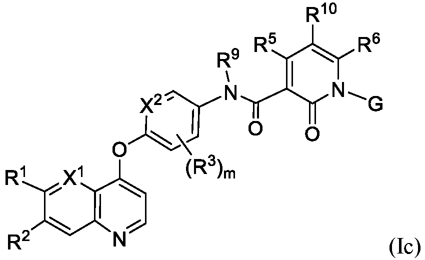 or a pharmaceutically acceptable salt, stereoisomer, or tautomer thereof.

Some embodiments provide for a compound, or a pharmaceutically acceptable salt, stereoisomer, or tautomer thereof, selected from Table 1.

Also provided herein are pharmaceutical compositions comprising a compound as described herein, or a pharmaceutically acceptable salt, stereoisomer, or tautomer thereof, and a pharmaceutically acceptable carrier or excipient.

The invention also provides a compound of the invention or a pharmaceutically acceptable salt, stereoisomer, or tautomer thereof, or a pharmaceutical composition of the invention, for use in a method of treating a disease, disorder, or syndrome which is mediated by modulating in vivo activity of a protein kinase, optionally wherein the protein kinase is AXL, KDR, Mer, or Met.

Also provided herein are compositions, including pharmaceutical compositions, kits that include the compounds, and method of using (or administering) and making the compounds. Also provided herein are compounds or compositions for use in a method of treating a disease, disorder, or condition that is mediated, at least in part, by c-Met, Axl, Mer and/or KDR activity.

The invention also provides a process for preparing a compound of Formula (Ia), Formula (Ib), or Formula (Ic), or a pharmaceutically acceptable salt, stereoisomer or tautomer thereof, wherein: the process comprises contacting a compound of Formula A-a, Formula A-b, or Formula A-c: 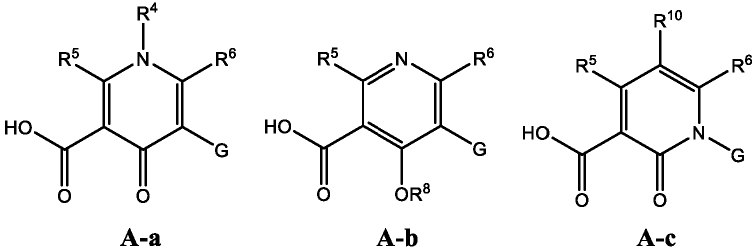
with a compound of Formula B-c: 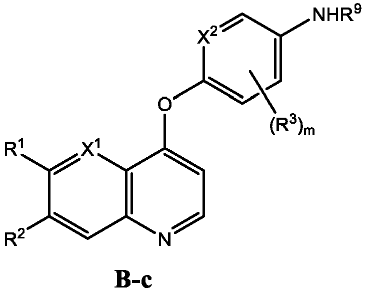
under amide bond forming conditions to provide the compound of Formula (Ia), Formula (Ib), or Formula (Ic), or a pharmaceutically acceptable salt, stereoisomer or tautomer thereof, wherein: X¹, X², R¹, R², R³, R⁴, R⁵, R⁶, R⁸, R⁹, R¹⁰, G and m are as defined herein.

### DETAILED DESCRIPTION

### Definitions

As used in the present specification, the following words, phrases and symbols are generally intended to have the meanings as set forth below, except to the extent that the context in which they are used indicates otherwise.

A dash ("-") that is not between two letters or symbols is used to indicate a point of attachment for a substituent. For example, -C(O)NH₂ is attached through the carbon atom. A dash at the front or end of a chemical group is a matter of convenience; chemical groups may be depicted with or without one or more dashes without losing their ordinary meaning. A wavy line or a dashed line drawn through or perpendicular across the end of a line in a structure indicates a specified point of attachment of a group. Unless chemically or structurally required, no directionality or stereochemistry is indicated or implied by the order in which a chemical group is written or named.

The prefix "Cᵤ₋ᵥ" indicates that the following group has from u to v carbon atoms. For example, "C₁₋₆ alkyl" indicates that the alkyl group has from 1 to 6 carbon atoms.

Reference to "about" a value or parameter herein includes (and describes) embodiments that are directed to that value or parameter per se. In certain embodiments, the term "about" includes the indicated amount ± 10%. In other embodiments, the term "about" includes the indicated amount ± 5%. In certain other embodiments, the term "about" includes the indicated amount ± 1%. Also, to the term "about X" includes description of "X". Also, the singular forms "a" and "the" include plural references unless the context clearly dictates otherwise. Thus, e.g., reference to "the compound" includes a plurality of such compounds and reference to "the assay" includes reference to one or more assays and equivalents thereof known to those skilled in the art.

"Alkyl" refers to an unbranched or branched saturated hydrocarbon chain. As used herein, alkyl has 1 to 20 carbon atoms (i.e., C₁₋₂₀ alkyl), 1 to 12 carbon atoms (i.e., C₁₋₁₂ alkyl), 1 to 8 carbon atoms (i.e., C₁₋₈ alkyl), 1 to 6 carbon atoms (i.e., C₁₋₆ alkyl) or 1 to 4 carbon atoms (i.e., C₁₋₄ alkyl). Examples of alkyl groups include, e.g., methyl, ethyl, propyl, isopropyl, n-butyl, sec-butyl, iso-butyl, tert-butyl, pentyl, 2-pentyl, isopentyl, neopentyl, hexyl, 2-hexyl, 3-hexyl and 3-methylpentyl. When an alkyl residue having a specific number of carbons is named by chemical name or identified by molecular formula, all positional isomers having that number of carbons may be encompassed; thus, for example, "butyl" includes n-butyl (i.e., -(CH₂)₃CH₃), sec-butyl (i.e., -CH(CH₃)CH₂CH₃), isobutyl (i.e., -CH₂CH(CH₃)₂) and tert-butyl (i.e., -C(CH₃)₃); and "propyl" includes n-propyl (i.e., -(CH₂)₂CH₃) and isopropyl (i.e., -CH(CH₃)₂).

Certain commonly used alternative chemical names may be used. For example, a divalent group such as a divalent "alkyl" group, a divalent "aryl" group, etc., may also be referred to as an "alkylene" group or an "alkylenyl" group, an "arylene" group or an "arylenyl" group, respectively. Also, unless indicated explicitly otherwise, where combinations of groups are referred to herein as one moiety, e.g., arylalkyl or aralkyl, the last mentioned group contains the atom by which the moiety is attached to the rest of the molecule.

"Alkenyl" refers to an alkyl group containing at least one carbon-carbon double bond and having from 2 to 20 carbon atoms (i.e., C₂₋₂₀ alkenyl), 2 to 8 carbon atoms (i.e., C₂₋₈ alkenyl), 2 to 6 carbon atoms (i.e., C₂₋₆ alkenyl) or 2 to 4 carbon atoms (i.e., C₂₋₄ alkenyl). Examples of alkenyl groups include, e.g., ethenyl, propenyl, butadienyl (including 1,2-butadienyl and 1,3-butadienyl).

"Alkynyl" refers to an alkyl group containing at least one carbon-carbon triple bond and having from 2 to 20 carbon atoms (i.e., C₂₋₂₀ alkynyl), 2 to 8 carbon atoms (i.e., C₂₋₈ alkynyl), 2 to 6 carbon atoms (i.e., C₂₋₆ alkynyl) or 2 to 4 carbon atoms (i.e., C₂₋₄ alkynyl). The term "alkynyl" also includes those groups having one triple bond and one double bond.

"Alkoxy" refers to the group "alkyl-O-". Examples of alkoxy groups include, e.g., methoxy, ethoxy, n-propoxy, iso-propoxy, n-butoxy, tert-butoxy, sec-butoxy, n-pentoxy, n-hexoxy and 1,2-dimethylbutoxy.

"Alkylthio" refers to the group "alkyl-S-". "Alkylsulfinyl" refers to the group "alkyl-S(O)-". "Alkylsulfonyl" refers to the group "alkyl-S(O)₂-". "Alkylsulfonylalkyl" refers to -alkyl-S(O)₂-alkyl.

"Acyl" refers to a group -C(O)R^{y}, wherein R^{y} is hydrogen, alkyl, alkenyl, alkynyl, cycloalkyl, heterocycloalkyl, aryl, heteroalkyl or heteroaryl; each of which may be optionally substituted, as defined herein. Examples of acyl include, e.g., formyl, acetyl, cyclohexylcarbonyl, cyclohexylmethyl-carbonyl and benzoyl.

"Amido" refers to both a "C-amido" group which refers to the group -C(O)NR^{y}R^{z} and an "N-amido" group which refers to the group -NR^{y}C(O)R^{z}, wherein R^{y} and R^{z} are independently hydrogen, alkyl, alkenyl, alkynyl, cycloalkyl, heterocycloalkyl, aryl, heteroalkyl or heteroaryl; each of which may be optionally substituted, as defined herein, or R^{y} and R^{z} are taken together to form a cycloalkyl or heterocycloalkyl; each of which may be optionally substituted, as defined herein.

"Amino" refers to the group -NR^{y}R^{z} wherein R^{y} and R^{z} are independently hydrogen, alkyl, alkenyl, alkynyl, cycloalkyl, heterocycloalkyl, aryl, heteroalkyl or heteroaryl; each of which may be optionally substituted, as defined herein.

"Amidino" refers to -C(NR^{y})(NR^{z}₂), wherein R^{y} and R^{z} are independently hydrogen, alkyl, alkenyl, alkynyl, cycloalkyl, heterocycloalkyl, aryl, heteroalkyl or heteroaryl; each of which may be optionally substituted, as defined herein.

"Aryl" refers to an aromatic carbocyclic group having a single ring (e.g., monocyclic) or multiple rings (e.g., bicyclic or tricyclic) including fused systems. As used herein, aryl has 6 to 20 ring carbon atoms (i.e., C₆₋₂₀ aryl), 6 to 12 carbon ring atoms (i.e., C₆₋₁₂ aryl), or 6 to 10 carbon ring atoms (i.e., C₆₋₁₀ aryl). Examples of aryl groups include, e.g., phenyl, naphthyl, fluorenyl and anthryl. Aryl, however, does not encompass or overlap in any way with heteroaryl defined below. If one or more aryl groups are fused with a heteroaryl, the resulting ring system is heteroaryl. If one or more aryl groups are fused with a heterocycloalkyl, the resulting ring system is heterocycloalkyl.

"Arylalkyl" or "Aralkyl" refers to the group "aryl-alkyl-".

"Carbamoyl" refers to -C(O)NR^{y}R^{z}. "O-carbamoyl" refers to -O-C(O)NR^{y}R^{z} and "N-carbamoyl" refers to -NR^{y}C(O)OR^{z}, wherein R^{y} and R^{z} are independently hydrogen, alkyl, alkenyl, alkynyl, cycloalkyl, heterocycloalkyl, aryl, heteroalkyl or heteroaryl; each of which may be optionally substituted, as defined herein.

"Carboxyl ester" or "ester" refer to both -OC(O)R^{x} and -C(O)OR^{x}, wherein R^{x} is alkyl, alkenyl, alkynyl, cycloalkyl, heterocycloalkyl, aryl, heteroalkyl or heteroaryl; each of which may be optionally substituted, as defined herein.

"Cycloalkyl" refers to a saturated or partially unsaturated cyclic alkyl group having a single ring or multiple rings including fused, bridged and spiro ring systems. The term "cycloalkyl" includes cycloalkenyl groups (i.e., the cyclic group having at least one double bond) and carbocyclic fused ring systems having at least one sp³ carbon atom (i.e., at least one non-aromatic ring). As used herein, cycloalkyl has from 3 to 20 ring carbon atoms (i.e., C₃₋₂₀ cycloalkyl), 3 to 12 ring carbon atoms (i.e., C₃₋₁₂ cycloalkyl), 3 to 10 ring carbon atoms (i.e., C₃₋₁₀ cycloalkyl), 3 to 8 ring carbon atoms (i.e., C₃₋₈ cycloalkyl), or 3 to 6 ring carbon atoms (i.e., C₃₋₆ cycloalkyl). Monocyclic groups include, for example, cyclopropyl, cyclobutyl, cyclopentyl, cyclohexyl, cycloheptyl and cyclooctyl. Polycyclic groups include, for example, bicyclo[2.2.1]heptanyl, bicyclo[2.2.2]octanyl, adamantyl, norbornyl, decalinyl, 7,7-dimethyl-bicyclo[2.2.1]heptanyl and the like. In some embodiments, one or more ring carbons of "cycloalkyl" can be optionally replaced by a carbonyl group. Examples of such cycloalkyl include cyclohexanone-4-yl, and the like. Further, the term cycloalkyl is intended to encompass moieties that have one or more aromatic ring fused (i.e., having a bond in common with) to the cycloalkyl ring, e.g., benzo or thienyl derivatives of cyclopentane, cyclohexane, and the like. A cycloalkyl group containing a fused aromatic ring can be attached through any ring-forming atom including a ring-forming atom of the fused aromatic ring. Still further, cycloalkyl also includes "spirocycloalkyl" when there are two positions for substitution on the same carbon atom, for example spiro[2.5]octanyl, spiro[4.5]decanyl, or spiro[5.5]undecanyl.

"Cycloalkylalkyl" refers to the group "cycloalkyl-alkyl-".

"Guanidino" refers to -NR^{y}C(=NR^{z})(NR^{y}R^{z}), wherein each R^{y} and R^{z} are independently hydrogen, alkyl, alkenyl, alkynyl, cycloalkyl, heterocycloalkyl, aryl, heteroalkyl or heteroaryl; each of which may be optionally substituted, as defined herein.

"Hydrazino" refers to -NHNH₂.

"Imino" refers to a group -C(NR^{y})R^{z}, wherein R^{y} and R^{z} are each independently hydrogen, alkyl, alkenyl, alkynyl, cycloalkyl, heterocycloalkyl, aryl, heteroalkyl or heteroaryl; each of which may be optionally substituted, as defined herein.

"Imido" refers to a group -C(O)NR^{y}C(O)R^{z}, wherein R^{y} and R^{z} are each independently hydrogen, alkyl, alkenyl, alkynyl, cycloalkyl, heterocycloalkyl, aryl, heteroalkyl or heteroaryl; each of which may be optionally substituted, as defined herein.

"Halogen" or "halo" refers to atoms occupying group VIIA of the periodic table, such as fluoro, chloro, bromo or iodo.

"Haloalkyl" refers to an unbranched or branched alkyl group as defined above, wherein one or more (e.g., 1 to 6 or 1 to 3) hydrogen atoms are replaced by a halogen. For example, where a residue is substituted with more than one halogen, it may be referred to by using a prefix corresponding to the number of halogen moieties attached. Dihaloalkyl and trihaloalkyl refer to alkyl substituted with two ("di") or three ("tri") halo groups, which may be, but are not necessarily, the same halogen. Examples of haloalkyl include, e.g., trifluoromethyl, difluoromethyl, fluoromethyl, trichloromethyl, 2,2,2-trifluoroethyl, 1,2-difluoroethyl, 3-bromo-2-fluoropropyl, 1,2-dibromoethyl and the like.

"Haloalkoxy" refers to an alkoxy group as defined above, wherein one or more (e.g., 1 to 6 or 1 to 3) hydrogen atoms are replaced by a halogen.

"Hydroxyalkyl" refers to an alkyl group as defined above, wherein one or more (e.g., 1 to 6 or 1 to 3) hydrogen atoms are replaced by a hydroxy group.

"Heteroalkyl" refers to an alkyl group in which one or more of the carbon atoms (and any associated hydrogen atoms) are each independently replaced with the same or different heteroatomic group, provided the point of attachment to the remainder of the molecule is through a carbon atom. The term "heteroalkyl" includes unbranched or branched saturated chain having carbon and heteroatoms. By way of example, 1, 2 or 3 carbon atoms may be independently replaced with the same or different heteroatomic group. Heteroatomic groups include, but are not limited to, -NR^{y}-, -O-, -S-, -S(O)-, -S(O)₂-, and the like, wherein R^{y} is hydrogen, alkyl, alkenyl, alkynyl, cycloalkyl, heterocycloalkyl, aryl, heteroalkyl or heteroaryl; each of which may be optionally substituted, as defined herein. Examples of heteroalkyl groups include, e.g., ethers (e.g., -CH₂OCH₃, -CH(CH₃)OCH₃, -CH₂CH₂OCH₃, -CH₂CH₂OCH₂CH₂OCH₃, etc.), thioethers (e.g., -CH₂SCH₃, -CH(CH₃)SCH₃, -CH₂CH₂SCH₃, -CH₂CH₂SCH₂CH₂SCH₃, etc.), sulfones (e.g., -CH₂S(O)₂CH₃, -CH(CH₃)S(O)₂CH₃, -CH₂CH₂S(O)₂CH₃, -CH₂CH₂S(O)₂CH₂CH₂OCH₃, etc.) and amines (e.g., -CH₂NR^{y}CH₃, -CH(CH₃)NR^{y}CH₃, -CH₂CH₂NR^{y}CH₃, -CH₂CH₂NR^{y}CH₂CH₂NR^{y}CH₃, etc., where R^{y} is hydrogen, alkyl, alkenyl, alkynyl, cycloalkyl, heterocycloalkyl, aryl, heteroalkyl, or heteroaryl; each of which may be optionally substituted, as defined herein). As used herein, heteroalkyl includes 1 to 10 carbon atoms, 1 to 8 carbon atoms, or 1 to 4 carbon atoms; and 1 to 3 heteroatoms, 1 to 2 heteroatoms, or 1 heteroatom.

"Heteroaryl" refers to an aromatic group having a single ring, multiple rings or multiple fused rings, with one or more ring heteroatoms independently selected from nitrogen, oxygen, boron, phosphorus and sulfur. As used herein, heteroaryl includes 1 to 20 ring carbon atoms (i.e., C₁₋₂₀ heteroaryl), 3 to 12 ring carbon atoms (i.e., C₃₋₁₂ heteroaryl), or 3 to 8 carbon ring atoms (i.e., C₃₋₈ heteroaryl), and 1 to 5 ring heteroatoms, 1 to 4 ring heteroatoms, 1 to 3 ring heteroatoms, 1 to 2 ring heteroatoms, or 1 ring heteroatom independently selected from nitrogen, oxygen and sulfur. In certain instances, heteroaryl includes 5-10 membered ring systems, 5-7 membered ring systems, or 5-6 membered ring systems, each independently having 1 to 4 ring heteroatoms, 1 to 3 ring heteroatoms, 1 to 2 ring heteroatoms, or 1 ring heteroatom independently selected from nitrogen, oxygen and sulfur. In some embodiments, the heteroaryl has 5-14 ring atoms including carbon atoms and 1, 2, 3 or 4 heteroatom ring members independently selected from nitrogen, sulfur and oxygen. In some embodiments, the heteroaryl has 5-14, or 5-10 ring atoms including carbon atoms and 1, 2, 3 or 4 heteroatom ring members independently selected from nitrogen, sulfur and oxygen. In some embodiments, the heteroaryl has 5-6 ring atoms and 1 or 2 heteroatom ring members independently selected from nitrogen, sulfur and oxygen. In some embodiments, the heteroaryl is a five-membered or six-membered heteroaryl ring. In other embodiments, the heteroaryl is an eight-membered, nine-membered or ten-membered fused bicyclic heteroaryl ring. Examples of heteroaryl groups include, e.g., acridinyl, benzimidazolyl, benzothiazolyl, benzindolyl, benzofuranyl, benzothiazolyl, benzothiadiazolyl, benzonaphthofuranyl, benzoxazolyl, benzothienyl (benzothiophenyl), benzotriazolyl, benzo[4,6]imidazo[1,2-a]pyridyl, carbazolyl, cinnolinyl, dibenzofuranyl, dibenzothiophenyl, furanyl, isothiazolyl, imidazolyl, indazolyl, indolyl, indazolyl, isoindolyl, isoquinolyl, isoxazolyl, naphthyridinyl, oxadiazolyl, oxazolyl, 1-oxidopyridinyl, 1-oxidopyrimidinyl, 1-oxidopyrazinyl, 1-oxidopyridazinyl, phenazinyl, phthalazinyl, pteridinyl, purinyl, pyrrolyl, pyrazolyl, pyridinyl, pyrazinyl, pyrimidinyl, pyridazinyl, quinazolinyl, quinoxalinyl, quinolinyl, quinuclidinyl, isoquinolinyl, thiazolyl, thiadiazolyl, triazolyl, tetrazolyl and triazinyl. In some embodiments, any ring-forming N in a heteroaryl moiety can be an N-oxide.

In certain instances, a fused heteroaryl refers to a heteroaryl ring fused to another heteroaryl ring. Examples of the fused-heteroaryl rings include, but are not limited to, benzo[d]thiazolyl, quinolinyl, isoquinolinyl, benzo[b]thiophenyl, indazolyl, benzo[d]imidazolyl, pyrazolo[1,5-a]pyridinyl and imidazo[1,5-a]pyridinyl, where the heteroaryl can be bound *via* either ring of the fused system. Any aromatic ring, having a single or multiple fused rings, containing at least one heteroatom, is considered a heteroaryl regardless of the attachment to the remainder of the molecule (i.e., through any one of the fused rings). Heteroaryl does not encompass or overlap with aryl as defined above.

"Heteroarylalkyl" refers to the group "heteroaryl-alkyl-".

"Heterocycloalkyl" or "heterocyclyl" refers to a saturated or partially unsaturated cyclic alkyl group, with one or more ring heteroatoms independently selected from boron, phosphorus, nitrogen, oxygen and sulfur. The term "heterocycloalkyl" includes heterocycloalkenyl groups (i.e., the heterocycloalkyl group having at least one double bond), bridged-heterocycloalkyl groups, fused-heterocycloalkyl groups and spiro-heterocycloalkyl groups. A heterocycloalkyl may be a single ring or multiple rings wherein the multiple rings may be fused, bridged or spiro. One or more ring carbon atoms and ring heteroatoms of a heterocycloalkyl group can be optionally oxidized to form an oxo or sulfido group or other oxidized linkage (*e.g.,* C(O), S(O), C(S) or S(O)₂, *N*-oxide *etc*.) or a nitrogen atom can be quaternized. The heterocycloalkyl group can be attached through a ring carbon atom or a ring heteroatom. Any non-aromatic ring containing at least one ring heteroatom is considered a heterocycloalkyl, regardless of the attachment (i.e., can be bound through a carbon atom or a heteroatom). As used herein, heterocycloalkyl has 2 to 20 ring carbon atoms (i.e., C₂₋₂₀ heterocycloalkyl), 2 to 12 ring carbon atoms (i.e., C₂₋₁₂ heterocycloalkyl), 2 to 10 ring carbon atoms (i.e., C₂₋₁₀ heterocycloalkyl), 2 to 8 ring carbon atoms (i.e., C₂₋₈ heterocycloalkyl), 3 to 12 ring carbon atoms (i.e., C₃₋₁₂ heterocycloalkyl), 3 to 8 ring carbon atoms (i.e., C₃₋₈ heterocyclyl), or 3 to 6 ring carbon atoms (i.e., C₃₋₆ heterocycloalkyl); having 1 to 5 ring heteroatoms, 1 to 4 ring heteroatoms, 1 to 3 ring heteroatoms, 1 to 2 ring heteroatoms, or 1 ring heteroatom independently selected from nitrogen, sulfur or oxygen. Examples of heterocycloalkyl groups include, e.g., azetidinyl, azepinyl, benzodioxolyl, benzo[b][1,4]dioxepinyl, 1,4-benzodioxanyl, benzopyranyl, benzodioxinyl, benzopyranonyl, benzofuranonyl, dioxolanyl, dihydropyranyl, hydropyranyl, thienyl[1,3]dithianyl, decahydroisoquinolyl, furanonyl, imidazolinyl, imidazolidinyl, indolinyl, indolizinyl, isoindolinyl, isothiazolidinyl, isoxazolidinyl, morpholinyl, octahydroindolyl, octahydroisoindolyl, 2-oxopiperazinyl, 2-oxopiperidinyl, 2-oxopyrrolidinyl, oxazolidinyl, oxiranyl, oxetanyl, phenothiazinyl, phenoxazinyl, piperidinyl, piperazinyl, 4-piperidonyl, pyrrolidinyl, pyrazolidinyl, quinuclidinyl, thiazolidinyl, tetrahydrofuryl, tetrahydropyranyl, trithianyl, tetrahydroquinolinyl, thiophenyl (i.e., thienyl), tetrahydropyranyl, thiomorpholinyl, thiamorpholinyl, 1-oxo-thiomorpholinyl and 1,1-dioxo-thiomorpholinyl. The term "heterocycloalkyl" also includes "spiroheterocycloalkyl" when there are two positions for substitution on the same carbon atom. Examples of the spiro-heterocycloalkyl rings include, e.g., bicyclic and tricyclic ring systems, such as 2-oxa-7-azaspiro[3.5]nonanyl, 2-oxa-6-azaspiro[3.4]octanyl and 6-oxa-1-azaspiro[3.3]heptanyl.

Further, the term heterocycloalkyl is intended to encompass any non-aromatic ring containing at least one heteroatom, which ring is fused to one or more aryl or heteroaryl rings, regardless of the attachment to the remainder of the molecule (i.e., a heterocycloalkyl group containing a fused aromatic ring can be attached through any ring atom including a ring atom of the fused aromatic ring). Examples of the fused-heterocycloalkyl rings include, but are not limited to, 1,2,3,4-tetrahydroisoquinolinyl, 4,5,6,7-tetrahydrothieno[2,3-c]pyridinyl, indolinyl and isoindolinyl, where the heterocycloalkyl can be bound *via* either ring of the fused system. Further, heterocycloalkyl, as defined herein, does not overlap with heteroaryl, as defined herein.

"Heterocycloalkylalkyl" refers to the group "heterocycloalkyl-alkyl-."

"Oxime" refers to the group -CR^{y}(=NOH) wherein R^{y} is hydrogen, alkyl, alkenyl, alkynyl, cycloalkyl, heterocycloalkyl, aryl, heteroalkyl or heteroaryl; each of which may be optionally substituted, as defined herein.

"Sulfonyl" refers to the group -S(O)₂R^{y}, where R^{y} is hydrogen, alkyl, alkenyl, alkynyl, cycloalkyl, heterocycloalkyl, aryl, heteroalkyl or heteroaryl; each of which may be optionally substituted, as defined herein. Examples of sulfonyl are methylsulfonyl, ethylsulfonyl, phenylsulfonyl and toluenesulfonyl.

"Sulfinyl" refers to the group -S(O)R^{y}, where R^{y} is hydrogen, alkyl, alkenyl, alkynyl, cycloalkyl, heterocycloalkyl, aryl, heteroalkyl or heteroaryl; each of which may be optionally substituted, as defined herein. Examples of sulfinyl are methylsulfinyl, ethylsulfinyl, phenylsulfinyl and toluenesulfinyl.

"Sulfonamido" refers to the groups -SO₂NR^{y}R^{z} and -NR^{y}SO₂R^{z}, where R^{y} and R^{z} are each independently hydrogen, alkyl, alkenyl, alkynyl, cycloalkyl, heterocycloalkyl, aryl, heteroalkyl or heteroaryl; each of which may be optionally substituted, as defined herein.

The terms "optional" or "optionally" means that the subsequently described event or circumstance may or may not occur and that the description includes instances where said event or circumstance occurs and instances in which it does not. Also, the term "optionally substituted" refers to any one or more (e.g., 1 to 5 or 1 to 3) hydrogen atoms on the designated atom or group may or may not be replaced by a moiety other than hydrogen.

The term "substituted" used herein means any of the above groups (i.e., alkyl, alkenyl, alkynyl, alkylene, alkoxy, haloalkyl, haloalkoxy, cycloalkyl, aryl, heterocycloalkyl, heteroaryl, and/or heteroalkyl) wherein at least one (e.g., 1 to 5 or 1 to 3) hydrogen atom is replaced by a bond to a non-hydrogen atom such as, but not limited to alkyl, alkenyl, alkynyl, alkoxy, alkylthio, acyl, amido, amino, amidino, aryl, aralkyl, azido, carbamoyl, carboxyl, carboxyl ester, cyano, cycloalkyl, cycloalkylalkyl, guanidino, halo, haloalkyl, haloalkoxy, hydroxyalkyl, heteroalkyl, heteroaryl, heteroarylalkyl, heterocycloalkyl, heterocycloalkylalkyl, -NHNH₂, =NNH₂, imino, imido, hydroxy, oxo, oxime, nitro, sulfonyl, sulfinyl, alkylsulfonyl, alkylsulfinyl, thiocyanate, -S(O)OH, -S(O)₂OH, sulfonamido, thiol, thioxo, N-oxide or -Si(R^{y})₃, wherein each R^{y} is independently hydrogen, alkyl, alkenyl, alkynyl, heteroalkyl, cycloalkyl, aryl, heteroaryl or heterocycloalkyl.

In certain embodiments, "substituted" includes any of the above alkyl, alkenyl, alkynyl, cycloalkyl, heterocycloalkyl, aryl or heteroaryl groups in which one or more (e.g., 1 to 5 or 1 to 3) hydrogen atoms are independently replaced with deuterium, halo, cyano, nitro, azido, oxo, alkyl, alkenyl, alkynyl, haloalkyl, cycloalkyl, heterocycloalkyl, aryl, heteroaryl, -NR^{q}R^{h}, -NR^{q}C(=O)R^{h}, -NR^{q}C(=O)NR^{q}R^{h}, -NR^{q}C(=O)OR^{h}, -NR^{q}S(=O)₁₋₂R^{h}, -C(=O)R^{q}, -C(=O)OR^{q}, -OC(=O)OR^{q}, -OC(=O)R^{q}, -C(=O)NR^{q}R^{h}, -OC(=O)NR^{q}R^{h}, -OR^{q}, -SR^{q}, -S(=O)R^{q}, -S(=O)₂R^{q}, -OS(=O)₁₋₂R^{q}, -S(=O)₁₋₂OR^{q}, -NR^{q}S(=O)₁₋₂NR^{q}R^{h}, =NSO₂R^{q}, =NOR^{q}, -S(=O)₁₋₂NR^{q}R^{h}, -SF₅, -SCF₃ or -OCF₃. In certain embodiments, "substituted" also means any of the above groups in which one or more (e.g., 1 to 5 or 1 to 3) hydrogen atoms are replaced with -C(=O)R^{q}, -C(=O)OR^{q}, -C(=O)NR^{q}R^{h}, -CH₂SO₂R^{q}, or -CH₂SO₂NR^{q}R^{h}. In the foregoing, R^{q} and R^{h} are the same or different and independently hydrogen, alkyl, alkenyl, alkynyl, alkoxy, thioalkyl, aryl, aralkyl, cycloalkyl, cycloalkylalkyl, haloalkyl, heterocycloalkyl, heterocycloalkylalkyl, heteroaryl, and/or heteroarylalkyl. In certain embodiments, "substituted" also means any of the above groups in which one or more (e.g., 1 to 5 or 1 to 3) hydrogen atoms are replaced by a bond to an amino, cyano, hydroxyl, imino, nitro, oxo, thioxo, halo, alkyl, alkoxy, alkylamino, thioalkyl, aryl, aralkyl, cycloalkyl, cycloalkylalkyl, haloalkyl, heterocycloalkyl, N-heterocycloalkyl, heterocycloalkylalkyl, heteroaryl, and/or heteroarylalkyl, or two of R^{q} and R^{h} and Rⁱ are taken together with the atoms to which they are attached to form a heterocycloalkyl ring optionally substituted with oxo, halo or alkyl optionally substituted with oxo, halo, amino, hydroxyl, or alkoxy.

Polymers or similar indefinite structures arrived at by defining substituents with further substituents appended ad infinitum (e.g., a substituted aryl having a substituted alkyl which is itself substituted with a substituted aryl group, which is further substituted by a substituted heteroalkyl group, etc.) are not intended for inclusion herein. Unless otherwise noted, the maximum number of serial substitutions in compounds described herein is three. For example, serial substitutions of substituted aryl groups with two other substituted aryl groups are limited to ((substituted aryl)substituted aryl) substituted aryl. Similarly, the above definitions are not intended to include impermissible substitution patterns (e.g., methyl substituted with 5 fluorines or heteroaryl groups having two adjacent oxygen ring atoms). Such impermissible substitution patterns are well known to the skilled artisan. When used to modify a chemical group, the term "substituted" may describe other chemical groups defined herein.

In certain embodiments, as used herein, the phrase "one or more" refers to one to five. In certain embodiments, as used herein, the phrase "one or more" refers to one to three.

Any compound or structure given herein, is intended to represent unlabeled forms as well as isotopically labeled forms (isotopologues) of the compounds. These forms of compounds may also be referred to as and include "isotopically enriched analogs." Isotopically labeled compounds have structures depicted herein, except that one or more atoms are replaced by an atom having a selected atomic mass or mass number. Examples of isotopes that can be incorporated into the disclosed compounds include isotopes of hydrogen, carbon, nitrogen, oxygen, phosphorous, fluorine, chlorine and iodine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ³⁶Cl, ¹²³I, and ¹²⁵I, respectively. Various isotopically labeled compounds of the present disclosure, for example those into which radioactive isotopes such as ³H and ¹⁴C are incorporated. Such isotopically labelled compounds may be useful in metabolic studies, reaction kinetic studies, detection or imaging techniques, such as positron emission tomography (PET) or single-photon emission computed tomography (SPECT) including drug or substrate tissue distribution assays or in radioactive treatment of patients.

The term "isotopically enriched analogs" includes "deuterated analogs" of compounds described herein in which one or more hydrogens is/are replaced by deuterium, such as a hydrogen on a carbon atom. Such compounds exhibit increased resistance to metabolism and are thus useful for increasing the half-life of any compound when administered to a mammal, particularly a human. See, for example, Foster, "Deuterium Isotope Effects in Studies of Drug Metabolism," Trends Pharmacol. Sci. 5(12):524-527 (1984). Such compounds are synthesized by means well known in the art, for example by employing starting materials in which one or more hydrogens have been replaced by deuterium.

Deuterium labelled or substituted therapeutic compounds of the disclosure may have improved DMPK (drug metabolism and pharmacokinetics) properties, relating to distribution, metabolism and excretion (ADME). Substitution with heavier isotopes such as deuterium may afford certain therapeutic advantages resulting from greater metabolic stability, for example increased *in vivo* half-life, reduced dosage requirements and/or an improvement in therapeutic index (see e.g., A. Kerekes et.al. J. Med. Chem. 2011, 54, 201-210; R. Xu et.al. J. Label Compd. Radiopharm. 2015, 58, 308-312). An ¹⁸F, ³H, ¹¹C labeled compound may be useful for PET or SPECT or other imaging studies. Isotopically labeled compounds of this disclosure can generally be prepared by carrying out the procedures disclosed in the schemes or in the examples and preparations described below by substituting a readily available isotopically labeled reagent for a non-isotopically labeled reagent. It is understood that deuterium in this context is regarded as a substituent in a compound described herein.

One or more constituent atoms of the compounds presented herein can be replaced or substituted with isotopes of the atoms in natural or non-natural abundance. In some embodiments, the compound includes at least one deuterium atom. For example, one or more hydrogen atoms in a compound presented herein can be replaced or substituted by deuterium (e.g., one or more hydrogen atoms of a C₁₋₆ alkyl group can be replaced by deuterium atoms, such as -CH₃ being replaced for -CD₃). In some embodiments, the compound includes two or more deuterium atoms. In some embodiments, the compound includes 1, 2, 3, 4, 5, 6, 7, 8, 9, 10, 11, or 12 deuterium atoms. In some embodiments, all of the hydrogen atoms in a compound can be replaced or substituted by deuterium atoms. Synthetic methods for including isotopes into organic compounds are known in the art (Deuterium Labeling in Organic Chemistry by Alan F. Thomas (New York, NY., Appleton-Century-Crofts, 1971; The Renaissance of H/D Exchange by Jens Atzrodt, Volker Derdau, Thorsten Fey and Jochen Zimmermann, Angew. Chem. Int. Ed. 2007, 7744-7765; The Organic Chemistry of Isotopic Labelling by James R. Hanson, Royal Society of Chemistry, 2011). Isotopically labeled compounds can be used in various studies such as NMR spectroscopy, metabolism experiments, and/or assays.

The concentration of such a heavier isotope, specifically deuterium, may be defined by an isotopic enrichment factor. In the compounds of this disclosure any atom not specifically designated as a particular isotope is meant to represent any stable isotope of that atom. Unless otherwise stated, when a position is designated specifically as "H" or "hydrogen", the position is understood to have hydrogen at its natural abundance isotopic composition. Accordingly, in the compounds of this disclosure any atom specifically designated as a deuterium (D) is meant to represent deuterium. Further, in some embodiments, the corresponding deuterated analog is provided.

In many cases, the compounds of this disclosure are capable of forming acid and/or base salts by virtue of the presence of amino and/or carboxyl groups or groups similar thereto.

Provided also are a pharmaceutically acceptable salt, isotopically enriched analog, deuterated analog, stereoisomer, tautomer, and a mixture of stereoisomers, of the compounds described herein.

"Pharmaceutically acceptable" or "physiologically acceptable" refer to compounds, salts, compositions, dosage forms and other materials which are useful in preparing a pharmaceutical composition that is suitable for veterinary or human pharmaceutical use.

The term "pharmaceutically acceptable salt" of a given compound refers to salts that retain the biological effectiveness and properties of the given compound and which are not biologically or otherwise undesirable. "Pharmaceutically acceptable salts" or "physiologically acceptable salts" include, for example, salts with inorganic acids and salts with an organic acid. In addition, if the compounds described herein are obtained as an acid addition salt, the free base can be obtained by basifying a solution of the acid salt. Conversely, if the product is a free base, an addition salt, particularly a pharmaceutically acceptable addition salt, may be produced by dissolving the free base in a suitable organic solvent and treating the solution with an acid, in accordance with conventional procedures for preparing acid addition salts from base compounds. Those skilled in the art will recognize various synthetic methodologies that may be used to prepare nontoxic pharmaceutically acceptable addition salts. Pharmaceutically acceptable acid addition salts may be prepared from non-toxic inorganic and organic acids. The pharmaceutically acceptable salts of the present invention can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, non-aqueous media like ether, ethyl acetate, alcohols (e.g., methanol, ethanol, iso-propanol or butanol) or acetonitrile (MeCN) are preferred. Lists of suitable salts are found in Remington's Pharmaceutical Sciences , 17th Ed., (Mack Publishing Company, Easton, 1985), p. 1418, Berge et al., J. Pharm. Sci., 1977, 66(1), 1-19 and in Stahl et al., Handbook of Pharmaceutical Salts: Properties, Selection, and Use, (Wiley, 2002).

The term "tautomer" means compounds produced by the phenomenon wherein a proton of one atom of a molecule shifts to another atom of the molecule. The tautomers also refer to one of two or more structural isomers that exist in equilibrium and are readily converted from one isomeric form to another. Non-limiting examples include enol-keto, imine-enamine, amide-imidic acid tautomers, the tautomeric forms of heteroaryl groups containing a -N=C(H)-NH- ring atom arrangement, such as pyrazoles, imidazoles, benzimidazoles, triazoles, and tetrazoles, and the tautomeric forms of hydroxy substituted 6-membered heteroaryl groups (e.g., hydroxy substituted pyridine, pyrimidine, pyrazine or pyridazine) such as 4-hydroxypyridine and puridin-4(1H)-one, and the like. The compounds described herein may have one or more tautomers and therefore include various isomers. A person of ordinary skill in the art would recognize that other tautomeric ring atom arrangements are possible. All such isomeric forms of these compounds are expressly included in the present disclosure.

Some of the compounds exist as tautomers. Tautomers are in equilibrium with one another. For example, amide containing compounds may exist in equilibrium with imidic acid tautomers. Regardless of which tautomer is shown and regardless of the nature of the equilibrium among tautomers, the compounds are understood by one of ordinary skill in the art to comprise both amide and imidic acid tautomers. Thus, the amide containing compounds are understood to include their imidic acid tautomers. Likewise, the imidic acid containing compounds are understood to include their amide tautomers.

The compounds of the invention, or their pharmaceutically acceptable salts include an asymmetric center and may thus give rise to enantiomers, diastereomers, and other stereoisomeric forms that may be defined, in terms of absolute stereochemistry, as (*R*)- or (*S*)- or, as (D)- or (L)- for amino acids. The present invention is meant to include all such possible isomers, as well as their racemic and optically pure forms. Optically active (+) and (-), (*R*)*-* and (*S*)-, or (D)- and (L)- isomers may be prepared using chiral synthons or chiral reagents, or resolved using conventional techniques, for example, chromatography and fractional crystallization. Conventional techniques for the preparation/isolation of individual enantiomers include chiral synthesis from a suitable optically pure precursor or resolution of the racemate (or the racemate of a salt or derivative) using, for example, chiral high pressure liquid chromatography (HPLC). When the compounds described herein contain olefinic double bonds or other centers of geometric asymmetry, and unless specified otherwise, it is intended that the compounds include both E and Z geometric isomers.

A "stereoisomer" refers to a compound made up of the same atoms bonded by the same bonds but having different three-dimensional structures, which are not interchangeable. The present invention contemplates various stereoisomers and mixtures thereof and includes "enantiomers," which refers to two stereoisomers whose molecules are nonsuperimposeable mirror images of one another.

"Diastereomers" are stereoisomers that have at least two asymmetric atoms, but which are not mirror-images of each other.

Relative centers of the compounds as depicted herein are indicated graphically using the "thick bond" style (bold or parallel lines) and absolute stereochemistry is depicted using wedge bonds (bold or parallel lines).

The term "leaving group" refers to an atom or a group of atoms that is displaced in a chemical reaction as stable species taking with it the bonding electrons. The non-limiting examples of a leaving group include, halo, methanesulfonyloxy, p-toluenesulfonyloxy, trifluoromethanesulfonyloxy, nonafluorobutanesulfonyloxy, (4-bromo-benzene)sulfonyloxy, (4-nitro-benzene)sulfonyloxy, (2-nitrobenzene)-sulfonyloxy, (4-isopropyl-benzene)sulfonyloxy, (2,4,6-tri-isopropyl-benzene)-sulfonyloxy, (2,4,6-trimethyl-benzene)sulfonyloxy, (4-*tert*-butyl-benzene)sulfonyloxy, benzenesulfonyloxy, (4-methoxy-benzene)sulfonyloxy, and the like.

The term "amide coupling conditions" refers to the reaction conditions under which an amine and a carboxylic acid couple to form an amide using a coupling reagent in presence of a base. The non-limiting examples of coupling reagents include 1-Ethyl-3-(3-dimethylaminopropyl) carbodiimide (EDC) with hydroxybenzotriazole monohydrate (HOBt), O-(7-Azabenzotriazole-1-yl)-*N,N,N,N*'-tetramethyluronium hexafluorophosphate (HATU), 1-hydroxy-7-azabenzotriazole, and the like. The non-limiting examples of the base include N-methylmorpholine, pyridine, morpholine, imidazole, and the like.

The term "protecting group" refers to a moiety of a compound that masks or alters the properties of a functional group or the properties of the compound as a whole. The chemical substructure of a protective group varies widely. One function of a protective group is to serve as an intermediate in the synthesis of the parental drug substance. Chemical protective groups and strategies for protection/deprotection are well known in the art. See: "Protective Groups in Organic Chemistry", Theodora W. Greene (John Wiley & Sons, Inc., New York, 1991. Protective groups are often utilized to mask the reactivity of certain functional groups, to assist in the efficiency of desired chemical reactions, e.g., making and breaking chemical bonds in an ordered and planned fashion. Protection of functional groups of a compound alters other physical properties besides the reactivity of the protected functional group, such as the polarity, lipophilicity (hydrophobicity), and other properties which can be measured by common analytical tools. Chemically protected intermediates may themselves be biologically active or inactive.

The non-limiting examples of protective groups for a hydroxy (i.e. a "hydroxy protecting group") include methoxymethyl ether, tetrahydropyranyl ether, t-butyl ether, allyl ether, benzyl ether, t-butyldiphenylsilyl ether, acetate ester, pivalate ester, benzoate ester, benzylidene acetal, acetonide, silyl ether, and the like.

### List of Abbreviations and Acronyms

| **Abbreviation** | **Meaning** |
|---|---|
| Ac | acetyl |
| ACN | acetonitrile |
| Amphos₂PdCl₂ | bis(di-tert-butyl(4-dimethylaminophenyl)phosphine)dichloropalladium(II) |
| anhyd. | anhydrous |
| aq. | aqueous |
| BINAP | 2,2'-Bis(diphenylphosphino)-1,1'-binaphthalene |
| cat | catalytical amount |
| δ | chemical shift (ppm) |
| dba | dibenzylideneacetone |
| DCE | dicholoroethane |
| DCM | dichloromethane |
| DIAD | diisopropyl azodicarboxylate |
| DIEA | diisopropylethylamine |
| DMAP | dimethylaminopyridine |
| DMF | dimethylformamide |
| DMSO | dimethylsulfoxide |
| dppf | 1,1'-ferrocenediyl-bis(diphenylphosphine) |
| eq or equiv. | equivalent(s) |
| EI | electron ionization |
| Et | ethyl |
| Et₂O | diethylether |
| EtOAc | ethyl acetate |
| EtOH | ethanol |
| h | hour |
| HATU | N-[(dimethylamino)-1H-1,2,3-triazolo-[4,5-b]pyridin-1-ylmethylene]-N-methylmethanaminium hexafluorophosphate N-oxide |
| HPLC | high performance liquid chromatography |
| LC-MS | Liquid chromatography - mass spectrometry |
| MeOH | methanol |
| NaOMe | sodium methoxide |
| Me | methyl |
| MS | Mass Spectrum |
| m/z | Mass to charge ratio |
| NaOAc | sodium acetate |
| NBS | N-bromosuccinimide |
| NIS | N-iodosuccinimide |
| NH₂Boc | tert-butyl carbamate |
| NMP | N-methyl-2-pyrrolidone |
| NMR | nuclear magnetic resonance spectroscopy |
| Pd(Amphos)₂Cl₂ | bis(di-tert-butyl(4-dimethylaminophenyl)phosphine)dichloropalladium(II) |
| Pd(PPh₃)₄ | tetrakis(triphenylphosphine)palladium(0) |
| Ph | phenyl |
| PMB- | 4-methoxybenzyl |
| PR₃ | tertiary organophosphine |
| Prep | preparatory |
| *rac-* | racemic |
| SPhos | 2-dicyclohexylphosphino-2',6'-dimethoxybiphenyl |
| ^{t}Bu or tBu | tert-butyl |
| TEA | triethylamine |
| THF | tetrahydrofuran |
| TLC | thin layer chromatography |
| XPhos Pd G2 | chloro(2-dicyclohexylphosphino-2',4',6'-triisopropyl-1,1'-biphenyl) [2-(2'-amino-1,1'-biphenyl)]palladium(II) |

### Compounds

Provided herein is are compounds of Formula (Ia), Formula (Ib), or Formula (Ic): 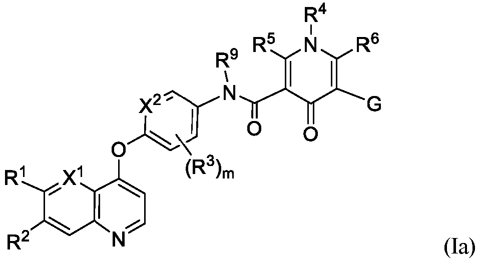 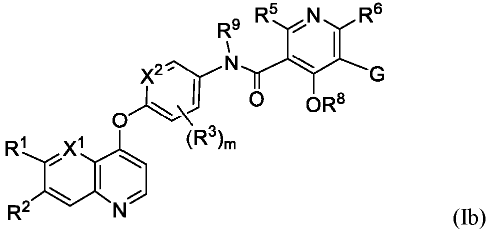 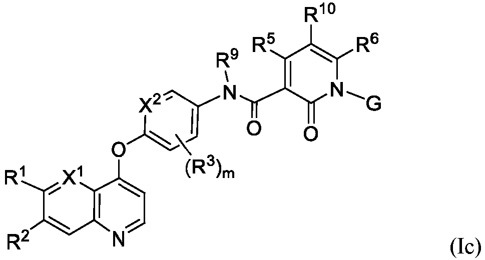 or a pharmaceutically acceptable salt, stereoisomer, or tautomer thereof, wherein:
G is C₃₋₁₀ cycloalkyl, 4- to 14-membered heterocycloalkyl, C₁₋₆ alkyl, C₂₋₆ alkenyl, C₁₋₆ alkoxy, cyano, halo, C(O)OR^{a}, or C(O)NR^{a}R^{a}, wherein the C₃₋₁₀ cycloalkyl, 4- to 14-membered heterocycloalkyl, C₁₋₆ alkyl, C₂₋₆ alkenyl and C₁₋₆ alkoxy of G are each optionally substituted with 1, 2, 3, or 4 independently selected R⁷ substituents;
X¹ is N;
X² is CH or CF;
R¹ and R² are each independently selected from H, halo, C₁₋₆ alkyl, C₂₋₆ alkenyl, C₂₋₆ alkynyl, C₁₋₆ haloalkyl, C₁₋₆ haloalkoxy, C₆₋₁₀ aryl, C₃₋₁₄ cycloalkyl, 5-14 membered heteroaryl, 4-14 membered heterocycloalkyl, C₆₋₁₀ aryl-C₁₋₄ alkylene-, C₃₋₁₄ cycloalkyl-C₁₋₄ alkylene-, (5-14 membered heteroaryl)-C₁₋₄ alkylene-, (4-14 membered heterocycloalkyl)-C₁₋₄ alkylene-, CN, NO₂, OR^{a}, SR^{a}, NHOR^{a}, C(O)R^{a}, C(O)NR^{a}R^{a}, C(O)OR^{a}, C(O)NR^{a}S(O)₂R^{a}, OC(O)R^{a}, OC(O)NR^{a}R^{a}, NHR^{a}, NR^{a}R^{a}, NR^{a}C(O)R^{a}, NR^{a}C(=NR^{a})R^{a}, NR^{a}C(O)OR^{a}, NR^{a}C(O)NR^{a}R^{a}, C(=NR^{a})R^{a}, C(=NOH)R^{a}, C(=NOH)NR^{a}, C(=NCN)NR^{a}R^{a}, NR^{a}C(=NCN)NR^{a}R^{a}, C(=NR^{a})NR^{a}R^{a}, NR^{a}C(=NR^{a})NR^{a}R^{a}, NR^{a}S(O)R^{a}, NR^{a}S(O)₂R^{a}, NR^{a}S(O)₂NR^{a}R^{a}, S(O)R^{a}, S(O)NR^{a}R^{a}, S(O)₂R^{a}, S(O)₂NR^{a}C(O)R^{a}, P(O)R^{a}R^{a}, P(O)(OR^{a})(OR^{a}), B(OH)₂, B(OR^{a})₂, and S(O)₂NR^{a}R^{a}, wherein the C₁₋₆ alkyl, C₂₋₆ alkenyl, C₂₋₆ alkynyl, C₆₋₁₀ aryl, C₃₋₁₄ cycloalkyl, 5-14 membered heteroaryl, 4-14 membered heterocycloalkyl, C₆₋₁₀ aryl-C₁₋₄ alkylene-, C₃₋₁₄ cycloalkyl-C₁₋₄ alkylene-, (5-14 membered heteroaryl)-C₁₋₄ alkylene-, and (4-14 membered heterocycloalkyl)-C₁₋₄ alkylene- of R¹ and R² are each optionally substituted with 1, 2, 3, 4 or 5 independently selected R^{b} substituents;
each R³ is independently selected from halo, OH, CN, -C(O)OH, -C(O)NH(C₁₋₆ alkyl), -SO₂(C₁₋₆ alkyl), -SO₂NH(C₁₋₆ alkyl), C₁-C₆ alkyl, C₁-C₆ alkoxy, C₁-C₆ haloalkoxy, NH₂, -NH(C₁-C₆ alkyl), -N(C₁-C₆ alkyl)₂, and C₃-C₆ cycloalkyl, wherein the C₁-C₆ alkyl, C₁-C₆ alkoxy, -NH(C₁-C₆alkyl), -N(C₁-C₆ alkyl)₂, and C₃-C₆ cycloalkyl of R³ are each optionally substituted with 1, 2, or 3 independently selected R^{g} substituents;
R⁴ is selected from H, halo, C₁₋₆ alkyl, C₂₋₆ alkenyl,
   C₂₋₆ alkynyl, C₁₋₆ haloalkyl, C₁₋₆ haloalkoxy, C₆₋₁₀ aryl, C₃₋₁₄ cycloalkyl, 5-14 membered heteroaryl, 4-14 membered heterocycloalkyl, C₆₋₁₀ aryl-C₁₋₄ alkylene-, C₃₋₁₄ cycloalkyl-C₁₋₄ alkylene-, (5-14 membered heteroaryl)-C₁₋₄ alkylene-, (4-14 membered heterocycloalkyl)-C₁₋₄ alkylene-, CN, NO₂, OR^{a}, SR^{a}, NHOR^{a}, C(O)R^{a}, C(O)NR^{a}R^{a}, C(O)OR^{a}, C(O)NR^{a}S(O)₂R^{a}, OC(O)R^{a}, OC(O)NR^{a}R^{a}, NHR^{a}, NR^{a}R^{a}, NR^{a}C(O)R^{a}*, N=C(NR^{a}R^{a})₂, NR^{a}C(=NR^{a})R^{a}, NR^{a}C(O)OR^{a}, NR^{a}C(O)NR^{a}R^{a}, C(=NR^{a})R^{a}, C(=NOH)R^{a}, C(=NOH)NR^{a}, C(=NCN)NR^{a}R^{a} , NR^{a}C(=NCN)NR^{a}R^{a}, C(=NR^{a})NR^{a}R^{a}, NR^{a}C(=NR^{a})NR^{a}R^{a}, NR^{a}S(O)R^{a}, NR^{a}S(O)₂R^{a}, NR^{a}S(O)₂NR^{a}R^{a}, S(O)R^{a}, S(O)NR^{a}R^{a}, S(O)₂R^{a}, S(O)₂NR^{a}C(O)R^{a}, P(O)R^{a}R^{a}, P(O)(OR^{a})(OR^{a}), B(OH)₂, B(OR^{a})₂, and S(O)₂NR^{a}R^{a}, wherein the C₁₋₆ alkyl, C₂₋₆ alkenyl, C₂₋₆ alkynyl, C₆₋₁₀ aryl, C₃₋₁₄ cycloalkyl, 5-14 membered heteroaryl, 4-14 membered heterocycloalkyl, C₆₋₁₀ aryl-C₁₋₄ alkylene-, C₃₋₁₄ cycloalkyl-C₁₋₄ alkylene-, (5-14 membered heteroaryl)-C₁₋₄ alkylene- and (4-14 membered heterocycloalkyl)-C₁₋₄ alkylene- of R⁴ are each optionally substituted with 1, 2, 3, 4 or 5 independently selected R^{b} substituents;
R⁵, R⁶ and R¹⁰ are each independently H, halo, C₁₋₆ alkyl, C₂₋₆ alkenyl, C₁₋₆ alkoxy, C₁₋₆ alkylthio, CN, C₁₋₄ haloalkyl, C₁₋₄ haloalkoxy, OH, C₁₋₄alkyl-C(O)-, C₁₋₄alkyl-OC(O)-, -C(O)NH(C₁₋₄ alkyl), NH₂, -NHC₁₋₄alkyl, or -N(C₁₋₄ alkyl)₂, wherein the C₁₋₆ alkyl, C₂₋₆ alkenyl, C₁₋₆ alkoxy, C₁₋₆ alkylthio, C₁₋₆ alkyl-C(O)- and C₁₋₄ alkyl of -NH(C₁₋₄alkyl), or -N(C₁₋₄ alkyl)₂ of R⁵, R⁶ and R¹⁰ are each optionally substituted with 1 or 2 independently selected R^{g} substituents;
each R⁷ is independently selected from halo, OH, C(O)OR^{a}, C(O)R^{a}, C(O)NR^{a}R^{a}, CN, NH₂, - NH(C₁₋C₆ alkyl), -N(C₁-C₆ alkyl)₂, C₁-C₆ alkyl, C₂₋C₆ alkenyl, C₁-C₆ alkoxy, C₁-C₆ haloalkyl, C₁₋C₆ haloalkoxy, C(O)NR^{a}R^{a}, NR^{a}C(O)R^{a}, NR^{a}C(O)NR^{a}R^{a}, SO₂R^{a}, NR^{a}S(O)₂R^{a}, NR^{a}S(O)₂NR^{a}R^{a}, C₃₋C₆ cycloalkyl, 4- to 6-membered heterocycloalkyl, phenyl, 5- or 6-membered heteroaryl, C₃₋C₆ cycloalkyl-C₁₋C₄ alkylene-, (4- to 6-membered heterocycloalkyl)-C₁-C₄ alkylene-, phenyl-C₁₋C₂ alkylene, and (5- or 6-membered heteroaryl)-C₁₋C₄ alkylene-, wherein the C₁-C₆ alkyl, C₁₋C₆ alkoxy, C₃₋C₆ cycloalkyl, 4- to 6-membered heterocycloalkyl, phenyl, 5- or 6-membered heteroaryl, C₃₋C₆ cycloalkyl-C₁₋C₄ alkylene-, (4-to 6-membered heterocycloalkyl)-C₁₋C₄ alkylene-, phenyl-C₁₋C₂ alkylene, and (5- or 6-membered heteroaryl)-C₁₋C₄ alkylene- of R⁷ are each optionally substituted with 1, 2, or 3 independently selected R^{f} substituents;
R⁸ is H, C₁₋₆ alkyl optionally substituted with 1 or 2 R^{g} substituents or a hydroxy protecting group;
R⁹ is H or C₁₋₆ alkyl optionally substituted with 1, 2, or 3 independently selected R^{g} substituents;
or R⁴ and R⁵ taken together with the atoms to which they are attached form 4- to 7-membered fused heterocycloalkyl or 5- to 6-membered fused heteroaryl, wherein the 4- to 7-membered fused heterocycloalkyl and 5- to 6-membered fused heteroaryl are each optionally substituted with 1 or 2 independently selected R^{g} substituents;
or R¹⁰ and R⁵ taken together with the atoms to which they are attached form fused C₃₋₇ cycloalkyl, 4- to 6-membered fused heterocycloalkyl, fused 5- or 6-membered heteroaryl or fused phenyl, wherein the fused C₃₋₇ cycloalkyl, 4- to 6-membered fused heterocycloalkyl, 5- or 6-membered heteroaryl, or fused phenyl is each optionally substituted with 1 or 2 independently selected R^{g} substituents, and wherein one or two ring carbon atoms of the fused C₃₋₇ cycloalkyl or fused heterocycloalkyl are optionally replaced by a carbonyl group;
or R¹² and R¹⁰ taken together with the atoms to which they are attached form 4- to 7-membered fused heterocycloalkyl or 5- to 6-membered fused heteroaryl, wherein the 4- to 7-membered fused heterocycloalkyl and 5- to 6-membered fused heteroaryl are each optionally substituted with 1 or 2 independently selected R^{g} substituents;
or R⁶ and R⁴ taken together with the atoms to which they are attached form 4- to 7-membered fused heterocycloalkyl or 5- to 6-membered fused heteroaryl, wherein the 4- to 7-membered fused heterocycloalkyl and 5- to 6-membered fused heteroaryl are each optionally substituted with 1 or 2 independently selected R^{g} substituents;
or R⁶ and R¹⁰ taken together with the atoms to which they are attached form fused C₃₋₇ cycloalkyl, 4- to 6-membered fused heterocycloalkyl, 5- to 6-membered fused heteroaryl, or fused heteroaryl, wherein the fused C₃₋₇ cycloalkyl, 4- to 6-membered fused heterocycloalkyl, 5- to 6-membered fused heteroaryl, and fused heteroaryl are each optionally substituted with 1 or 2 independently selected R^{g} substituents and wherein one or two ring carbon atoms of the fused C₃₋₇ cycloalkyl or 4- to 6-membered fused heterocycloalkyl are optionally replaced by a carbonyl;
each R^{a} is independently selected from H, CN, C₁-C₆ alkyl, C₁-C₆ haloalkyl, C₂-C₆ alkenyl, C₂-C₆ alkynyl, C₆-C₁₀ aryl, C₃-C₁₀ cycloalkyl, 5-14 membered heteroaryl, 4-14 membered heterocycloalkyl, C₆-C₁₀ aryl-C₁₋C₄ alkylene-, C₃-C₁₀ cycloalkyl-C₁₋C₄ alkylene-, (5-14 membered heteroaryl)-C₁₋C₄ alkylene-, and (4-14 membered heterocycloalkyl)-C₁₋C₄ alkylene-; wherein the C₁-C₆ alkyl, C₁-C₆ haloalkyl, C₂-C₆ alkenyl, C₂-C₆ alkynyl, C₆-C₁₀ aryl, C₃-C₁₀ cycloalkyl, 5-14 membered heteroaryl, 4-14 membered heterocycloalkyl, C₆-C₁₀ aryl-C₁₋C₄ alkylene-, C₃-C₁₀ cycloalkyl-C₁₋C₄ alkylene-, (5-14 membered heteroaryl)-C₁₋C₄ alkylene-, and (4-14 membered heterocycloalkyl)-C₁₋C₄ alkylene- of R^{a} are each optionally substituted with 1, 2, 3, 4, or 5 independently selected R^{d} substituents;
or any two R^{a} substituents together with the nitrogen atom to which they are attached form 4-, 5-, 6-, 7-, 8-, 9-, or 10-membered heterocycloalkyl, each of which is optionally substituted with 1, 2, or 3 independently selected R^{f} substituents;
each R^{b} is independently selected from halo, C₁-C₆ alkyl, C₂-C₆ alkenyl, C₂-C₆ alkynyl, C₁-C₆ haloalkyl, C₁-C₆ haloalkoxy, C₆-C₁₀ aryl, C₃-C₁₀ cycloalkyl, 5-10 membered heteroaryl, 4-10 membered heterocycloalkyl, C₆₋C₁₀ aryl-C₁₋C₄ alkylene-, C₃₋C₁₀ cycloalkyl-C₁₋C₄ alkylene-, (5-10 membered heteroaryl)-C₁₋C₄ alkylene-, (4-10 membered heterocycloalkyl)-C₁₋C₄ alkylene-, CN, OH, NH₂, NO₂, NHOR^{c}, OR^{c}, SR^{c}, C(O)R^{a}, C(O)NR^{c}R^{c}, C(O)OR^{c}, C(O)NR^{c}S(O)₂R^{c}, OC(O)R^{c}, OC(O)NR^{c}R^{c}, C(=NOH)R^{c}, C(=NOH)NR^{c}, C(=NCN)NR^{c}R^{c}, NR^{c}C(=NCN)NR^{c}R^{c}, C(=NR^{c})NR^{c}R^{c}, NR^{c}C(=NR^{c})NR^{c}R^{c}, NHR^{c}, NR^{c}R^{c}, NR^{c}C(O)R^{c}, NR^{c}C(=NR^{c})R^{c}, NR^{c}C(O)OR^{c}, NR^{c}C(O)NR^{c}R^{c}, NR^{c}S(O)R^{c}, NR^{c}S(O)₂R^{c}, NR^{c}S(O)₂NR^{c}R^{c}, S(O)R^{c}, S(O)NR^{c}R^{c}, S(O)₂R^{c}, S(O)₂NR^{c}C(O)R^{c}, Si(R^{c})₃, P(O)R^{c}R^{c}, P(O)(OR^{c})(OR^{c}), B(OH)₂, B(OR^{c})₂, and S(O)₂NR^{c}R^{c}; wherein the C₁-C₆ alkyl, C₂-C₆ alkenyl, C₂-C₆ alkynyl, C₆-C₁₀ aryl, C₃-C₁₀ cycloalkyl, 5-10 membered heteroaryl, 4-10 membered heterocycloalkyl, C₆-C₁₀ aryl-C₁₋C₄ alkylene-, C₃₋C₁₀ cycloalkyl-C₁₋C₄ alkylene-, (5-10 membered heteroaryl)-C₁₋C₄ alkylene-, and (4-10 membered heterocycloalkyl)-C₁-C₄ alkylene- of R^{b} are each further optionally substituted with 1, 2, or 3 independently selected R^{d} substituents;
each R^{c} is independently selected from H, C₁-C₆ alkyl, C₁-C₆ haloalkyl, C₂-C₆ alkenyl, C₂-C₆ alkynyl, C₆-C₁₀ aryl, C₃-C₁₀ cycloalkyl, 5-10 membered heteroaryl, 4-10 membered heterocycloalkyl, C₆-C₁₀ aryl-C₁₋C₄ alkylene-, C₃-C₁₀ cycloalkyl-C₁₋C₄ alkylene-, (5-10 membered heteroaryl)-C₁₋C₄ alkylene-, and (4-10 membered heterocycloalkyl)-C₁₋C₄ alkylene-; wherein the C₁-C₆ alkyl, C₂-C₆ alkenyl, C₂-C₆ alkynyl, C₆-C₁₀ aryl, C₃-C₁₀ cycloalkyl, 5-10 membered heteroaryl, 4-10 membered heterocycloalkyl, C₆-C₁₀ aryl-C₁₋C₄ alkylene-, C₃-C₁₀ cycloalkyl-C₁₋C₄ alkylene-, (5-10 membered heteroaryl)-C₁₋C₄ alkylene-, and (4-10 membered heterocycloalkyl)-C₁₋C₄ alkylene- of R^{c} are each optionally substituted with 1, 2, 3, 4, or 5 independently selected R^{f} substituents;
or any two R^{c} substituents together with the nitrogen atom to which they are attached form 4-, 5-, 6-, 7-, 8-, 9-, or 10-membered heterocycloalkyl, each of which is optionally substituted with 1, 2, or 3 independently selected R^{f} substituents;
each R^{d} is independently selected from C₁-C₆ alkyl, C₁₋C₆ haloalkyl, halo, C₆₋C₁₀ aryl, 5-10 membered heteroaryl, C₃₋C₁₀ cycloalkyl, 4-10 membered heterocycloalkyl, C₆₋C₁₀ aryl-C₁₋C₄ alkylene-, C₃₋C₁₀ cycloalkyl-C₁₋C₄ alkylene-, (5-10 membered heteroaryl)-C₁₋C₄ alkylene-, (4-10 membered heterocycloalkyl)-C₁₋C₄ alkylene-, CN, NH₂, NHOR^{e}, OR^{e}, SR^{e}, C(O)R^{e}, C(O)NR^{e}R^{e}, C(O)OR^{e}, OC(O)R^{e}, OC(O)NR^{e}R^{e}, NHR^{e}, NR^{e}R^{e}, NR^{e}C(O)R^{e}, NR^{e}C(O)NR^{e}R^{e}, NR^{e}C(O)OR^{e}, C(=NR^{e})NR^{e}R^{e}, NR^{e}C(=NR^{e})NR^{e}R^{e}, NR^{e}C(=NOH)NR^{e}R^{e}, NR^{e}C(=NCN)NR^{e}R^{e}, S(O)R^{e}, S(O)NR^{e}R^{e}, S(O)₂R^{e}, NR^{e}S(O)₂R^{e}, NR^{e}S(O)₂NR^{e}R^{e}, and S(O)₂NR^{e}R^{e}; wherein the C₁-C₆ alkyl, C₆₋C₁₀ aryl, 5-10 membered heteroaryl, C₃₋C₁₀ cycloalkyl, 4-10 membered heterocycloalkyl, C₆₋C₁₀ aryl-C₁₋C₄ alkylene-, C₃₋C₁₀ cycloalkyl-C₁₋C₄alkylene-, (5-10 membered heteroaryl)-C₁₋C₄ alkylene-, and (4-10 membered heterocycloalkyl)-C₁₋C₄ alkylene- of R^{d} are each optionally substituted with 1, 2, or 3 independently selected R^{f} substituents;
each R^{e} is independently selected from H, C₁-C₆ alkyl, C₁₋C₆ alkoxy, C₃₋C₆ cycloalkyl, C₃₋C₆ cycloalkyl-C₁₋C₄ alkylene-, C₆₋C₁₀ aryl, C₆₋C₁₀ aryl-C₁₋C₄ alkylene-, 5- or 6-membered heteroaryl, (5- or 6-membered heteroaryl)-C₁₋C₄ alkylene-, 4-7-membered heterocycloalkyl, (4-7-membered heterocycloalkyl)-C₁₋C₄ alkylene-, C₁-C₆ haloalkyl, C₁₋C₆ haloalkoxy, C₂₋C₄ alkenyl, and C₂₋C₄ alkynyl, wherein the C₁-C₆ alkyl, C₁-C₆ alkoxy, C₃₋C₆ cycloalkyl, C₆₋C₁₀ aryl, 5 or 6-membered heteroaryl, 4-7-membered heterocycloalkyl, C₆₋C₁₀ aryl-C₁₋C₄ alkylene-, (5- or 6-membered heteroaryl)-C₁₋C₄ alkylene-, (4-7-membered heterocycloalkyl)-C₁₋C₄ alkylene-, C₂₋C₄ alkenyl, and C₂₋C₄ alkynyl of R^{e} are each optionally substituted with 1, 2, or 3 R^{f} substituents;
or any two R^{e} substituents together with the nitrogen atom to which they are attached form a 4-, 5-, 6-, 7-, 8-, 9-, or 10-membered heterocycloalkyl, each of which is optionally substituted with 1, 2, or 3 independently selected R^{f} substituents;
each R^{f} is independently selected from t halo, OH, CN, C(O)OH, NH₂, -NH(C₁₋C₆ alkyl), -N(C₁₋C₆ alkyl)₂, C₁₋C₆ alkyl, vinyl, C₁₋C₆ alkoxy, C₁₋C₆ alkylthio, C₁₋C₆ haloalkyl, C₁₋C₆ haloalkoxy, phenyl, 5-6 membered heteroaryl, 4-6 membered heterocycloalkyl, and C₃₋C₆ cycloalkyl, wherein the C₁₋C₆ alkyl, C₁₋C₆ alkoxy, C₁₋C₆ alkylthio, phenyl, C₃₋C₆ cycloalkyl, 4-6 membered heterocycloalkyl, and 5-6 membered heteroaryl of R^{f} are each optionally substituted with 1, 2, or 3 substituents selected from halo, OH, CN, - C(O)OH, -NH₂, C₁₋C₄ alkyl, C₁₋C₄ alkoxy, C₁₋C₄ haloalkyl, C₁₋C₄ haloalkoxy, phenyl, C₃₋C₁₀ cycloalkyl, 5-6 membered heteroaryl, and 4-6 membered heterocycloalkyl;
each R^{g} is independently selected from halo, OH, CN, C(O)OH, -C(O)O-C₁₋C₄ alkyl, NH₂, - NH(C₁₋C₆ alkyl), -N(C₁₋C₆ alkyl)₂, C₁₋C₆ alkyl, C₁₋C₆ alkoxy, C₁₋C₆ alkylthio, C₁₋C₆ haloalkyl, C₁₋C₆ haloalkoxy, phenyl, 5-6 membered heteroaryl, 4-6 membered heterocycloalkyl, and C₃₋C₆ cycloalkyl; and
the subscript m is 0.

In some embodiments, R¹ is H or alkoxy. In some embodiments, R¹ is H or C₁₋₆ alkoxy. In some embodiments, R² is H or alkoxy. In some embodiments, R² is H or C₁₋₆ alkoxy optionally substituted with C₁₋₆ alkoxy. In some embodiments, one of R¹ and R² is H and the other of R¹ and R² is alkoxy. In some embodiments, both R¹ and R² are alkoxy.

In some embodiments, R¹ is H, C₁₋₆ alkyl, C₁₋₆ alkoxy, halo, NH₂, -NH(C₁₋₆ alkyl), -N(C₁₋₆ alkyl)₂, C₁₋₆ alkylNHC(O)-, or C₁₋₆alkylSO₂NH-. In some embodiments, R² is H, C₁₋₆ alkyl, C₁₋₆ alkoxy, halo, OH, NH₂, -NH(C₁₋₆ alkyl), -N(C₁₋₆ alkyl)₂, -C₁₋₆ alkylNHC(O)-, CF₃, C₁₋₆ alkylOC(O)-, pyridyl, C₁₋₆alkylSO₂NH- or 1H-pyrazol-4-yl optionally substituted with R^{g}.

In some embodiments, one of R¹ and R² is OR^{a} and R^{a} is C₁₋₆ alkyl substituted 1 R^{d}. In some embodiments, R² is OR^{a} and R^{a} is C₁₋₆ alkyl substituted 1 R^{d}. In some embodiments, R^{d} is OR^{e} or C₃₋₁₀ cycloalkyl. In some embodiments, R^{e} is C₁₋₆ alkyl.

In some embodiments, in the compound of Formula (Ia), Formula (Ib), or Formula (Ic), or a pharmaceutically acceptable salt, stereoisomer, or tautomer thereof, G is C₃₋₆ cycloalkyl or 4- to 6-membered heterocycloalkyl.

In some embodiments, in the compound of Formula (Ia), Formula (Ib), or Formula (Ic), or a pharmaceutically acceptable salt, stereoisomer, or tautomer thereof, G is cyclopropyl, cyclobutyl, cyclopentyl, cyclohexyl, cyclobutene-1-yl, cyclopenten-1-yl, cyclohexten-1-yl, 3,6-dihydro-2H-pyran-4-yl, piperidin-1-yl, pyrrolidine-1-yl or morpholino.

In some embodiments, in the compound of Formula (Ia), Formula (Ib), or Formula (Ic), or a pharmaceutically acceptable salt, stereoisomer, or tautomer thereof, G is C₁₋₆ alkyl, C₂₋₆ alkenyl, C₁₋₆alkoxy or halo.

In some embodiments, in the compound of Formula (Ia), Formula (Ib), or Formula (Ic), or a pharmaceutically acceptable salt, stereoisomer, or tautomer thereof, G is C₂₋₆ alkenyl substituted with C₁₋C₆ alkyl or C₃₋C₆ cycloalkyl.

In some embodiments, in the compound of Formula (Ia), Formula (Ib), or Formula (Ic), or a pharmaceutically acceptable salt, stereoisomer, or tautomer thereof, G is cyano, C(O)OR^{a}, or C(O)NR^{a}R^{a}.

In some embodiments, in the compound of Formula (Ia), Formula (Ib), or Formula (Ic), or a pharmaceutically acceptable salt, stereoisomer, or tautomer thereof, G is C₁₋₆ alkyl, vinyl, methoxy or halo.

In some embodiments, G is ring B as described below.

In some embodiments, the compound is a compound of formula (Ia): 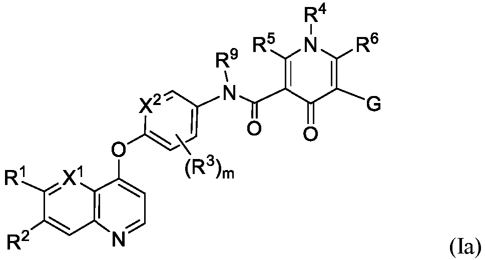 or a pharmaceutically acceptable salt, stereoisomer, or tautomer thereof.

In some embodiments, the compound is a compound of formula (Ia-1): 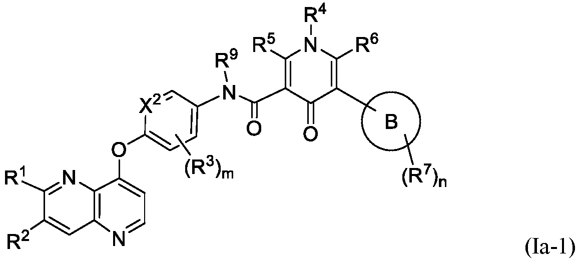 or a pharmaceutically acceptable salt, stereoisomer, or tautomer thereof, wherein ring B is cyclopentene-1-yl, cyclohexen-1-yl or 3,6-dihydro-2H-pyran-4-yl and the subscript n is 0, 1, 2, 3 or 4.

In some embodiments, the compound is a compound of formula (Ib): 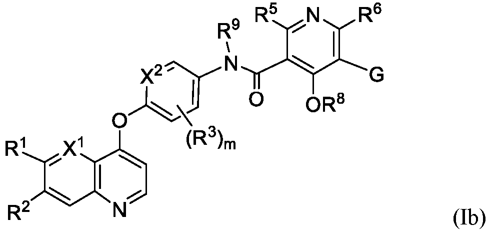 or a pharmaceutically acceptable salt, stereoisomer, or tautomer thereof.

In some embodiments, the compound is a compound of formula (Ib-1): 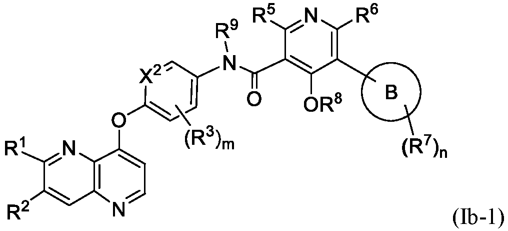 or a pharmaceutically acceptable salt, stereoisomer, or tautomer thereof, wherein ring B is cyclopentene-1-yl, cyclohexen-1-yl or 3,6-dihydro-2H-pyran-4-yl and the subscript n is 0, 1, 2, 3 or 4.

In some embodiments, the compound is a compound of formula (Ic): 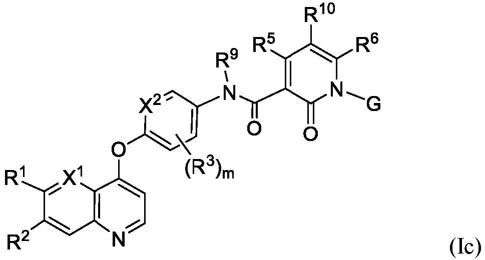 or a pharmaceutically acceptable salt, stereoisomer, or tautomer thereof.

In some embodiments, the compound is a compound of formula (Ic-1): 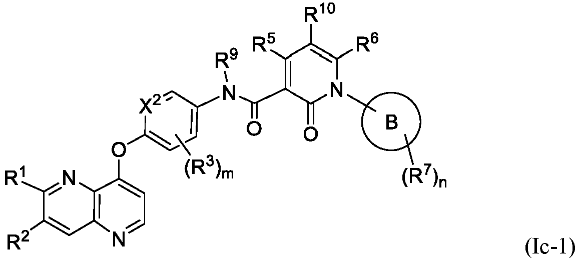 or a pharmaceutically acceptable salt, stereoisomer, or tautomer thereof, wherein ring B is cyclopentene-1-yl, cyclohexen-1-yl or 3,6-dihydro-2H-pyran-4-yl and the subscript n is 0, 1, 2, 3 or 4.

In some embodiments of a compound of Formula (Ia), Formula (Ib), or Formula (Ic) or any sub-formula thereof, or a pharmaceutically acceptable salt, stereoisomer, or tautomer thereof, R¹ is H, C₁₋₆ alkyl, C₁₋₆ alkoxy, halo, NH₂, -NH(C₁₋₆ alkyl), -N(C₁₋₆ alkyl)₂, C₁₋₆ alkylNHC(O)-, or C₁₋₆alkylSO₂NH-.

In some embodiments of a compound of Formula (Ia), Formula (Ib), or Formula (Ic) or any sub-formula thereof, or a pharmaceutically acceptable salt, stereoisomer, or tautomer thereof, R¹ is H or C₁₋₆ alkoxy.

In some embodiments of a compound of Formula (Ia), Formula (Ib), or Formula (Ic) or any sub-formula thereof, or a pharmaceutically acceptable salt, stereoisomer, or tautomer thereof, R² is H, C₁₋₆ alkyl, C₁₋₆ alkoxy, halo, OH, NH₂, -NH(C₁₋₆ alkyl), -N(C₁₋₆ alkyl)₂, -C₁₋₆ alkylNHC(O)-, CF₃, C₁₋₆ alkylOC(O)-, pyridyl,
C₁₋₆alkylSO₂NH- or 1H-pyrazol-4-yl optionally substituted with R^{g}.

In some embodiments of a compound of Formula (Ia), Formula (Ib), or Formula (Ic) or any sub-formula thereof, or a pharmaceutically acceptable salt, stereoisomer, or tautomer thereof, R² is H or C₁₋₆ alkoxy optionally substituted with C₁₋₆ alkoxy.

In some embodiments of a compound of Formula (Ia), Formula (Ib), or Formula (Ic) or any sub-formula thereof, or a pharmaceutically acceptable salt, stereoisomer, or tautomer thereof, R³ is H or halo.

In some embodiments of a compound of Formula (Ia), Formula (Ib), or Formula (Ic) or any sub-formula thereof, or a pharmaceutically acceptable salt, stereoisomer, or tautomer thereof, R⁷ is H, halo, C₁₋₆ alkyl or
C₁₋₆ alkoxy.

In some embodiments of a compound of Formula (Ia), Formula (Ib), or Formula (Ic) or any sub-formula thereof, or a pharmaceutically acceptable salt, stereoisomer, or tautomer thereof, R⁹ is H or methyl. In some embodiments of a compound of Formula (Ia), Formula (Ib), or Formula (Ic) or any sub-formula thereof, or a pharmaceutically acceptable salt, stereoisomer, or tautomer thereof, R⁹ is H.

In compounds of Formula (Ia), Formula (Ib), or Formula (Ic) or any sub-formula thereof, or a pharmaceutically acceptable salt, stereoisomer, or tautomer thereof, X¹ is N.

In compounds of Formula (Ia), Formula (Ib), or Formula (Ic) or any sub-formula thereof, or a pharmaceutically acceptable salt, stereoisomer, or tautomer thereof, X² is CH or CF and m is 0.

In some embodiments of a compound of Formula (Ia), Formula (Ib), or Formula (Ic) or any sub-formula thereof, or a pharmaceutically acceptable salt, stereoisomer, or tautomer thereof, R⁴ is selected from H, C₁₋₆ alkyl,
C₁₋₆ alkoxy, OH, C₃₋₆ cycloalkyl, C₁₋₆ haloalkyl, C₃₋₆ cycloalkyl-C₁₋₄ alkylene-, 4-6 membered heterocycloalkyl, (4-6 membered heterocycloalkyl)-C₁₋₄ alkylene-, 5-6 membered heteroaryl, (5-6 membered heteroaryl)-C₁₋₄ alkylene-, and N=C[N(C₁₋₆ alkyl)(C₁₋₆ alkyl)]₂, wherein the C₁₋₆ alkyl, C₁₋₆ alkoxy, C₃₋₆ cycloalkyl, C₃₋₆ cycloalkyl-C₁₋₄ alkylene-, 4-6 membered heterocycloalkyl, (4-6 membered heterocycloalkyl)-C₁₋₄ alkylene-, 5-6 membered heteroaryl, (5-6 membered heteroaryl)-C₁₋₄ alkylene-, and N=C[N(C₁₋₆ alkyl)(C₁₋₆ alkyl)]₂ of R⁴ are each optionally substituted with 1 or 2 independently selected R^{b} or R^{g} substituents.

In some embodiments of a compound of Formula (Ia), Formula (Ib), or Formula (Ic) or any sub-formula thereof, or a pharmaceutically acceptable salt, stereoisomer, or tautomer thereof, R⁴ is C₁₋₆ alkyl or C₁₋₆ haloalkyl.

In some embodiments of a compound of Formula (Ia), Formula (Ib), or Formula (Ic) or any sub-formula thereof, or a pharmaceutically acceptable salt, stereoisomer, or tautomer thereof, R⁴ and R⁵ taken together with the atoms to which they are attached form 4- to 7-membered fused heterocycloalkyl.

In some embodiments of a compound of Formula (Ia), Formula (Ib), or Formula (Ic) or any sub-formula thereof, or a pharmaceutically acceptable salt, stereoisomer, or tautomer thereof, R⁵ and R⁶ are each independently selected from H, CH₃, propen-2-yl, Br, Cl, CN, methoxy, 2-fluoroethyl, isopropyl, CH₃C(O)-, OH,
t-butyl, ethyl, hydroxymethyl, isopropylthio, and methoxymethyl.

In some embodiments of a compound of Formula (Ia), Formula (Ib), or Formula (Ic) or any sub-formula thereof, or a pharmaceutically acceptable salt, stereoisomer, or tautomer thereof, R¹⁰ is H, CH₃, propen-2-yl, Br, Cl, CN, methoxy, 2-fluoroethyl, isopropyl, CH₃C(O)-, OH, t-butyl, ethyl, hydroxymethyl, isopropylthio or methoxymethyl.

In some embodiments of a compound of Formula (Ia), Formula (Ib), or Formula (Ic)) or any sub-formula thereof, or a pharmaceutically acceptable salt, stereoisomer, or tautomer thereof, each R⁸ is independently H or
C₁₋₆ alkyl.

The invention also provides a process for preparing a compound of Formula (Ia), Formula (Ib), or Formula (Ic), or a pharmaceutically acceptable salt, stereoisomer or tautomer thereof, wherein: the process comprises contacting a compound of Formula A-a, Formula A-b, or Formula A-c: 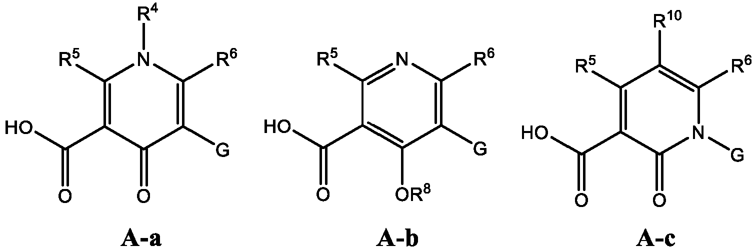 with a compound of Formula B-c: 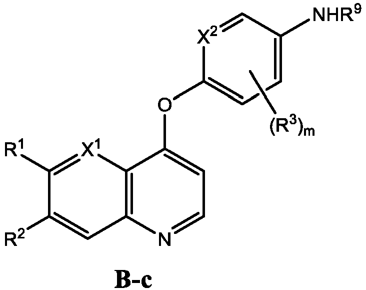 under amide bond forming conditions to provide the compound of Formula (Ia), Formula (Ib), or Formula (Ic), or a pharmaceutically acceptable salt, stereoisomer or tautomer thereof, wherein: X¹, X², R¹, R², R³, R⁴, R⁵, R⁶, R⁸, R⁹, R¹⁰, G and m are as defined above. In some embodiments, the amide bond forming conditions comprise HATU in the presence of a base in an organic solvent.

In some embodiments, a compound of the invention is selected from:
7-(cyclopenten-1-yl)-N-[4-[(6,7-dimethoxy-1,5-naphthyridin-4-yl)oxy]-3-fluorophenyl]-8-oxo-3,4-dihydro-1H-pyrido[2, 1-c][1,4]oxazine-9-carboxamide;
5-(cyclopenten-1-yl)-N-[4-[(6,7-dimethoxy-1,5-naphthyridin-4-yl)oxy]-3-fluorophenyl]-4-hydroxy-6-methylpyridine-3-carboxamide;
5-(cyclohexen-1-yl)-N-[4-[(6,7-dimethoxy-1,5-naphthyridin-4-yl)oxy]-3-fluorophenyl]-4-hydroxy-6-methylpyridine-3-carboxamide;
7-(cyclohexen-1-yl)-N-[4-[(6,7-dimethoxy-1,5-naphthyridin-4-yl)oxy]-3-fluorophenyl]-8-oxo-3,4-dihydro-1H-pyrido[2, 1-c][1,4]oxazine-9-carboxamide;
7-(cyclohexen-1-yl)-N-[4-[(6,7-dimethoxy-1,5-naphthyridin-4-yl)oxy]phenyl]-8-oxo-3,4-dihydro-1H-pyrido[2, 1-c] [1,4]oxazine-9-carboxamide;
7-(cyclopenten-1-yl)-N-[4-[(6,7-dimethoxy-1,5-naphthyridin-4-yl)oxy]phenyl]-8-oxo-3,4-dihydro-1H-pyrido[2, 1-c] [1,4]oxazine-9-carboxamide;
7-(3,6-dihydro-2H-pyran-4-yl)-N-[4-[(6,7-dimethoxy-1,5-naphthyridin-4-yl)oxy]-3-fluorophenyl]-8-oxo-3,4-dihydro-1H-pyrido[2,1-c][1,4]oxazine-9-carboxamide;
7-(3,6-dihydro-2H-pyran-4-yl)-N-[4-[(6,7-dimethoxy-1,5-naphthyridin-4-yl)oxy]phenyl]-8-oxo-3,4-dihydro-1H-pyrido[2, 1-c][1,4]oxazine-9-carboxamide;
5-(cyclopenten-1-yl)-N-[4-[(6,7-dimethoxy-1,5-naphthyridin-4-yl)oxy]-3-fluorophenyl]-4-hydroxy-2,6-dimethylpyridine-3-carboxamide;
5-(3,6-dihydro-2H-pyran-4-yl)-N-[4-[(6,7-dimethoxy-1,5-naphthyridin-4-yl)oxy]-3-fluorophenyl]-4-hydroxy-6-methylpyridine-3-carboxamide;
5-(3,6-dihydro-2H-pyran-4-yl)-N-[3-fluoro-4-[(7-methoxy-1,5-naphthyridin-4-yl)oxy]phenyl]-4-hydroxy-6-methylpyridine-3-carboxamide;
5-(3,6-dihydro-2H-pyran-4-yl)-N-[3-fluoro-4-[(6-methoxy-1,5-naphthyridin-4-yl)oxy]phenyl]-4-hydroxy-6-methylpyridine-3-carboxamide;
5-(cyclohexen-1-yl)-N-[3-fluoro-4-[(6-methoxy-1,5-naphthyridin-4-yl)oxy]phenyl]-4-hydroxy-6-methylpyridine-3-carboxamide;
5-(cyclopenten-1-yl)-N-[3-fluoro-4-[(6-methoxy-1,5-naphthyridin-4-yl)oxy]phenyl]-4-hydroxy-6-methylpyridine-3-carboxamide;
5-(cyclohexen-1-yl)-N-[3-fluoro-4-[(7-methoxy-1,5-naphthyridin-4-yl)oxy]phenyl]-4-hydroxy-6-methylpyridine-3-carboxamide;
5-(cyclopenten-1-yl)-N-[3-fluoro-4-[(7-methoxy-1,5-naphthyridin-4-yl)oxy]phenyl]-4-hydroxy-6-methylpyridine-3-carboxamide;
5-(cyclohexen-1-yl)-N-[4-[(6,7-dimethoxy-1,5-naphthyridin-4-yl)oxy]-3-fluorophenyl]-4-hydroxy-2,6-dimethylpyridine-3-carboxamide;
or a pharmaceutically acceptable salt, stereoisomer or tautomer thereof.

In some embodiments a compound of the invention is selected from
5-(cyclopenten-1-yl)-N-[4-[(6,7-dimethoxy-1,5-naphthyridin-4-yl)oxy]-3-fluorophenyl]-1,6-dimethyl-4-oxopyridine-3-carboxamide;
5-cyclopentyl-N-[4-[(6,7-dimethoxy-1,5-naphthyridin-4-yl)oxy]-3-fluorophenyl]-1,6-dimethyl-4-oxopyridine-3-carboxamide;
N-[3-fluoro-4-[(7-methoxy-1,5-naphthyridin-4-yl)oxy]phenyl]-4-hydroxy-2,6-dimethyl-5-prop-1-en-2-ylpyridine-3-carboxamide;
N-[4-[(6,7-dimethoxy-1,5-naphthyridin-4-yl)oxy]phenyl]-4-hydroxy-2,6-dimethyl-5-prop-1-en-2-ylpyridine-3-carboxamide;
N-[4-[(6,7-dimethoxy-1,5-naphthyridin-4-yl)oxy]-3-fluorophenyl]-4-hydroxy-2,6-dimethyl-5-prop-1-en-2-ylpyridine-3-carboxamide;
N-[4-[(6,7-dimethoxy-1,5-naphthyridin-4-yl)oxy]-2,5-difluorophenyl]-4-hydroxy-2,6-dimethyl-5-prop-1-en-2-ylpyridine-3-carboxamide;
5-(3,6-dihydro-2H-pyran-4-yl)-4-hydroxy-N-[4-[(7-methoxy-1,5-naphthyridin-4-yl)oxy]phenyl]-6-methylpyridine-3-carboxamide;
5-(cyclohexen-1-yl)-N-[4-[(6,7-dimethoxy-1,5-naphthyridin-4-yl)oxy]-3-fluorophenyl]-1-(2-fluoroethyl)-6-methyl-4-oxopyridine-3-carboxamide;
N-[4-[(6,7-dimethoxy-1,5-naphthyridin-4-yl)oxy]phenyl]-4-hydroxy-2,6-dimethyl-5-propan-2-ylpyridine-3-carboxamide;
N-[4-[(6,7-dimethoxy-1,5-naphthyridin-4-yl)oxy]-2,5-difluorophenyl]-4-hydroxy-2,6-dimethyl-5-propan-2-ylpyridine-3-carboxamide;
5-(cyclopenten-1-yl)-N-[4-[(6,7-dimethoxy-1,5-naphthyridin-4-yl)oxy]-3-fluorophenyl]-1-(2-fluoroethyl)-6-methyl-4-oxopyridine-3-carboxamide;
5-(3,6-dihydro-2H-pyran-4-yl)-N-[3-fluoro-4-[(7-methoxy-1,5-naphthyridin-4-yl)oxy]phenyl]-1,6-dimethyl-4-oxopyridine-3-carboxamide;
5-cyclopentyl-N-[4-[(6,7-dimethoxy-1,5-naphthyridin-4-yl)oxy]-3-fluorophenyl]-1-(2-fluoroethyl)-6-methyl-4-oxopyridine-3-carboxamide;
1-(cyclopenten-1-yl)-N-[4-[(6,7-dimethoxy-1,5-naphthyridin-4-yl)oxy]-3-fluorophenyl]-4,6-dimethyl-2-oxopyridine-3-carboxamide;
1-cyclopentyl-N-[4-[(6,7-dimethoxy-1,5-naphthyridin-4-yl)oxy]-3-fluorophenyl]-4,6-dimethyl-2-oxopyridine-3-carboxamide;
N-[4-[(6,7-dimethoxy-1,5-naphthyridin-4-yl)oxy]-3-fluorophenyl]-4,6-dimethyl-2-oxo-1-prop-1-en-2-ylpyridine-3-carboxamide;
N-[3-fluoro-4-[(7-methoxy-1,5-naphthyridin-4-yl)oxy]phenyl]-4,6-dimethyl-2-oxo-1-prop-1-en-2-ylpyridine-3-carboxamide;
5-bromo-1-cyclopentyl-N-[4-[(6,7-dimethoxy-1,5-naphthyridin-4-yl)oxy]-3-fluorophenyl]-4,6-dimethyl-2-oxopyridine-3-carboxamide;
5-[(E)-2-cyclopentylethenyl]-N-[4-[(6,7-dimethoxy-1,5-naphthyridin-4-yl)oxy]-3-fluorophenyl]-4-hydroxy-6-methylpyridine-3-carboxamide;
5-[(E)-2-cyclopropylethenyl]-N-[4-[(6,7-dimethoxy-1,5-naphthyridin-4-yl)oxy]-3-fluorophenyl]-4-hydroxy-6-methylpyridine-3-carboxamide;
N-[4-[(6,7-dimethoxy-1,5-naphthyridin-4-yl)oxy]-3-fluorophenyl]-5-[(E)-3,3-dimethylbut-1-enyl]-4-hydroxy-6-methylpyridine-3-carboxamide;
N-[4-[(6,7-dimethoxy-1,5-naphthyridin-4-yl)oxy]-3-fluorophenyl]-4-hydroxy-6-methyl-5-[(E)-4-methylpent-1-enyl]pyridine-3-carboxamide;
N-[4-[(6,7-dimethoxy-1,5-naphthyridin-4-yl)oxy]-3-fluorophenyl]-4-hydroxy-2,6-dimethyl-5-propan-2-ylpyridine-3-carboxamide;
N-[3-fluoro-4-[(7-methoxy-1,5-naphthyridin-4-yl)oxy]phenyl]-4-hydroxy-2,6-dimethyl-5-propan-2-ylpyridine-3-carboxamide;
5-(cyclopenten-1-yl)-N-[3-fluoro-4-[[6-methoxy-7-(2-methoxyethoxy)-1,5-naphthyridin-4-yl]oxy]phenyl]-4-hydroxy-6-methylpyridine-3-carboxamide;
5-(cyclohexen-1-yl)-N-[3-fluoro-4-[[6-methoxy-7-(2-methoxyethoxy)-1,5-naphthyridin-4-yl]oxy]phenyl]-4-hydroxy-6-methylpyridine-3-carboxamide;
5-(3,6-dihydro-2H-pyran-4-yl)-N-[3-fluoro-4-[[6-methoxy-7-(2-methoxyethoxy)-1,5-naphthyridin-4-yl]oxy]phenyl]-4-hydroxy-6-methylpyridine-3-carboxamide;
5-[(E)-2-cyclopentylethenyl]-N-[3-fluoro-4-[[6-methoxy-7-(2-methoxyethoxy)-1,5-naphthyridin-4-yl]oxy]phenyl]-4-hydroxy-6-methylpyridine-3-carboxamide;
5-[(E)-2-cyclopropylethenyl]-N-[3-fluoro-4-[[6-methoxy-7-(2-methoxyethoxy)-1,5-naphthyridin-4-yl]oxy]phenyl]-4-hydroxy-6-methylpyridine-3-carboxamide;
N-[4-[(6,7-dimethoxy-1,5-naphthyridin-4-yl)oxy]-3-fluorophenyl]-4-methyl-2-oxo-1-propan-2-ylpyridine-3-carboxamide;
N-[4-[(6,7-dimethoxy-1,5-naphthyridin-4-yl)oxy]phenyl]-4-methyl-2-oxo-1-propan-2-ylpyridine-3-carboxamide;
5-[(E)-3,3-dimethylbut-1-enyl]-N-[3-fluoro-4-[[6-methoxy-7-(2-methoxyethoxy)-1,5-naphthyridin-4-yl]oxy]phenyl]-4-hydroxy-6-methylpyridine-3-carboxamide;
5-(cyclopenten-1-yl)-N-[3-fluoro-4-[[6-methoxy-7-(2-methoxyethoxy)-1,5-naphthyridin-4-yl]oxy]phenyl]-4-hydroxy-2,6-dimethylpyridine-3-carboxamide;
5-(cyclopenten-1-yl)-N-[3-fluoro-4-[[6-methoxy-7-(2-methoxyethoxy)-1,5-naphthyridin-4-yl]oxy]phenyl]-1,2,6-trimethyl-4-oxopyridine-3-carboxamide;
5-(3,6-dihydro-2H-pyran-4-yl)-N-[3-fluoro-4-[[6-methoxy-7-(2-methoxyethoxy)-1,5-naphthyridin-4-yl]oxy]phenyl]-4-hydroxy-2,6-dimethylpyridine-3-carboxamide;
5-(3,6-dihydro-2H-pyran-4-yl)-N-[3-fluoro-4-[[6-methoxy-7-(2-methoxyethoxy)-1,5-naphthyridin-4-yl]oxy]phenyl]-1,2,6-trimethyl-4-oxopyridine-3-carboxamide;
5-[(E)-3,3-dimethylbut-1-enyl]-N-[3-fluoro-4-[[6-methoxy-7-(2-methoxyethoxy)-1,5-naphthyridin-4-yl]oxy]phenyl]-1,2,6-trimethyl-4-oxopyridine-3-carboxamide;
5-cyclopropyl-N-[4-[(6,7-dimethoxy-1,5-naphthyridin-4-yl)oxy]-3-fluorophenyl]-1,2,6-trimethyl-4-oxopyridine-3-carboxamide;
5-cyclopropyl-N-[4-[(6,7-dimethoxy-1,5-naphthyridin-4-yl)oxy]-2,5-difluorophenyl]-1,2,6-trimethyl-4-oxopyridine-3-carboxamide;
5-cyclopropyl-N-[4-[(6,7-dimethoxy-1,5-naphthyridin-4-yl)oxy]phenyl]-1,2,6-trimethyl-4-oxopyridine-3-carboxamide;
N-[4-[(6,7-dimethoxy-1,5-naphthyridin-4-yl)oxy]-3-fluorophenyl]-5-[(E)-3,3-dimethylbut-1-enyl]-1,2,6-trimethyl-4-oxopyridine-3-carboxamide;
5-[(E)-2-cyclopropylethenyl]-N-[4-[(6,7-dimethoxy-1,5-naphthyridin-4-yl)oxy]-3-fluorophenyl]-1,2,6-trimethyl-4-oxopyridine-3-carboxamide;
5-[(E)-2-cyclopropylethenyl]-N-[3-fluoro-4-[[6-methoxy-7-(2-methoxyethoxy)-1,5-naphthyridin-4-yl]oxy]phenyl]-1,2,6-trimethyl-4-oxopyridine-3-carboxamide;
N-[4-[(6,7-dimethoxy-1,5-naphthyridin-4-yl)oxy]-3-fluorophenyl]-1,2,6-trimethyl-4-oxo-5-prop-1-en-2-ylpyridine-3-carboxamide;
5-(3,6-dihydro-2H-pyran-4-yl)-N-[4-[(6,7-dimethoxy-1,5-naphthyridin-4-yl)oxy]-3-fluorophenyl]-1,2,6-trimethyl-4-oxopyridine-3-carboxamide;
5-[(E)-2-cyclopentylethenyl]-N-[3-fluoro-4-[[6-methoxy-7-(2-methoxyethoxy)-1,5-naphthyridin-4-yl]oxy]phenyl]-1,2,6-trimethyl-4-oxopyridine-3-carboxamide;
N-[4-[(6,7-dimethoxy-1,5-naphthyridin-4-yl)oxy]phenyl]-1,2,6-trimethyl-4-oxo-5-prop-1-en-2-ylpyridine-3-carboxamide;
N-[4-[(6,7-dimethoxy-1,5-naphthyridin-4-yl)oxy]-3-fluorophenyl]-1,2,6-trimethyl-4-oxo-5-propan-2-ylpyridine-3-carboxamide;
N-[4-[(6,7-dimethoxy-1,5-naphthyridin-4-yl)oxy]phenyl]-1,2,6-trimethyl-4-oxo-5-propan-2-ylpyridine-3-carboxamide;
5-(cyclohexen-1-yl)-N-[3-fluoro-4-[[6-methoxy-7-(2-methoxyethoxy)-1,5-naphthyridin-4-yl]oxy]phenyl]-1,2,6-trimethyl-4-oxopyridine-3-carboxamide;
4-hydroxy-5-methoxy-N-[4-[(7-methoxy-1,5-naphthyridin-4-yl)oxy]phenyl]-2,6-dimethylpyridine-3-carboxamide;
N-[3-fluoro-4-[(7-methoxy-1,5-naphthyridin-4-yl)oxy]phenyl]-4-hydroxy-5-methoxy-2,6-dimethylpyridine-3-carboxamide;
N-[4-[(6,7-dimethoxy-1,5-naphthyridin-4-yl)oxy]phenyl]-4-hydroxy-5-methoxy-2,6-dimethylpyridine-3-carboxamide;
N-[4-[(6,7-dimethoxy-1,5-naphthyridin-4-yl)oxy]-3-fluorophenyl]-4-hydroxy-5-methoxy-2,6-dimethylpyridine-3-carboxamide;
5-bromo-N-[4-[(6,7-dimethoxy-1,5-naphthyridin-4-yl)oxy]-2,5-difluorophenyl]-6-ethyl-1-methyl-4-oxopyridine-3-carboxamide;
5-bromo-6-ethyl-N-[4-[[6-methoxy-7-(2-methoxyethoxy)-1,5-naphthyridin-4-yl]oxy]phenyl]-1-methyl-4-oxopyridine-3-carboxamide;
5-(cyclopenten-1-yl)-N-[3-fluoro-4-[[7-(2-methoxyethoxy)-1,5-naphthyridin-4-yl]oxy]phenyl]-1,2,6-trimethyl-4-oxopyridine-3-carboxamide;
5-(cyclohexen-1-yl)-N-[3-fluoro-4-[[7-(2-methoxyethoxy)-1,5-naphthyridin-4-yl]oxy]phenyl]-1,2,6-trimethyl-4-oxopyridine-3-carboxamide;
5-(3,6-dihydro-2H-pyran-4-yl)-N-[3-fluoro-4-[[7-(2-methoxyethoxy)-1,5-naphthyridin-4-yl]oxy]phenyl]-1,2,6-trimethyl-4-oxopyridine-3-carboxamide;
N-[4-[(6,7-dimethoxy-1,5-naphthyridin-4-yl)oxy]-2,5-difluorophenyl]-4-hydroxy-5-methoxy-2,6-dimethylpyridine-3-carboxamide;
7-(cyclopenten-1-yl)-N-[4-[(6,7-dimethoxy-1,5-naphthyridin-4-yl)oxy]-3-fluorophenyl]-6-methyl-8-oxo-3,4-dihydro-1H-pyrido[2, 1-c][1,4]oxazine-9-carboxamide;
N-[3-fluoro-4-[[6-methoxy-7-(2-methoxyethoxy)-1,5-naphthyridin-4-yl]oxy]phenyl]-4-hydroxy-5-methoxy-2,6-dimethylpyridine-3-carboxamide;
4-hydroxy-5-methoxy-N-[4-[[6-methoxy-7-(2-methoxyethoxy)-1,5-naphthyridin-4-yl]oxy]phenyl]-2,6-dimethylpyridine-3-carboxamide;
7-(3,6-dihydro-2H-pyran-4-yl)-N-[4-[(6,7-dimethoxy-1,5-naphthyridin-4-yl)oxy]phenyl]-6-methyl-8-oxo0-3,4-dihydro-1H-pyrido[2,1-c][1,4]oxazine-9-carboxamide;
N-[4-[[7-(2-cyclobutylethoxy)-6-methoxy-1,5-naphthyridin-4-yl]oxy]-3-fluorophenyl]-5-(cyclopenten-1-yl)-1,2,6-trimethyl-4-oxopyridine-3-carboxamide;
N-[3-fluoro-4-[(7-methoxy-1,5-naphthyridin-4-yl)oxy]phenyl]-5-methoxy-1,2,6-trimethyl-4-oxopyridine-3-carboxamide;
N-[4-[(6,7-dimethoxy-1,5-naphthyridin-4-yl)oxy]phenyl]-5-methoxy-1,2,6-trimethyl-4-oxopyridine-3-carboxamide;
N-[4-[(6,7-dimethoxy-1,5-naphthyridin-4-yl)oxy]-3-fluorophenyl]-5-methoxy-1,2,6-trimethyl-4-oxopyridine-3-carboxamide;
5-cyano-N-[4-[(6,7-dimethoxy-1,5-naphthyridin-4-yl)oxy]-3-fluorophenyl]-1,2,6-trimethyl-4-oxopyridine-3-carboxamide;
7-(3,6-dihydro-2H-pyran-4-yl)-N-[4-[(6,7-dimethoxy-1,5-naphthyridin-4-yl)oxy]-3-fluorophenyl]-6-methyl-8-oxo-3,4-dihydro-1H-pyrido[2,1-c][1,4]oxazine-9-carboxamide;
7-(cyclohexen-1-yl)-N-[4-[(6,7-dimethoxy-1,5-naphthyridin-4-yl)oxy]-3-fluorophenyl]-6-methyl-8-oxo-3,4-dihydro-1H-pyrido[2, 1-c][1,4]oxazine-9-carboxamide;
5-cyano-N-[3-fluoro-4-[(7-methoxy-1,5-naphthyridin-4-yl)oxy]phenyl]-1,2,6-trimethyl-4-oxopyridine-3-carboxamide;
5-cyano-N-[4-[(6,7-dimethoxy-1,5-naphthyridin-4-yl)oxy]phenyl]-1,2,6-trimethyl-4-oxopyridine-3-carboxamide;
5-cyano-N-[2,5-difluoro-4-[[6-methoxy-7-(2-methoxyethoxy)-1,5-naphthyridin-4-yl]oxy]phenyl]-1,2,6-trimethyl-4-oxopyridine-3-carboxamide;
3-N-[4-[(6,7-dimethoxy-1,5-naphthyridin-4-yl)oxy]-3-fluorophenyl]-4-hydroxy-2,6-dimethylpyridine-3,5-dicarboxamide;
3-N-[4-[(6,7-dimethoxy-1,5-naphthyridin-4-yl)oxy]phenyl]-4-hydroxy-2,6-dimethylpyridine-3,5-dicarboxamide;
3-N-[2,5-difluoro-4-[[6-methoxy-7-(2-methoxyethoxy)-1,5-naphthyridin-4-yl]oxy]phenyl]-4-hydroxy-2,6-dimethylpyridine-3,5-dicarboxamide;
5-cyano-N-[4-[(6,7-dimethoxy-1,5-naphthyridin-4-yl)oxy]-3-fluorophenyl]-4-hydroxy-2,6-dimethylpyridine-3-carboxamide;
N-[4-[(6,7-dimethoxy-1,5-naphthyridin-4-yl)oxy]-3-fluorophenyl]-1,2,6-trimethyl-5-morpholin-4-yl-4-oxopyridine-3-carboxamide;
N-[4-[(6,7-dimethoxy-1,5-naphthyridin-4-yl)oxy]-3-fluorophenyl]-1,2,6-trimethyl-4-oxo-5-pyrrolidin-1-ylpyridine-3-carboxamide;
N-[4-[(6,7-dimethoxy-1,5-naphthyridin-4-yl)oxy]-3-fluorophenyl]-1,2,6-trimethyl-4-oxo-5-piperidin-1-ylpyridine-3-carboxamide;
3-N-[3-fluoro-4-[(7-methoxy-1,5-naphthyridin-4-yl)oxy]phenyl]-4-hydroxy-2,6-dimethylpyridine-3,5-dicarboxamide;
5-[[4-[(6,7-dimethoxy-1,5-naphthyridin-4-yl)oxy]-3-fluorophenyl]carbamoyl]-1,2,6-trimethyl-4-oxopyridine-3-carboxylic acid;
3-N-[4-[(6,7-dimethoxy-1,5-naphthyridin-4-yl)oxy]-3-fluorophenyl]-4-hydroxy-5-N,5-N,2,6-tetramethylpyridine-3,5-dicarboxamide;
3-N-[4-[(6,7-dimethoxy-1,5-naphthyridin-4-yl)oxy]-3-fluorophenyl]-4-hydroxy-2,6-dimethyl-5-N-propan-2-ylpyridine-3,5-dicarboxamide;
5-N-[4-[(6,7-dimethoxy-1,5-naphthyridin-4-yl)oxy]-3-fluorophenyl]-3-N,3-N,1,2,6-pentamethyl-4-oxopyridine-3,5-dicarboxamide;
3-N-cyclopropyl-5-N-[4-[(6,7-dimethoxy-1,5-naphthyridin-4-yl)oxy]-3-fluorophenyl]-1,2,6-trimethyl-4-oxopyridine-3,5-dicarboxamide;
3-N-[4-[(6,7-dimethoxy-1,5-naphthyridin-4-yl)oxy]-3-fluorophenyl]-4-hydroxy-5-N,2,6-trimethylpyridine-3,5-dicarboxamide;
5-N-cyclopropyl-3-N-[4-[(6,7-dimethoxy-1,5-naphthyridin-4-yl)oxy]-3-fluorophenyl]-4-hydroxy-2,6-dimethylpyridine-3,5-dicarboxamide;
7-(cyclopenten-1-yl)-N-[4-[(6,7-dimethoxy-1,5-naphthyridin-4-yl)oxy]phenyl]-6-methyl-8-oxo-3,4-dihydro-1H-pyrido[2,1-c][1,4]oxazine-9-carboxamide; and
7-(cyclohexen-1-yl)-N-[4-[(6,7-dimethoxy-1,5-naphthyridin-4-yl)oxy]phenyl]-6-methyl-8-oxo-3,4-dihydro-1H-pyrido[2,1-c][1,4]oxazine-9-carboxamide;
or a pharmaceutically acceptable salt, stereoisomer or tautomer thereof.

In some embodiments a compound of the invention is
5-(cyclopenten-1-yl)-N-[3-fluoro-4-[[7-(2-methoxyethoxy)-1,5-naphthyridin-4-yl]oxy]phenyl]-4-hydroxy-2,6-dimethylpyridine-3-carboxamide
or a pharmaceutically acceptable salt, stereoisomer or tautomer thereof.

In some embodiments, provided is a compound, or a pharmaceutically acceptable salt, stereoisomer, or tautomer thereof, selected from Table 1:

**Table 1**

| **Cpd #** | **Structure** | **Name** |
|---|---|---|
| G1-1 | 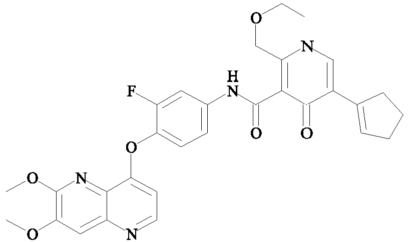 | 7-(cyclopenten-1-yl)-N-[4-[(6,7-dimethoxy-1,5-naphthyridin-4-yl)oxy]-3-fluorophenyl]-8-oxo-3,4-dihydro-1H-pyrido[2,1-c][1,4]oxazine-9-carboxamide |
| G1-2 | 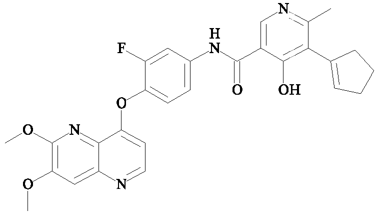 | 5-(cyclopenten-1-yl)-N-[4-[(6,7-dimethoxy-1,5-naphthyridin-4-yl)oxy]-3-fluorophenyl]-4-hydroxy-6-methylpyridine-3-carboxamide |
| G1-3 | 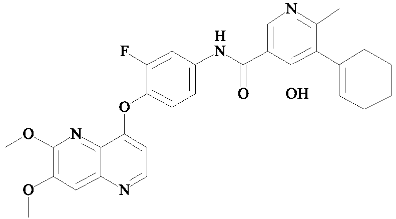 | 5-(cyclohexen-1-yl)-N-[4-[(6,7-dimethoxy-1,5-naphthyridin-4-yl)oxy]-3-fluorophenyl]-4-hydroxy-6-methylpyridine-3-carboxamide |
| G1-4 | 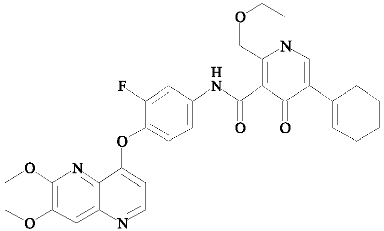 | 7-(cyclohexen-1-yl)-N-[4-[(6,7-dimethoxy-1,5-naphthyridin-4-yl)oxy]-3-fluorophenyl]-8-oxo-3,4-dihydro-1H-pyrido[2,1-c][1,4]oxazine-9-carboxamide |
| G1-5 | 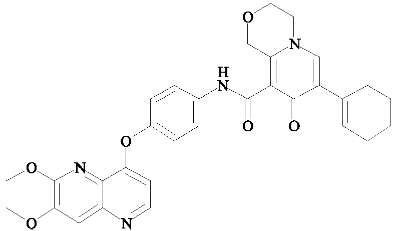 | 7-(cyclohexen-1-yl)-N-[4-[(6,7-dimethoxy-1,5-naphthyridin-4-yl)oxy]phenyl]-8-oxo-3,4-dihydro-1H-pyrido[2,1-c][1,4]oxazine-9-carboxamide |
| G1-6 | 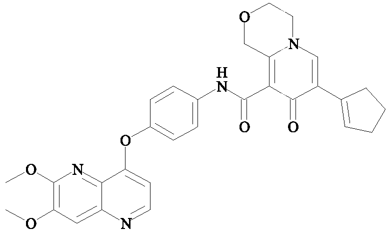 | 7-(cyclopenten-1-yl)-N-[4-[(6,7-dimethoxy-1,5-naphthyridin-4-yl)oxy]phenyl]-8-oxo-3,4-dihydro-1H-pyrido[2,1-c][1,4]oxazine-9-carboxamide |
| G1-7 | 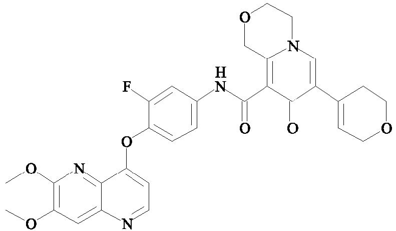 | 7-(3,6-dihydro-2H-pyran-4-yl)-N-[4-[(6,7-dimethoxy-1,5-naphthyridin-4-yl)oxy]-3-fluorophenyl]-8-oxo-3,4-dihydro-1H-pyrido[2,1-c][1,4]oxazine-9-carboxamide |
| G1-8 | 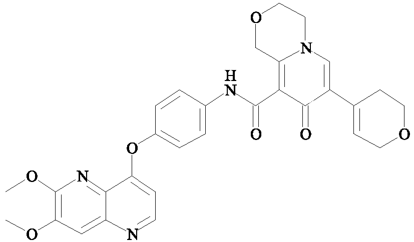 | 7-(3,6-dihydro-2H-pyran-4-yl)-N-[4-[(6,7-dimethoxy-1,5-naphthyridin-4-yl)oxy]phenyl]-8-oxo-3,4-dihydro-1H-pyrido[2,1-c][1,4]oxazine-9-carboxamide |
| 1-2 | 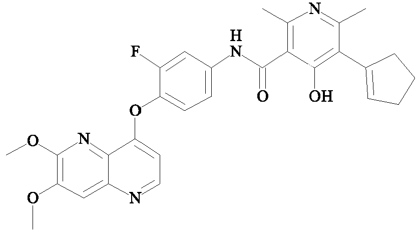 | 5-(cyclopenten-1-yl)-N-[4-[(6,7-dimethoxy-1,5-naphthyridin-4-yl)oxy]-3-fluorophenyl]-4-hydroxy-2,6-dimethylpyridine-3-carboxamide |
| 1-3 | 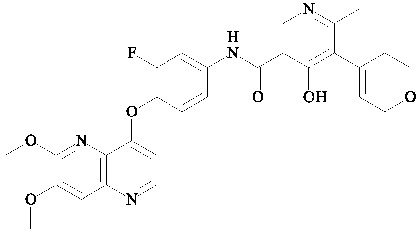 | 5-(3,6-dihydro-2H-pyran-4-yl)-N-[4-[(6,7-dimethoxy-1,5-naphthyridin-4-yl)oxy]-3-fluorophenyl]-4-hydroxy-6-methylpyridine-3-carboxamide |
| 1-4 | 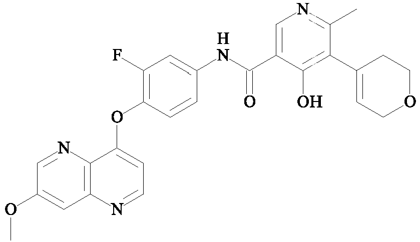 | 5-(3,6-dihydro-2H-pyran-4-yl)-N-[3-fluoro-4-[(7-methoxy-1,5-naphthyridin-4-yl)oxy]phenyl]-4-hydroxy-6-methylpyridine-3-carboxamide |
| 1-5 | 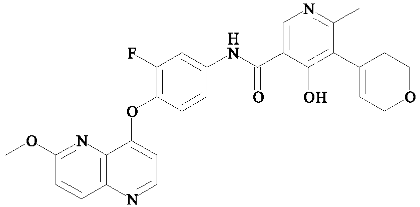 | 5-(3,6-dihydro-2H-pyran-4-yl)-N-[3-fluoro-4-[(6-methoxy-1,5-naphthyridin-4-yl)oxy]phenyl]-4-hydroxy-6-methylpyridine-3-carboxamide |
| 1-6 | 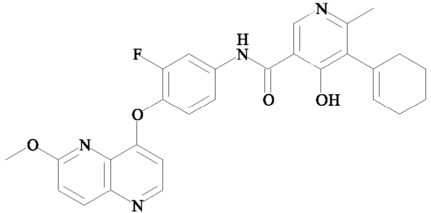 | 5-(cyclohexen-1-yl)-N-[3-fluoro-4-[(6-methoxy-1,5-naphthyridin-4-yl)oxy]phenyl]-4-hydroxy-6-methylpyridine-3-carboxamide |
| 1-7 | 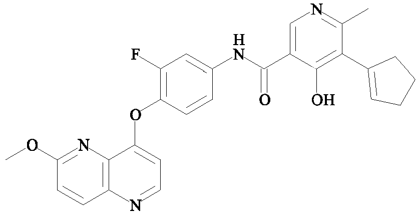 | 5-(cyclopenten-1-yl)-N-[3-fluoro-4-[(6-methoxy-1,5-naphthyridin-4-yl)oxy]phenyl]-4-hydroxy-6-methylpyridine-3-carboxamide |
| 1-8 | 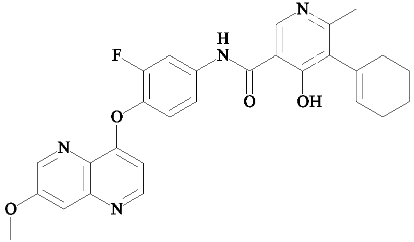 | 5-(cyclohexen-1-yl)-N-[3-fluoro-4-[(7-methoxy-1,5-naphthyridin-4-yl)oxy]phenyl]-4-hydroxy-6-methylpyridine-3-carboxamide |
| 1-9 | 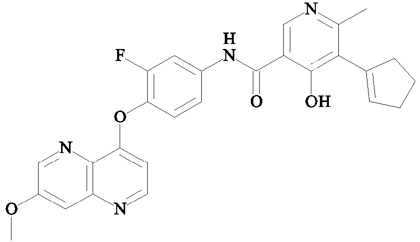 | 5-(cyclopenten-1-yl)-N-[3-fluoro-4-[(7-methoxy-1,5-naphthyridin-4-yl)oxy]phenyl]-4-hydroxy-6-methylpyridine-3-carboxamide |
| 2-3 | 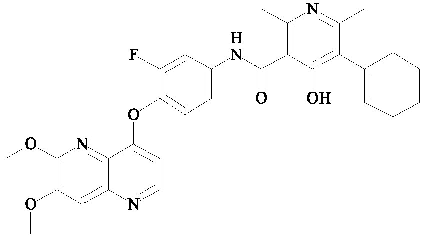 | 5-(cyclohexen-1-yl)-N-[4-[(6,7-dimethoxy-1,5-naphthyridin-4-yl)oxy]-3-fluorophenyl]-4-hydroxy-2,6-dimethylpyridine-3-carboxamide |
| 1 | 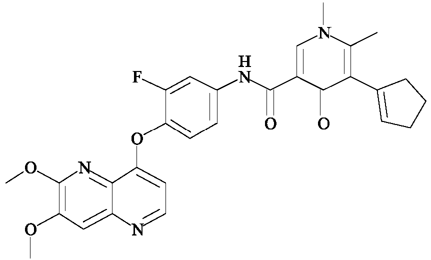 | 5-(cyclopenten-1-yl)-N-[4-[(6,7-dimethoxy-1,5-naphthyridin-4-yl)oxy]-3-fluorophenyl]-1,6-dimethyl-4-oxopyridine-3-carboxamide |
| 2 | 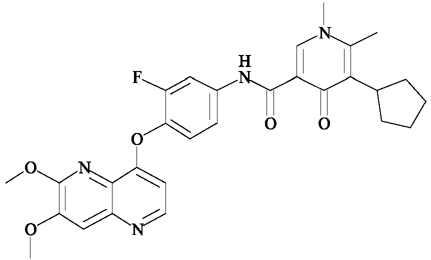 | 5-cyclopentyl-N-[4-[(6,7-dimethoxy-1,5-naphthyridin-4-yl)oxy]-3-fluorophenyl]-1,6-dimethyl-4-oxopyridine-3-carboxamide |
| 3 | 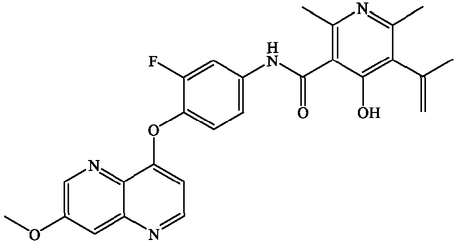 | N-[3-fluoro-4-[(7-methoxy-1,5-naphthyridin-4-yl)oxy]phenyl]-4-hydroxy-2,6-dimethyl-5-prop-1-en-2-ylpyridine-3-carboxamide |
| 4 | 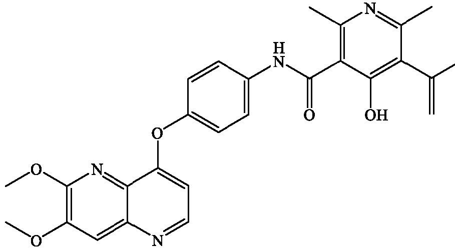 | N-[4-[(6,7-dimethoxy-1,5-naphthyridin-4-yl)oxy]phenyl]-4-hydroxy-2,6-dimethyl-5-prop-1-en-2-ylpyridine-3-carboxamide |
| 5 | 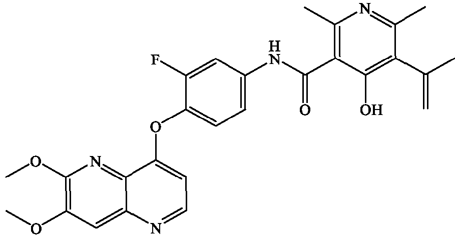 | N-[4-[(6,7-dimethoxy-1,5-naphthyridin-4-yl)oxy]-3-fluorophenyl]-4-hydroxy-2,6-dimethyl-5-prop-1-en-2-ylpyridine-3-carboxamide |
| 6 | 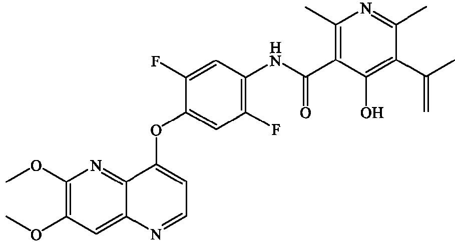 | N-[4-[(6,7-dimethoxy-1,5-naphthyridin-4-yl)oxy]-2,5-difluorophenyl]-4-hydroxy-2,6-dimethyl-5-prop-1-en-2-ylpyridine-3-carboxamide |
| 7 | 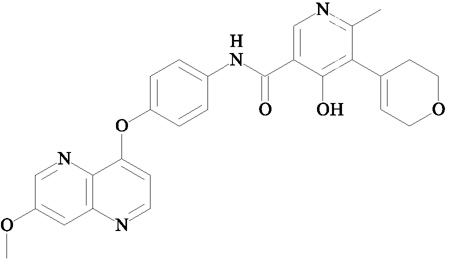 | 5-(3,6-dihydro-2H-pyran-4-yl)-4-hydroxy-N-[4-[(7-methoxy-1,5-naphthyridin-4-yl)oxy]phenyl]-6-methylpyridine-3-carboxamide |
| 8 | 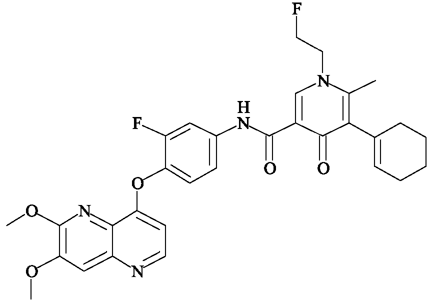 | 5-(cyclohexen-1-yl)-N-[4-[(6,7-dimethoxy-1,5-naphthyridin-4-yl)oxy]-3-fluorophenyl]-1-(2-fluoroethyl)-6-methyl-4-oxopyridine-3-carboxamide |
| 9 | 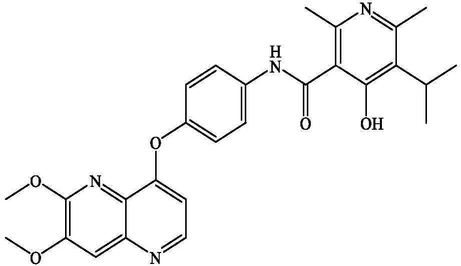 | N-[4-[(6,7-dimethoxy-1,5-naphthyridin-4-yl)oxy]phenyl]-4-hydroxy-2,6-dimethyl-5-propan-2-ylpyridine-3-carboxamide |
| 10 | 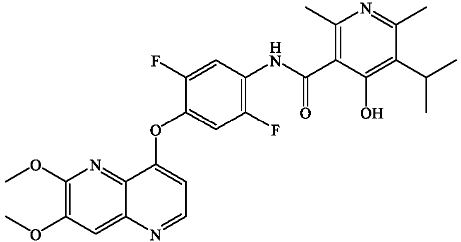 | N-[4-[(6,7-dimethoxy-1,5-naphthyridin-4-yl)oxy]-2,5-difluorophenyl]-4-hydroxy-2,6-dimethyl-5-propan-2-ylpyridine-3-carboxamide |
| 11 | 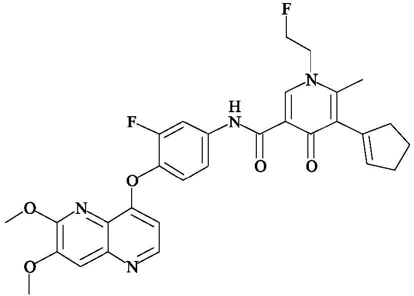 | 5-(cyclopenten-1-yl)-N-[4-[(6,7-dimethoxy-1,5-naphthyridin-4-yl)oxy]-3-fluorophenyl]-1-(2-fluoroethyl)-6-methyl-4-oxopyridine-3-carboxamide |
| 12 | 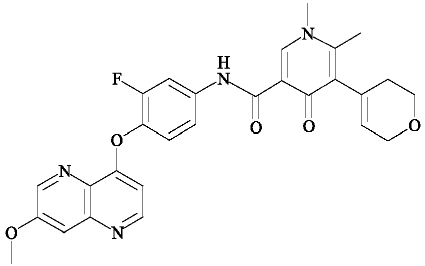 | 5-(3,6-dihydro-2H-pyran-4-yl)-N-[3-fluoro-4-[(7-methoxy-1,5-naphthyridin-4-yl)oxy]phenyl]-1,6-dimethyl-4-oxopyridine-3-carboxamide |
| 13 | 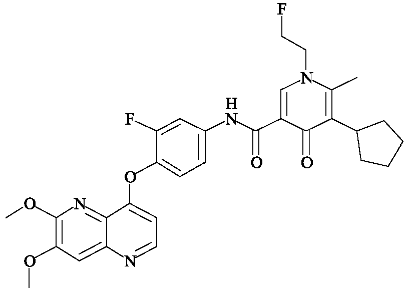 | 5-cyclopentyl-N-[4-[(6,7-dimethoxy-1,5-naphthyridin-4-yl)oxy]-3-fluorophenyl]-1-(2-fluoroethyl)-6-methyl-4-oxopyridine-3-carboxamide |
| 14 | 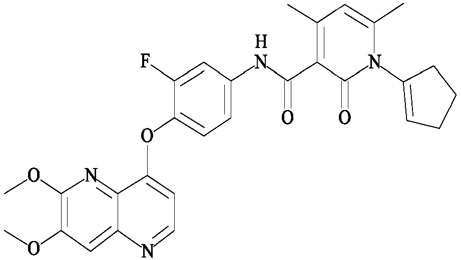 | 1-(cyclopenten-1-yl)-N-[4-[(6,7-dimethoxy-1,5-naphthyridin-4-yl)oxy]-3-fluorophenyl]-4,6-dimethyl-2-oxopyridine-3-carboxamide |
| 15 | 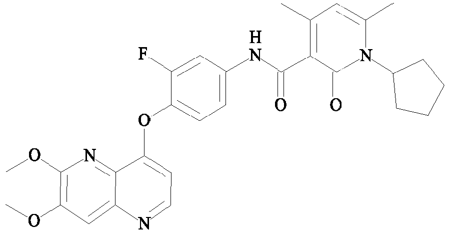 | 1-cyclopentyl-N-[4-[(6,7-dimethoxy-1,5-naphthyridin-4-yl)oxy]-3-fluorophenyl]-4,6-dimethyl-2-oxopyridine-3-carboxamide |
| 16 | 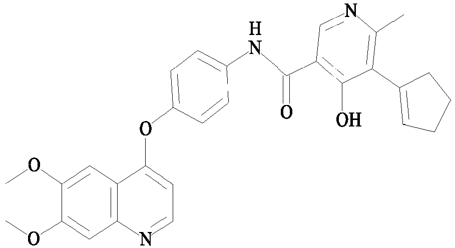 | 5-(cyclopenten-1-yl)-N-[4-(6,7-dimethoxyquinolin-4-yl)oxyphenyl]-4-hydroxy-6-methylpyridine-3-carboxamide |
| 17 | 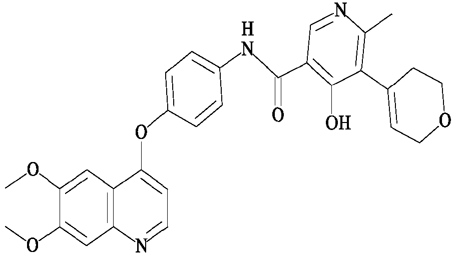 | 5-(3,6-dihydro-2H-pyran-4-yl)-N-[4-(6,7-dimethoxyquinolin-4-yl)oxyphenyl]-4-hydroxy-6-methylpyridine-3-carboxamide |
| 18 | 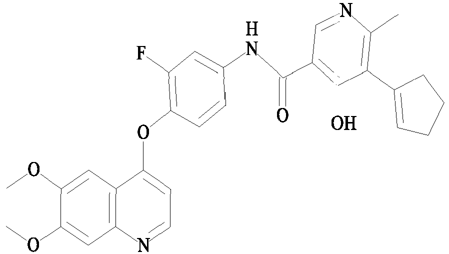 | 5-(cyclopenten-1-yl)-N-[4-(6,7-dimethoxyquinolin-4-yl)oxy-3-fluorophenyl]-4-hydroxy-6-methylpyridine-3-carboxamide |
| 19 | 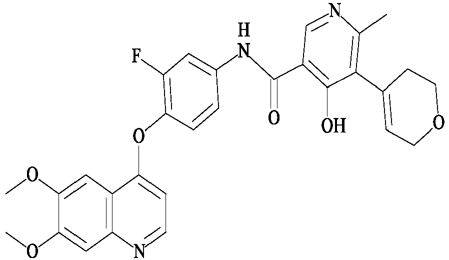 | 5-(3,6-dihydro-2H-pyran-4-yl)-N-[4-(6,7-dimethoxyquinolin-4-yl)oxy-3-fluorophenyl]-4-hydroxy-6-methylpyridine-3-carboxamide |
| 20 | 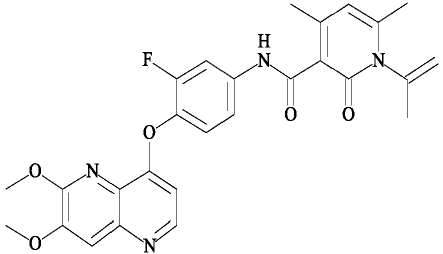 | N-[4-[(6,7-dimethoxy-1,5-naphthyridin-4-yl)oxy]-3-fluorophenyl]-4,6-dimethyl-2-oxo-1-prop-1-en-2-ylpyridine-3-carboxamide |
| 21 | 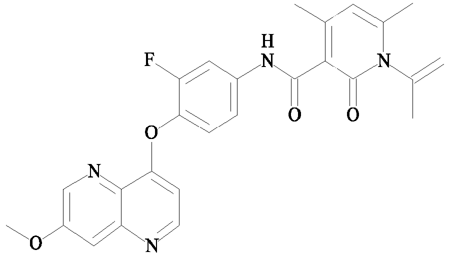 | N-[3-fluoro-4-[(7-methoxy-1,5-naphthyridin-4-yl)oxy]phenyl]-4,6-dimethyl-2-oxo-1-prop-1-en-2-ylpyridine-3-carboxamide |
| 22 | 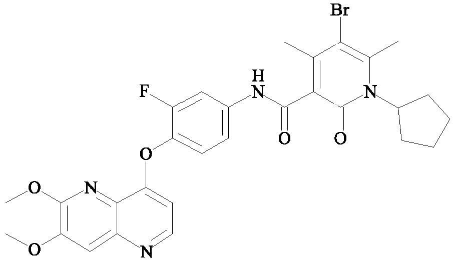 | 5-bromo-1-cyclopentyl-N-[4-[(6,7-dimethoxy-1,5-naphthyridin-4-yl)oxy]-3-fluorophenyl]-4,6-dimethyl-2-oxopyridine-3-carboxamide |
| 23 | 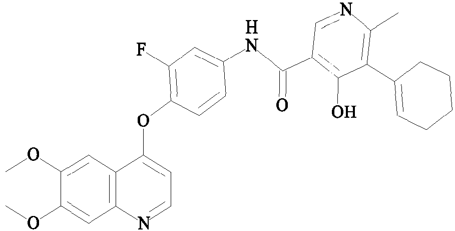 | 5-(cyclohexen-1-yl)-N-[4-(6,7-dimethoxyquinolin-4-yl)oxy-3-fluorophenyl]-4-hydroxy-6-methylpyridine-3-carboxamide |
| 24 | 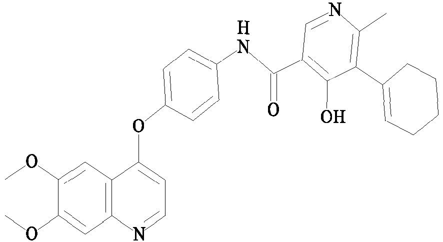 | 5-(cyclohexen-1-yl)-N-[4-(6,7-dimethoxyquinolin-4-yl)oxyphenyl]-4-hydroxy-6-methylpyridine-3-carboxamide |
| 25 | 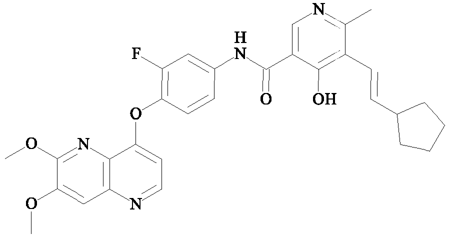 | 5-[(E)-2-cyclopentylethenyl]-N-[4-[(6,7-dimethoxy-1,5-naphthyridin-4-yl)oxy]-3-fluorophenyl]-4-hydroxy-6-methylpyridine-3-carboxamide |
| 26 | 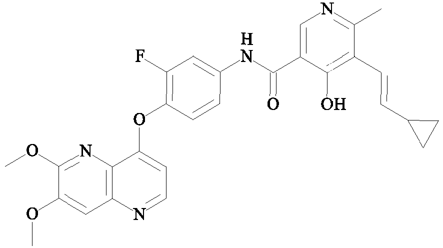 | 5-[(E)-2-cyclopropylethenyl]-N-[4-[(6,7-dimethoxy-1,5-naphthyridin-4-yl)oxy]-3-fluorophenyl]-4-hydroxy-6-methylpyridine-3-carboxamide |
| 27 | 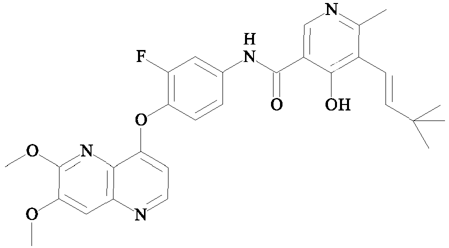 | N-[4-[(6,7-dimethoxy-1,5-naphthyridin-4-yl)oxy]-3-fluorophenyl]-5-[(E)-3,3-dimethylbut-1-enyl]-4-hydroxy-6-methylpyridine-3-carboxamide |
| 28 | 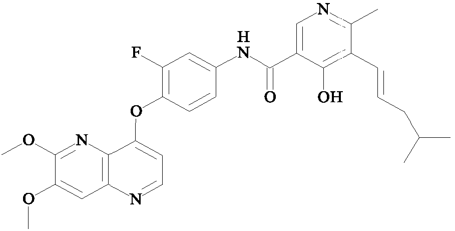 | N-[4-[(6,7-dimethoxy-1,5-naphthyridin-4-yl)oxy]-3-fluorophenyl]-4-hydroxy-6-methyl-5-[(E)-4-methylpent-1-enyl]pyridine-3-carboxamide |
| 29 | 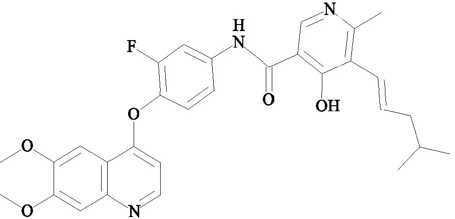 | N-[4-(6,7-dimethoxyquinolin-4-yl)oxy-3-fluorophenyl]-4-hydroxy-6-methyl-5-[(E)-4-methylpent-1-enyl]pyridine-3-carboxamide |
| 30 | 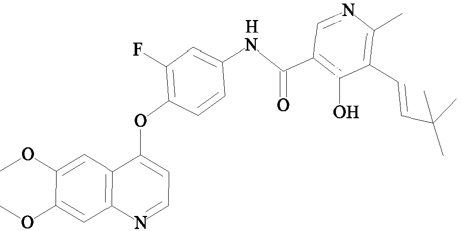 | N-[4-(6,7-dimethoxyquinolin-4-yl)oxy-3-fluorophenyl]-5-[(E)-3,3-dimethylbut-1-enyl]-4-hydroxy-6-methylpyridine-3-carboxamide |
| 31 | 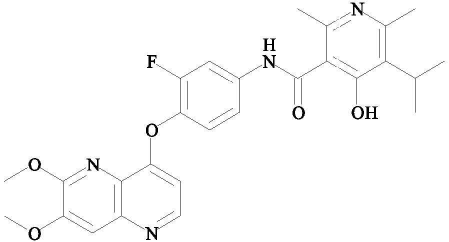 | N-[4-[(6,7-dimethoxy-1,5-naphthyridin-4-yl)oxy]-3-fluorophenyl]-4-hydroxy-2,6-dimethyl-5-propan-2-ylpyridine-3-carboxamide |
| 32 | 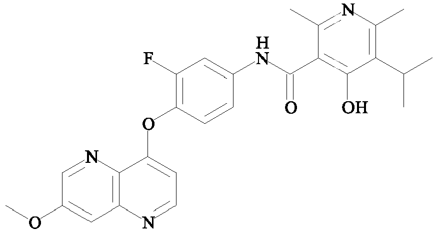 | N-[3-fluoro-4-[(7-methoxy-1,5-naphthyridin-4-yl)oxy]phenyl]-4-hydroxy-2,6-dimethyl-5-propan-2-ylpyridine-3-carboxamide |
| 33 | 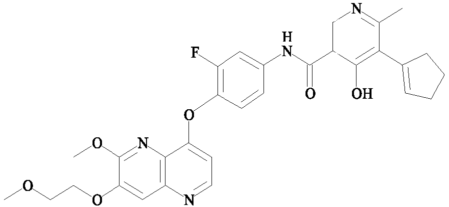 | 5-(cyclopenten-1-yl)-N-[3-fluoro-4-[[6-methoxy-7-(2-methoxyethoxy)-1,5-naphthyridin-4-yl]oxy]phenyl]-4-hydroxy-6-methylpyridine-3-carboxamide |
| 34 | 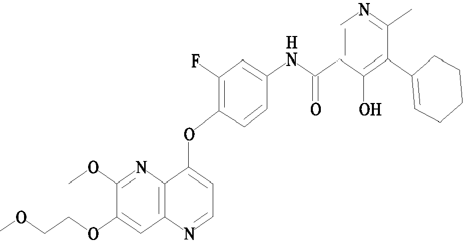 | 5-(cyclohexen-1-yl)-N-[3-fluoro-4-[[6-methoxy-7-(2-methoxyethoxy)-1,5-naphthyridin-4-yl]oxy]phenyl]-4-hydroxy-6-methylpyridine-3-carboxamide |
| 35 | 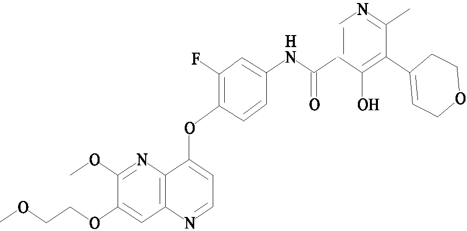 | 5-(3,6-dihydro-2H-pyran-4-yl)-N-[3-fluoro-4-[[6-methoxy-7-(2-methoxyethoxy)-1,5-naphthyridin-4-yl]oxy]phenyl]-4-hydroxy-6-methylpyridine-3-carboxamide |
| 36 | 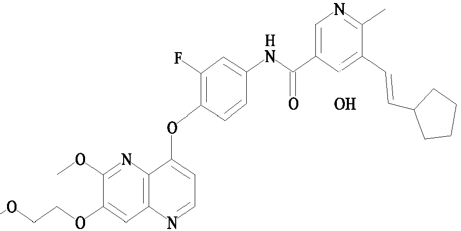 | 5-[(E)-2-cyclopentylethenyl]-N-[3-fluoro-4-[[6-methoxy-7-(2-methoxyethoxy)-1,5-naphthyridin-4-yl]oxy]phenyl]-4-hydroxy-6-methylpyridine-3-carboxamide |
| 37 | 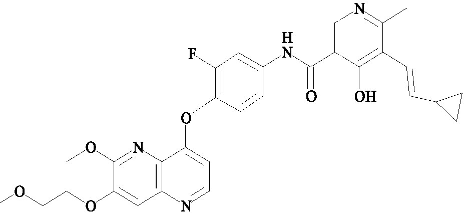 | 5-[(E)-2-cyclopropylethenyl]-N-[3-fluoro-4-[[6-methoxy-7-(2-methoxyethoxy)-1,5-naphthyridin-4-yl]oxy]phenyl]-4-hydroxy-6-methylpyridine-3-carboxamide |
| 38 | 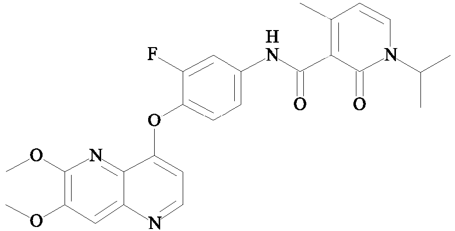 | N-[4-[(6,7-dimethoxy-1,5-naphthyridin-4-yl)oxy]-3-fluorophenyl]-4-methyl-2-oxo-1-propan-2-ylpyridine-3-carboxamide |
| 39 | 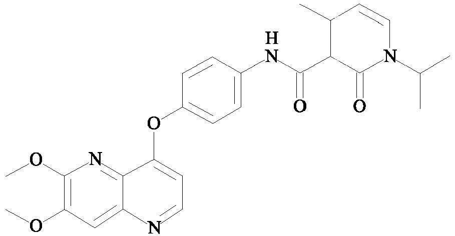 | N-[4-[(6,7-dimethoxy-1,5-naphthyridin-4-yl)oxy]phenyl]-4-methyl-2-oxo-1-propan-2-ylpyridine-3-carboxamide |
| 40 | 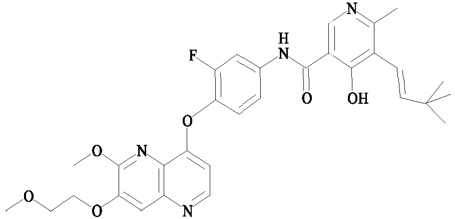 | 5-[(E)-3,3-dimethylbut-1-enyl]-N-[3-fluoro-4-[[6-methoxy-7-(2-methoxyethoxy)-1,5-naphthyridin-4-yl]oxy]phenyl]-4-hydroxy-6-methylpyridine-3-carboxamide |
| 41 | 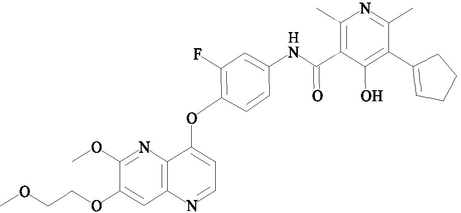 | 5-(cyclopenten-1-yl)-N-[3-fluoro-4-[[6-methoxy-7-(2-methoxyethoxy)-1,5-naphthyridin-4-yl]oxy]phenyl]-4-hydroxy-2,6-dimethylpyridine-3-carboxamide |
| 42 | 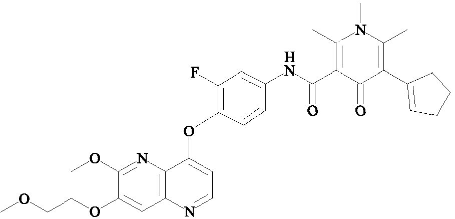 | 5-(cyclopenten-1-yl)-N-[3-fluoro-4-[[6-methoxy-7-(2-methoxyethoxy)-1,5-naphthyridin-4-yl]oxy]phenyl]-1,2,6-trimethyl-4-oxopyridine-3-carboxamide |
| 43 | 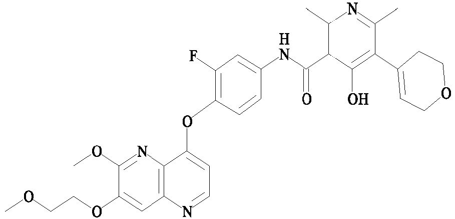 | 5-(3,6-dihydro-2H-pyran-4-yl)-N-[3-fluoro-4-[[6-methoxy-7-(2-methoxyethoxy)-1,5-naphthyridin-4-yl]oxy]phenyl]-4-hydroxy-2,6-dimethylpyridine-3-carboxamide |
| 44 | 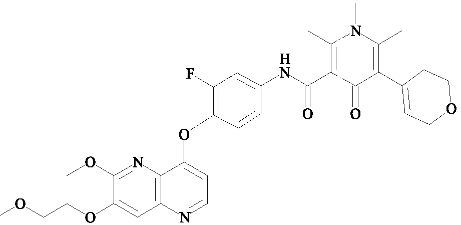 | 5-(3,6-dihydro-2H-pyran-4-yl)-N-[3-fluoro-4-[[6-methoxy-7-(2-methoxyethoxy)-1,5-naphthyridin-4-yl]oxy]phenyl]-1,2,6-trimethyl-4-oxopyridine-3-carboxamide |
| 45 | 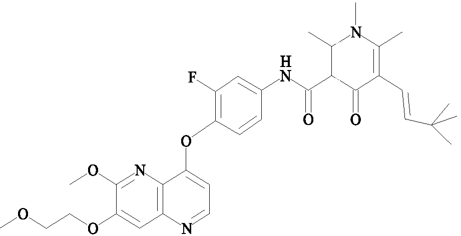 | 5-[(E)-3,3-dimethylbut-1-enyl]-N-[3-fluoro-4-[[6-methoxy-7-(2-methoxyethoxy)-1,5-naphthyridin-4-yl]oxy]phenyl]-1,2,6-trimethyl-4-oxopyridine-3-carboxamide |
| 46 | 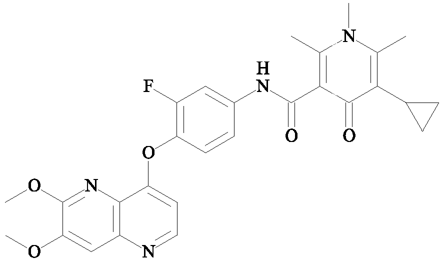 | 5-cyclopropyl-N-[4-[(6,7-dimethoxy-1,5-naphthyridin-4-yl)oxy]-3-fluorophenyl]-1,2,6-trimethyl-4-oxopyridine-3-carboxamide |
| 47 | 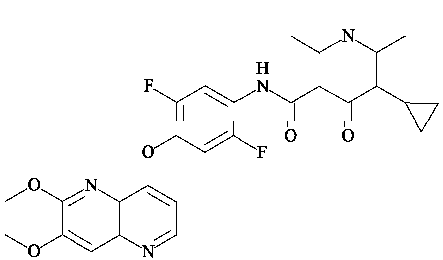 | 5-cyclopropyl-N-[4-[(6,7-dimethoxy-1,5-naphthyridin-4-yl)oxy]-2,5-difluorophenyl]-1,2,6-trimethyl-4-oxopyridine-3-carboxamide |
| 48 | 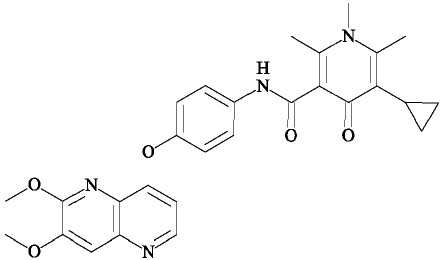 | 5-cyclopropyl-N-[4-[(6,7-dimethoxy-1,5-naphthyridin-4-yl)oxy]phenyl]-1,2,6-trimethyl-4-oxopyridine-3-carboxamide |
| 49 | 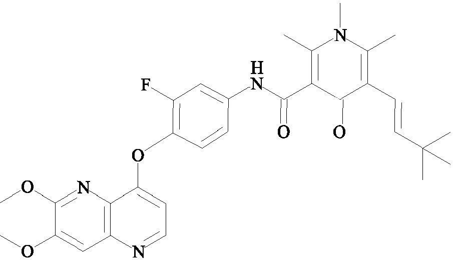 | N-[4-[(6,7-dimethoxy-1,5-naphthyridin-4-yl)oxy]-3-fluorophenyl]-5-[(E)-3,3-dimethylbut-1-enyl]-1,2,6-trimethyl-4-oxopyridine-3-carboxamide |
| 50 | 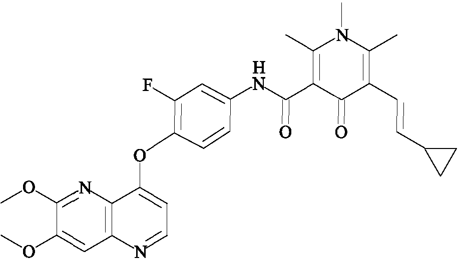 | 5-[(E)-2-cyclopropylethenyl]-N-[4-[(6,7-dimethoxy-1,5-naphthyridin-4-yl)oxy]-3-fluorophenyl]-1,2,6-trimethyl-4-oxopyridine-3-carboxamide |
| 51 | 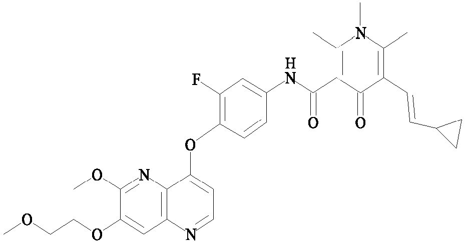 | 5-[(E)-2-cyclopropylethenyl]-N-[3-fluoro-4-[[6-methoxy-7-(2-methoxyethoxy)-1,5-naphthyridin-4-yl]oxy]phenyl]-1,2,6-trimethyl-4-oxopyridine-3-carboxamide |
| 52 | 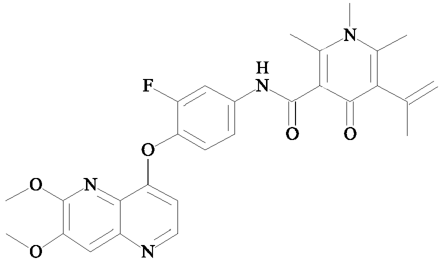 | N-[4-[(6,7-dimethoxy-1,5-naphthyridin-4-yl)oxy]-3-fluorophenyl]-1,2,6-trimethyl-4-oxo-5-prop-1-en-2-ylpyridine-3-carboxamide |
| 53 | 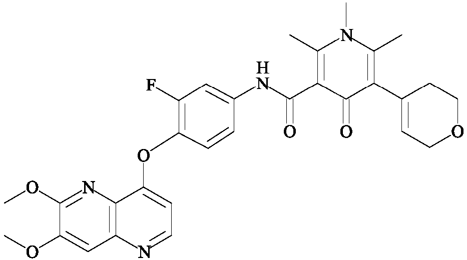 | 5-(3,6-dihydro-2H-pyran-4-yl)-N-[4-[(6,7-dimethoxy-1,5-naphthyridin-4-yl)oxy]-3-fluorophenyl]-1,2,6-trimethyl-4-oxopyridine-3-carboxamide |
| 54 | 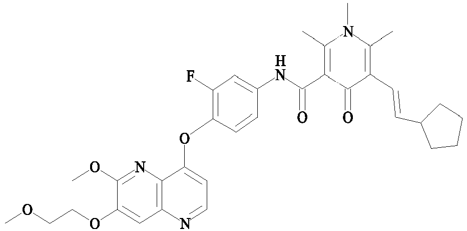 | 5-[(E)-2-cyclopentylethenyl]-N-[3-fluoro-4-[[6-methoxy-7-(2-methoxyethoxy)-1,5-naphthyridin-4-yl]oxy]phenyl]-1,2,6-trimethyl-4-oxopyridine-3-carboxamide |
| 55 | 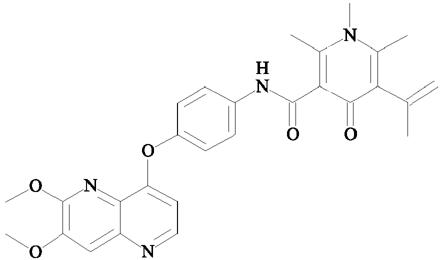 | N-[4-[(6,7-dimethoxy-1,5-naphthyridin-4-yl)oxy]phenyl]-1,2,6-trimethyl-4-oxo-5-prop-1-en-2-ylpyridine-3-carboxamide |
| 56 | 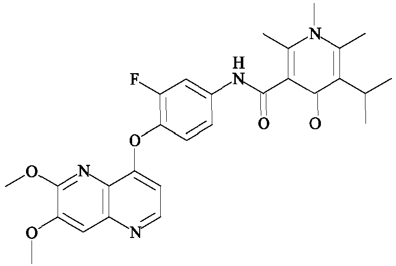 | N-[4-[(6,7-dimethoxy-1,5-naphthyridin-4-yl)oxy]-3-fluorophenyl]-1,2,6-trimethyl-4-oxo-5-propan-2-ylpyridine-3-carboxamide |
| 57 | 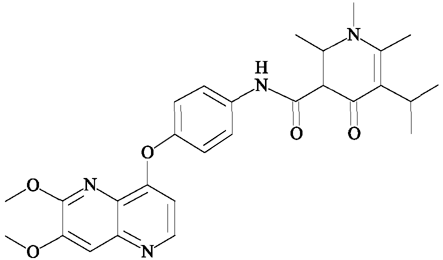 | N-[4-[(6,7-dimethoxy-1,5-naphthyridin-4-yl)oxy]phenyl]-1,2,6-trimethyl-4-oxo-5-propan-2-ylpyridine-3-carboxamide |
| 58 | 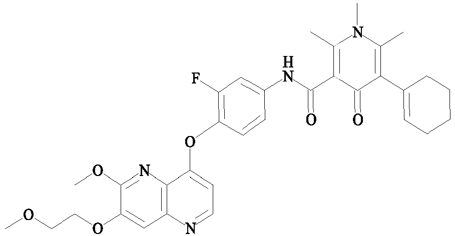 | 5-(cyclohexen-1-yl)-N-[3-fluoro-4-[[6-methoxy-7-(2-methoxyethoxy)-1,5-naphthyridin-4-yl]oxy]phenyl]-1,2,6-trimethyl-4-oxopyridine-3-carboxamide |
| 59 | 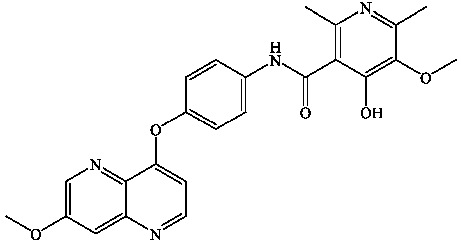 | 4-hydroxy-5-methoxy-N-[4-[(7-methoxy-1,5-naphthyridin-4-yl)oxy]phenyl]-2,6-dimethylpyridine-3-carboxamide |
| 60 | 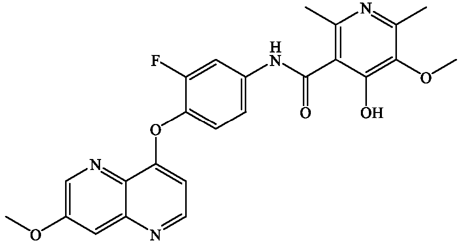 | N-[3-fluoro-4-[(7-methoxy-1,5-naphthyridin-4-yl)oxy]phenyl]-4-hydroxy-5 -methoxy-2,6-dimethylpyridine-3-carboxamide |
| 61 | 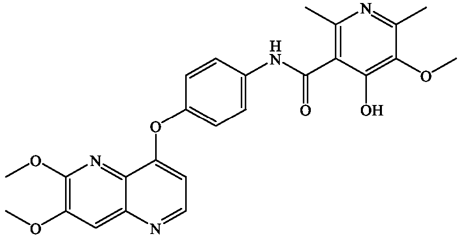 | N-[4-[(6,7-dimethoxy-1,5-naphthyridin-4-yl)oxy]phenyl]-4-hydroxy-5-methoxy-2,6-dimethylpyridine-3-carboxamide |
| 62 | 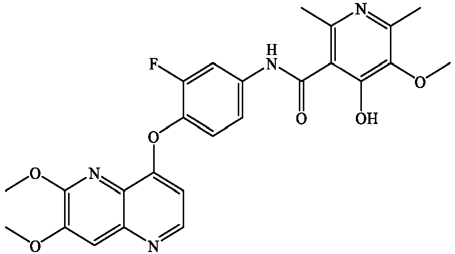 | N-[4-[(6,7-dimethoxy-1,5-naphthyridin-4-yl)oxy]-3-fluorophenyl]-4-hydroxy-5-methoxy-2,6-dimethylpyridine-3-carboxamide |
| 63 | 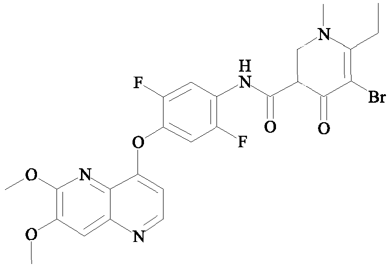 | 5-bromo-N-[4-[(6,7-dimethoxy-1,5-naphthyridin-4-yl)oxy]-2,5-difluorophenyl]-6-ethyl-1-methyl-4-oxopyridine-3-carboxamide |
| 64 | 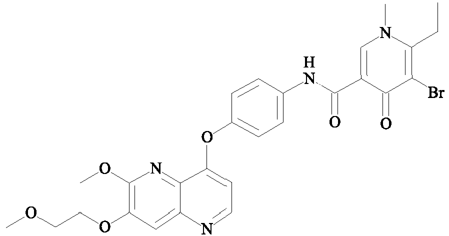 | 5-bromo-6-ethyl-N-[4-[[6-methoxy-7-(2-methoxyethoxy)-1,5-naphthyridin-4-yl]oxy]phenyl]-1-methyl-4-oxopyridine-3-carboxamide |
| 65 | 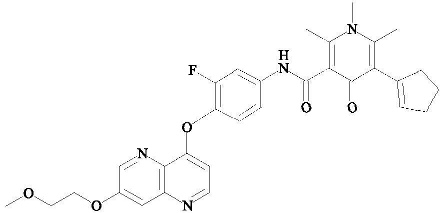 | 5-(cyclopenten-1-yl)-N-[3-fluoro-4-[[7-(2-methoxyethoxy)-1,5-naphthyridin-4-yl]oxy]phenyl]-1,2,6-trimethyl-4-oxopyridine-3-carboxamide |
| 66 | 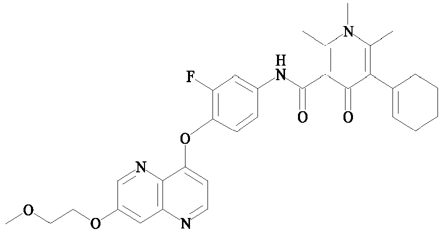 | 5-(cyclohexen-1-yl)-N-[3-fluoro-4-[[7-(2-methoxyethoxy)-1,5-naphthyridin-4-yl]oxy]phenyl]-1,2,6-trimethyl-4-oxopyridine-3-carboxamide |
| 67 | 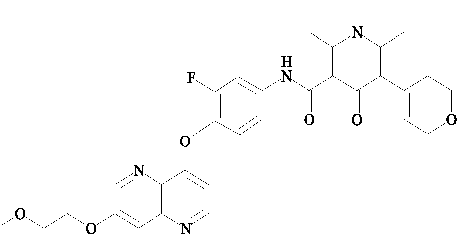 | 5-(3,6-dihydro-2H-pyran-4-yl)-N-[3-fluoro-4-[[7-(2-methoxyethoxy)-1,5-naphthyridin-4-yl]oxy]phenyl]-1,2,6-trimethyl-4-oxopyridine-3-carboxamide |
| 68 | 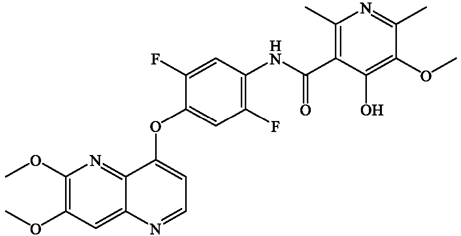 | N-[4-[(6,7-dimethoxy-1,5-naphthyridin-4-yl)oxy]-2,5-difluorophenyl]-4-hydroxy-5-methoxy-2,6-dimethylpyridine-3-carboxamide |
| 69 | 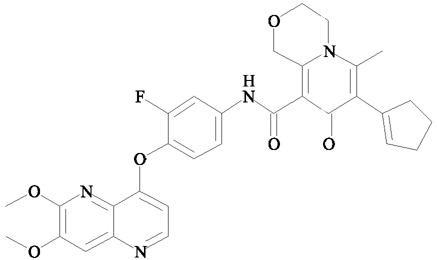 | 7-(cyclopenten-1-yl)-N-[4-[(6,7-dimethoxy-1,5-naphthyridin-4-yl)oxy]-3-fluorophenyl]-6-methyl-8-oxo-3,4-dihydro-1H-pyrido[2,1-c][1,4]oxazine-9-carboxamide |
| 70 | 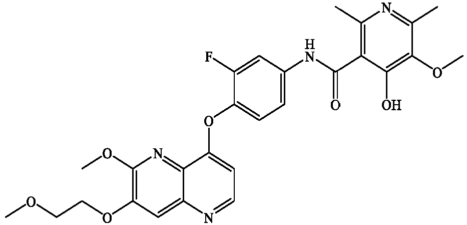 | N-[3-fluoro-4-[[6-methoxy-7-(2-methoxyethoxy)-1,5-naphthyridin-4-yl]oxy]phenyl]-4-hydroxy-5-methoxy-2,6-dimethylpyridine-3-carboxamide |
| 71 | 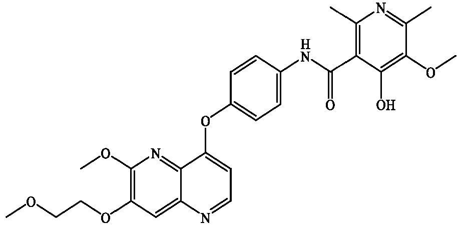 | 4-hydroxy-5-methoxy-N-[4-[[6-methoxy-7-(2-methoxyethoxy)-1,5-naphthyridin-4-yl]oxy]phenyl]-2,6-dimethylpyridine-3-carboxamide |
| 72 | 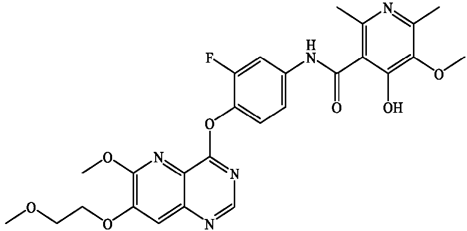 | N-[3-fluoro-4-[6-methoxy-7-(2-methoxyethoxy)pyrido[3,2-d]pyrimidin-4-yl]oxyphenyl]-4-hydroxy-5-methoxy-2,6-dimethylpyridine-3-carboxamide |
| 73 | 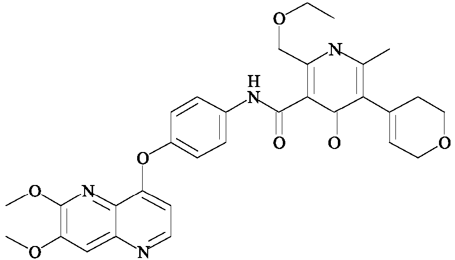 | 7-(3,6-dihydro-2H-pyran-4-yl)-N-[4-[(6,7-dimethoxy-1,5-naphthyridin-4-yl)oxy]phenyl]-6-methyl-8-oxo-3,4-dihydro-1H-pyrido[2,1-c][1,4]oxazine-9-carboxamide |
| 74 | 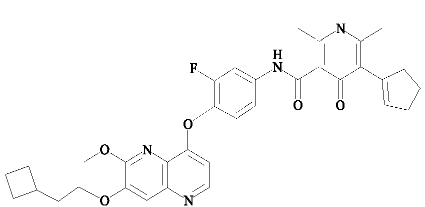 | N-[4-[[7-(2-cyclobutylethoxy)-6-methoxy-1,5-naphthyridin-4-yl]oxy]-3-fluorophenyl]-5-(cyclopenten-1-yl)-1,2,6-trimethyl-4-oxopyridine-3-carboxamide |
| 75 | 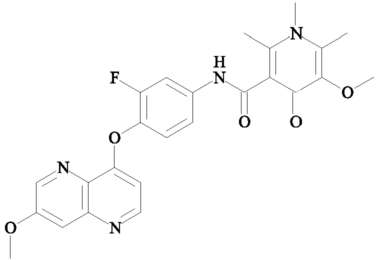 | N-[3-fluoro-4-[(7-methoxy-1,5-naphthyridin-4-yl)oxy]phenyl]-5-methoxy-1,2,6-trimethyl-4-oxopyridine-3-carboxamide |
| 76 | 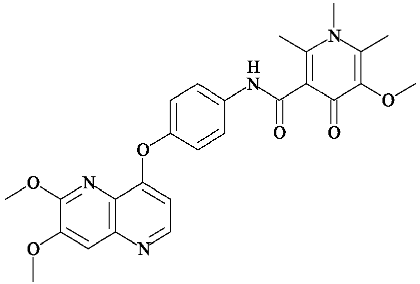 | N-[4-[(6,7-dimethoxy-1,5-naphthyridin-4-yl)oxy]phenyl]-5-methoxy-1,2,6-trimethyl-4-oxopyridine-3-carboxamide |
| 77 | 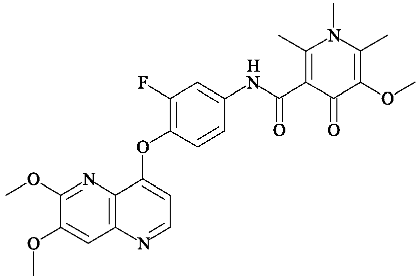 | N-[4-[(6,7-dimethoxy-1,5-naphthyridin-4-yl)oxy]-3-fluorophenyl]-5-methoxy-1,2,6-trimethyl-4-oxopyridine-3-carboxamide |
| 78 | 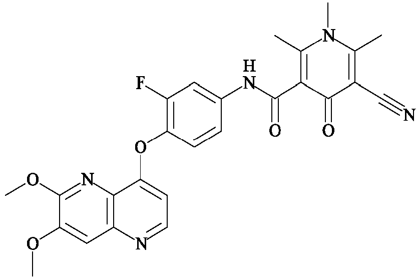 | 5-cyano-N-[4-[(6,7-dimethoxy-1,5-naphthyridin-4-yl)oxy]-3-fluorophenyl]-1,2,6-trimethyl-4-oxopyridine-3-carboxamide |
| 79 | 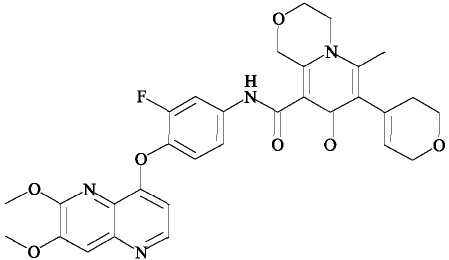 | 7-(3,6-dihydro-2H-pyran-4-yl)-N-[4-[(6,7-dimethoxy-1,5-naphthyridin-4-yl)oxy]-3-fluorophenyl]-6-methyl-8-oxo-3,4-dihydro-1H-pyrido[2,1-c][1,4]oxazine-9-carboxamide |
| 80 | 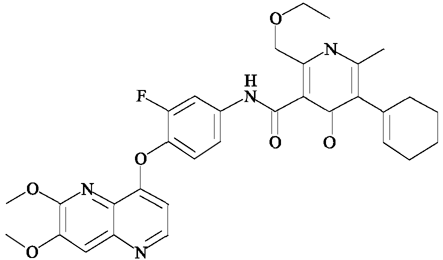 | 7-(cyclohexen-1-yl)-N-[4-[(6,7-dimethoxy-1,5-naphthyridin-4-yl)oxy]-3-fluorophenyl]-6-methyl-8-oxo-3,4-dihydro-1H-pyrido[2,1-c][1,4]oxazine-9-carboxamide |
| 81 | 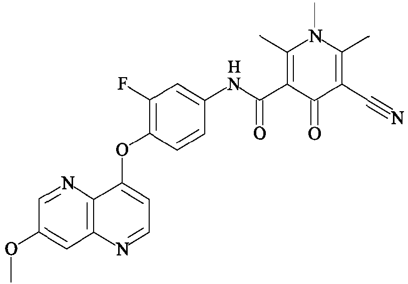 | 5-cyano-N-[3-fluoro-4-[(7-methoxy-1,5-naphthyridin-4-yl)oxy]phenyl]-1,2,6-trimethyl-4-oxopyridine-3-carboxamide |
| 82 | 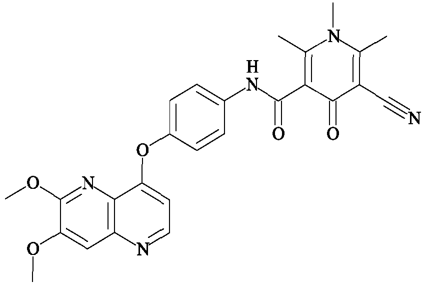 | 5-cyano-N-[4-[(6,7-dimethoxy-1,5-naphthyridin-4-yl)oxy]phenyl]-1,2,6-trimethyl-4-oxopyridine-3-carboxamide |
| 83 | 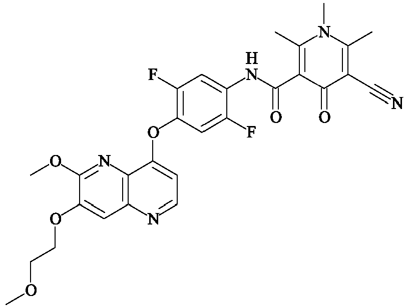 | 5-cyano-N-[2,5-difluoro-4-[[6-methoxy-7-(2-methoxyethoxy)-1,5-naphthyridin-4-yl]oxy]phenyl]-1,2,6-trimethyl-4-oxopyridine-3-carboxamide |
| 84 | 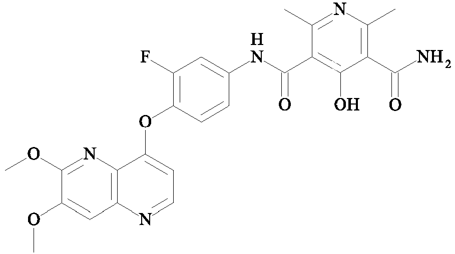 | 3-N-[4-[(6,7-dimethoxy-1,5-naphthyridin-4-yl)oxy]-3-fluorophenyl]-4-hydroxy-2,6-dimethylpyridine-3,5-dicarboxamide |
| 85 | 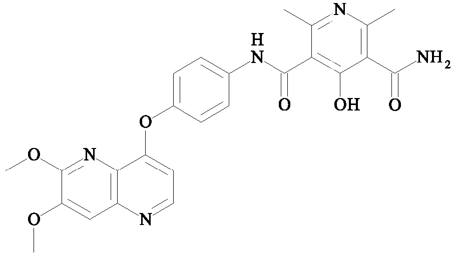 | 3-N-[4-[(6,7-dimethoxy-1,5-naphthyridin-4-yl)oxy]phenyl]-4-hydroxy-2,6-dimethylpyridine-3,5-dicarboxamide |
| 86 | 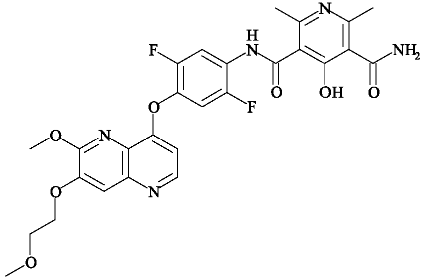 | 3-N-[2,5-difluoro-4-[[6-methoxy-7-(2-methoxyethoxy)-1,5-naphthyridin-4-yl]oxy]phenyl]-4-hydroxy-2,6-dimethylpyridine-3,5-dicarboxamide |
| 87 | 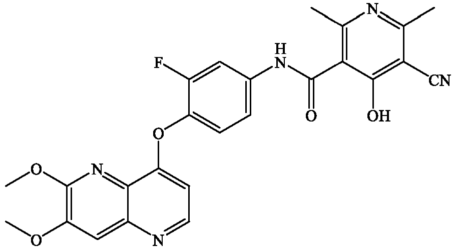 | 5-cyano-N-[4-[(6,7-dimethoxy-1,5-naphthyridin-4-yl)oxy]-3-fluorophenyl]-4-hydroxy-2,6-dimethylpyridine-3-carboxamide |
| 88 | 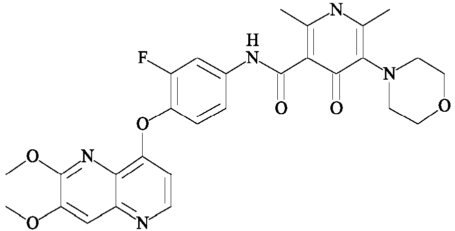 | N-[4-[(6,7-dimethoxy-1,5-naphthyridin-4-yl)oxy]-3-fluorophenyl]-1,2,6-trimethyl-5-morpholin-4-yl-4-oxopyridine-3-carboxamide |
| 89 | 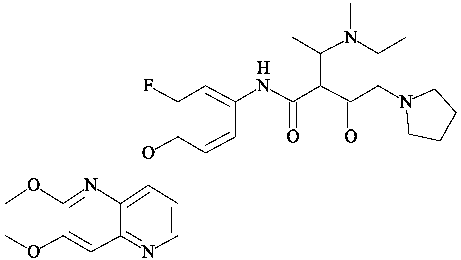 | N-[4-[(6,7-dimethoxy-1,5-naphthyridin-4-yl)oxy]-3-fluorophenyl]-1,2,6-trimethyl-4-oxo-5-pyrrolidin-1-ylpyridine-3-carboxamide |
| 90 | 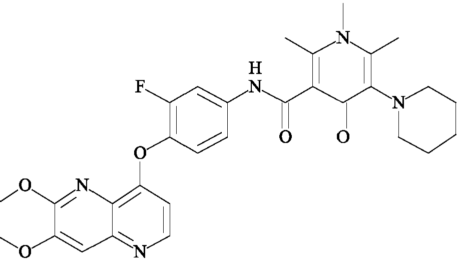 | N-[4-[(6,7-dimethoxy-1,5-naphthyridin-4-yl)oxy]-3-fluorophenyl]-1,2,6-trimethyl-4-oxo-5-piperidin-1-ylpyridine-3-carboxamide |
| 91 | 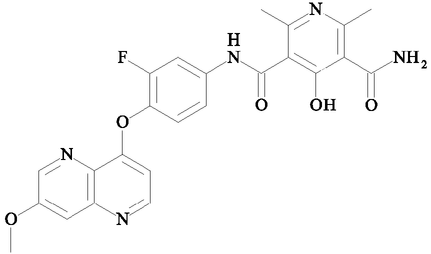 | 3-N-[3-fluoro-4-[(7-methoxy-1,5-naphthyridin-4-yl)oxy]phenyl]-4-hydroxy-2,6-dimethylpyridine-3,5-dicarboxamide |
| 92 | 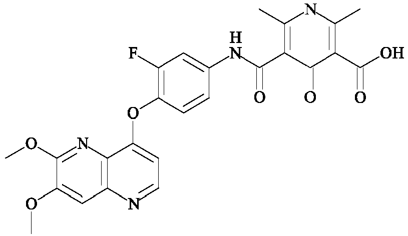 | 5-[[4-[(6,7-dimethoxy-1,5-naphthyridin-4-yl)oxy]-3-fluorophenyl]carbamoyl]-1,2,6-trimethyl-4-oxopyridine-3-carboxylic acid |
| 93 | 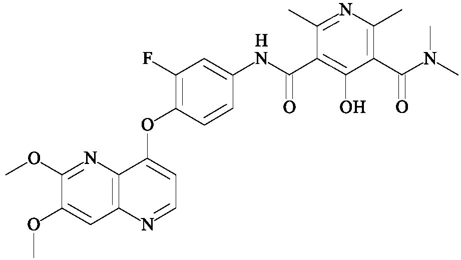 | 3-N-[4-[(6,7-dimethoxy-1,5-naphthyridin-4-yl)oxy]-3-fluorophenyl]-4-hydroxy-5-N,5-N,2,6-tetramethylpyridine-3,5-dicarboxamide |
| 94 | 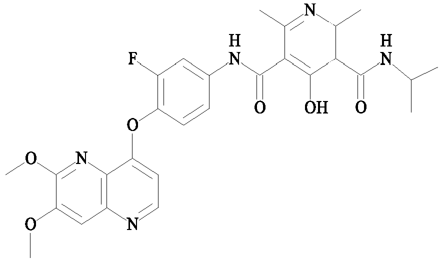 | 3-N-[4-[(6,7-dimethoxy-1,5-naphthyridin-4-yl)oxy]-3-fluorophenyl]-4-hydroxy-2,6-dimethyl-5-N-propan-2-ylpyridine-3,5-dicarboxamide |
| 95 | 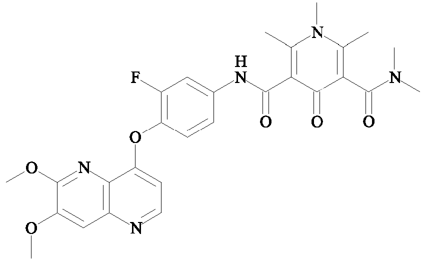 | 5-N-[4-[(6,7-dimethoxy-1,5-naphthyridin-4-yl)oxy]-3-fluorophenyl]-3-N,3-N,1,2,6-pentamethyl-4-oxopyridine-3,5-dicarboxamide |
| 96 | 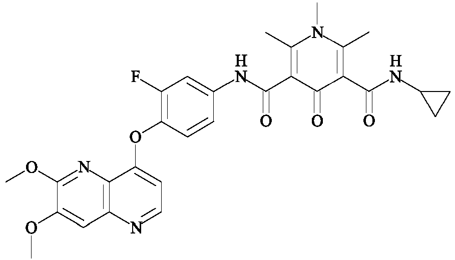 | 3-N-cyclopropyl-5-N-[4-[(6,7-dimethoxy-1,5-naphthyridin-4-yl)oxy]-3-fluorophenyl]-1,2,6-trimethyl-4-oxopyridine-3,5-dicarboxamide |
| 97 | 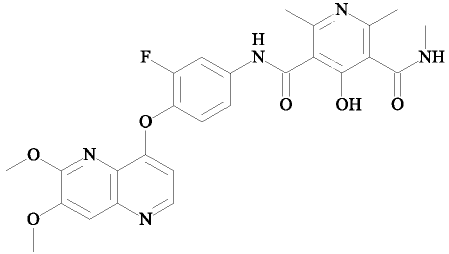 | 3-N-[4-[(6,7-dimethoxy-1,5-naphthyridin-4-yl)oxy]-3-fluorophenyl]-4-hydroxy-5-N,2,6-trimethylpyridine-3,5-dicarboxamide |
| 98 | 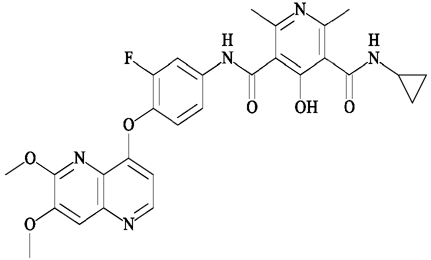 | 5-N-cyclopropyl-3-N-[4-[(6,7-dimethoxy-1,5-naphthyridin-4-yl)oxy]-3-fluorophenyl]-4-hydroxy-2,6-dimethylpyridine-3,5-dicarboxamide |
| 99 | 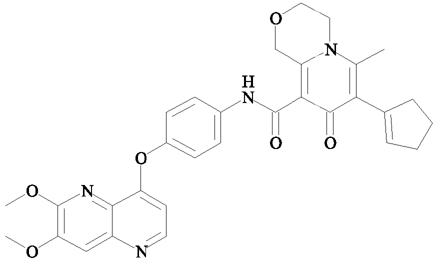 | 7-(cyclopenten-1-yl)-N-[4-[(6,7-dimethoxy-1,5-naphthyridin-4-yl)oxy]phenyl]-6-methyl-8-oxo-3,4-dihydro-1H-pyrido[2,1-c][1,4]oxazine-9-carboxamide |
| 100 | 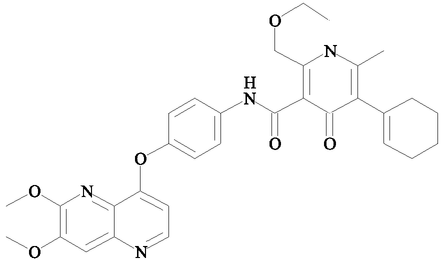 | 7-(cyclohexen-1-yl)-N-[4-[(6,7-dimethoxy-1,5-naphthyridin-4-yl)oxy]phenyl]-6-methyl-8-oxo-3,4-dihydro-1H-pyrido[2,1-c][1,4]oxazine-9-carboxamide |
| 101 | 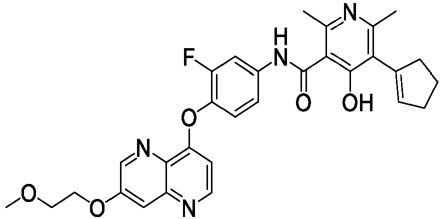 | 5-(cyclopenten-1-yl)-N-[3-fluoro-4-[[7-(2-methoxyethoxy)-1,5-naphthyridin-4-yl]oxy]phenyl]-4-hydroxy-2,6-dimethylpyridine-3-carboxamide |

### Treatment Methods and Uses

"Treatment" or "treating" is an approach for obtaining beneficial or desired results including clinical results. Beneficial or desired clinical results may include one or more of the following: a) inhibiting the disease or condition (e.g., decreasing one or more symptoms resulting from the disease or condition, and/or diminishing the extent of the disease or condition); b) slowing or arresting the development of one or more clinical symptoms associated with the disease or condition (e.g., stabilizing the disease or condition, preventing or delaying the worsening or progression of the disease or condition, and/or preventing or delaying the spread (e.g., metastasis) of the disease or condition); and/or c) relieving the disease, that is, causing the regression of clinical symptoms (e.g., ameliorating the disease state, providing partial or total remission of the disease or condition, enhancing effect of another medication, delaying the progression of the disease, increasing the quality of life, and/or prolonging survival.

"Prevention" or "preventing" means any treatment of a disease or condition that causes the clinical symptoms of the disease or condition not to develop. Compounds may be administered to a subject (including a human) who is at risk or has a family history of the disease or condition.

"Subject" refers to an animal, such as a mammal (including a human), that has been or will be the object of treatment, observation or experiment. The methods described herein may be useful in human therapy and/or veterinary applications. The subject may be a mammal. The subject may be a human.

The term "therapeutically effective amount" or "effective amount" of a compound described herein or a pharmaceutically acceptable salt, tautomer, stereoisomer, mixture of stereoisomers, or deuterated analog thereof means an amount sufficient to effect treatment when administered to a subject, to provide a therapeutic benefit such as amelioration of symptoms or slowing of disease progression. For example, a therapeutically effective amount may be an amount sufficient to decrease a symptom of a sickle cell disease. The therapeutically effective amount may vary depending on the subject, and disease or condition being treated, the weight and age of the subject, the severity of the disease or condition, and the manner of administering, which can readily be determined by one or ordinary skill in the art.

The methods described herein may be applied to cell populations in vivo or ex vivo. "In vivo" means within a living individual, as within an animal or human. In this context, the methods described herein may be used therapeutically in an individual. "Ex vivo" means outside of a living individual. Examples of ex vivo cell populations include in vitro cell cultures and biological samples including fluid or tissue samples obtained from individuals. Such samples may be obtained by methods well known in the art. Exemplary biological fluid samples include blood, cerebrospinal fluid, urine, and saliva. In this context, the compounds and compositions described herein may be used for a variety of purposes, including therapeutic and experimental purposes. For example, the compounds and compositions described herein may be used *ex vivo* to determine the optimal schedule and/or dosing of administration of a compound of the present disclosure for a given indication, cell type, individual, and other parameters. Information gleaned from such use may be used for experimental purposes or in the clinic to set protocols for *in vivo* treatment. Other *ex vivo* uses for which the compounds and compositions described herein may be suited are described below or will become apparent to those skilled in the art. The selected compounds may be further characterized to examine the safety or tolerance dosage in human or non-human subjects. Such properties may be examined using commonly known methods to those skilled in the art.

The method may be a method of modulating in vivo activity of a protein kinase in a subject, the method comprising: administering to the subject a therapeutically effective amount of a compound as described herein, or a pharmaceutically acceptable salt, stereoisomer, or tautomer thereof, or a pharmaceutical composition as described herein.

The methods may be methods of modulating in vivo activity of a protein kinase in a subject, the method comprising administering to the subject a therapeutically effective amount of a compound as described herein, or a pharmaceutically acceptable salt, stereoisomer, or tautomer thereof, or a pharmaceutical composition as described herein.

The method may be a method of treating a disease, disorder, or syndrome in a subject, the method comprising: administering to the subject in need thereof a therapeutically effective amount of a compound as described herein, or a pharmaceutically acceptable salt, stereoisomer, or tautomer thereof, or a pharmaceutical composition as described herein, wherein the disease, disorder, or syndrome is mediated at least in part by modulating in vivo activity of a protein kinase.

The methods may be methods of treating a disease, disorder, or syndrome in a subject, the method comprising administering to the subject in need thereof a therapeutically effective amount of a compound as described herein, or a pharmaceutically acceptable salt, stereoisomer, or tautomer thereof, or a pharmaceutical composition as described herein, wherein the disease, disorder, or syndrome is mediated at least in part by modulating in vivo activity of a protein kinase.

The protein kinase may be AXL, KDR, Mer, or Met. The disease may be cancer.

Also described herein are methods of treating a disease, disorder, or syndrome in a subject, the method comprising: administering to the subject in need thereof a therapeutically effective amount of a compound as described herein, or a pharmaceutically acceptable salt, stereoisomer, or tautomer thereof, or a pharmaceutical composition as described herein, in combination with a therapeutic agent or therapy.

Also described herein are methods of treating a disease, disorder, or syndrome in a subject, the method comprising: administering to the subject in need thereof a therapeutically effective amount of a compound as described herein, or a pharmaceutically acceptable salt, stereoisomer, or tautomer thereof, or a pharmaceutical composition as described herein, in combination with a therapeutic agent or therapy.

The therapeutic agent may be an immunotherapeutic agent or a cancer vaccine. The immunotherapeutic agent may be an anti-PD-1 antibody or anti-PD-L1 antibody.

Also described herein are methods for treating cancer.

"Cancer" includes tumor types such as tumor types including breast, colon, renal, lung, squamous cell myeloid leukemia, hemangiomas, melanomas, astrocytomas, and glioblastomas as well as other cellular-proliferative disease states, including but not limited to: Cardiac: sarcoma (angiosarcoma, fibrosarcoma, rhabdomyosarcoma, liposarcoma), myxoma, rhabdomyoma, fibroma, lipoma and teratoma; Lung: bronchogenic carcinoma (squamous cell, undifferentiated small cell, undifferentiated large cell, adenocarcinoma), alveolar (bronchiolar) carcinoma, bronchial adenoma, sarcoma, lymphoma, chondromatous hanlartoma, inesothelioma; Gastrointestinal: esophagus (squamous cell carcinoma, adenocarcinoma, leiomyosarcoma, lymphoma), stomach (carcinoma, lymphoma, leiomyosarcoma), pancreas (ductal adenocarcinoma, insulinoma, glucagonoma, gastrinoma, carcinoid tumors, vipoma), small bowel (adenocarcinoma, lymphoma, carcinoid tumors, Karposi's sarcoma, leiomyoma, hemangioma, lipoma, neurofibroma, fibroma), large bowel (adenocarcinoma, tubular adenoma, villous adenoma, hamartoma, leiomyoma); Genitourinary tract: kidney (adenocarcinoma, Wilm's tumor [nephroblastoma], lymphoma, leukemia, renal cell carcinoma), bladder and urethra (squamous cell carcinoma, transitional cell carcinoma, adenocarcinoma), prostate (adenocarcinoma, sarcoma, small cell carcinoma of the prostate), testis (seminoma, teratoma, embryonal carcinoma, teratocarcinoma, choriocarcinoma, sarcoma, interstitial cell carcinoma, fibroma, fibroadenoma, adenomatoid tumors, lipoma); Liver: hepatoma (hepatocellular carcinoma), cholangiocarcinoma, hepatoblastoma, angiosarcoma, hepatocellular adenoma, hemangioma; Bone: osteogenic sarcoma (osteosarcoma), fibrosarcoma, malignant fibrous histiocytoma, chondrosarcoma, Ewing's sarcoma, malignant lymphoma (reticulum cell sarcoma), malignant giant cell tumor chordoma, osteochronfroma (osteocartilaginous exostoses), benign chondroma, chondroblastoma, chondromyxofibroma, osteoid osteoma and giant cell tumors; Nervous system: skull (osteoma, hemangioma, granuloma, xanthoma, osteitis defornians), meninges (meningioma, meningiosarcoma, gliomatosis), brain (astrocytoma, medulloblastoma, glioma, ependymoma, germinoma (pinealoma), glioblastoma multiform, oligodendroglioma, schwannoma, retinoblastoma, congenital tumors), spinal cord neurofibroma, meningioma, glioma, sarcoma); Gynecological: uterus (endometrial carcinoma), cervix (cervical carcinoma, pre-tumor cervical dysplasia), ovaries (ovarian carcinoma [serous cystadenocarcinoma, mucinous cystadenocarcinoma, unclassified carcinoma], granulosa-thecal cell tumors, Sertoli-Leydig cell tumors, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, botryoid sarcoma (embryonal rhabdomyosarcoma], fallopian tubes (carcinoma); Hematologic: blood (myeloid leukemia [acute and chronic], acute lymphoblastic leukemia, chronic lymphocytic leukemia, myeloproliferative diseases, multiple myeloma, myelodysplastic syndrome), Hodgkin's disease, non-Hodgkin's lymphoma [malignant lymphoma]; Skin: malignant melanoma, basal cell carcinoma, squamous cell carcinoma, Karposi's sarcoma, moles dysplastic nevi, lipoma, angioma, dermatofibroma, keloids, psoriasis; and Adrenal glands: neuroblastoma; as well as cancers of the thyroid including medullary thyroid cancer. Thus, the term "cancerous cell," as provided herein, includes a cell afflicted by any one of the above-identified conditions.

The cancer may be selected from ovarian cancer, prostate cancer, lung cancer, medullary thyroid cancer, liver cancer, gastrointestinal cancer, pancreatic cancer, bone cancer, hematologic cancer, skin cancer, kidney cancer, breast cancer, colon cancer, and fallopian tube cancer.

The cancer may clear cell carcinoma, clear cell renal cell carcinoma, non-clear cell carcinoma, non-clear cell renal cell carcinoma, urothelial carcinoma, salivary gland cancer, penile squamous cell carcinoma, neuroendocrine tumors, adrenocortical carcinoma, or merkel cell carcinoma.

The disease or disorder may be ovarian cancer.

The disease or disorder may be prostate cancer.

The disease or disorder may be lung cancer.

The disease or disorder may be medullary thyroid cancer.

The disease or disorder may be liver cancer.

The disease or disorder may be gastrointestinal cancer.

The disease or disorder may be pancreatic cancer.

The disease or disorder may be bone cancer.

The disease or disorder may be hematologic cancer.

The disease or disorder may be skin cancer.

The disease or disorder may be kidney cancer.

The disease or disorder may be breast cancer.

The disease or disorder may be colon cancer. The disease or disorder may be fallopian cancer. The disease or disorder may be liver cancer, wherein the liver cancer is hepatocellular carcinoma, cholangiocarcinoma, hepatoblastoma, angiosarcoma, hepatocellular adenoma, or hemagioma.

The disease or disorder may be gastrointestinal cancer, wherein the gastrointestinal cancer is cancer of the esophagus which is squamous cell carcinoma, adenocarcinoma, or leiomyosarcoma; cancer of the stomach which is carcinoma, or lymphoma; cancer of the pancreas, which is ductal adenocarcinoma, insulinoma, gucagonoma, gastrinoma, carcinoid tumors, or vipoma; cancer of the small bowel, which is adenocarcinoma, lymphoma, carcinoid tumors, Karposi's sarcoma, leiomyoma, hemagioma, lipoma, or cancer of the large bowel, which is adenocarcinoma, tubular adenoma, villous adenoma, hamartoma, or leiomyoma.

The disease or disorder may be cancer of the pancreas, wherein the cancer of the pancreas is ductal adenocarcinoma, insulinoma, gucagonoma, gastrinoma, carcinoid tumors, or vipoma.

The disease or disorder may be bone cancer, wherein the bone cancer is osteosarcoma, fibrosarcoma, malignant fibrous histiocytoma, chondrosarcoma, Ewing's sarcoma, malignant reticulum cell sarcoma, multiple myeloma, malignant giant cell tumor chordoma, osteocartiliginous exostoses, chondroblastoma, chondromyxofibroma, or osteoid osteoma.

The disease or disorder may be hematologic cancer, wherein the hematologic cancer is myeloid leukemia, acute lymphoblastic leukemia, chronic lymphocytic leukemia, myeloproliferative diseases, multiple myeloma, or myelodysplastic syndrome.

The disease or disorder may be skin cancer, wherein the skin cancer is malignant melanoma, basal cell carcinoma, squamous cell carcinoma, or Karposi's sarcoma.

The disease or disorder may be a renal tumor or renal cell carcinoma.

The disease or disorder may be breast cancer.

The disease or disorder may be a colon cancer tumor.

The disease or disorder may be fallopian tube carcinoma.

### Combination Therapies

A compound as disclosed herein can be administered as a single therapy or in combination ("co-administered") with one or more additional therapies for the treatment of a disease or disorder, for instance a disease or disorder associated with hyper-proliferation such as cancer. Therapies that may be used in combination with a compound disclosed herein include: (i) surgery; (ii) radiotherapy (for example, gamma radiation, neutron beam radiotherapy, electron beam radiotherapy, proton therapy, brachytherapy, and systemic radioactive isotopes); (iii) endocrine therapy; (iv) adjuvant therapy, immunotherapy, CAR T- cell therapy; and (v) other chemotherapeutic agents.

The term "co-administered" ("co-administering") refers to either simultaneous administration, or any manner of separate sequential administration, of a compound as described herein, and a further active pharmaceutical ingredient or ingredients, including cytotoxic agents and radiation treatment. If the administration is not simultaneous, the compounds are administered in a close time proximity to each other. Furthermore, it does not matter if the compounds are administered in the same dosage form, e.g. one compound may be administered topically and another compound may be administered orally.

Typically, any agent that has activity against a disease or condition being treated may be co-administered. Examples of such agents for cancer treatment can be found, for instance, at https://www.cancer.gov/about-cancer/treatment/drugs and in publicly available sources such as Cancer Principles and Practice of Oncology by V. T. Devita and S. Hellman (editors), 1 Ith edition (2018), Lippincott Williams & Wilkins Publishers. A person of ordinary skill in the art would be able to discern which combinations of agents would be useful based on the particular characteristics of the drugs and the disease involved.

The treatment method may include the co-administration of a compound as disclosed herein, or a pharmaceutically acceptable salt, stereoisomer, or tautomer thereof, and at least one immunotherapy. Immunotherapy (also called biological response modifier therapy, biologic therapy, biotherapy, immune therapy, or biological therapy) is treatment that uses parts of the immune system to fight disease. Immunotherapy can help the immune system recognize cancer cells, or enhance a response against cancer cells. Immunotherapies include active and passive immunotherapies. Active immunotherapies stimulate the body's own immune system while passive immunotherapies generally use immune system components created outside of the body.

Examples of active immunotherapies include, but are not limited to vaccines including cancer vaccines, tumor cell vaccines (autologous or allogeneic), dendritic cell vaccines, antigen vaccines, anti-idiotype vaccines, DNA vaccines, viral vaccines, or Tumor- Infiltrating Lymphocyte (TIL) Vaccine with Interleukin-2 (IL-2) or Lymphokine- Activated Killer (LAK) Cell Therapy.

Examples of passive immunotherapies include but are not limited to monoclonal antibodies and targeted therapies containing toxins. Monoclonal antibodies include naked antibodies and conjugated monoclonal antibodies (also called tagged, labeled, or loaded antibodies). Naked monoclonal antibodies do not have a drug or radioactive material attached whereas conjugated monoclonal antibodies are joined to, for example, a chemotherapy drug (chemolabeled), a radioactive particle (radiolabeled), or a toxin (immunotoxin). Examples of these naked monoclonal antibody drugs include, but are not limited to rituximab (Rituxan), an antibody against the CD20 antigen used to treat, for example, B cell non-Hodgkin lymphoma; trastuzumab (Herceptin), an antibody against the HER2 protein used to treat, for example, advanced breast cancer; alemtuzumab (Campath), an antibody against the CD52 antigen used to treat, for example, B cell chronic lymphocytic leukemia (B-CLL); cetuximab (Erbitux), an antibody against the EGFR protein used, for example, in combination with irinotecan to treat, for example, advanced colorectal cancer and head and neck cancers; and bevacizumab (Avastin) which is an antiangiogenesis therapy that works against the VEGF protein and is used, for example, in combination with chemotherapy to treat, for example, metastatic colorectal cancer. Examples of the conjugated monoclonal antibodies include, but are not limited to Radiolabeled antibody ibritumomab tiuxetan (Zevalin) which delivers radioactivity directly to cancerous B lymphocytes and is used to treat, for example, B cell non-Hodgkin lymphoma; radiolabeled antibody tositumomab (Bexxar) which is used to treat, for example, certain types of non-Hodgkin lymphoma; and immunotoxin gemtuzumab ozogamicin (Mylotarg) which contains calicheamicin and is used to treat, for example, acute myelogenous leukemia (AML). BL22 is a conjugated monoclonal antibody for treating, for example, hairy cell leukemia, immunotoxins for treating, for example, leukemias, lymphomas, and brain tumors, and radiolabeled antibodies such as OncoScint for example, for colorectal and ovarian cancers and ProstaScint for example, for prostate cancers.

Further examples of therapeutic antibodies that can be used include, but are not limited to, HERCEPTIN^{®} (trastuzumab) (Genentech, Calif.) which is a humanized anti- HER2 monoclonal antibody for the treatment of patients with metastatic breast cancer; REOPRO^{®} (abciximab) (Centocor) which is an anti-glycoprotein Ilb/IIIa receptor on the platelets for the prevention of clot formation; ZENAPAX^{™} (daclizumab) (Roche Pharmaceuticals, Switzerland) which is an immunosuppressive, humanized anti-CD25 monoclonal antibody for the prevention of acute renal allograft rejection; PANOREX^{™} which is a murine anti-17-IA cell surface antigen IgG2a antibody (Glaxo Wellcome/Centocor); BEC2 which is a murine anti-idiotype (GD3epitope) IgG antibody (ImClone System); IMC-C225 which is a chimeric anti-EGFR IgG antibody (ImClone System); VITAXIN^{™} which is a humanized anti-alpha V beta 3 integrin antibody (Applied Molecular Evolution/Medlmmune); Campath 1H/LDP-03 which is a humanized anti CD52 IgG1 antibody (Leukosite); Smart M195 which is a humanized anti-CD33 IgG antibody (Protein Design Lab/Kanebo); RITETXAN^{™} which is a chimeric anti-CD20 IgG1 antibody (IDEC Pharm/Genentech, Roche/Zettyaku); LYMPHOCIDE^{™} which is a humanized anti- CD22 IgG antibody (Immunomedics); LYMPHOCIDE^{™} Y-90 (Immunomedics); Lymphoscan (Tc-99m-labeled; radioimaging; Immunomedics); Nuvion (against CD3; Protein Design Labs); CM3 is a humanized anti-ICAM3 antibody (ICOS Pharm); IDEC-1 14 is a primatized anti-CD80 antibody (IDEC Pharm/Mitsubishi); ZEVALIN^{™} is a radiolabeled murine anti-CD20 antibody (IDEC/Schering AG); IDEC-131 is a humanized anti-CD40L antibody (IDEC/Eisai); IDEC-151 is a primatized anti-CD4 antibody (IDEC); IDEC-152 is a primatized anti-CD23 antibody (IDEC/Seikagaku); SMART anti-CD3 is a humanized anti- CD3 IgG (Protein Design Lab); 5G1.1 is a humanized anti-complement factor 5 (C5) antibody (Alexion Pharm); D2E7 is a humanized anti-TNF-alpha antibody (CAT/BASF); CDP870 is a humanized anti-TNF-alpha. Fab fragment (Celltech); IDEC-1 51 is a primatized anti-CD4 IgG1 antibody (IDEC Pharm/SmithKline Beecham); MDX-CD4 is a human anti-CD4 IgG antibody (Medarex/Eisai/Genmab); CD20-sreptdavidin (+biotin-yttrium 90; NeoRx); CDP571 is a humanized anti-TNF-alpha. IgG4 antibody (Celltech); LDP-02 is a humanized anti-alpha4 beta7 antibody (LeukoSite/Genentech); OrthoClone OKT4A is a humanized anti-CD4 IgG antibody (Ortho Biotech); ANTOVA (ruplizumab) is a humanized anti-CD40L IgG antibody (Biogen); ANTEGREN^{™} is a humanized anti-VLA-4 IgG antibody (Elan); and CAT-152 is a human anti-TGF-beta₂ antibody (Cambridge Ab Tech).

Immunotherapies that can be used in combination with a compound as disclosed herein include adjuvant immunotherapies. Examples include cytokines, such as granulocyte- macrophage colony-stimulating factor (GM-CSF), granulocyte-colony stimulating factor (G-CSF), macrophage inflammatory protein (MIP)-l -alpha, interleukins (including IL-1, IL-2, IL-4, IL-6, IL-7, IL-12, IL-15, IL-18, IL-21, and IL-27), tumor necrosis factors (including TNF-alpha), and interferons (including IFN-alpha, IFN-beta, and IFN-gamma); aluminum hydroxide (alum); Bacille Calmette-Guerin (BCG); Keyhole limpet hemocyanin (KLH); Incomplete Freund's adjuvant (IF A); QS-21; DETOX; Levamisole; and Dinitrophenyl (DNP), and combinations thereof, such as, for example, combinations of, interleukins, for example, IL-2 with other cytokines, such as IFN-alpha.

An immunological therapy or an immunological therapeutic agent can include, one or more of the following: an adoptive cell transfer, an angiogenesis inhibitor, Bacillus Calmette-Guerin therapy, biochemotherapy, a cancer vaccine, a chimeric antigen receptor (CAR) T-cell therapy, a cytokine therapy, gene therapy, an immune checkpoint modulator, an immunoconjugate, a radioconjugate, an oncolytic virus therapy, or a targeted drug therapy. The function or at least one of the functions of the immunological therapy or immunological therapeutic agent, collectively referred to herein as an "immunotherapeutic agent."

An exemplary immunotherapeutic agent is an immune cell (e.g. T-cell, dendritic cell, a natural killer cell and the like) modulator chosen from an agonist or an activator of a costimulatory molecule, wherein the modulator is a monoclonal antibody, a bispecific antibody comprising one or more immune checkpoint antigen binding moieties, a trispecific antibody, or an immune cell-engaging multivalent antibody/fusion protein/construct known in the art). The immunotherapeutic agent can be an antibody that modulates a costimulatory molecule, bind to an antigen on the surface of an immune cell, or a cancer cell. The antibody modulator can be a monoclonal antibody, a polyclonal antibody, a bispecific antibody, a trispecific or multispecific format antibody, a fusion protein, or a fragment thereof, for example, a Diabody, a Single-chain (sc)- diabody (scFv)2, a Miniantibody, a Minibody, a Bamase-barstar, a scFv-Fc, a sc(Fab)2, a Trimeric antibody construct, a Triabody antibody construct, a Trimerbody antibody construct, a Tribody antibody construct, a Collabody antibody construct, a (scFv-TNFa)3, or a F(ab)3/DNL antibody construct.

The immunotherapeutic agent may be an agent that modulates immune responses, for example, a checkpoint inhibitor or a checkpoint agonist. The immunotherapeutic agent may be an agent that enhances anti-tumor immune responses. The immunotherapeutic agent may be an agent that increases cell-mediated immunity. The immunotherapeutic agent may be an agent that increases T-cell activity. The immunotherapeutic agent may be an agent that increases cytolytic T-cell (CTL) activity. The immunotherapeutic agent may be an antibody modulator that targets PD-1, PD-L1, PD-L2, CEACAM (e g., CEACAM-1, -3 and/or -5), CTLA-4, TIM-3, LAG-3, VISTA, BTLA, TIGIT, LAIR1, CD160, 2B4, TGF beta, 0X40, 41BB, LIGHT, CD40, GITR, TGF-beta, TIM-3, SIRP-alpha, VSIG8, BTLA, SIGLEC7, SIGLEC9, ICOS, B7H3, B7H4, FAS, and/or BTNL2 among others known in the art. The immunotherapeutic agent may be an agent that increases natural killer (NK) cell activity. The immunotherapeutic agent may be an agent that inhibits suppression of an immune response. The immunotherapeutic agent may be an agent that inhibits suppressor cells or suppressor cell activity.The immunotherapeutic agent may be an agent or therapy that inhibits Treg activity. The immunotherapeutic agent may be an agent that inhibits the activity of inhibitory immune checkpoint receptors.

The immunotherapeutic agent may include a T cell modulator chosen from an agonist or an activator of a costimulatory molecule. The agonist of the costimulatory molecule may be chosen from an agonist (e.g., an agonistic antibody or antigen-binding fragment thereof, or a soluble fusion) of GITR, 0X40, ICOS, SLAM (e.g., SLAMF7), HVEM, LIGHT, CD2, CD27, CD28, CDS, ICAM-1, LFA-1 (CD1 la/CDl8), ICOS (CD278), 4-1BB (CD137), CD30, CD40, BAFFR, CD7, NKG2C, NKp80, CD160, B7-H3, or CD83 ligand. The effector cell combination may include a bispecific T cell engager (e.g., a bispecific antibody molecule that binds to CD3 and a tumor antigen (e.g., EGFR, PSCA, PSMA, EpCAM, HER2 among others).

The immunotherapeutic agent may be a modulator of PD-1 activity, a modulator of PD-L 1 activity, a modulator of PD-L2 activity, a modulator of CTLA-4 activity, a modulator of CD28 activity, a modulator of CD80 activity, a modulator of CD86 activity, a modulator of 4-1BB activity, an modulator of 0X40 activity, a modulator of KIR activity, a modulator of Tim-3 activity, a modulator of LAG3 activity, a modulator of CD27 activity, a modulator of CD40 activity, a modulator of GITR activity, a modulator of TIGIT activity, a modulator of CD20 activity, a modulator of CD96 activity, a modulator of IDO1 activity, a modulator of SIRP-alpha activity, a modulator of TIGIT activity, a modulator of VSIG8 activity, a modulator of BTLA activity, a modulator of SIGLEC7 activity, a modulator of SIGLEC9 activity, a modulator of ICOS activity, a modulator of B7H3 activity, a modulator of B7H4 activity, a modulator of FAS activity, a modulator of BTNL2 activity, a cytokine, a chemokine, an interferon, an interleukin, a lymphokine, a member of the tumor necrosis factor (TNF) family, or an immunostimulatory oligonucleotide. The immunotherapeutic agent may be an immune checkpoint modulator (e.g., an immune checkpoint inhibitor e.g. an inhibitor of PD-1 activity, a modulator of PD-L 1 activity, a modulator of PD-L2 activity, a modulator of CTLA-4, or a CD40 agonist (e.g., an anti-CD40 antibody molecule), (xi) an 0X40 agonist (e.g., an anti- 0X40 antibody molecule), or (xii) a CD27 agonist (e.g., an anti-CD27 antibody molecule). The immunomodulator may be an inhibitor of PD-1, PD-L1, PD-L2, CTLA-4, TIM-3, LAG-3, CEACAM (e.g., CEACAM-1, -3 and/or -5), VISTA, BTLA, TIGIT, LAIR1, CD 160, 2B4 and/or TGF beta. The inhibitor of an immune checkpoint molecule may inhibit PD-1, PD-L1, LAG-3, TIM-3, CEACAM (e.g., CEACAM-1, -3 and/or -5), CTLA-4, or any combination thereof.

Inhibition of an inhibitory molecule can be performed at the DNA, RNA or protein level. An inhibitory nucleic acid (e.g., a dsRNA, siRNA or shRNA), can be used to inhibit expression of an inhibitory molecule. The inhibitor of an inhibitory signal may be, a polypeptide e.g., a soluble ligand (e.g., PD-1-Ig or CTLA-4 Ig), or an antibody or antigen-binding fragment thereof,, for example, a monoclonal antibody, a bispecific antibody comprising one or more immune checkpoint antigen binding moieties, a trispecific antibody, or an immune cell-engaging multivalent antibody/fusion protein/construct known in the art that binds to the inhibitory molecule; e.g., an antibody or fragment thereof (also referred to herein as "an antibody molecule") that binds to PD-1, PD-L1, PD-L2, CEACAM (e.g., CEACAM-1, -3 and/or -5), CTLA-4, TIM-3, LAG-3, VISTA, BTLA, TIGIT, LAIR1, CD 160, 2B4, TGF beta, or a combination thereof.

The treatment method may include the co-administration of a compound as disclosed herein or a pharmaceutically acceptable salt thereof and at least one cytotoxic agent. The term "cytotoxic agent" as used herein refers to a substance that inhibits or prevents a cellular function and/or causes cell death or destruction. Cytotoxic agents include, but are not limited to, radioactive isotopes (e.g., At²¹¹, 1¹³¹, 1¹²⁵, Y⁹⁰, Re¹⁸⁶, Re¹⁸⁸, Sm¹⁵³, Bi²¹², P³², Pb²¹² and radioactive isotopes of Lu); chemotherapeutic agents; growth inhibitory agents; enzymes and fragments thereof such as nucleolytic enzymes; and toxins such as small molecule toxins or enzymatically active toxins of bacterial, fungal, plant or animal origin, including fragments and/or variants thereof.

Exemplary cytotoxic agents can be selected from anti -microtubule agents, platinum coordination complexes, alkylating agents, antibiotic agents, topoisomerase II inhibitors, antimetabolites, topoisomerase I inhibitors, hormones and hormonal analogues, signal transduction pathway inhibitors, non-receptor tyrosine kinase angiogenesis inhibitors, immunotherapeutic agents, proapoptotic agents, inhibitors of LDH-A; inhibitors of fatty acid biosynthesis; cell cycle signaling inhibitors; HDAC inhibitors, proteasome inhibitors; and inhibitors of cancer metabolism.

"Chemotherapeutic agents" include chemical compounds useful in the treatment of cancer. Examples of chemotherapeutic agents include erlotinib (TARCEVA^{®}, Genentech/OSI Pharm.), bortezomib (VELCADE^{®}, Millennium Pharm.), disulfiram, epigallocatechin gallate, salinosporamide A, carfilzomib, 17-AAG(geldanamycin), radicicol, lactate dehydrogenase A (LDH-A), fulvestrant (FASLODEX^{®}, AstraZeneca), sunitib (SETTENT^{®}, Pfizer/Sugen), letrozole (FEMARA^{®}, Novartis), imatinib mesylate (GLEEVEC^{®}, Novartis), finasunate (VATALANIB^{®}, Novartis), oxaliplatin (ELOXATIN^{®}, Sanofi), 5-FET (5-fluorouracil), leucovorin, rapamycin (Sirolimus, RAPAMUNE^{®}, Wyeth), lapatinib (TYKERB^{®}, GSK572016, Glaxo Smith Kline), lonafamib (SCH 66336), sorafenib (NEXAVAR^{®}, Bayer Labs), gefitinib (IRESSA^{®}, AstraZeneca), AG1478; alkylating agents such as thiotepa and CYTOXAN^{®}); cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphoramide and trimethylomelamine; acetogenins (especially bullatacin and bullatacinone); a camptothecin (including topotecan and irinotecan); bryostatin; cally statin; CC-1065 (including its adozelesin, carzelesin and bizelesin synthetic analogs); cryptophycins (particularly cryptophycin 1 and cryptophycin 8); adrenocorticosteroids (including prednisone and prednisolone); cyproterone acetate; 5 alpha-reductases including finasteride and dutasteride); vorinostat, romidepsin, panobinostat, valproic acid, mocetinostat dolastatin; aldesleukin, talc duocarmycin (including the synthetic analogs, KW-2189 and CB1-TM1); eleutherobin; pancrati statin; a sarcodictyin; spongistatin; nitrogen mustards such as chlorambucil, chlomaphazine, chlorophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, fotemustine, lomustine, nimustine, and ranimnustine; antibiotics such as the enediyne antibiotics (e.g., calicheamicin, especially calicheamicin gamma II and calicheamicin omega I (Angew Chem. Inti. Ed. Engl. 1994 33: 183-186); dynemicin, including dynemicin A; bisphosphonates, such as clodronate; an esperamicin; as well as neocarzinostatin chromophore and related chromoprotein enediyne antibiotic chromophores), aclacinomysins, actinomycin, authramycin, azaserine, bleomycins, cactinomycin, carabicin, caminomycin, carzinophilin, chromomycinis, dactinomycin, daunorubicin, detorubicin, 6-diazo-5-oxo-L-norleucine, ADRIAMYCIN^{®} (doxorubicin), morpholino-doxorubicin, cyanomorpholino- doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, porfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogs such as denopterin, methotrexate, pteropterin, trimetrexate; purine analogs such as fludarabine, 6-mercaptopurine, thiamiprine, thioguanine; pyrimidine analogs such as ancitabine, azacitidine, azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfomithine; elliptinium acetate; an epothilone; etoglucid; gallium nitrate; hydroxyurea; lentinan; lonidainine; maytansinoids such as maytansine and ansamitocins; mitoguazone; mitoxantrone; mopidamnol; nitraerine; pentostatin; phenamet; pirarubicin; losoxantrone; podophyllinic acid; 2-ethylhydrazide; procarbazine; PSK^{®} polysaccharide complex (JHS Natural Products, Eugene, Ore.); razoxane; rhizoxin; sizofuran; spirogermanium; tenuazonic acid; triaziquone; 2,2',2"-trichlorotriethylamine; trichothecenes especially T-2 toxin, verracurin A, roridin A and anguidine); urethan; vindesine; dacarbazine; mannomustine; mitobronitol; mitolactol; pipobroman; gacytosine; arabinoside "Ara-C"); cyclophosphamide; thiotepa; taxoids, e.g., TAXOL (paclitaxel; Bristol-Myers Squibb Oncology, Princeton, N.J.), ABRAXANE^{®} (Cremophor-free), albumin-engineered nanoparticle formulations of paclitaxel (American Pharmaceutical Partners, Schaumberg, Ill.), and TAXOTERE^{®} (docetaxel, doxetaxel; Sanofi-Aventis); chloranmbucil; GEMZAR^{®} (gemcitabine); 6-thioguanine; mercaptopurine; methotrexate; platinum analogs such as cisplatin and carboplatin; vinblastine; etoposide (VP-16); ifosfamide; mitoxantrone; vincristine; NAVELBINE^{®} (vinorelbine); novantrone; teniposide; edatrexate; daunomycin; aminopterin; capecitabine (XELODA^{®}); ibandronate; CPT-1 1; topoisomerase inhibitor RFS 2000; difluorom ethyl ornithine (DMFO); retinoids such as retinoic acid; and pharmaceutically acceptable salts, acids and derivatives of any of the above.

Chemotherapeutic agent also includes (i) anti-hormonal agents that act to regulate or inhibit hormone action on tumors such as anti-estrogens and selective estrogen receptor modulators (SERMs), including, for example, tamoxifen (including NOLVADEX^{®}; tamoxifen citrate), raloxifene, droloxifene, iodoxyfene, 4-hydroxytamoxifen, trioxifene, keoxifene, LY117018, onapristone, and FARESTON^{®} (toremifme citrate); (ii) aromatase inhibitors that inhibit the enzyme aromatase, which regulates estrogen production in the adrenal glands, such as, for example, 4(5)-imidazoles, aminoglutethimide, MEGASE^{®} (megestrol acetate), AROMASIN^{®} (exemestane; Pfizer), formestanie, fadrozole, RIVISOR^{®} (vorozole), FEMARA^{®} (letrozole; Novartis), and ARIMIDEX^{®} (anastrozole; AstraZeneca); (iii) anti-androgens such as flutamide, nilutamide, bicalutamide, leuprolide and goserelin; buserelin, tripterelin, medroxyprogesterone acetate, diethylstilbestrol, premarin, fluoxymesterone, all transretionic acid, fenretinide, as well as troxacitabine (a 1,3-dioxolane nucleoside cytosine analog); (iv) protein kinase inhibitors; (v) lipid kinase inhibitors; (vi) antisense oligonucleotides, particularly those which inhibit expression of genes in signaling pathways implicated in aberrant cell proliferation, such as, for example, PKC-alpha, Ralf and H-Ras; (vii) ribozymes such as VEGF expression inhibitors (e.g., ANGIOZYME^{®}) and HER2 expression inhibitors; (viii) vaccines such as gene therapy vaccines, for example, ALLOVECTIN^{®}, LEUVECTIN^{®}, and VAXID^{®}; PROLEUKIN^{®}, rIL-2; a topoisomerase 1 inhibitor such as LEIRTOTECAN^{®}; ABARELIX^{®}; and (ix) pharmaceutically acceptable salts, acids and derivatives of any of the above.

Chemotherapeutic agents also include antibodies, as described above, including alemtuzumab (Campath), bevacizumab (AVASTIN^{®}, Genentech); cetuximab (ERBITETX^{®}, Imclone); panitumumab (VECTIBIX^{®}, Amgen), rituximab (RITETXAN^{®}, Genentech/Biogen Idee), pertuzumab (OMNITARG^{®}, 2C4, Genentech), trastuzumab (HERCEPTIN^{®}, Genentech), tositumomab (Bexxar, Corixia), and the antibody drug conjugate, gemtuzumab ozogamicin (MYLOTARG^{®}, Wyeth). Additional humanized monoclonal antibodies with therapeutic potential as agents in combination with the compounds of the invention include: apolizumab, aselizumab, atlizumab, bapineuzumab, bivatuzumab mertansine, cantuzumab mertansine, cedelizumab, certolizumab pegol, cidfusituzumab, cidtuzumab, daclizumab, eculizumab, efalizumab, epratuzumab, erlizumab, felvizumab, fontolizumab, gemtuzumab ozogamicin, inotuzumab ozogamicin, ipilimumab, labetuzumab, lintuzumab, matuzumab, mepolizumab, motavizumab, motovizumab, natalizumab, nimotuzumab, nivolumab, nolovizumab, numavizumab, ocrelizumab, omalizumab, palivizumab, pascolizumab, pecfusituzumab, pectuzumab, pexelizumab, ralivizumab, ranibizumab, reslivizumab, reslizumab, resyvizumab, rovelizumab, ruplizumab, sibrotuzumab, siplizumab, sontuzumab, tacatuzumab tetraxetan, tadocizumab, talizumab, tefibazumab, tocilizumab, toralizumab, tucotuzumab celmoleukin, tucusituzumab, umavizumab, urtoxazumab, ustekinumab, visilizumab, and the anti-interleukin-l2 (ABT-8744695, Wyeth Research and Abbott Laboratories) which is a recombinant exclusively human-sequence, full-length IgG₁ λ antibody genetically modified to recognize interleukin- 12 p40 protein.

Chemotherapeutic agents also include "tyrosine kinase inhibitors" including the EGFR inhibitors; small molecule HER2 tyrosine kinase inhibitor such as mubritonib (TAK165, Takeda); CP-724.714, (Axon Medchem BV, an oral selective inhibitor of the ErbB2 receptor tyrosine kinase); dual-HER inhibitors such as EKB-569 (available from Wyeth) which preferentially binds EGFR but inhibits both HER2 and EGFR-overexpressing cells; lapatinib (GSK572016; available from Glaxo- SmithKline), an oral HER2 and EGFR tyrosine kinase inhibitor; PKI-166 (available from Novartis); pan-HER inhibitors such as canertinib (CI-1033; Pharmacia); Raf-1 inhibitors such as antisense agent ISIS-5132 available from ISIS Pharmaceuticals which inhibit Raf-1 signaling; non-HER targeted TK inhibitors such as imatinib mesylate (GLEEVEC^{®}, available from Glaxo SmithKline); multi -targeted tyrosine kinase inhibitors such as sunitinib (SLTTENT^{®}, available from Pfizer); VEGF receptor tyrosine kinase inhibitors such as vatalanib (PTK787/ZK222584, available from Novartis/Schering AG); MAPK extracellular regulated kinase 1 inhibitor Cl- 1040 (available from Pharmacia); quinazolines, such as PD 153035, 4-(3-chloroanilino) quinazoline; pyridopyrimidines; pyrimidopyrimidines; pyrrolopyrimidines, such as CGP 59326, CGP 60261 and CGP 62706; pyrazolopyrimidines, 4-(phenylamino)-7H-pyrrolo[2,3-d] pyrimidines; curcumin (diferuloyl methane, 4,5-bis (4-fluoroanilino)phthalimide); tyrphostines containing nitrothiophene moieties; antisense molecules (e.g. those that bind to HER-encoding nucleic acid); quinoxalines (U.S. Pat. No. 5,804,396); tryphostins (U.S. Pat. No. 5,804,396); affmitac (ISIS 3521; Isis/Lilly); PKI166 (Novartis); Semaxinib (Pfizer); INC-1C11 (Imclone), rapamycin (sirolimus, RAPAMUNE^{®}); or as described in any of the following patent publications: U.S. Pat. No. 5,804,396; WO 1999/09016 (American Cyanamid); WO 1998/43960 (American Cyanamid); WO 1997/38983 (Warner Lambert); WO 1999/06378 (Warner Lambert); WO 1999/06396 (Warner Lambert); WO 1996/30347 (Pfizer, Inc); WO 1996/33978 (Zeneca); WO 1996/3397 (Zeneca) and WO 1996/33980 (Zeneca). Tyrosine kinase inhibitors also include erlotinib (Tarceva^{®}), gefitinib (Iressa^{®}), dasatinib (Sprycel^{®}), nilotinib (Tasigna^{®}), crizotinib (Xalkori^{®}), ruxolitinib (Jakafi^{®}), vemurafenib (Zelboraf^{®}), Vandetanib (Caprelsa^{®}), pazopanib (Votrient^{®}), afatinib, alisertib, amuvatinib, axitinib, bosutinib, brivanib, canertinib, cabozantinib, cediranib, crenolanib, dabrafenib, dacomitinib, danusertib, dovitinib, foretinib, ganetespib, ibrutinib, iniparib, lenvatinib, linifanib, linsitinib, masitinib, momelotinib, motesanib, neratinib, niraparib, oprozomib, olaparib, pictilisib, ponatinib, quizartinib, regorafenib, rigosertib, rucaparib, saracatinib, saridegib, tandutinib, tasocitinib, telatinib, tivantinib, tivozanib, tofacitinib, trametinib, veliparib, vismodegib, volasertib, cobimetinib (Cotellic^{®}), and others.

Chemotherapeutic agents also include dexamethasone, interferons, colchicine, metoprine, cyclosporine, amphotericin, metronidazole, alemtuzumab, alitretinoin, allopurinol, amifostine, arsenic trioxide, asparaginase, BCG live, bevacuzimab, bexarotene, cladribine, clofarabine, darbepoetin alfa, denileukin, dexrazoxane, epoetin alfa, elotinib, filgrastim, histrelin acetate, ibritumomab, interferon alfa-2a, interferon alfa-2b, lenalidomide, levamisole, mesna, methoxsalen, nandrolone, nelarabine, nofetumomab, oprelvekin, palifermin, pamidronate, pegademase, pegaspargase, pegfilgrastim, pemetrexed disodium, plicamycin, porfimer sodium, quinacrine, rasburicase, sargramostim, temozolomide, VM-26, 6-TG, toremifene, tretinoin, ATRA, valrubicin, zoledronate, and zoledronic acid, and pharmaceutically acceptable salts thereof.

Chemotherapeutic agents also include hydrocortisone, hydrocortisone acetate, cortisone acetate, tixocortol pivalate, triamcinolone acetonide, triamcinolone alcohol, mometasone, amcinonide, budesonide, desonide, fluocinonide, fluocinolone acetonide, betamethasone, betamethasone sodium phosphate, dexamethasone, dexamethasone sodium phosphate, fluocortolone, hydrocortisone-17-butyrate, hydrocortisone- 17-valerate, aclometasone dipropionate, betamethasone valerate, betamethasone dipropionate, prednicarbate, clobetasone-1 7-butyrate, clobetasol-1 7-propionate, fluocortolone caproate, fluocortolone pivalate and fluprednidene acetate; immune selective anti-inflammatory peptides (ImSAIDs) such as phenylalanine-glutamine-glycine (FEG) and its D-isomeric form (feG) (IMULAN BioTherapeutics, LLC); anti-rheumatic drugs such as azathioprine, ciclosporin (cyclosporine A), D-penicillamine, gold salts, hydroxychloroquine, leflunomideminocycline, sulfasalazine, tumor necrosis factor alpha (TNF alpha) blockers such as etanercept (Enbrel), infliximab (Remicade), adalimumab (1-Iumira), certolizumab pegol (Cimzia), golimumab (Simponi), Interleukin 1 (IL-1) blockers such as anakinra (Kineret), T cell costimulation blockers such as abatacept (Orencia), Interleukin 6 (IL-6) blockers such as tocilizumab (ACTEMERA^{®}); Interleukin 13 (IL-1 3) blockers such as lebrikizumab; Interferon alpha (IFN) blockers such as Rontalizumab; Beta 7 integrin blockers such as rhuMAb Beta7; IgE pathway blockers such as Anti-Ml prime; Secreted homotrimeric LTa3 and membrane bound heterotrimer LTal/l32 blockers such as Anti-lymphotoxin alpha (LTa); miscellaneous investigational agents such as thioplatin, PS-341, phenylbutyrate, ET- I8-OCH3, or famesyl transferase inhibitors (L-739749, L-744832); polyphenols such as quercetin, resveratrol, piceatannol, epigallocatechine gallate, theaflavins, flavanols, procyanidins, betulinic acid and derivatives thereof; autophagy inhibitors such as chloroquine; delta-9-tetrahydrocannabinol (dronabinol, MARINOL^{®}); beta-lapachone; lapachol; colchicines; betulinic acid; acetylcamptothecin, scopolectin, and 9- aminocamptothecin); podophyllotoxin; tegafur (UFTORAL^{®}); bexarotene (TARGRETIN^{®}); bisphosphonates such as clodronate (for example, BONEFOS^{®} or OSTAC^{®}), etidronate (DIDROCAL^{®}), NE-58095, zoledronic acid/zoledronate (ZOMETA^{®}), alendronate (FOSAMAX^{®}), pamidronate (AREDIA^{®}), tiludronate (SKELID^{®}), or risedronate (ACTONEL^{®}); and epidermal growth factor receptor (EGF-R); vaccines such as THERATOPE^{®} vaccine; perifosine, COX-2 inhibitor (e.g. celecoxib or etoricoxib), proteosome inhibitor (e.g. PS341); CCI-779; tipifamib (R11577); orafenib, ABT510; Bcl-2 inhibitor such as oblimersen sodium (GENASENSE) pixantrone; farnesyltransferase inhibitors such as lonafamib (SCH 6636, SARASAR^{™}); and pharmaceutically acceptable salts, acids or derivatives of any of the above; as well as combinations of two or more of the above such as CHOP, an abbreviation for a combined therapy of cyclophosphamide, doxorubicin, vincristine, and prednisolone; and FOLFOX, an abbreviation for a treatment regimen with oxaliplatin (ELOXATIN^{™}) combined with 5-FU and leucovorin. [000354] Chemotherapeutic agents also include Poly ADP ribose polymerase (PARP) inhibitors: olaparib (Lynparza^{®}), rucaprib (Rubraca^{®}) niraparib (Zejula^{®}), talzoparib (Talzenna^{®}).

Compounds as disclosed herein may be used in combination therapy with any of the kinase inhibitors disclosed herein for the treatment of diseases such as cancer. Exemplary kinase inhibitors include imatinib, baricitinib gefitinib, erlotinib, sorafenib, dasatinib, sunitinib, lapatinib, nilotinib, pirfenidone, pazopanib, crizotinib, vemurafenib, vandetanib, ruxolitinib, axitinib, bosutinib, regorafenib, tofacitinib, cabozantinib, ponatinib, trametinib, dabrafenib, afatinib, ibrutinib, ceritinib, idelalisib, nintedanib, palbociclib, lenvatinib, cobimetinib, abemaciclib, acalabrutinib, alectinib, binimetinib, brigatinib, encorafenib, erdafitinib, everolimus, fostamatinib, gilter, larotrectinib, lorlatinib, netarsudil, osimertinib, pexidartinib, ribociclib, temsirolimus, XL-092, XL-147, XL-765, XL-499, and XL-880. In some embodiments, a compound as described herein can be used in combination with a HSP90 inhibitor (e.g., XL888), liver X receptor (LXR) modulators, retinoid-related orphan receptor gamma (RORy) modulators, a CK1 inhibitor, a CKl-a inhibitor, a Wnt pathway inhibitor (e.g., SST-215), or a mineralocorticoid receptor inhibitor, (e.g., esaxerenone or XL- 550) for the treatment of a disease disclosed herein such as cancer.

For treatment of cancer, compounds as disclosed herein may be used in combination with inhibitors of PD-1 or inhibitors of PD-L1, e.g., an anti-PD-1 monoclonal antibody, an anti-PD-1 bispecific antibody or an anti-PD-L 1 monoclonal antibody, an anti-PD-L1 bispecific antibody, for example, nivolumab (Opdivo), pembrolizumab (Keytruda, MK-3475), atezolizumab, avelumab, AB122, AMP-224, AMP- 514, PDR001, durvalumab, pidilizumab (Imfinzi^{®}, CT-011), CK-301, BMS 936559, and MPDL3280A; CTLA-4 inhibitors, e.g., an anti-CTLA-4 antibody, for example, ipilimumab (Yervoy) and tremelimumab; and phosphatidylserine inhibitors, for example, bavituximab (PGN401); antibodies to cytokines (IL-10, TGF-b, and the like.); other anti-cancer agents such as cemiplimab. The anti-PD-1 monoclonal antibody may be nivolumab or pembrolizumab.

A compound as described herein can be used in combination with a vaccination protocol for the treatment of cancer. A compound as described herein can be used in combination with vaccines, to stimulate the immune response to pathogens, toxins, and self-antigens. Examples of pathogens for which this therapeutic approach may be particularly useful, include pathogens for which there is currently no effective vaccine, or pathogens for which conventional vaccines are less than completely effective. These include, but are not limited to, HIV, Hepatitis (A, B, & C), Influenza, Herpes, Giardia, Malaria, Leishmania, Staphylococcus aureus, Pseudomonas Aeruginosa.

Compounds as disclosed herein may be used in combination with inhibitors of PARP, for example, olaparib (Lynparza^{®}), rucaprib (Rubraca ^{®}), niraparib (Zejula^{®}), talzoparib (Talzenna^{®}) for the treatment of cancer.

Compounds as disclosed herein may be used in combination with esaxerenone (XL-550) or XL-888 for the treatment of cancer.

The compounds as disclosed herein can be combined with one or more inhibitors of the following kinases for the treatment of cancer: Akt1, Akt2, Akt3, TGF-βR, PKA, PKG, PKC, CaM-kinase, phosphorylase kinase, MEKK, ERK, MAPK, mTOR, EGFR, HER2, HER3, HER4, 1NS-R, IGF-1R, IR-R, PDGFαR, PDGFβ/R, CSFIR, KIT, FLK-II, KDR/FLK-1, FLK-4, flt-1, FGFR1, FGFR2, FGFR3, FGFR4, Ron, Sea, TRKA, TRKB, TRKC, FLT3, VEGFR/Flt2, Flt4, EphAl, EphA2, EphA3, EphB2, EphB4, Tie2, Src, Fyn, Lck, Fgr, Btk, Fak, SYR, FRK, JAK, ABL, ALK, CDK7, CDK12, KRAS, and B-Raf.

### Pharmaceutical Compositions and Modes of Administration

Compounds provided herein are usually administered in the form of pharmaceutical compositions. Thus, provided herein are also pharmaceutical compositions that comprise one or more of the compounds described herein (e.g., compounds of Formula (Ia), Formula (Ib), or Formula (Ic) or subformulas thereof) or a pharmaceutically acceptable salt, stereoisomer, or tautomer thereof, and one or more pharmaceutically acceptable vehicles selected from carriers, adjuvants and excipients. Suitable pharmaceutically acceptable vehicles may include, for example, inert solid diluents and fillers, diluents, including sterile aqueous solution and various organic solvents, permeation enhancers, solubilizers and adjuvants. Such compositions are prepared in a manner well known in the pharmaceutical art. See, e.g., Remington's Pharmaceutical Sciences, Mace Publishing Co., Philadelphia, Pa. 17th Ed. (1985); and Modern Pharmaceutics, Marcel Dekker, Inc. 3rd Ed. (G.S. Banker & C.T. Rhodes, Eds.).

The pharmaceutical compositions may be administered in either single or multiple doses. The pharmaceutical composition may be administered by various methods including, for example, rectal, buccal, intranasal and transdermal routes. In certain embodiments, the pharmaceutical composition may be administered by intra-arterial injection, intravenously, intraperitoneally, parenterally, intramuscularly, subcutaneously, orally, topically, or as an inhalant.

One mode for administration is parenteral, for example, by injection. The forms in which the pharmaceutical compositions described herein may be incorporated for administration by injection include, for example, aqueous or oil suspensions, or emulsions, with sesame oil, corn oil, cottonseed oil, or peanut oil, as well as elixirs, mannitol, dextrose, or a sterile aqueous solution, and similar pharmaceutical vehicles.

Oral administration may be another route for administration of the compounds described herein. Administration may be via, for example, capsule or enteric coated tablets. In making the pharmaceutical compositions that include at least one compound described herein or a pharmaceutically acceptable salt, a stereoisomer, or tautomer thereof, the active ingredient is usually diluted by an excipient and/or enclosed within such a carrier that can be in the form of a capsule, sachet, paper or other container. When the excipient serves as a diluent, it can be in the form of a solid, semi-solid, or liquid material, which acts as a vehicle, carrier or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing, for example, up to 10% by weight of the active compound, soft and hard gelatin capsules, sterile injectable solutions, and sterile packaged powders.

Some examples of suitable excipients include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, sterile water, syrup, and methyl cellulose. The formulations can additionally include lubricating agents such as talc, magnesium stearate, and mineral oil; wetting agents; emulsifying and suspending agents; preserving agents such as methyl and propylhydroxybenzoates; sweetening agents; and flavoring agents.

The compositions that include at least one compound described herein or a pharmaceutically acceptable salt, a stereoisomer, or tautomer thereof can be formulated so as to provide quick, sustained or delayed release of the active ingredient after administration to the subject by employing procedures known in the art. Controlled release drug delivery systems for oral administration include osmotic pump systems and dissolutional systems containing polymer-coated reservoirs or drug-polymer matrix formulations. Examples of controlled release systems are given in U.S. Patent Nos. 3,845,770; 4,326,525; 4,902,514; and 5,616,345. Another formulation for use in the methods disclosed herein employ transdermal delivery devices ("patches"). Such transdermal patches may be used to provide continuous or discontinuous infusion of the compounds described herein in controlled amounts. The construction and use of transdermal patches for the delivery of pharmaceutical agents is well known in the art. See, e.g., U.S. Patent Nos. 5,023,252, 4,992,445 and 5,001,139. Such patches may be constructed for continuous, pulsatile, or on demand delivery of pharmaceutical agents.

For preparing solid compositions such as tablets, the principal active ingredient may be mixed with a pharmaceutical excipient to form a solid preformulation composition containing a homogeneous mixture of a compound described herein or a pharmaceutically acceptable salt, a stereoisomer, or tautomer thereof. When referring to these preformulation compositions as homogeneous, the active ingredient may be dispersed evenly throughout the composition so that the composition may be readily subdivided into equally effective unit dosage forms such as tablets, pills and capsules.

The tablets or pills of the compounds described herein may be coated or otherwise compounded to provide a dosage form affording the advantage of prolonged action, or to protect from the acid conditions of the stomach. For example, the tablet or pill can include an inner dosage and an outer dosage component, the latter being in the form of an envelope over the former. The two components can be separated by an enteric layer that serves to resist disintegration in the stomach and permit the inner component to pass intact into the duodenum or to be delayed in release. A variety of materials can be used for such enteric layers or coatings, such materials including a number of polymeric acids and mixtures of polymeric acids with such materials as shellac, cetyl alcohol, and cellulose acetate.

Compositions for inhalation or insufflation may include solutions and suspensions in pharmaceutically acceptable, aqueous or organic solvents, or mixtures thereof, and powders. The liquid or solid compositions may contain suitable pharmaceutically acceptable excipients as described herein. In some embodiments, the compositions are administered by the oral or nasal respiratory route for local or systemic effect. In other embodiments, compositions in pharmaceutically acceptable solvents may be nebulized by use of inert gases. Nebulized solutions may be inhaled directly from the nebulizing device or the nebulizing device may be attached to a facemask tent, or intermittent positive pressure breathing machine. Solution, suspension, or powder compositions may be administered, preferably orally or nasally, from devices that deliver the formulation in an appropriate manner.

### Dosing

The specific dose level of a compound of the present application for any particular subject will depend upon a variety of factors including the activity of the specific compound employed, the age, body weight, general health, sex, diet, time of administration, route of administration, and rate of excretion, drug combination and the severity of the particular disease in the subject undergoing therapy. For example, a dosage may be expressed as a number of milligrams of a compound described herein per kilogram of the subject's body weight (mg/kg). Dosages of between about 0.1 and 150 mg/kg may be appropriate. In some embodiments, about 0.1 and 100 mg/kg may be appropriate. In other embodiments a dosage of between 0.5 and 60 mg/kg may be appropriate. Normalizing according to the subject's body weight is particularly useful when adjusting dosages between subjects of widely disparate size, such as occurs when using the drug in both children and adult humans or when converting an effective dosage in a non-human subject such as dog to a dosage suitable for a human subject.

### Synthesis of the Compounds

The compounds may be prepared using the methods disclosed herein and routine modifications thereof, which will be apparent given the disclosure herein and methods well known in the art. Conventional and well-known synthetic methods may be used in addition to the teachings herein. The synthesis of typical compounds described herein may be accomplished as described in the following examples. If available, reagents may be purchased commercially, e.g., from Sigma Aldrich or other chemical suppliers.

Typical embodiments of compounds described herein may be synthesized using the general reaction schemes described below. It will be apparent given the description herein that the general schemes may be altered by substitution of the starting materials with other materials having similar structures to result in products that are correspondingly different. Descriptions of syntheses follow to provide numerous examples of how the starting materials may vary to provide corresponding products. Given a desired product for which the substituent groups are defined, the necessary starting materials generally may be determined by inspection. Starting materials are typically obtained from commercial sources or synthesized using published methods. For synthesizing compounds which are embodiments described in the present disclosure, inspection of the structure of the compound to be synthesized will provide the identity of each substituent group. The identity of the final product will generally render apparent the identity of the necessary starting materials by a simple process of inspection, given the examples herein. In general, compounds described herein are typically stable and isolatable at room temperature and pressure.

Preparation of compounds as disclosed herein can involve the protection and deprotection of various chemical groups. The need for protection and deprotection, and the selection of appropriate protecting groups, can be readily determined by one skilled in the art. The chemistry of protecting groups is described, *e.g.,* in Kocienski, Protecting Groups, (Thieme, 2007); Robertson, Protecting Group Chemistry, (Oxford University Press, 2000); Smith et al., March's Advanced Organic Chemistry: Reactions, Mechanisms, and Structure, 6th Ed. (Wiley, 2007); Peturssion et al., "Protecting Groups in Carbohydrate Chemistry," J. Chem. Educ., 1997, 74(11), 1297; and Wuts et al., Protective Groups in Organic Synthesis, 4th Ed., (Wiley, 2006).

The Schemes below provide general guidance in connection with preparing the compounds of the invention. One skilled in the art would understand that the preparations shown in the Schemes can be modified or optimized using general knowledge of organic chemistry to prepare various compounds of the invention.

Compounds of the invention, or any subformulas as disclosed herein and certain intermediates can be prepared, for example, using a process as illustrated in Schemes 1A-5B. The variables employed in the Schemes below are as defined throughout the specification.

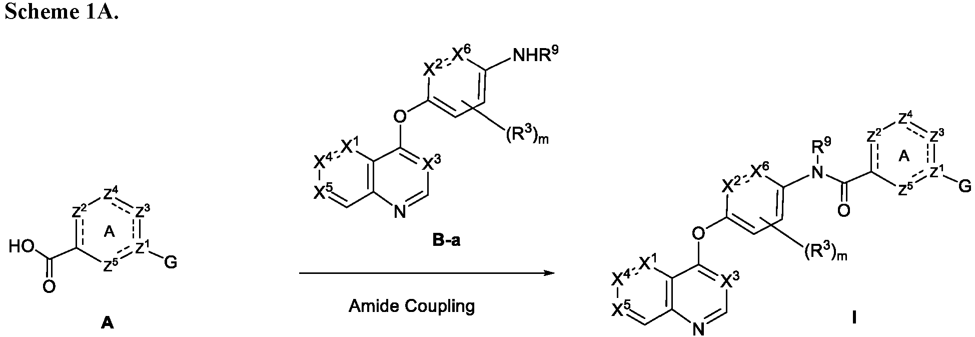

As shown in Scheme 1A, a compound of formula (I) can be synthesized from carboxylic acid **A** and aniline **B-a** by standard methods to form amide bonds using coupling agents appropriate for this transformation that are well known in the art such as HATU in the presence of a base such as DIEA in organic solvents such as DMF at room or elevated temperatures.

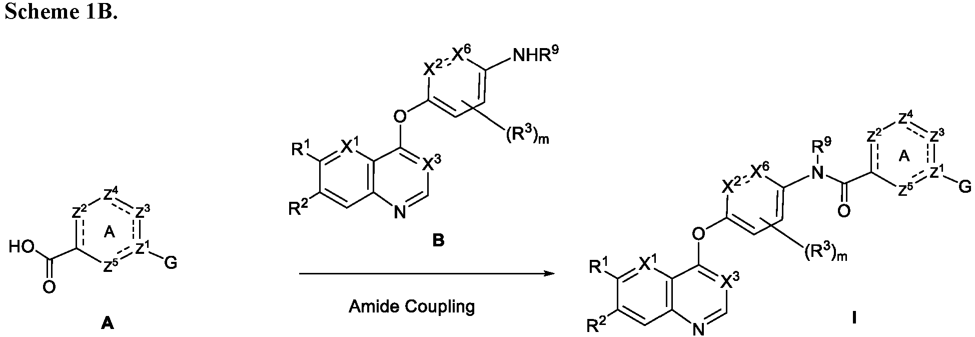

As shown in Scheme 1B, a compound of formula (I) can be synthesized from carboxylic acid **A** and aniline **B** by standard methods to form amide bonds using coupling agents appropriate for this transformation that are well known in the art such as HATU in the presence of a base such as DIEA in organic solvents such as DMF at room or elevated temperatures.

In some embodiments, provided is a process for preparing a compound of formula (I), comprising contacting a compound of formula A with a compound of formula B-b, under conditions suitable to provide a compound of formula (I).

In some embodiments, provided is a process for preparing a compound of formula (I), comprising contacting a compound of formula A with a compound of formula B, under conditions suitable to provide a compound of formula (I).

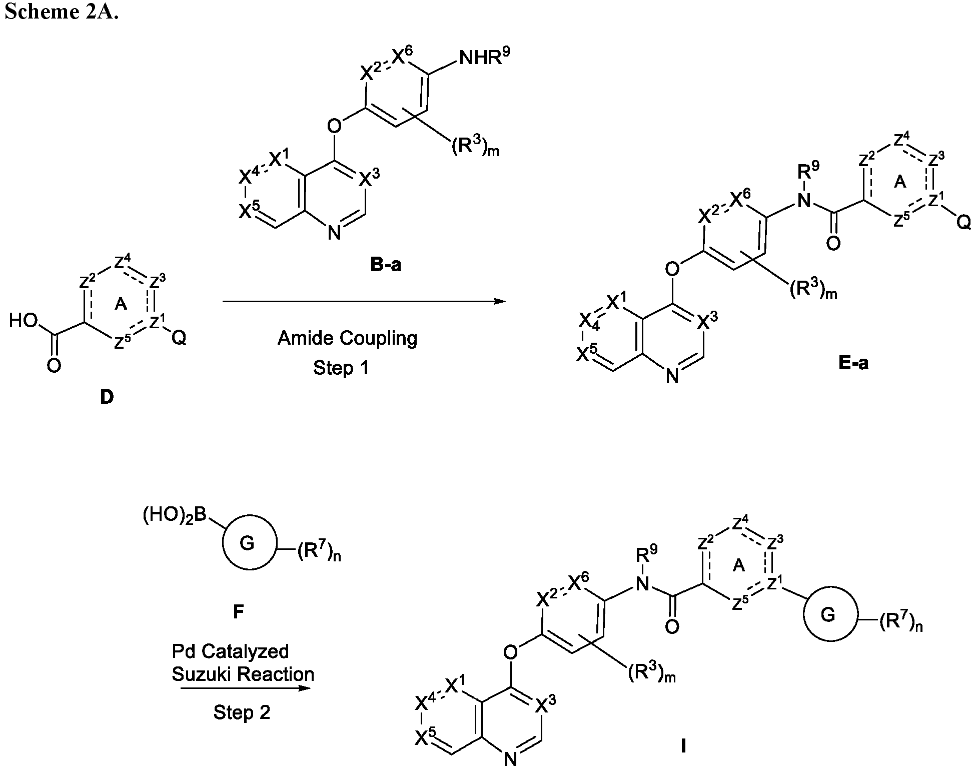

As shown in Scheme 2A, a compound of formula (I) can be made from a two-step process starting from bromocarboxylic acid **D,** where Q is a leaving group (including Cl, Br, I, triflate and the like), and aniline **B-a** which are coupled together by standard methods to form amide bonds using coupling agents appropriate for this transformation that are well known in the art such as HATU in the presence of a base such as DIEA in organic solvents such as DMF at room or elevated temperatures to form a compound of formula E-a. In a second step, compounds of formula **E-a** can be converted to compounds of formula **(I)** by coupling with boron compounds of the formula **F** using coupling chemistry known to those skilled in the art. Typical procedures to accomplish this type of coupling involve the use palladium-containing complexes as a catalyst in the presence of an inorganic base such as tripotassium phosphate in a mixture of water and a water-miscible solvent such as dioxane.

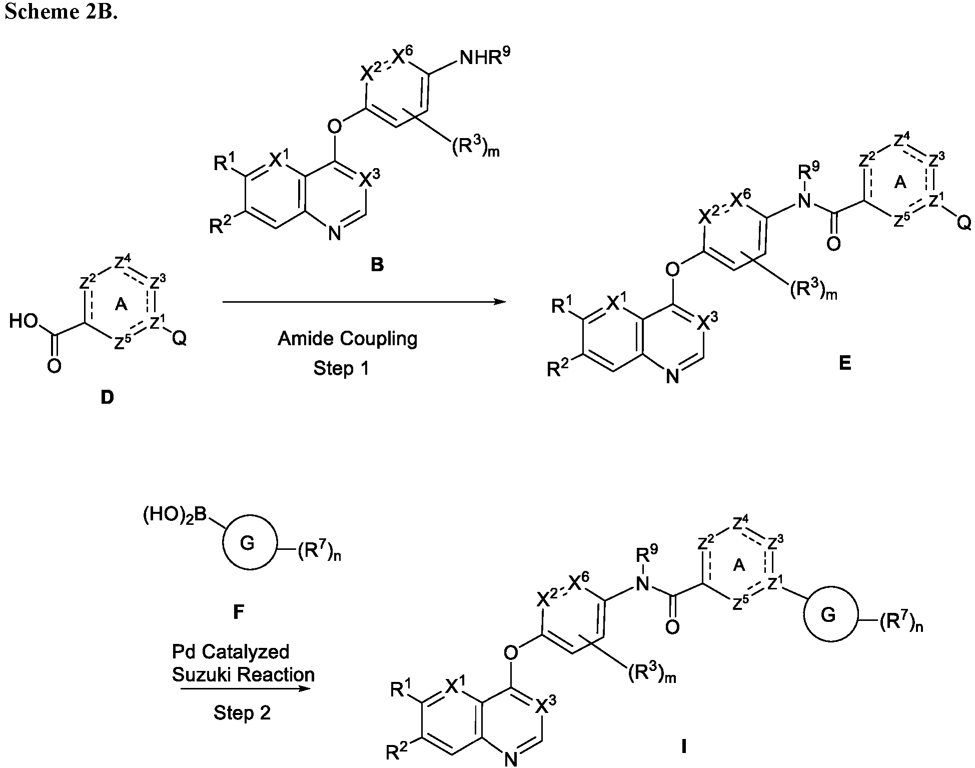

As shown in Scheme 2B, a compound of formula **(I)** can be made from a two-step process starting from bromocarboxylic acid **D,** where Q is a leaving group (including Cl, Br, I, triflate and the like), and aniline **B** which are coupled together by standard methods to form amide bonds using coupling agents appropriate for this transformation that are well known in the art such as HATU in the presence of a base such as DIEA in organic solvents such as DMF at room or elevated temperatures to form a compound of formula **E.** In a second step, compounds of formula **E** can be converted to compounds of formula **(I)** by coupling with boron compounds of the formula **F** using coupling chemistry known to those skilled in the art. Typical procedures to accomplish this type of coupling involve the use palladium-containing complexes as a catalyst in the presence of an inorganic base such as tripotassium phosphate in a mixture of water and a water-miscible solvent such as dioxane.

In some embodiments, provided is a process for preparing a compound of formula (I), comprising:
contacting a compound of formula D with a compound of formula B-a, under conditions suitable to provide a compound of formula E-a; and
contacting a compound of formula E-a with a compound of formula F, under conditions suitable to provide a compound of Formula (I).

In some embodiments, provided is a process for preparing a compound of formula (I), comprising:
contacting a compound of formula D with a compound of formula B, under conditions suitable to provide a compound of formula E; and
contacting a compound of formula E with a compound of formula F, under conditions suitable to provide a compound of Formula (I).

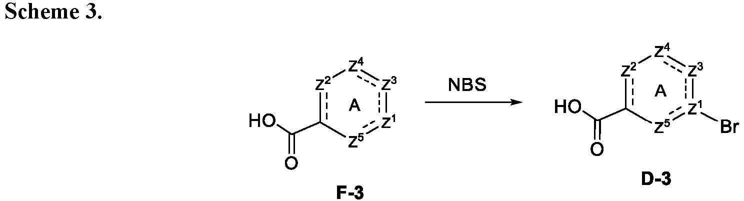

As shown in Scheme 3, a compound of formula **D-3** (Q = Br) can be prepared from carboxylic acid **F-3** through treatment with NBS in an appropriate solvent typically at room temperature.

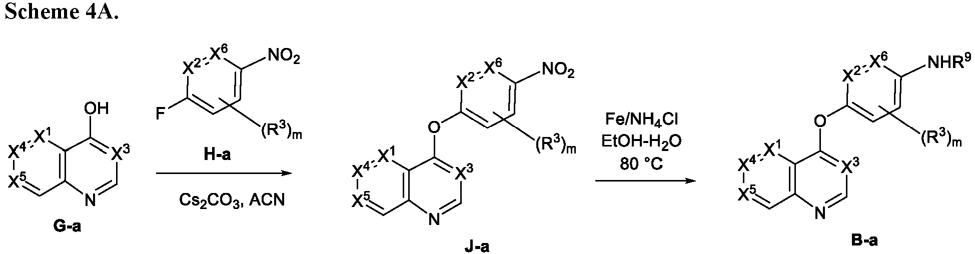

As shown in Scheme 4A, a compound of formula **J-a** can be prepared by reacting a compound of formula **G-a** with a compound of formula **H-a** in the presence of a base such as cesium carbonate in an appropriate organic solvent, typically at room temperature. A compound of formula **B-a** can be made from a compound of formula **J-a** by reducing the nitro group with a mixture of ammonium chloride and iron typically in a solvent mixture of water and an alcohol such as methanol or ethanol at elevated temperatures.

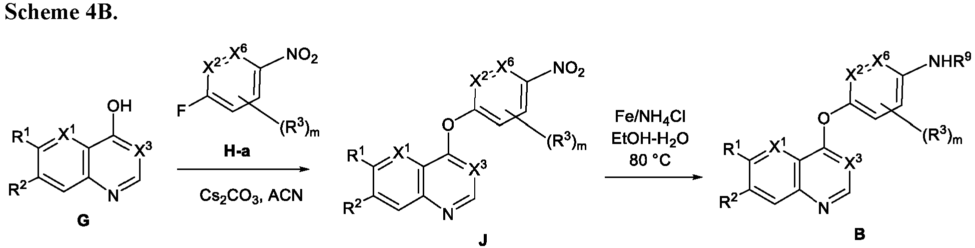

As shown in Scheme 4B, a compound of formula **J** can be prepared by reacting a compound of formula **G** with a compound of formula **H** in the presence of a base such as cesium carbonate in an appropriate organic solvent, typically at room temperature. A compound of formula **B** can be made from a compound of formula **J** by reducing the nitro group with a mixture of ammonium chloride and iron typically in a solvent mixture of water and an alcohol such as methanol or ethanol at elevated temperatures.

In some embodiments, provided is a process for preparing a compound of formula B-a, comprising:
contacting a compound of formula G-a with a compound of formula H-a, under conditions suitable to provide a compound of formula J-a; and
reducing a compound of formula J-a under conditions suitable to provide a compound of formula B-a.

In some embodiments, provided is a process for preparing a compound of formula B, comprising:
contacting a compound of formula G with a compound of formula H, under conditions suitable to provide a compound of formula J; and
reducing a compound of formula J under conditions suitable to provide a compound of formula B.

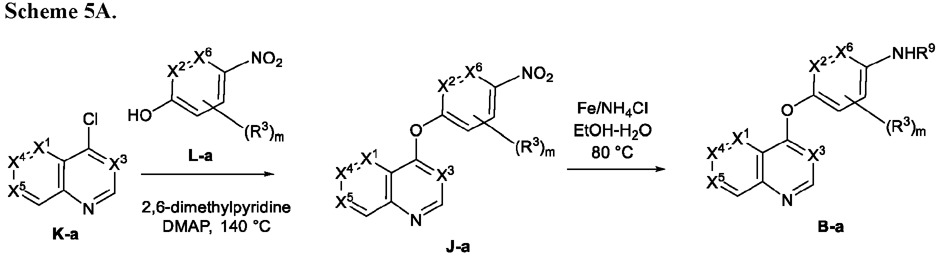

As shown in Scheme 5A, a compound of formula **J-a** can also be synthesized by reacting a compound of formula **K-a** with a compound of formula **L-a** in an appropriate solvent such as 2,6-dimethylpyridine in the presence of a catalytic amount of dimethylaminopyridine at elevated temperatures. A compound of formula **B-a** can be prepared from a compound of formula **J-a** by reducing the nitro group with a mixture of ammonium chloride and iron typically in a solvent mixture of water and an alcohol such as methanol or ethanol at elevated temperatures.

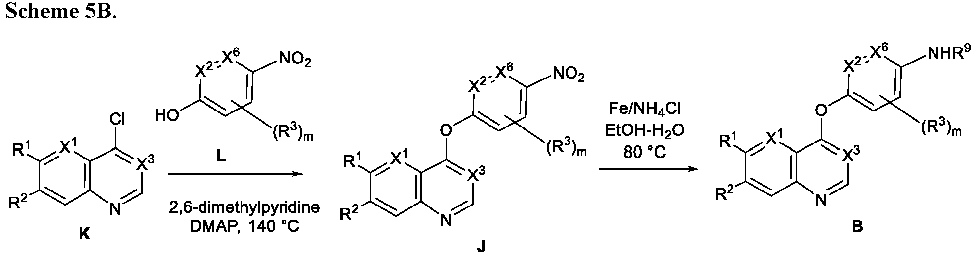

As shown in Scheme 5B, a compound of formula **J** can also be synthesized by reacting a compound of formula **K** with a compound of formula L in an appropriate solvent such as 2,6-dimethylpyridine in the presence of a catalytic amount of dimethylaminopyridine at elevated temperatures. A compound of formula **B** can be prepared from a compound of formula **J** by reducing the nitro group with a mixture of ammonium chloride and iron typically in a solvent mixture of water and an alcohol such as methanol or ethanol at elevated temperatures.

In some embodiments, provided is a process for preparing a compound of formula B-a, comprising:
contacting a compound of formula K-a with a compound of formula L-a, under conditions suitable to provide a compound of formula J-a; and
reducing a compound of formula J-a under conditions suitable to provide a compound of formula B-a.

In some embodiments, provided is a process for preparing a compound of formula B, comprising:
contacting a compound of formula K with a compound of formula L, under conditions suitable to provide a compound of formula J; and
reducing a compound of formula J under conditions suitable to provide a compound of formula B.

### EXAMPLES

The following examples are provided for the purpose of further illustration and are not intended to limit the scope of the claimed invention.

The following examples are included to demonstrate specific embodiments of the disclosure. It should be appreciated by those of skill in the art that the techniques disclosed in the examples which follow represent techniques to function well in the practice of the disclosure, and thus can be considered to constitute specific modes for its practice. However, those of skill in the art should, in light of the present disclosure, appreciate that many changes can be made in the specific embodiments which are disclosed and still obtain a like or similar result without departing from the spirit and scope of the disclosure.

### Synthetic Examples

### General Procedure A1: 4-((1,5-Naphthyridin-4-yl)oxy)anilines

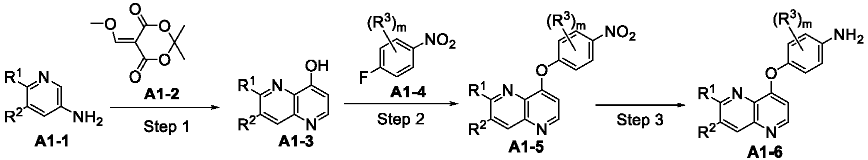

**Step 1:** A mixture of Compound **A1-1** (32 mmol, 1 eq) and Compound **A1-2** (5.92 g, 32 mmol, 1 eq) in toluene (50 mL, 1.5-1.6 mL/mmol of **A1-1** used) was stirred at 105 °C for 1.5 h and then cooled to room temperature. Hexanes (50 mL, 1.5-1.6 mL/mmol of **A1-1** used) was added and the suspension was filtered. This material was mixed with Ph₂O (50 mL, 1.5-1.6 mL/mmol of **A1-1** used) and the resulting mixture was stirred at 220-230 °C for 1 h, cooled to room temperature and poured into Et₂O (100 mL, 3.0-3.2 mL/mmol of **A1-1** used). The resulting suspension was filtered, washed with Et₂O, and dried to give Compound **A1-3.** Compound **A1-2** can easily be generated from heating 2,2-dimethyl-1,3-dioxane-4,6-dione (1 eq) in trimethyl orthoformate (10 eq) at 110°C for 1-2 h.

**Step 2:** A mixture of Compound **A1-3** (4.8 mmol, 1 eq), Compound **A1-4** (6.8 mmol, 1.4 eq), and Cs₂CO₃ (6.6 g, 20 mmol, 4.2 eq) in acetonitrile (ACN) (20 mL, 4.2 mL/mmol of **A1-3** used) was stirred at room temperature overnight. EtOAc (80 mL, 16-17 mL/mmol of **A1-3** used) was added and the resulting mixture filtered. The filtrate was evaporated, and residue purified by silica gel column chromatography to give Compound **A1-5.**

**Step 3:** A mixture of Compound **A1-5** (1.8 mmol, 1 eq), NH₄Cl (500 mg, 9.3 mmol, 5.2 eq), and Fe powder (260 mg, 4.6 mmol, 2.6 eq) in 4:1 MeOH:water (13.8 mL/mmol of **A1-5** used) was refluxed for 1 h and then cooled to room temperature. The resulting mixture was filtered through Celite and the filtrate concentrated to remove MeOH. To the residue was added aq saturated NaHCO₃ (6 mL, 3.3 mL/mmol of **A1-5** used) and the resulting aqueous mixture was extracted with EtOAc. The organic extract was dried over anhyd. Na₂SO₄ and evaporated give Compound **A1-6.**

### Example of General Procedure A1: 4-((6,7-Dimethoxy-1,5-naphthyridin-4-yl)oxy)-3-fluoroaniline (A1-10)

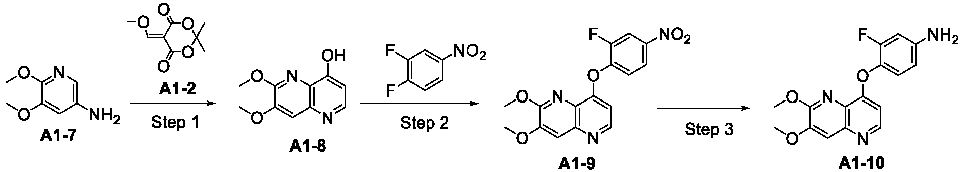

**Step 1: 6,7-Dimethoxy-1,5-naphthyridin-4-ol (A1-8):** A mixture of 2,2-dimethyl-1,3-dioxane-4,6-dione (2.7 g, 18.7 mmol, 1 eq) in trimethyl orthoformate (19.6 g, 185 mmol, 20.3 mL, 10 eq) was stirred at 110 °C for 1.5 h to form a yellow solution of Compound **A1-2.** Compound **A1-7** (2.8 g, 18.5 mmol, 1 eq) was added to the above solution and the mixture was stirred at 110 °C for 0.5 h. The resulting brown suspension was filtered and the solid washed with petroleum ether (2 x 30 mL) and dried under vacuum to give the intermediate 5-[(E)-(5,6-dimethoxy-3-pyridyl)iminomethyl]-2,2-dimethyl-1,3-dioxane-4,6-dione. A portion of this compound (2.2 g, 7.1 mmol) in Ph₂O (25 mL) was stirred at 230 °C for 0.5 h. Upon cooling to room temperature, to the reaction mixture was added methyl *tert*-butyl ether (MTBE) (100 mL) and the mixture stirred for 5 min and then filtered. The resulting solid was washed with MTBE (2 x 30 mL) and dried under vacuum to give Compound A1-8. ¹H NMR (400 MHz, DMSO-*d*₆) δ 11.62 (br s, 1H), 7.93-7.79 (m, 1H), 7.27 (s, 1H), 6.28-6.08 (m, 1H), 3.94 (s, 3H), 3.88 (s, 3H).

**Step 2: 8-(2-Fluoro-4-nitrophenoxy)-2,3-dimethoxy-1,5-naphthyridine (A1-9):** To mixture of Compound **A1-8** (2.1 g, 10.2 mmol, 1 eq) and 1,2-difluoro-4-nitrobenzene (1.6 g, 10.2 mmol, 1.13 mL, 1 eq) in ACN (50 mL) was added Cs₂CO₃ (6.6 g, 20.4 mmol, 2 eq) and the resulting mixture was stirred at room temperature for 15 h. The reaction mixture was filtered, and any solids washed with ACN (2 x 30 mL) and the resulting filtrate was concentrated under reduced pressure. The residue was purified by column chromatography on silica gel (petroleum ether:EtOAc) to give Compound **A1-9.** ¹H NMR (400 MHz, CDCl₃) δ 8.70 (d, 1H), 8.12 (dd, 1H), 8.01-7.96 (m, 1H), 7.53 (s, 1H), 7.17 (d, 1H), 7.03 (dd, 1H), 4.02 (s, 3H), 3.75 (s, 3H).

**Step 3: 4-((6,7-Dimethoxy-1,5-naphthyridin-4-yl)oxy)-3-fluoroaniline (A1-10):** To a mixture of Compound **A1-9** (1.4 g, 3.9 mmol, 1 eq) in EtOH (20 mL) and water (5 mL) was added Fe powder (1.1 g, 19 mmol, 5 eq) and NH₄Cl (2.1 g, 39 mmol, 10 eq) and the resulting mixture was stirred at 80 °C for 15 h. The reaction mixture was filtered, and the filter cake was washed with hot MeOH (2 x 30 mL) and the filtrate concentrated under reduced pressure. The resulting solid was washed with water (2 x 50 mL) and dried under vacuum to give Compound **A1-10,** which was used in subsequent reactions without further purification. MS of C₁₆H₁₄FN₃O₃: *m*/*z*: 315.9 (MH+).

The following additional intermediates were made following **General Procedure A1** for the synthesis of 4-((1,5-Naphthyridin-4-yl)oxy)anilines **A1-6:**

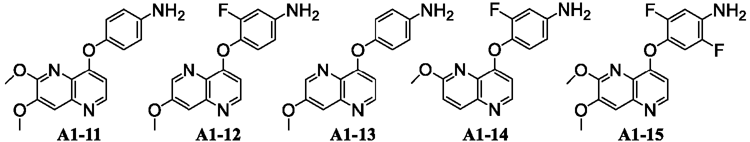

**4-((6,7-Dimethoxy-1,5-naphthyridin-4-yl)oxy)aniline (A1-11):** For the synthesis of Compound **A1-11,** in **General Procedure A1,** Step 1, Compound **A1-1** = 5,6-dimethoxypyridin-3-amine and, in Step 2, Compound **A1-4** = 1-fluoro-4-nitrobenzene. MS for C₁₆H₁₅N₃O₃: *m*/*z* 298 (MH+).

**3-Fluoro-4-((7-methoxy-1,5-naphthyridin-4-yl)oxy)aniline (A1-12):** For the synthesis of Compound **A1-12,** in **General Procedure A1,** Step 1, Compound **A1-1** = 5-methoxypyridin-3-amine and, in Step 2, Compound **A1-4** = 1,2-difluoro-4-nitrobenzene.. MS for C₁₅H₁₂FN₃O₂: *m*/*z* 286 (MH+).

**4-((7-Methoxy-1,5-naphthyridin-4-yl)oxy)aniline (A1-13):** For the synthesis of Compound **A1-13,** in **General Procedure A1,** Step 1, Compound **A1-1** = 5-methoxypyridin-3-amine and, in Step 2, Compound **A1-4** = 1-fluoro-4-nitrobenzene. MS for C₁₅H₁₃N₃O₂: *m*/*z* 268 (MH+).

**3-Fluoro-4-((6-methoxy-1,5-naphthyridin-4-yl)oxy)aniline (A1-14):** For the synthesis of Compound **A1-14,** in **General Procedure A1,** Step 1, Compound **A1-1** = 6-methoxypyridin-3-amine and, in Step 2, Compound **A1-4** = 1,2,4-trifluoro-5-nitrobenzene. MS for C₁₅H₁₂FN₃O₂: m/z 286.0 (MH+).

**4-((6,7-Dimethoxy-1,5-naphthyridin-4-yl)oxy)-2,5-difluoroaniline (A1-15):** For the synthesis of Compound **A1-15,** in **General Procedure A1,** Step 1, Compound **A1-1** = 5,6-dimethoxypyridin-3-amine and, in Step 2, Compound **A1-4** = 2,4,5-trifluoroaniline. MS for C₁₆H₁₃F₂N₃O₃: *m*/*z* 334.0 (MH+).

### Procedure A2: Alternative Method for 4-((1,5-Naphthyridin-4-yl)oxy)anilines

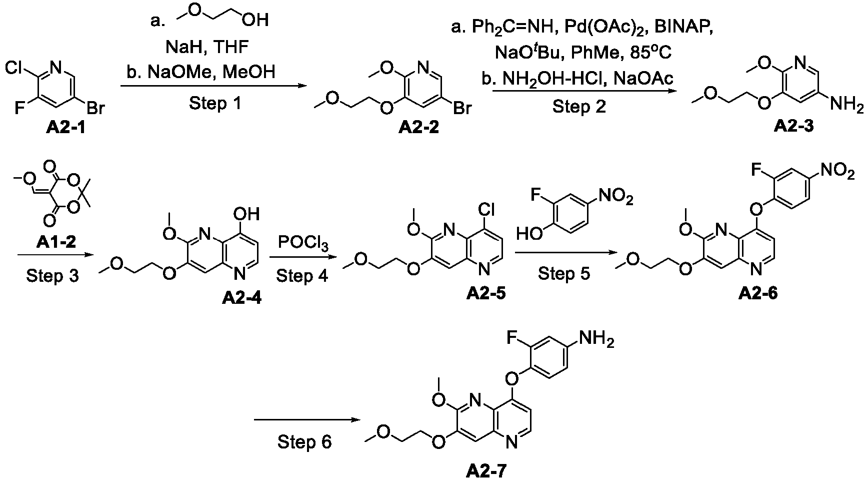

**Step 1: 5-Bromo-2-methoxy-3-(2-methoxyethoxy)pyridine (A2-2):** 2-Methoxyethanol (8.68 g, 114 mmol, 1.2 eq) was slowly added to a cooled mixture of 60% NaH (4.56 g, 114. mmol, 1.2 eq) in THF (200 mL) at 0 °C. A solution of Compound **A2-1** (20 g, 95 mmol, 1 eq) in THF (20 mL) was then added and the resulting mixture was stirred at 25 °C for 12 h. The reaction mixture was diluted with water (200 mL) and extracted with EtOAc (3 x 200 mL). The combined organic layers were washed with aq saturated NaCl (3 x 10 mL), dried over anhyd. Na₂SO₄ and concentrated under vacuum to give 5-bromo-2-chloro-3-(2-methoxyethoxy)pyridine as a light yellow solid. The crude 5-bromo-2-chloro-3-(2-methoxyethoxy)pyridine was dissolved in MeOH (300 mL) to which was added NaOMe (25.3 g, 469 mmol, 5 eq). The reaction mixture was heated to 80 °C with stirring for 12 h. The reaction mixture was concentrated to remove the solvent and the resulting mixture was diluted with water (200 mL) and extracted with EtOAc (3 x 200 mL). The combined organic layers were washed with aq saturated NaCl (20 mL), dried over anhyd. Na₂SO₄ and concentrated under vacuum to give crude Compound **A2-2** as a light yellow solid (25 g, 99% yield) which was used for next step without purification. ¹H NMR (400 MHz, DMSO-*d₆*) δ = 7.80 (d, 1H), 7.51 (d, 1H), 4.14 (dd, 2H), 3.85 (s, 3H), 3.65 (dd, 2H), 3.29 (s, 3H); MS for C₉H₁₂BrNO₃: *m*/*z* 261.9 (MH+).

**Step 2: 6-Methoxy-5-(2-methoxyethoxy)pyridin-3-amine (A2-3):** A mixture of diphenylmethanimine (41.6 g, 229 mmol, 2.4 eq), Compound **A2-2** (25 g, 95 mmol, 1 eq), Pd(OAc)₂ (2.6 g, 11.4 mmol, 0.12 eq), *rac*-BINAP (10.7 g, 17.2 mmol, 0.18 eq) and KOtBu (19.8 g, 176 mmol, 1.85 eq) in toluene (300 mL) was stirred at 85 °C for 12 h under an atmosphere of nitrogen. The reaction was washed with water (300 mL) and extracted with EtOAc (3 x 300 mL). The combined organic extracts were concentrated to dryness. The resulting brown oil was dissolved in MeOH (300 mL) to which was added NH₂OH-HCl (13.4 g, 193 mmol, 2 eq) and NaOAc (20.6 g, 251 mmol, 2.6 eq). The mixture was stirred at 25 °C for 12 h. The mixture was concentrated under vacuum and the residue was dissolved in DCM(100 mL). The resulting mixture was treated with aq 2 M HCl solution until pH = 3-4. The aqueous layer was separated. Aq saturated NaHCO₃ (200 mL) was added with the resulting pH > 7. The mixture was extracted with DCM (3 x 300 mL). The combined organic extracts were dried over anhyd. Na₂SO₄ and concentrated under vacuum to give Compound **A2-3** as a brown oil (10 g, 52% yield) which was used for next step without purification. MS for C₉H₁₄N₂O₃: *m*/*z* 198.9 (MH+).

**Step 3: 6-Methoxy-7-(2-methoxyethoxy)-1,5-naphthyridin-4-ol (A2-4):** Compound **A2-4** was synthesized from Compound **A2-3** using Step 1 of **General Procedure A1.** ¹H NMR (400 MHz, DMSO-*d₆*) 11.59 (br s, 1H), 7.77 (br d, 1H), 7.25 (br s, 1H), 6.09 (br d, 1H), 4.20 (br s, 2H), 4.02 (s, 3H), 3.77 - 3.70 (m, 2H), 3.34 (s, 3H).

**Step 4: 8-Chloro-2-methoxy-3-(2-methoxyethoxy)-1,5-naphthyridine (A2-5):** To a solution of Compound **A2-4** (8.0 g, 32 mmol, 1 eq) in ACN (40 mL) was added POCl₃ (19.6 g, 128 mmol, 4 eq) and the reaction mixture was stirred at 90 °C for 2 h. After cooling to ambient temperature, the reaction mixture was concentrated and then quenched with water (100 mL). The pH was adjusted to 7 with aq NaHCO₃ (100 mL). The resulting suspension was filtered and the solid was dried to give Compound **A2-5** as a brown solid (7.3 g, 85% yield) which was used in the next step without further purification.

**Step 5: 8-(2-Fluoro-4-nitrophenoxy)-2-methoxy-3-(2-methoxyethoxy)-1,5-naphthyridine (A2-6):** To a mixture of Compound **A2-5** (3.3 g, 12.3 mmol, 1 eq) and 2-fluoro-4-nitrophenol (3.1 g, 19.6 mmol, 1.6 eq) in Ph₂O (30 mL) was added DIEA (4.76 g, 36.8 mmol, 3 eq) and the resulting mixture was heated to 170 °C with stirring for 3 h. The reaction mixture was cooled and petroleum ether (100 mL) was added. The resulting suspension was filtered. The solid was dried and then purified by flash silica gel chromatography (0~80% EtOAc/petroleum ether) to give Compound **A2-6** as a yellow solid (2.6 g, 54% yield). MS for C₁₈H₁₆FN₃O₆: *m*/*z* 390.0 (MH+).

**Step 6: 3-Fluoro-4-((6-methoxy-7-(2-methoxyethoxy)-1,5-naphthyridin-4-yl)oxy)aniline (A2-7):** Compound **A2-7** was synthesized from Compound **A2-6** using Step 3 of **General Procedure A1.** ¹H NMR (400 MHz, DMSO-d6) δ 8.47 (d, 1H), 7.64 (s, 1H), 7.05 (t, 1H), 6.61 - 6.50 (m, 2H), 6.46 (dd, 1H), 5.47 (s, 2H), 4.31 (t, 2H), 4.04 (s, 3H), 3.76 (t, 2H), 3.34 (s, 3H); MS for C₁₈H₁₈FN₃O₄: m/z 360.2 (MH+).

The following additional intermediates were made following **Procedure A2** for the alternative synthesis of 4-((1,5-Naphthyridin-4-yl)oxy)anilines:

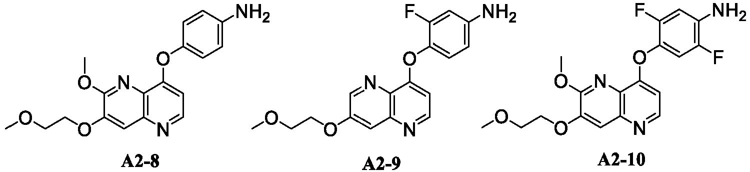

**4-((6-Methoxy-7-(2-methoxyethoxy)-1,5-naphthyridin-4-yl)oxy)aniline (A2-8):** The 2-fluoro-4-nitrophenol in step 5 was replaced with 4-nitrophenol. ¹H NMR (400 MHz, DMSO-d6) δ 8.52 - 8.39 (m, 1H), 7.75 - 7.52 (m, 1H), 7.03 - 6.47 (m, 5H), 5.12 (br s, 2H), 4.30 (br d, 2H), 4.03 (br s, 3H), 3.75 (br d, 2H), 3.36 (br s, 3H); MS for C₁₈H₁₉N₃O₄: *m*/*z* 342.2 (MH+).

**3-Fluoro-4-((7-(2-methoxyethoxy)-1,5-naphthyridin-4-yl)oxy)aniline (A2-9):** Step 1 was replaced with the following method: To a mixture of 5-bromopyridin-3-ol (2 g, 11.5 mmol, 1 eq) and 1-bromo-2-methoxy-ethane (2.4 g, 17 mmol, 11.5 eq) in DMF (20 mL) was added Cs₂CO₃ (4.9 g, 14.9 mmol, 1.3 eq). The mixture was stirred at 80 °C for 12 h. The reaction mixture was diluted with EtOAc (10 mL) and washed with water (5 x 30 mL), washed with aq saturated NaCl (30 mL), dried over anhyd. Na₂SO₄ and concentrated under reduced pressure to give 3-bromo-5-(2-methoxyethoxy)pyridine as a yellow solid (4.2 g, crude) which was used to replace Compound **A2-2** in Step 2. ¹H NMR (400 MHz, DMSO-*d₆*) δ 8.72 (d, 1H), 8.63 (d, 1H), 7.77 (d, 1H), 6.93 (d, 2H), 6.68 (d, 2H), 6.58 (d, 1H), 5.21 (br s, 2H), 4.35 (dd, 2H), 3.80 - 3.74 (m, 2H), 3.35 (s, 3H); MS for C₁₇H₁₇N₃O₃: *m*/*z* 312.1 (MH+).

**2,5-Difluoro-4-((6-methoxy-7-(2-methoxyethoxy)-1,5-naphthyridin-4-yl)oxy)aniline (A2-10):** The 2-fluoro-4-nitrophenol in step 5 was replaced with 2,5-difluoro-4-nitrophenol. ¹H NMR (400 MHz, DMSO-*d₆*) δ 8.50 (d, 1H), 7.65 (br s, 1H), 7.18-7.31 (m, 1H), 6.72-6.82 (m, 1H), 6.69 (br s, 1H), 5.49 (br s, 2H), 4.31 (br s, 2H), 4.02 (br s, 3H), 3.75 (br s, 2H), 3.33 (br s, 3H).; MS for C₁₈H₁₇F₂N₃O₄: *m*/*z* 378.2 (MH+).

### General Procedure B1: 4-(Quinolin-4-yloxy)anilines

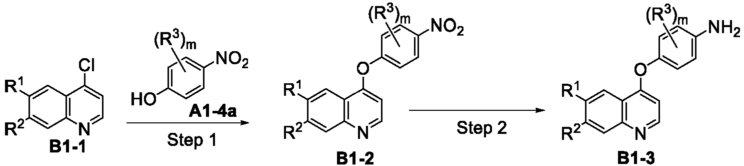

**Step 1:** A mixture of Compound **B1-1** (1 eq) and Compound **A1-4a** (1.2-1.4 eq) in an appropriate solvent such as, but not limited to, 2,6-dimethylpyridine or diphenyl ether (1.1-2.2 mL/mmol of **B1-1** used) was stirred at 140 °C for typically 36-66 h. DMAP (0.2 eq) can also be optionally added as a catalyst. Upon completion of the reaction as monitored by LC-MS and/or TLC, the reaction mixture was allowed to cool to room temperature and typically worked up by one of the following methods or a similar variation. Method 1: MeOH (0.9 mL/mmol of **B1-1** used) was added, followed by aq 6.5% K₂CO₃ (1.4 mL/mmol of **B1-1** used). The resulting mixture was stirred at 0 °C for 2 h. The resulting mixture was filtered and washed with water (4.5 mL/mmol of **B1-1** used) to give Compound **B1-2.** Method 2: The mixture was diluted with MTBE (2.2 mL/mmol of **B1-1** used) and filtered. The resulting solid was washed with MTBE (0.4 mL/mmol of **B1-1** used) and dried under vacuum to give Compound **B1-2.** Regardless of the method of work up, the crude Compound **B1-2** was generally used in subsequent reactions without further purification.

**Step 2:** To a mixture of Compound **B1-2** (1 eq) in EtOH (4.5-6.5 mL/mmol of **B1-2**) and water (1.1-1.3 mL/mmol of **B1-2)** was added Fe powder (5.0 eq) and NH₄Cl (8-10 eq). The mixture was stirred at 85 °C for 3-4 h. Upon completion of the reaction as monitored by LC-MS and/or TLC, the reaction mixture was allowed to cool to room temperature and typically worked up by one of the following methods or a similar variation. Method 1: The reaction was filtered, and the filtrate was dried over anhyd. Na₂SO₄ and concentrated to give crude product. To this crude product was added EtOAc (25 mL/mmol of **B1-2** used) and DCM (25 mL/mmol of **B1-2** used). The resulting mixture was filtered, and the filtrate was concentrated to give Compound **B1-3.** Method 2: The mixture was filtered through Celite, the filtrate was concentrated under vacuum and the residue was dissolved in EtOAc (11.2 mL/mmol of **B1-2** used). The organic layer was washed with aq saturated NaHCO₃ (6.7 mL/mmol of **B1-2** used), washed with water (6.7 mL/mmol of **B1-2** used), washed with aq saturated NaCl (6.7 mL/mmol of B1-2 used), dried over anhyd. Na₂SO₄ and concentrated under vacuum to give Compound **B1-3.** Regardless of the method of work up, the crude Compound **B1-3** was generally used in subsequent reactions without further purification.

### Example of General Procedure B1: 4-((6,7-Dimethoxyquinolin-4-yl)oxy)-3-fluoroaniline (B1-6)

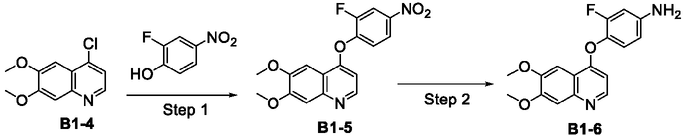

**Step 1: 4-(2-Fluoro-4-nitrophenoxy)-6,7-dimethoxyquinoline (B1-5):** A suspension of Compound **B1-4** (10 g, 45 mmol, 1 eq) and 2-fluoro-4-nitro-phenol (8.4 g, 54 mmol, 1.2 eq) in Ph₂O (100 mL) was heated and stirred at 140 °C for 66 h. After cooling to room temperature, the mixture was diluted with MTBE (100 mL) and filtered. The filter cake was washed with MTBE (20 mL) and dried under vacuum to give Compound **B1-5,** which was used in subsequent steps without further purification. ¹H NMR (400 MHz, DMSO-*d₆*) δ 8.89 (d, 1H), 8.56 (dd, 1H), 8.35-8.27 (m, 1H), 7.89 (t, 1H), 7.79-7.71 (m, 2H), 7.17 (d, 1H), 4.04 (d, 6H); MS for C₁₇H₁₃FN₂O₅: *m*/*z* 344.9 (MH+).

**Step 2: 4-((6,7-Dimethoxyquinolin-4-yl)oxy)-3-fluoroaniline (B1-6):** Fe powder (12.5 g, 223 mmol, 5 eq) was added to a mixture of Compound **B1-5** (16.2 g, 45 mmol, 1 eq) and NH₄Cl (23.9 g, 447 mmol, 10 eq) in EtOH (200 mL) and water (50 mL). The mixture was heated and stirred at 85 °C for 3.5 h. After cooling to room temperature, the mixture was filtered through a pad of Celite. The filtrate was concentrated under vacuum and the residue was dissolved in EtOAc (500 mL). The organic layer was washed with aq NaHCO₃ (300 mL), washed with water (300 mL), washed with aq saturated NaCl (300 mL), dried over anhyd. Na₂SO₄ and concentrated under vacuum to give Compound **B1-6,** which was used in subsequent reactions without further purification. MS for C₁₇H₁₅FN₂O₃: *m*/*z* 315.0 (MH+).

The following additional intermediate was made following **General Procedure B1** for the synthesis of 4-(quinolin-4-yloxy)anilines **B1-3:**

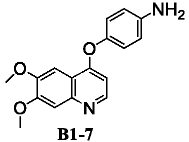

**4-((6,7-Dimethoxyquinolin-4-yl)oxy)aniline (B1-7):** For the synthesis of Compound **B1-7,** in **General Procedure B1,** Step 1, Compound **B1-1** = 4-chloro-6,7-dimethoxyquinoline and Compound **A1-4a** = 4-nitrophenol. MS for C₁₇H₁₆N₂O₃: *m*/*z* 297.2 (MH+).

### Procedure C1: 4-(Pyrido[3,2-d]pyrimidin-4-yloxy)anilines

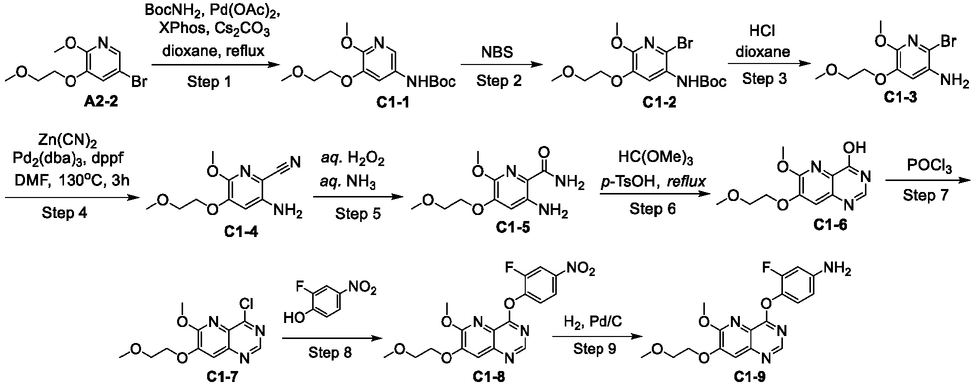

**Step 1: tert-Butyl (6-methoxy-5-(2-methoxyethoxy)pyridin-3-yl)carbamate (C1-1):** Cs₂CO₃ (12.1 g, 37 mmol, 2.1 eq) was added to a mixture of Compound **A2-2** (6.13 g, 18 mmol, 1.0 eq), NH₂Boc (3.33 g, 28 mmol, 1.6 eq), Pd(OAc)₂ (211 mg, 0.94 mmol, 0.05 eq) and XPhos (1.0 g, 2.1 mmol, 0.12 eq) in anhyd. 1,4-dioxane (80 mL) under an atmosphere of N₂. The resulting mixture was heated to 120 °C under N₂ with stirring for 36 h. The reaction mixture was partitioned between EtOAc (180 mL) and water (60 mL). The organic phase was washed with aq saturated NaCl (2 x 65 mL), dried over anhyd. Na₂SO₄ and concentrated under reduced pressure. The crude product was purified by flash silica gel chromatography (21-31% EtOAc in petroleum ether) to give Compound **C1-1** as a red gum (3.73 g, 70% yield). ¹H NMR (400 MHz, CDCl₃) δ 7.61 (s, 1H), 7.49 (d, 1H), 6.38 (s, 1H), 4.21-4.15 (m, 2H), 3.95 (s, 3H), 3.82-3.76 (m, 2H), 3.44 (s, 3H), 1.51 (s, 9H); MS for C₁₄H₂₂N₂O₅: *m*/*z* 299.1 (MH+).

**Step 2: tert-Butyl (2-bromo-6-methoxy-5-(2-methoxyethoxy)pyridin-3-yl)carbamate (C1-2):** To a solution of **C1-1** (3.73 g, 12.4 mmol, 1.0 eq) in ACN (61 mL) was added NBS (2.34 g, 13.1 mmol, 1.06 eq) in portions at 25 °C. The resulting mixture stirred at 25 °C for 3 h. The reaction mixture was quenched with aq saturated Na₂SO₃ solution (100 mL) and extracted with EtOAc (2 x 120 mL). The combined organic extracts were washed with water (100 mL), washed with aq saturated NaCl (150 mL), dried over anhyd. Na₂SO₄ and concentrated under reduced pressure to give Compound **C1-2** as a red gum (4.58 g, 98% yield) which was used directly in the next step. ¹H NMR (400 MHz, CDCl₃) δ 8.04 (brs, 1H), 6.70 (s, 1H), 4.22-4.16 (m, 2H), 3.95 (s, 3H), 3.81-3.75 (m, 2H), 3.44 (s, 3H), 1.52 (s, 9H); MS for C₁₄H₂₁BrN₂O₅: *m*/*z* 379.1 (MH+).

**Step 3: 2-Bromo-6-methoxy-5-(2-methoxyethoxy)pyridin-3-amine (C1-3):** To a solution of Compound **C1-2** (4.58 g, 12.1 mmol, 1.0 eq) in anhyd. DCM (12 mL) was added 4 M HCl in 1,4-dioxane (28 mL, 9.23 eq) at 20 °C. The resulting mixture was stirred at 20 °C for 2 h. The resulting reaction mixture was slowly added to a solution of aq 2 M K₂CO₃ solution (80 mL) with vigorously stirring. After gas evolution ceased, the mixture was extracted with DCM, first with 150 mL and then with 90 mL. The combined organic layers were washed with aq saturated NaCl (100 mL), dried over anhyd. Na₂SO₄ and concentrated under reduced pressure to give Compound **C1-3** as a purple gum (3.14 g, 89% yield) which was used directly in the next step. MS for C₉H₁₃BrN₂O₃: *m*/*z* 279.0 (MH+).

**Step 4: 3-Amino-6-methoxy-5-(2-methoxyethoxy)picolinonitrile (C1-4):** To a solution of Compound **C1-3** (3.14 g, 10.8 mmol, 1.0 eq) in anhyd. DMF (27 mL) was added Pd₂(dba)₃ (248 mg, 0.271 mmol, 0.025 eq), dppf (239 mg, 0.43 mmol, 0.040 eq) and zinc cyanide (860 mg, 7.32 mmol, 0.68 eq). The resulting mixture was degassed and purged with N₂, then heated to 130 °C with stirring for 2.75 h. The reaction mixture was allowed to cool to ambient temperature and then concentrated under reduced pressure to remove most of the volatiles. The resulting residue was diluted with 14% aq ammonia (40 mL) and extracted with EtOAc, first with 60 mL, then with 30 mL. The combined organic layers were washed with aq saturated NaCl (50 mL), dried over anhyd. Na₂SO₄ and concentrated under reduced pressure. The crude product was purified by flash silica gel chromatography (21-41% EtOAc in petroleum ether) to give Compound **C1-4** as a red solid (2.19 g, 90% yield). MS for C₁₀H₁₃N₃O₃: m/z 224.0 (MH+).

**Step 5: 3-Amino-6-methoxy-5-(2-methoxyethoxy)picolinamide (C1-5):** Compound **C1-4** (2.19 g, 9.7 mmol, 1.0 eq) was suspended in a mixture of hydrogen peroxide (30% aqueous, 8.5 mL, 88 mmol, 9.1 eq) and aq NH₄OH (14 M, 34.0 mL, 49 eq). The resulting mixture was stirred at 25 °C for 4 h. The reaction mixture was then filtered, and the collected solid was washed with water (2 x 5 mL) and dried under vacuum to give Compound **C1-5** (2.20 g, 92% yield) which was used directly in the next step. MS for C₁₀H₁₅N₃O₄: *m*/*z* 242.0 (MH+).

**Step 6: 6-Methoxy-7-(2-methoxyethoxy)pyrido[3,2-d]pyrimidin-4-ol (C1-6):** To a suspension of Compound **C1-5** (2.20 g, 8.9 mmol, 1.0 eq) in anhyd. toluene (37.5 mL) was added triethyl orthoformate (7.5 mL,45.1 mmol, 5 eq) and *p*-toluenesulfonic acid monohydrate (153 mg, 0.80 mmol, 0.09 eq). The resulting mixture was heated to 120 °C with stirring for 5 h. The reaction mixture was concentrated under reduced pressure. The resulting residue was triturated with 4:1 petroleum ether:EtOAc (15 mL). The resulting solid was filtered and dried under high vacuum to give Compound **C1-6** as a yellow solid (1.93 g, 83% yield). ¹H NMR (400 MHz, DMSO-*d₆*) δ 12.36 (brs, 1H), 8.03 (d, 1H), 7.42 (s, 1H), 4.31-4.23 (m, 2H), 3.98 (s, 3H), 3.76-3.66 (m, 2H), 3.32 (s, 3H); MS for C₁₁H₁₃N₃O₄: *m*/*z* 252.0 (MH+).

**Step 7: 4-Chloro-6-methoxy-7-(2-methoxyethoxy)pyrido[3,2-d]pyrimidine (C1-7):** A suspension of Compound **C1-6** (1.93 g, 7.37 mmol, 1.0 eq) in phosphorus oxychloride (32.0 mL, 344 mmol, 47 eq) was heated to 120 °C with stirring for 3 h. The reaction mixture was concentrated under reduced pressure to remove most of the volatiles. The resulting residue was suspended in DCM (100 mL) and neutralized with aq saturated NaHCO₃ solution (150 mL), then extracted with DCM 2 x (50 mL×2). The combined extracts were washed with aq saturated NaCl (150 mL), dried over anhyd. Na₂SO₄ and concentrated under reduced pressure to give Compound **C1-7** as a light brown solid (1.73 g, 87% yield) which was used directly in the next step. ¹H NMR (400 MHz, CDCl₃) δ 8.88 (s, 1H), 7.42 (s, 1H), 4.39-4.30 (m, 2H), 4.22 (s, 3H), 3.94-3.84 (m, 2H), 3.48 (s, 3H); MS for C₁₁H₁₂ClN₃O₃: *m*/*z* 270.1 (MH+).

**Step 8: 4-(2-Fluoro-4-nitrophenoxy)-6-methoxy-7-(2-methoxyethoxy)pyrido[3,2-d]pyrimidine (C1-8):** A mixture of Compound **C1-7** (1.73 g, 6.41 mmol, 1.0 eq) and 2-fluoro-4-nitrophenol (1.11 g, 6.93 mmol, 1.1 eq) in o-xylene (43 mL) was heated to 137 °C with stirring for 37 h. The reaction mixture was cooled to ambient temperature and concentrated under reduced pressure. The resulting residue was triturated with 8:1 MTBE:MeOH (9 mL) for 30 min. The resulting solid was collected by filtration, washed with petroleum ether (2 x 1.5 mL) and dried under vacuum to give crude Compound **C1-8** as a yellow solid (2.72 g) crude) which was used directly in the next step without further purification. ¹H NMR (400 MHz, DMSO-*d₆*) δ 8.63 (s, 1H), 8.41 (dd, 1H), 8.27-8.20 (m, 1H), 7.84 (dd, 1H), 7.72 (s, 1H), 4.42-4.35 (m, 2H), 4.09 (s, 3H), 3.79-3.73 (m, 2H), 3.34 (s, 3H); MS for C₁₇H₁₅FN₄O₆: *m*/*z* 391.0 (MH+).

**Step 9: 3-Fluoro-4-((6-methoxy-7-(2-methoxyethoxy)pyrido[3,2-d]pyrimidin-4-yl)oxy)aniline (C1-9):** To a stainless autoclave charged with a solution of crude Compound **C1-8** (2.72 g) in THF (150 mL) was added 10% Pd/C (600 mg) under argon. The resulting mixture was degassed and purged with argon. The atmosphere was exchanged with H₂ (50 psi) 3 times. The resulting mixture was heated to 60 °C with stirring for 45 h under H₂ (50 psi). The reaction mixture was cooled down and filtered through a Celite pad. The filtrate was concentrated under reduced pressure. The resulting residue was purified by flash silica gel chromatography (41 ~ 83% EtOAc in petroleum ether) to give Compound **C1-9** as a light yellow solid (1.06 g, 46% yield over 2 steps). ¹H NMR (400 MHz, DMSO-*d₆*) δ 8.61 (s, 1H), 7.41 (s, 1H), 7.10 (t, 1H), 6.58-6.53 (m, 1H), 6.53-6.47 (m, 1H), 4.38-4.29 (m, 2H), 4.22 (s, 3H), 3.95-3.85 (m, 2H), 3.77 (brs, 2H), 3.48 (s, 3H); MS for C₁₇H₁₇FN₄O₄: *m*/*z* 361.2 (MH+).

### General Procedure D1: Halogenation

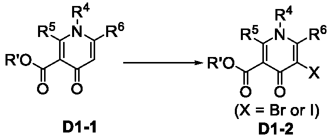

**Step 1:** Compound **D1-1** can be a carboxylic acid or an ester. Compound **D1-1** was halogenated using NBS or NIS in an appropriate organic solvent such as, but not limited to, DCE, NMP, DMF or ACN. To a solution of Compound **D1-1** (1 eq) in solvent (1.1-3.2 mL/mmol of **D1-1)** was added solid NBS (1-1.6 eq) in portions. The resulting mixture was stirred at room temperature to 80 °C (30 min to overnight). Upon completion of the reaction as monitored by LC-MS and/or TLC, in cases where heating was used, the reaction mixture was allowed to cool to room temperature. The reaction mixture was then typically worked up by one of the following methods or a very similar variation. Method 1: The reaction mixture was partitioned between an aqueous solvent such as, but not limited to, water or aq saturated NaHCO₃ and an organic solvent such as, but not limited to, EtOAc or DCM. The organic phase was further washed with aq saturated NaCl, dried over anhyd. Na₂SO₄ and concentrated to give crude brominated product **D1-2.** Method 2: To the reaction mixture was added water and the resulting mixture was stirred at room temperature for 15 min. The resulting precipitate was filtered, washed with water, and allowed to air-dry to give crude brominated product **D1-2.** Method 3: If the desired product precipitated out of the reaction mixture without diluting with water, the reaction mixture was filtered, the collected solid washed with the same solvent the reaction was run in or a similar solvent and then the solid was airdried. Regardless of the method of work up, the crude **D1-2** was generally used in subsequent reactions without further purification.

### Example of General Procedure D1: Methyl 7-bromo-8-oxo-1,3,4,8-tetrahydropyrido[2,1-c][1,4]oxazine-9-carboxylate (D1-4)

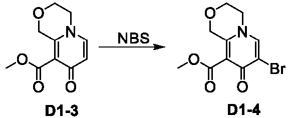

**Methyl 7-bromo-8-oxo-1,3,4,8-tetrahydropyrido[2,1-c][1,4]oxazine-9-carboxylate (D1-4):** To a solution of Compound **D1-3** (210 mg, 1.00 mmol, 1 eq) in DMF (2 mL) was added NBS (214 mg, 1.20 mmol, 1.2 eq) and the resulting mixture was stirred at 25 °C for 2 h. The resulting mixture was diluted with aq saturated NaHCO₃ (50 mL) and extracted with DCM (3 x 50 mL). The combined DCM extracts were washed with aq saturated NaCl (20 mL), dried over anhyd. Na₂SO₄ and concentrated to give Compound **D1-4** as a yellow solid (260 mg, 90% yield). MS for C₁₀H₁₀BrNO₄: *m*/*z* 289.7 (MH+). See Example 3 for the synthesis of Compound **D1-3.**

The following additional compounds were made in a similar fashion using **General Procedure D1:**

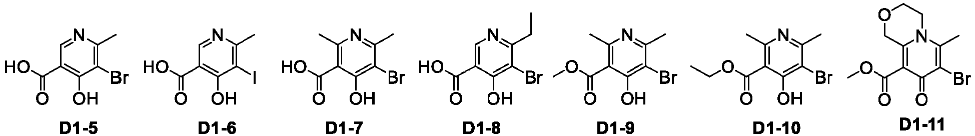

**5-Bromo-4-hydroxy-6-methylnicotinic acid (D1-5):** Compound **D1-3** was replaced with 4-hydroxy-6-methylnicotinic acid. MS for C₇H₆BrNO₃: *m*/*z* 232/234 (MH+).

**4-Hydroxy-5-iodo-6-methylnicotinic acid (D1-6):** Compound **D1-3** was replaced with 4-hydroxy-6-methylnicotinic acid and the NBS was replaced with NIS. MS for C₇H₆INO₃: *m*/*z* 279.7 (MH+).

**5-Bromo-4-hydroxy-2,6-dimethylnicotinic acid (D1-7):** Compound **D1-3** was replaced with 4-hydroxy-2,6-dimethylnicotinic acid (See **Example 4** for the synthesis of 4-hydroxy-2,6-dimethylnicotinic acid (Compound **2-1)).** MS for C₈H₈BrNO₃: *m*/*z* 246 (MH+).

**5-Bromo-6-ethyl-4-hydroxynicotinic acid (D1-8):** See the procedure used in Bannen, L., et. al. WO2021062245, which is incorporated by reference.

**Methyl 5-bromo-4-hydroxy-2,6-dimethylnicotinate (D1-9):** Compound **D1-3** was replaced with methyl 4-hydroxy-2,6-dimethylnicotinate (can be prepared by the method of Bradbury, R.H.; et al J. Med. Chem 1993, 36, 1245-54, which is incorporated by reference herein). MS for C₉H₁₀BrNO₃: *m*/*z* 260/262 (MH+).

**Ethyl 5-bromo-4-hydroxy-2,6-dimethylnicotinate (D1-10):** Compound **D1-3** was replaced with ethyl 4-hydroxy-2,6-dimethylnicotinate (can be prepared by the method of Dean, A.; et al Inorganica Chimica Acta 2011,373, 179-186, which is incorporated by reference herein). MS for C₁₀H₁₂BrNO₃: *m*/*z* 274/276 (MH+).

**Methyl 7-bromo-6-methyl-8-oxo-1,3,4,8-tetrahydropyrido[2,1-c][1,4]oxazine-9-carboxylate (D1-11):** Compound **D1-3** was replaced with Compound **26-2.** MS for C₁₁H₁₂BrNO₄: *m*/*z* 301.9/303.9 (MH+)

### General Procedure E1: Suzuki Reaction

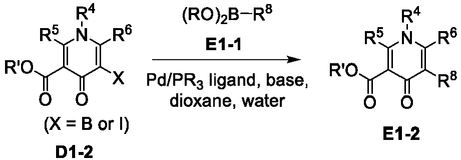

A mixture of the Compound **D1-2** (1 eq), boronic acid/ester **E1-1** (1-5 eq), catalytic Pd/PR₃ complex such as, but not limited to, Pd(PPh₃)₄ (5-10 mol%), Pd(dppf)Cl₂ (mol10-20%), Pd(Amphos)₂Cl₂ (10-20 mol%)/SPhos (1 eq), and a base (2-5 eq) such as, but not limited to, Cs₂CO₃, K₂CO₃, NaOH, Na₂CO₃, K₃PO₄, KF, in dioxane/water (1/1 to 5/1) (1.5-5 mL/mmol of **D1-2** used) was degassed and purged with nitrogen 3 times. The resulting mixture was stirred at 80 - 160 °C, with or without microwave irradiation, under an atmosphere of nitrogen until the starting material **D1-2** was consumed (0.5-20 h) as monitored by LC-MS and/or TLC. The reaction mixture was then concentrated under reduced pressure. To the resulting residue was added water and resulting mixture was washed with EtOAc, followed by DCM. The aqueous phase was acidified with aq 2 N HCl to pH 2 - 5. If a suspension resulted, the mixture was filtered, the solid washed with water and dried under reduced pressure to give crude Compound **E1-2.** If a filterable solid did not result, the acidic aqueous phase was extracted with an organic solvent such as, but not limited to, EtOAc or DCM. The combined organic extracts were dried over anhyd. Na₂SO₄ or MgSO₄ and concentrated to provide crude Compound **E1-2.** Crude Compound **E1-2** was either purified by silica gel chromatography or used directly in subsequent steps without further purification (9 - 97% yield).

### Example of General Procedure E1: Methyl 7-(cyclopent-1-en-1-yl)-8-oxo-1,3,4,8-tetrahydropyrido[2,1-c][1,4]oxazine-9-carboxylate (E1-3)

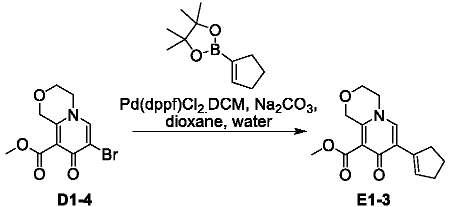

Methyl **7-(cyclopent-1-en-1-yl)-8-oxo-1,3,4,8-tetrahydropyrido[2,1-c][1,4]oxazine-9-carboxylate (E1-3):** To a solution of Compound **D1-4** (60 mg, 0.21 mmol, 1 eq) and 2-(cyclopenten-1-yl)-4, 4, 5, 5-tetramethyl-1, 3, 2-dioxaborolane (121 mg, 0.62 mmol, 3 eq) in 1,4-dioxane (3 mL) and water (0.3 mL) was added Pd(dppf)Cl₂.CH₂Cl₂ (17.0 mg, 0.021 mmol, 0.1 eq) and Na₂CO₃ (66.2 mg, 0.62 mmol, 3 eq). The mixture was stirred at 100 °C for 12 h. The reaction mixture was concentrated under reduced pressure to remove solvent. The resulting residue was purified by flash silica gel chromatography (0~10% DCM/MeOH) to give Compound **E1-3** as a yellow solid (55 mg, 96% yield). MS for C₁₅H₁₇NO₄: *m*/*z* 275.9 (MH+).

The following additional compounds were made using **General Procedure E1:**

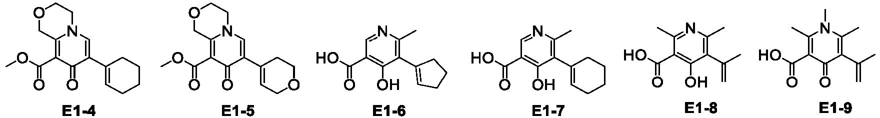

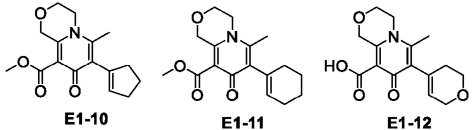

**Methyl 7-(cyclohex-1-en-1-yl)-8-oxo-1,3,4,8-tetrahydropyrido[2,1-c][1,4]oxazine-9-carboxylate (E1-4):** The 2-(cyclopenten-1-yl)-4, 4, 5, 5-tetramethyl-1, 3, 2-dioxaborolane was replaced with cyclohexen-1-ylboronic acid. MS for C₁₆H₁₉NO₄: *m*/*z* 289.9 (MH+).

**Methyl 7-(3,6-dihydro-2H-pyran-4-yl)-8-oxo-1,3,4,8-tetrahydropyrido[2,1-c][1,4]oxazine-9-carboxylate (E1-5):** The 2-(cyclopenten-1-yl)-4, 4, 5, 5-tetramethyl-1, 3, 2-dioxaborolane was replaced with 2-(3,6-dihydro-2H-pyran-4-yl)-4,4,5,5-tetramethyl-1,3,2-dioxaborolane. MS for C₁₅H₁₇NO₅: *m*/*z* 291.9 (MH+).

**5-(Cyclopent-1-en-1-yl)-4-hydroxy-6-methylnicotinic acid (E1-6):** Compound **D1-4** was replaced with Compound **D1-5.** MS for C₁₂H₁₃NO₃: *m*/*z* 220.2 (MH+).

**5-(Cyclohex-1-en-1-yl)-4-hydroxy-6-methylnicotinic acid (E1-7):** Compound **D1-4** was replaced with Compound **D1-6** and the 2-(cyclopenten-1-yl)-4, 4, 5, 5-tetramethyl-1, 3, 2-dioxaborolane was replaced with 2-(cyclohexen-1-yl)-4,4,5,5-tetramethyl-1,3,2-dioxaborolane. MS for C₁₃H₁₅NO₃: *m*/*z* 233.8 (MH+).

**4-Hydroxy-2,6-dimethyl-5-(prop-1-en-2-yl)nicotinic acid (E1-8):** Compound **D1-4** was replaced with Compound **D1-9** and the 2-(cyclopenten-1-yl)-4, 4, 5, 5-tetramethyl-1, 3, 2-dioxaborolane was replaced with isopropenylboronic acid. Prior to work up of the reaction, the resulting methyl ester product was hydrolyzed by adding aq NaOH solution to the crude reaction mixture with stirring at 100 °C until hydrolysis was complete. MS for C₁₁H₁₃NO₃: *m*/*z* 208 (MH+).

**1,2,6-Trimethyl-4-oxo-5-(prop-1-en-2-yl)-1,4-dihydropyridine-3-carboxylic acid (E1-9):** Compound **D1-4** was replaced with Compound **9-3** and the 2-(cyclopenten-1-yl)-4, 4, 5, 5-tetramethyl-1, 3, 2-dioxaborolane was replaced with 2 4,4,5,5-tetramethyl-2-(prop-1-en-2-yl)-1,3,2-dioxaborolane. MS for C₁₂H₁₅NO₃: *m*/*z* 222 (MH+).

**Methyl 7-(cyclopent-1-en-1-yl)-6-methyl-8-oxo-1,3,4,8-tetrahydropyrido[2,1-c][1,4]oxazine-9-carboxylate (E1-10):** Compound **D1-4** was replaced with Compound D-11. MS for C₁₆H₁₉NO₄: *m*/*z* 290.1 (MH+).

**Methyl 7-(cyclohex-1-en-1-yl)-6-methyl-8-oxo-1,3,4,8-tetrahydropyrido[2,1-c][1,4]oxazine-9-carboxylate (E1-11):** Compound **D1-4** was replaced with Compound **D-11** and the 2-(cyclopenten-1-yl)-4, 4, 5, 5-tetramethyl-1, 3, 2-dioxaborolane was replaced with cyclohexen-1-ylboronic acid. MS for C₁₇H₂₁NO₄: *m*/*z* 304.1 (MH+).

**7-(3,6-Dihydro-2H-pyran-4-yl)-4-methyl-8-oxo-1,3,4,8-tetrahydropyrido[2,1-c][1,4]oxazine-9-carboxylic acid (E1-12):** Compound **D1-4** was replaced with Compound **D-11** and the 2-(cyclopenten-1-yl)-4, 4, 5, 5-tetramethyl-1, 3, 2-dioxaborolane was replaced with 2-(3,6-dihydro-2H-pyran-4-yl)-4,4,5,5-tetramethyl-1,3,2-dioxaborolane. The methyl ester was hydrolyzed during the course of the reaction. MS for C₁₅H₁₇NO₅: *m*/*z* 292.1 (MH+).

### Procedure F1: Ester Hydrolysis: 7-(Cyclopent-1-en-1-yl)-8-oxo-1,3,4,8-tetrahydropyrido[2,1-c][1,4]oxazine-9-carboxylic acid (F1-1)

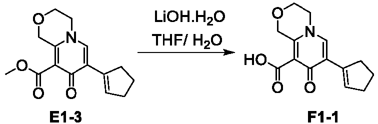

**7-(Cyclopent-1-en-1-yl)-8-oxo-1,3,4,8-tetrahydropyrido[2,1-c][1,4]oxazine-9-carboxylic acid (F1-1):** To a solution of Compound **E1-3** (55 mg, 0.20 mmol, 1 eq) in THF (2 mL) and water (2 mL) was added LiOH.H₂O (25.2 mg, 0.60 mmol, 3 eq). The mixture was stirred at 25 °C for 12 h. The reaction mixture was concentrated under reduced pressure to remove THF. The aqueous phase was acidified to pH = 3 with aq 2 N HCl. The resulting mixture was diluted with water (5 mL) and extracted with EtOAc (3 x 10 mL). The combined organic layers were washed with aq saturated NaCl (10 mL), dried over anhyd. Na₂SO₄ and concentrated under reduced pressure to give Compound **F1-1** as a yellow solid (40 mg, 77% yield). MS for C₁₄H₁₅NO₄: *m*/*z* 261.9 (MH+).

The following additional compounds were made using **General Procedure F1:**

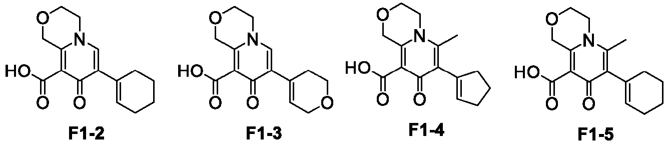

**7-(Cyclohex-1-en-1-yl)-8-oxo-1,3,4,8-tetrahydropyrido[2,1-c][1,4]oxazine-9-carboxylic acid (F1-2):** Compound **E1-3** was replaced with Compound **E1-4.** MS for C₁₅H₁₇NO₄: *m*/*z* 276.0 (MH+).

**7-(3,6-Dihydro-2H-pyran-4-yl)-8-oxo-1,3,4,8-tetrahydropyrido[2,1-c] [1,4]oxazine-9-carboxylic acid (F1-3):** Compound **E1-3** was replaced with Compound **E1-5.** MS for C₁₄H₁₅NO₅: *m*/*z* 277.9 (MH+).

**7-(Cyclopent-1-en-1-yl)-6-methyl-8-oxo-1,3,4,8-tetrahydropyrido[2,1-c][1,4]oxazine-9-carboxylic acid (F1-4):** Compound **E1-3** was replaced with Compound **E1-10.** MS for C₁₅H₁₇NO₄: *m*/*z* 276.1 (MH+).

**7-(Cyclohex-1-en-1-yl)-6-methyl-8-oxo-1,3,4,8-tetrahydropyrido[2,1-c][1,4]oxazine-9-carboxylic acid (F1-5):** Compound **E1-3** was replaced with Compound **E1-11.** MS for C₁₆₆H₁₉NO₄: *m*/*z* 290.1 (MH+).

### Procedure F2: Double Bond Reduction: 5-Isopropyl-2,6-dimethyl-4-oxo-1,4-dihydropyridine-3-carboxylic acid (F2-1)

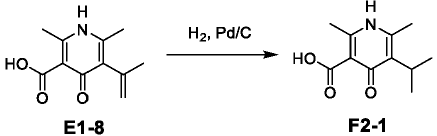

**5-Isopropyl-2,6-dimethyl-4-oxo-1,4-dihydropyridine-3-carboxylic acid (F2-1):** Compound E1-8 (200 mg, 0.97 mmol) and Pd/C (10%, wet, 80 mg) in EtOH (10 ml) was stirred at room temperature under H₂ (1 atm) until hydrogenation was complete. The mixture was filtered through Celite and the filtrate was concentrated to give Compound **F2-1** (162 mg, 80% yield). MS for C₁₁H₁₅NO₃: *m*/*z* 210 (MH+).

The following compounds were made using standard hydrogenation conditions such as that used in **General Procedure F2** as exemplified by the conversion of Compound **E1-8** to Compound **F2-1:**

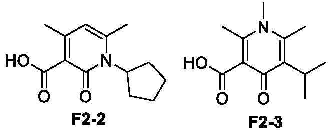

**1-Cyclopentyl-4,6-dimethyl-2-oxo-pyridine-3-carboxylic acid (F2-2):** Compound **E1-8** was replaced with Compound **12-3.** MS for C₁₃H₁₇NO₃: *m*/*z* 236.0 (MH+).

**5-Isopropyl-1,2,6-trimethyl-4-oxo-1,4-dihydropyridine-3-carboxylic acid (F2-3):** Compound **E1-8** was replaced with Compound **E1-9.** MS for C₁₂H₁₇NO₃: *m*/*z* 224 (MH+).

### General Procedure G1: HATU Coupling

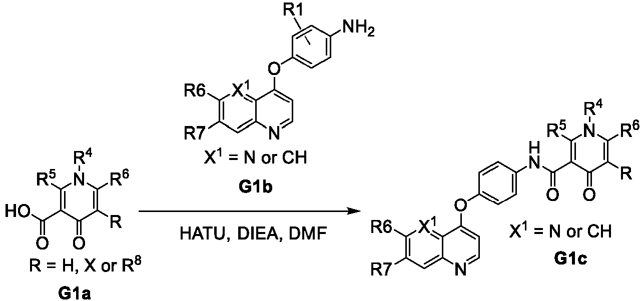

To a solution of Compound **G1a** (1 eq) in DMF (2-5 mL/mmol of **G1a** used) was added the aniline **G1b** (0.7-1.1 eq), HATU (1.1-2 eq), and DIEA (3-5 eq). The mixture was stirred (room temperature to 40 °C) until Compound **G1a** was consumed based on LC-MS and/or TLC (4-15 h). The reaction was quenched with water and extracted with EtOAc twice. The combined organic extracts were washed with aq saturated NaCl (3 - 5 times), dried over anhyd. Na₂SO₄ and concentrated under reduced pressure. The resulting residue was purified by flash silica gel chromatography to give the Compound **G1c** (6 - 63% yield).

### Example of General Procedure G1: 7-(Cyclopenten-1-yl)-N-[4-[(6,7-dimethoxy-1,5-naphthyridin-4-yl)oxy]-3-fluorophenyl]-8-oxo-3,4-dihydro-1H-pyrido[2,1-c][1,4]oxazine-9-carboxamide (G1-1)

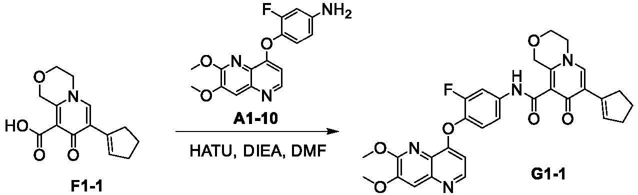

**7-(Cyclopenten-1-yl)-N-[4-[(6,7-dimethoxy-1,5-naphthyridin-4-yl)oxy]-3-fluorophenyl]-8-oxo-3,4-dihydro-1H-pyrido[2,1-c][1,4]oxazine-9-carboxamide (G1-1):** To a solution of Compound **F1-1** (20 mg, 0.076 mmol, 1 eq) and Compound **A1-10** (31 mg, 0.099 mmol, 1.3 eq) in DMF (2 mL) was added HATU (38 mg, 0.0995 mmol, 1.3 eq) and DIEA (30 mg, 0.23 mmol, 3 eq). The mixture was stirred at 16 °C for 12 h. The reaction mixture was concentrated under reduced pressure to remove solvent. The residue was purified by prep-HPLC to give Compound G1-1 as a white solid (13.2 mg, 31% yield). ¹H NMR (400 MHz, DMSO-*d₆*) δ 13.20 (s, 1H), 8.54 (d, 1H), 7.97 (m, 1H), 7.77 (s, 1H), 7.65 (s, 1H), 7.38-7.43 (m, 1H), 7.29-7.36 (m, 1H), 7.18 (br s, 1H), 6.80 (d, 1H), 5.19 (s, 2H), 4.18-4.23 (m, 2H), 4.05 (br m, 2H), 3.96 (d, 6H), 2.62 (br d, 2H), 2.44 (br s, 2H), 1.88 (m, 2H); MS for C₃₀H₂₇FN₄O₆: *m*/*z* 559.2 (MH+).

The following additional compounds were made using **General Procedure G1:**
**5-(Cyclopenten-1-yl)-N-[4-[(6,7-dimethoxy-1,5-naphthyridin-4-yl)oxy]-3-fluorophenyl]-4-hydroxy-6-methylpyridine-3-carboxamide hydrochloride (G1-2):** Compound **F1-1** was replaced with Compound **E1-6.** Compound **G1-2** was isolated as the hydrochloride salt from prep HPLC purification using a mobile phase that contained HCl. ¹H NMR (400 MHz, DMSO-*d₆*) δ 13.29 (s, 1H), 12.65 - 12.57 (m, 1H), 8.73 (d, 1H), 8.43 (d, 1H), 8.07 (dd, 1H), 7.75 (s, 1H), 7.54 - 7.40 (m, 2H), 7.06 (br d, 1H), 5.64 (s, 1H), 4.07 - 3.99 (m, 6H), 2.47 (br s, 4H), 2.30 (s, 3H), 2.01 - 1.89 (m, 2H); MS for C₂₈H₂₅FN₄O₅: *m*/*z* 517.2 (MH+).

**5-(Cyclohexen-1-yl)-N-[4-[(6,7-dimethoxy-1,5-naphthyridin-4-yl)oxy]-3-fluorophenyl]-4-hydroxy-6-methylpyridine-3-carboxamide (G1-3):** Compound **F1-1** was replaced with Compound **E1-7.** ¹H NMR (400 MHz, DMSO-*d₆*) δ 13.34 (s, 1H), 12.60 (br d, 1H), 8.79 (d, 1H), 8.43 (d, 1H), 8.10 (dd, 1H), 7.81 (s, 1H), 7.58 - 7.51 (m, 1H), 7.51 - 7.42 (m, 1H), 7.13 (d, 1H), 5.49 (br s, 1H), 4.05 (d, 6H), 2.29 (s, 3H), 2.17 - 2.07 (m, 4H), 1.76 - 1.57 (m, 4H); MS for C₂₉H₂₇FN₄O₅: *m*/*z* 531.0 (MH+).

**7-(Cyclohexen-1-yl)-N-[4-[(6,7-dimethoxy-1,5-naphthyridin-4-yl)oxy]-3-fluorophenyl]-8-oxo-3,4-dihydro-1H-pyrido[2,1-c][1,4]oxazine-9-carboxamide (G1-4):** Compound **F1-1** was replaced with Compound **F1-2.** ¹H NMR (400 MHz, DMSO-*d₆*) δ 13.37 (s, 1H), 8.54 (d, 1H), 7.89-8.04 (m, 1H), 7.72 (s, 1H), 7.65 (s, 1H), 7.37-7.42 (m, 1H), 7.27-7.34 (m, 1H), 6.80 (d, 1H), 6.07 (br s, 1H), 5.21 (s, 2H), 4.12-4.19 (m, 2H), 4.03 (m, 2H), 3.96 (d, 6H), 2.33-2.39 (m, 2H), 2.14 (br s, 2H), 1.64 (br d, 4H); MS for C₃₁H₂₉FN₄O₆: *m*/*z* 573.1 (MH+).

**7-(Cyclohexen-1-yl)-N-[4-[(6,7-dimethoxy-1,5-naphthyridin-4-yl)oxy]phenyl]-8-oxo-3,4-dihydro-1H-pyrido[2,1-c][1,4]oxazine-9-carboxamide (G1-5):** Compound **F1-1** was replaced with Compound **F1-2** and Compound **A1-10** was replaced with Compound **A1-11.** ¹H NMR (400 MHz, DMSO-*d₆*) δ 13.18 (s, 1H), 8.53 (d, 1H), 7.70-7.78 (m, 3H), 7.64 (s, 1H), 7.17 (br d, 2H), 6.78 (d, 1H), 6.07 (br s, 1H), 5.22 (s, 2H), 4.15 (br d, 2H), 4.03 (br d, 2H), 3.96 (d, 6H), 2.34 (br d, 2H), 2.14 (br s, 2H), 1.64 (br d, 4H); MS for C₃₁H₃₀N₄O₆: *m*/*z* 555.2 (MH+).

**7-(Cyclopenten-1-yl)-N-[4-[(6,7-dimethoxy-1,5-naphthyridin-4-yl)oxy]phenyl]-8-oxo-3,4-dihydro-1H-pyrido[2,1-c][1,4]oxazine-9-carboxamide (G1-6):** Compound **A1-10** was replaced with Compound **A1-11.** ¹H NMR (400 MHz, DMSO-*d₆*) δ 13.01 (s, 1H), 8.53 (d, 1H), 7.73-7.80 (m, 3H), 7.64 (s, 1H), 7.15-7.21 (m, 3H), 6.78 (d, 1H), 5.20 (s, 2H), 4.17-4.22 (m, 2H), 4.02-4.06 (m, 2H), 3.96 (d, 6H), 2.62 (br d, 2H), 2.37-2.44 (m, 2H), 1.83-1.93 (m, 2H); MS for C₃₀H₂₈N₄O₆: *m*/*z* 541.2 (MH+).

**7-(3,6-Dihydro-2H-pyran-4-yl)-N-[4-[(6,7-dimethoxy-1,5-naphthyridin-4-yl)oxy]-3-fluorophenyl]-8-oxo-3,4-dihydro-1H-pyrido[2,1-c][1,4]oxazine-9-carboxamide (G1-7):** Compound **F1-1** was replaced with Compound **F1-3.** ¹H NMR (400 MHz, DMSO-*d₆*) δ 13.15 (s, 1H), 8.54 (d, 1H), 7.96 (m, 1H), 7.80 (s, 1H), 7.65 (s, 1H), 7.36-7.43 (m, 1H), 7.29-7.35 (m, 1H), 6.80 (d, 1H), 6.54 (br s, 1H), 5.19 (s, 2H), 4.13-4.25 (m, 4H), 4.01-4.08 (m, 2H), 3.96 (d, 6H), 3.79 (m, 2H), 2.44 (br s, 2H); MS for C₃₀H₂₇FN₄O₇: *m*/*z* 575.2 (MH+).

**7-(3,6-Dihydro-2H-pyran-4-yl)-N-[4-[(6,7-dimethoxy-1,5-naphthyridin-4-yl)oxy]phenyl]-8-oxo-3,4-dihydro-1H-pyrido[2,1-c][1,4]oxazine-9-carboxamide (G1-8):** Compound **F1-1** was replaced with Compound **F1-3** and Compound **A1-10** was replaced with Compound **A1-11.** ¹H NMR (400 MHz, DMSO-*d₆*) δ 12.96 (s, 1H), 8.53 (d, 1H), 7.71-7.83 (m, 3H), 7.64 (s, 1H), 7.17 (d, 2H), 6.77 (d, 1H), 6.54 (s, 1H), 5.20 (s, 2H), 4.15-4.23 (m, 4H), 4.04 (m, 2H), 3.96 (d, 6H), 3.79 (m, 2H), 2.41-2.44 (m, 2H); MS for C₃₀H₂₈N₄O₇: *m*/*z* 557.2 (MH+).

### Example 1: 5-(Cyclopenten-1-yl)-N-[4-[(6,7-dimethoxy-1,5-naphthyridin-4-yl)oxy]-3-fluorophenyl]-4-hydroxy-2,6-dimethylpyridine-3-carboxamide (1-2):

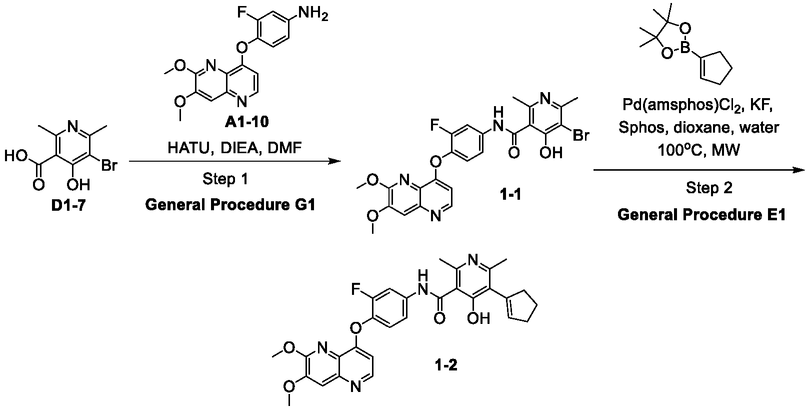

**Step 1: 5-Bromo-N-(4-((6,7-dimethoxy-1,5-naphthyridin-4-yl)oxy)-3-fluorophenyl)-4-hydroxy-2,6-dimethylnicotinamide (1-1):** Compound **1-1** was synthesized from Compound **D1-7** and Compound **A1-10** using **General Procedure G1.** MS for C₂₄H₂₀BrFN₄O₅: *m*/*z* 545.1 (MH+).

**Step 2: 5-(Cyclopenten-1-yl)-N-[4-[(6,7-dimethoxy-1,5-naphthyridin-4-yl)oxy]-3-fluorophenyl]-4-hydroxy-2,6-dimethylpyridine-3-carboxamide hydrochloride (1-2):** Compound **1-2** was synthesized from Compound **1-1** using **General Procedure** E1. Compound **1-2** was isolated as the hydrochloride salt from prep HPLC purification using a mobile phase that contained HCl. ¹H NMR (400 MHz, DMSO-*d₆*) δ 13.45 (s, 1H), 12.10 (br s, 1H), 8.80 (d, 1H), 8.12 - 8.03 (m, 1H), 7.78 (s, 1H), 7.52 - 7.43 (m, 2H), 7.13 (d, 1H), 5.63 (d, 1H), 4.06 (d, 6H), 2.71 (s, 3H), 2.50 - 2.48 (m, 4H), 2.30 (s, 3H), 1.99 - 1.91 (m, 2H); MS for C₂₉H₂₇FN₄O₅: *m*/*z* 531.1 (MH+).

The following compounds were made using the same two step procedure, using **General Procedure G1** followed by **General Procedure E1,** as exemplified by the synthesis of Compound **1-2** in

### Example 1.

**5-(3,6-Dihydro-2H-pyran-4-yl)-N-[4-[(6,7-dimethoxy-1,5-naphthyridin-4-yl)oxy]-3-fluorophenyl]-4-hydroxy-6-methylpyridine-3-carboxamide (1-3):** In Step 1, Compound **D1-7** was replaced with Compound **D1-5.** In Step 2, 2-(cyclopent-1-en-1-yl)-4,4,5,5-tetramethyl-1,3,2-dioxaborolane was replaced with 2-(3,6-dihydro-2H-pyran-4-yl)-4,4,5,5-tetramethyl-1,3,2-dioxaborolane or the corresponding boronic acid. ¹H NMR (400 MHz, DMSO-*d₆*) δ 13.16 (s, 1H), 12.42 (s, 1H), 8.48 (d, 1H), 8.39 (s, 1H), 7.95 (dd, 1H), 7.59 (s, 1H), 7.42 - 7.31 (m, 1H), 7.26 (t, 1H), 6.75 (d, 1H), 5.53 (t, 1H), 4.10 (q, 2H), 3.88 (s, 6H), 3.73 (s, 2H), 2.23 (s, 3H), 2.16 (s, 2H). MS for C₂₈H₂₅FN₄O₆: 533.1 (MH+).

**5-(3,6-Dihydro-2H-pyran-4-yl)-N-[3-fluoro-4-[(7-methoxy-1,5-naphthyridin-4-yl)oxy]phenyl]-4-hydroxy-6-methylpyridine-3-carboxamide (1-4):** ): In Step 1, Compound **D1-7** was replaced with Compound **D1-5** and Compound **A1-10** was replaced with Compound **A1-12.** In Step 2, 2-(cyclopent-1-en-1-yl)-4,4,5,5-tetramethyl-1,3,2-dioxaborolane was replaced with 2-(3,6-dihydro-2H-pyran-4-yl)-4,4,5,5-tetramethyl-1,3,2-dioxaborolane or the corresponding boronic acid. ¹H NMR (400 MHz, DMSO-*d₆*) δ 13.22 (s, 1H), 12.50 (d, 1H), 8.83 - 8.62 (m, 2H), 8.47 (d, 1H), 8.07 (dd, 1H), 7.81 (d, 1H), 7.56 - 7.28 (m, 2H), 6.76 (dd, 1H), 5.61 (s, 1H), 4.18 (d, 2H), 4.01 (s, 3H), 3.81 (t, 2H), 2.31 (s, 3H), 2.24 (s, 2H). MS for C₂₇H₂₃FN₄O₅: 503.1 (MH+).

**5-(3,6-Dihydro-2H-pyran-4-yl)-N-[3-fluoro-4-[(6-methoxy-1,5-naphthyridin-4-yl)oxy]phenyl]-4-hydroxy-6-methylpyridine-3-carboxamide (1-5):** ): In Step 1, Compound **D1-7** was replaced with Compound **D1-5** and Compound **A1-10** was replaced with Compound **A1-14.** In Step 2, 2-(cyclopent-1-en-1-yl)-4,4,5,5-tetramethyl-1,3,2-dioxaborolane was replaced with 2-(3,6-dihydro-2H-pyran-4-yl)-4,4,5,5-tetramethyl-1,3,2-dioxaborolane or the corresponding boronic acid. ¹H NMR (400 MHz, DMSO-*d₆*) δ 13.12 (s, 1H), 12.41 (s, 1H), 8.55 (d, 1H), 8.39 (s, 1H), 8.22 (d, 1H), 7.97 (dd, 1H), 7.43 - 7.31 (m, 1H), 7.26 (dd, 2H), 6.90 (d, 1H), 5.54 (s, 1H), 4.11 (d, 2H), 3.87 (s, 3H), 3.74 (s, 2H), 2.24 (s, 3H), 2.16 (s, 2H). MS for C₂₇H₂₃FN₄O₅: 503.1 (MH+).

**5-(Cyclohexen-1-yl)-N-[3-fluoro-4-[(6-methoxy-1,5-naphthyridin-4-yl)oxy]phenyl]-4-hydroxy-6-methylpyridine-3-carboxamide (1-6):** ): In Step 1, Compound **D1-7** was replaced with Compound **D1-5** and Compound **A1-10** was replaced with Compound **A1-14.** In Step 2, 2-(cyclopent-1-en-1-yl)-4,4,5,5-tetramethyl-1,3,2-dioxaborolane was replaced with 2-(cyclohex-1-en-1-yl)-4,4,5,5-tetramethyl-1,3,2-dioxaborolane or the corresponding boronic acid. ¹H NMR (400 MHz, DMSO-*d₆*) δ 13.27 (s, 1H), 12.41 (d, 1H), 8.63 (d, 1H), 8.43 (d, 1H), 8.30 (d, 1H), 8.04 (dd, 1H), 7.57 - 7.07 (m, 3H), 6.97 (d, 1H), 5.56 - 5.30 (m, 1H), 3.94 (s, 3H), 2.28 (s, 3H), 2.12 (s, 4H), 1.80 - 1.56 (m, 4H). MS for C₂₈H₂₅FN₄O₄: 501 (MH+).

**5-(Cyclopenten-1-yl)-N-[3-fluoro-4-[(6-methoxy-1,5-naphthyridin-4-yl)oxy]phenyl]-4-hydroxy-6-methylpyridine-3-carboxamide (1-7):** ): In Step 1, Compound **D1-7** was replaced with Compound **D1-5** and Compound **A1-10** was replaced with Compound **A1-14.** ¹H NMR (400 MHz, DMSO-*d₆*) δ 13.17 (s, 1H), 12.40 (d, 1H), 8.55 (d, 1H), 8.37 (d, 1H), 8.22 (d, 1H), 7.97 (dd, 1H), 7.45 - 7.20 (m, 3H), 6.90 (d, 1H), 5.57 (t, 1H), 3.87 (s, 3H), 2.49 (s, 2H), 2.41 (d, 2H), 2.22 (s, 3H), 1.89 (dd, 2H). MS for C₂₇H₂₃FN₄O₄: 487.1 (MH+).

**5-(Cyclohexen-1-yl)-N-[3-fluoro-4-[(7-methoxy-1,5-naphthyridin-4-yl)oxy]phenyl]-4-hydroxy-6-methylpyridine-3-carboxamide (1-8):** ): In Step 1, Compound **D1-7** was replaced with Compound **D1-5** and Compound **A1-10** was replaced with Compound **A1-12.** In Step 2, 2-(cyclopent-1-en-1-yl)-4,4,5,5-tetramethyl-1,3,2-dioxaborolane was replaced with 2-(cyclohex-1-en-1-yl)-4,4,5,5-tetramethyl-1,3,2-dioxaborolane or the corresponding boronic acid. ¹H NMR (400 MHz, DMSO-*d₆*) δ 13.23 (s, 1H), 12.35 (s, 1H), 8.82 - 8.55 (m, 2H), 8.37 (s, 1H), 8.00 (dd, 1H), 7.74 (d, 1H), 7.51 - 7.15 (m, 2H), 6.68 (dd, 1H), 5.53 - 5.33 (m, 1H), 3.94 (s, 3H), 2.21 (s, 3H), 2.05 (s, 4H), 1.60 (d, 4H). MS for C₂₈H₂₅FN₄O₄: 501.2 (MH+).

**5-(Cyclopenten-1-yl)-N-[3-fluoro-4-[(7-methoxy-1,5-naphthyridin-4-yl)oxy]phenyl]-4-hydroxy-6-methylpyridine-3-carboxamide (1-9):** ): In Step 1, Compound **D1-7** was replaced with Compound **D1-5** and Compound **A1-10** was replaced with Compound **A1-12.** ¹H NMR (400 MHz, DMSO-*d₆*) δ 13.26 (s, 1H), 12.43 (s, 1H), 8.84 - 8.51 (m, 2H), 8.38 (s, 1H), 8.00 (dd, 1H), 7.74 (d, 1H), 7.52 - 7.26 (m, 2H), 6.68 (dd, 1H), 5.56 (s, 1H), 3.94 (s, 3H), 2.49 (s, 2H), 2.40 (s, 2H), 2.22 (s, 3H), 1.89 (q, 2H). MS for C₂₇H₂₃FN₄O₄: 487.2 (MH+).

**5-(3,6-Dihydro-2H-pyran-4-yl)-4-hydroxy-N-[4-[(7-methoxy-1,5-naphthyridin-4-yl)oxy]phenyl]-6-methylpyridine-3-carboxamide (7):** In Step 1, Compound D1-7 was replaced with Compound D1-5 and Compound A1-10 was replaced with Compound A1-13. In Step 2, 2-(cyclopent-1-en-1-yl)-4,4,5,5-tetramethyl-1,3,2-dioxaborolane was replaced with 2-(3,6-dihydro-2H-pyran-4-yl)-4,4,5,5-tetramethyl-1,3,2-dioxaborolane or the corresponding boronic acid. ¹H NMR (400 MHz, DMSO-*d₆*) *δ* 12.97 (s, 1H), 12.37 (s, 1H), 8.66 (d, 1H), 8.63 (d, 1H), 8.38 (s, 1H), 7.76 (d, 2H), 7.72 (d, 1H), 7.17 (d, 2H), 6.67 (d, 1H), 5.54 (s, 1H), 4.11 (d, 2H), 3.93 (s, 3H), 3.74 (t, 2H), 2.24 (s, 3H), 2.17 (s, 2H). MS (EI) for C₂₇H₂₄N₄O₅, found 485.2 (MH+).

**5-(3,6-Dihydro-2H-pyran-4-yl)-N-[3-fluoro-4-[(7-methoxy-1,5-naphthyridin-4-yl)oxy]phenyl]-1,6-dimethyl-4-oxopyridine-3-carboxamide (12):** In Step 1, Compound **A1-10** was replaced with Compound **A1-12** and Compound **D1-7** was replaced with 5-bromo-1,6-dimethyl-4-oxo-1,4-dihydropyridine-3-carboxylic acid (Bannen, L., et. al. WO2021173591, which is incorporated by reference). In Step 2, 2-(cyclopent-1-en-1-yl)-4,4,5,5-tetramethyl-1,3,2-dioxaborolane was replaced with 2-(3,6-dihydro-2H-pyran-4-yl)-4,4,5,5-tetramethyl-1,3,2-dioxaborolane or the corresponding boronic acid. ¹H NMR (400 MHz, DMSO-*d₆*) δ 13.17 (s, 1H), 8.69 (d, 1H), 8.64 (d, 2H), 7.99 (dd, 1H), 7.74 (d, 1H), 7.50 - 7.39 (m, 1H), 7.35 (t, 1H), 6.68 (d, 1H), 5.50 (s, 1H), 4.12 (d, 2H), 3.94 (s, 3H), 3.77 (d, 5H), 2.32 (s, 3H), 2.31 - 2.23 (m, 2H). MS (EI) for C₂₈H₂₅FN₄O₅, found 517.1 (MH+).

**5-(Cyclopenten-1-yl)-N-[4-(6,7-dimethoxyquinolin-4-yl)oxyphenyl]-4-hydroxy-6-methylpyridine-3-carboxamide (16):** In Step 1, Compound **A1-10** was replaced with Compound **B1-7** and Compound **D1-7** was replaced with Compound **D1-5.** ¹H NMR (400 MHz, DMSO-*d₆*) δ 13.03 (s, 1H), 12.34 (d, 1H), 8.44 (d, 1H), 8.36 (d, 1H), 7.85 - 7.67 (m, 2H), 7.47 (s, 1H), 7.34 (s, 1H), 7.29 - 7.12 (m, 2H), 6.47 (d, 1H), 5.57 (s, 1H), 3.88 (d, 6H), 2.48 (d, 2H), 2.38 (bm, 2H), 2.23 (s, 3H), 1.89 (p, 2H). MS (EI) for C₂₉H₂₇N₃O₅, found 498.1 (MH+).

**5-(3,6-Dihydro-2H-pyran-4-yl)-N-[4-(6,7-dimethoxyquinolin-4-yl)oxyphenyl]-4-hydroxy-6-methylpyridine-3-carboxamide (17):** In Step 1, Compound **A1-10** was replaced with Compound **B1-7** and Compound **D1-7** was replaced with Compound **D1-5.** In Step 2, 2-(cyclopent-1-en-1-yl)-4,4,5,5-tetramethyl-1,3,2-dioxaborolane was replaced with 2-(3,6-dihydro-2H-pyran-4-yl)-4,4,5,5-tetramethyl-1,3,2-dioxaborolane or the corresponding boronic acid. ¹H NMR (400 MHz, DMSO-*d₆*) δ 12.98 (s, 1H), 12.36 (d, 1H), 8.42 (d, 1H), 8.38 (d, 1H), 7.78 (d, 1H), 7.76 (s, 1H), 7.46 (s, 1H), 7.33 (s, 1H), 7.19 (d, 2H), 6.44 (d, 1H), 5.54 (s, 1H), 4.11 (s, 2H), 3.88 (d, 6H), 3.74 (t, 2H), 2.24 (s, 3H), 2.17 (s, 2H). MS (EI) for C₂₉H₂₇N₃O₆, found 514.1 (MH+).

**5-(Cyclopenten-1-yl)-N-[4-(6,7-dimethoxyquinolin-4-yl)oxy-3-fluorophenyl]-4-hydroxy-6-methylpyridine-3-carboxamide (18):** In Step 1, Compound **A1-10** was replaced with Compound **B1-6** and Compound **D1-7** was replaced with Compound **D1-5.** ¹H NMR (400 MHz, DMSO-*d₆*) δ 13.23 (s, 1H), 12.45 (s, 1H), 8.42 (d, 1H), 8.38 (s, 1H), 7.99 (dd, 1H), 7.47 (s, 1H), 7.41 (d, 1H), 7.38 (d, 1H), 7.34 (s, 1H), 6.42 (dd, 1H), 5.57 (t, 1H), 3.89 (s, 6H), 2.53 - 2.46 (m, 2H), 2.39 (s, 2H), 2.23 (s, 3H), 1.89 (p, 2H). MS (EI) for C₂₉H₂₆FN₃O₅, found 516.25 (MH+).

**5-(3,6-Dihydro-2H-pyran-4-yl)-N-[4-(6,7-dimethoxyquinolin-4-yl)oxy-3-fluorophenyl]-4-hydroxy-6-methylpyridine-3-carboxamide (19):** In Step 1, Compound **A1-10** was replaced with Compound **B1-6** and Compound **D1-7** was replaced with Compound **D1-5.** In Step 2, 2-(cyclopent-1-en-1-yl)-4,4,5,5-tetramethyl-1,3,2-dioxaborolane was replaced with 2-(3,6-dihydro-2H-pyran-4-yl)-4,4,5,5-tetramethyl-1,3,2-dioxaborolane or the corresponding boronic acid. ¹H NMR (400 MHz, DMSO-*d₆*) δ 13.16 (s, 1H), 12.42 (s, 1H), 8.51 - 8.26 (m, 2H), 7.99 (dd, 1H), 7.56 - 7.21 (m, 4H), 6.42 (dd, 1H), 5.54 (s, 1H), 4.11 (d, 2H), 3.89 (d, 6H), 3.74 (t, 2H), 2.24 (s, 3H), 2.17 (s, 2H). MS (EI) for C₂₉H₂₆FN₃O₆, found 532.5 (MH+).

**5-(Cyclohexen-1-yl)-N-[4-(6,7-dimethoxyquinolin-4-yl)oxy-3-fluorophenyl]-4-hydroxy-6-methylpyridine-3-carboxamide (23):** In Step 1, Compound **A1-10** was replaced with Compound **B1-6** and Compound **D1-7** was replaced with Compound **D1-5.** In Step 2, 2-(cyclopent-1-en-1-yl)-4,4,5,5-tetramethyl-1,3,2-dioxaborolane was replaced with 2-(cyclohex-1-en-1-yl)-4,4,5,5-tetramethyl-1,3,2-dioxaborolane or the corresponding boronic acid. ¹H NMR (400 MHz, DMSO-*d₆*) δ 13.24 (s, 1H), 12.36 (s, 1H), 8.42 (d, 1H), 8.37 (s, 1H), 7.99 (dd, 1H), 7.47 (s, 1H), 7.45 (s, 1H), 7.38 (d, 1H), 7.35 (s, 1H), 6.42 (dd, 1H), 5.42 (s, 1H), 3.89 (d, 6H), 2.21 (s, 3H), 2.05 (s, 4H), 1.60 (d, 4H). MS (EI) for C₃₀H₂₈FN₃O₅, found 530.2 (MH+).

**5-(Cyclohexen-1-yl)-N-[4-(6,7-dimethoxyquinolin-4-yl)oxyphenyl]-4-hydroxy-6-methylpyridine-3-carboxamide (24):** In Step 1, Compound **A1-10** was replaced with Compound **B1-7** and Compound **D1-7** was replaced with Compound **D1-5.** In Step 2, 2-(cyclopent-1-en-1-yl)-4,4,5,5-tetramethyl-1,3,2-dioxaborolane was replaced with 2-(cyclohex-1-en-1-yl)-4,4,5,5-tetramethyl-1,3,2-dioxaborolane or the corresponding boronic acid. ¹H NMR (400 MHz, DMSO-*d₆*) δ 13.04 (s, 1H), 12.28 (s, 1H), 8.41 (d, 1H), 8.35 (s, 1H), 7.77 (d, 2H), 7.45 (s, 1H), 7.33 (s, 1H), 7.18 (d, 2H), 6.43 (d, 1H), 5.48 - 5.26 (m, 1H), 3.87 (d, 6H), 2.21 (s, 3H), 2.14 - 1.92 (m, 4H), 1.60 (d, 4H). MS (EI) for C₃₀H₂₉N₃O₅, found 512.2 (MH+).

**5-[(E)-2-Cyclopentylethenyl]-N-[4-[(6,7-dimethoxy-1,5-naphthyridin-4-yl)oxy]-3-**fluorophenyl]-4-hydroxy-6-methylpyridine-3-carboxamide (25): In Step 1, Compound D1-7 was replaced with Compound **D1-5.** In Step 2, 2-(cyclopent-1-en-1-yl)-4,4,5,5-tetramethyl-1,3,2-dioxaborolane was replaced with (E)-(2-cyclopentylvinyl)boronic acid or the corresponding boronic acid pinacol ester. ¹H NMR (400 MHz, DMSO-*d₆*) δ 13.25 (s, 1H), 12.43 (s, 1H), 8.48 (d, 1H), 8.32 (s, 1H), 7.96 (dd, 1H), 7.59 (s, 1H), 7.37 (ddd, 1H), 7.26 (t, 1H), 6.76 (d, 1H), 6.61 (dd, 1H), 6.24 (dd, 1H), 3.90 (s, 3H), 3.88 (s, 3H), 2.51 (q, 1H), 2.35 (s, 3H), 1.84 - 1.70 (m, 2H), 1.70 - 1.46 (m, 4H), 1.31 (dt, 2H). MS (EI) for C₃₀H₂₀FN₄O₅, found 545.20 (MH+).

**5-[(E)-2-Cyclopropylethenyl]-N-[4-[(6,7-dimethoxy-1,5-naphthyridin-4-yl)oxy]-3-fluorophenyl]-4-hydroxy-6-methylpyridine-3-carboxamide (26):** In Step 1, Compound **D1-7** was replaced with Compound **D1-5.** In Step 2, 2-(cyclopent-1-en-1-yl)-4,4,5,5-tetramethyl-1,3,2-dioxaborolane was replaced with (E)-(2-cyclopropylvinyl)boronic acid or the corresponding boronic acid pinacol ester. ¹H NMR (400 MHz, DMSO-*d₆*) δ 13.22 (s, 1H), 12.38 (d, 1H), 8.51 (d, 1H), 8.30 (d, 1H), 7.96 (dd, 1H), 7.59 (s, 1H), 7.39 - 7.31 (m, 1H), 7.28 (t, 1H), 6.79 (d, 1H), 6.42 - 6.24 (m, 2H), 3.91 (s, 3H), 3.89 (s, 3H), 2.35 (s, 3H), 1.51 (tt, 1H), 0.75 (dd, 2H), 0.43 (dd, 2H). MS (EI) for C₂₈H₂₅FN₄O₅, found 517.0 (MH+).

**N-[4-[(6,7-Dimethoxy-1,5-naphthyridin-4-yl)oxy]-3-fluorophenyl]-5-[(E)-3,3-dimethylbut-1-enyl]-4-hydroxy-6-methylpyridine-3-carboxamide (27):** In Step 1, Compound **D1-7** was replaced with Compound **D1-5.** In Step 2, 2-(cyclopent-1-en-1-yl)-4,4,5,5-tetramethyl-1,3,2-dioxaborolane was replaced with (E)-(3,3-dimethylbut-1-en-1-yl)boronic acid or the corresponding boronic acid pinacol ester. ¹H NMR (400 MHz, DMSO-*d₆*) δ 13.23 (s, 1H), 12.41 (s, 1H), 8.48 (d, 1H), 8.33 (s, 1H), 7.97 (dd, 1H), 7.59 (s, 1H), 7.39 (ddd, 1H), 7.26 (t, 1H), 6.76 (dd, 1H), 6.51 (d, 1H), 6.17 (d, 1H), 3.89 (d, 6H), 2.35 (s, 3H), 1.04 (s, 9H). MS (EI) for C₂₉H₂₉FN₄O₅, found 533.2 (MH+).

**N-[4-[(6,7-Dimethoxy-1,5-naphthyridin-4-yl)oxy]-3-fluorophenyl]-4-hydroxy-6-methyl-5-[(E)-4-methylpent-1-enyl]pyridine-3-carboxamide (28):** In Step 1, Compound **D1-7** was replaced with Compound **D1-5.** In Step 2, 2-(cyclopent-1-en-1-yl)-4,4,5,5-tetramethyl-1,3,2-dioxaborolane was replaced with (E)-(4-methylpent-1-en-1-yl)boronic acid or the corresponding boronic acid pinacol ester. ¹H NMR (400 MHz, DMSO-*d₆*) δ 13.20 (s, 1H), 12.38 (s, 1H), 8.48 (d, 1H), 8.33 (s, 1H), 7.96 (dd, 1H), 7.59 (s, 1H), 7.37 (ddd, 1H), 7.26 (t, 1H), 6.76 (dd, 1H), 6.57 (dt, 1H), 6.23 (d, 1H), 3.90 (s, 3H), 3.88 (s, 3H), 2.36 (s, 3H), 2.04 (td, 2H), 1.64 (dt, 1H), 0.88 (s, 3H), 0.86 (s, 3H). MS (EI) for C₂₉H₂₉FN₄O₅, found 533.2 (MH+).

**N-[4-(6,7-Dimethoxyquinolin-4-yl)oxy-3-fluorophenyl]-4-hydroxy-6-methyl-5-[(E)-4-methylpent-1-enyl]pyridine-3-carboxamide (29):** In Step 1, Compound **A1-10** was replaced with Compound **B1-6** and Compound **D1-7** was replaced with Compound **D1-5.** In Step 2, 2-(cyclopent-1-en-1-yl)-4,4,5,5-tetramethyl-1,3,2-dioxaborolane was replaced with (E)-(4-methylpent-1-en-1-yl)boronic acid or the corresponding boronic acid pinacol ester. ¹H NMR (400 MHz, DMSO-*d₆*) δ 13.26 (s, 1H), 12.42 (s, 1H), 8.42 (d, 1H), 8.34 (s, 1H), 7.99 (dd, 1H), 7.52 - 7.25 (m, 4H), 6.58 (dd, 1H), 6.42 (dd, 1H), 6.23 (dt, 1H), 3.88 (s, 6H), 2.36 (s, 3H), 2.04 (td, 2H), 1.65 (dt, 1H), 0.88 (s, 3H), 0.86 (s, 3H). MS (EI) for C₃₀H₃₀FN₃O₅, found 532.0 (MH+).

**N-[4-(6,7-Dimethoxyquinolin-4-yl)oxy-3-fluorophenyl]-5-[(E)-3,3-dimethylbut-1-enyl]-4-hydroxy-6-methylpyridine-3-carboxamide (30):** In Step 1, Compound **A1-10** was replaced with Compound **B1-6** and Compound **D1-7** was replaced with Compound **D1-5.** In Step 2, 2-(cyclopent-1-en-1-yl)-4,4,5,5-tetramethyl-1,3,2-dioxaborolane was replaced with (E)-(3,3-dimethylbut-1-en-1-yl)boronic acid or the corresponding boronic acid pinacol ester. ¹H NMR (400 MHz, DMSO-*d₆*) δ 13.24 (s, 1H), 12.43 (s, 1H), 8.42 (d, 1H), 8.34 (s, 1H), 8.00 (dd, 1H), 7.54 - 7.41 (m, 2H), 7.41 - 7.27 (m, 2H), 6.52 (d, 1H), 6.43 (dd, 1H), 6.17 (d, 1H), 3.89 (s, 6H), 2.36 (s, 3H), 1.04 (s, 9H). MS (EI) for C₃₀H₃₀FN₃O₅, found 532.25 (MH+).

**5-(Cyclopenten-1-yl)-N-[3-fluoro-4-[[6-methoxy-7-(2-methoxyethoxy)-1,5-naphthyridin-4-yl]oxy]phenyl]-4-hydroxy-6-methylpyridine-3-carboxamide (33):** In Step 1, Compound **A1-10** was replaced with Compound **A2-7** and Compound **D1-7** was replaced with Compound **D1-5.** ¹H NMR (400 MHz, DMSO-*d₆*) δ 13.16 (s, 1H), 12.38 (s, 1H), 8.47 (d, 1H), 8.37 (s, 1H), 7.95 (dd, 1H), 7.61 (s, 1H), 7.36 (ddd, 1H), 7.26 (t, 1H), 6.75 (dd, 1H), 5.56 (t, 1H), 4.30 - 4.15 (m, 2H), 3.89 (s, 3H), 3.74 - 3.62 (m, 2H), 3.27 (s, 3H), 2.48 (d, 2H), 2.40 (s, 2H), 2.22 (s, 3H), 1.96 - 1.72 (m, 2H). MS (EI) for C₃₀H₂₀FN₄O₆, found 561 (MH+).

**5-(Cyclohexen-1-yl)-N-[3-fluoro-4-[[6-methoxy-7-(2-methoxyethoxy)-1,5-naphthyridin-4-yl]oxy]phenyl]-4-hydroxy-6-methylpyridine-3-carboxamide (34):** In Step 1, Compound **A1-10** was replaced with Compound **A2-7** and Compound **D1-7** was replaced with Compound **D1-5.** In Step 2, 2-(cyclopent-1-en-1-yl)-4,4,5,5-tetramethyl-1,3,2-dioxaborolane was replaced with 2-(cyclohex-1-en-1-yl)-4,4,5,5-tetramethyl-1,3,2-dioxaborolane or the corresponding boronic acid. ¹H NMR (400 MHz, DMSO-*d₆*) δ 13.18 (s, 1H), 12.33 (s, 1H), 8.48 (d, 1H), 8.36 (s, 1H), 7.95 (dd, 1H), 7.61 (s, 1H), 7.38 (ddd, 1H), 7.25 (t, 1H), 6.75 (dd, 1H), 5.41 (s, 1H), 4.42 - 4.20 (m, 2H), 3.89 (s, 3H), 3.76 - 3.62 (m, 2H), 3.27 (s, 3H), 2.20 (s, 3H), 2.04 (s, 4H), 1.59 (d, 4H). MS (EI) for C₃₁H₃₁FN₄O₆, found 575 (MH+).

**5-(3,6-Dihydro-2H-pyran-4-yl)-N-[3-fluoro-4-[[6-methoxy-7-(2-methoxyethoxy)-1,5-naphthyridin-4-yl]oxy]phenyl]-4-hydroxy-6-methylpyridine-3-carboxamide (35):** In Step 1, Compound **A1-10** was replaced with Compound **A2-7** and Compound **D1-7** was replaced with Compound **D1-5.** In Step 2, 2-(cyclopent-1-en-1-yl)-4,4,5,5-tetramethyl-1,3,2-dioxaborolane was replaced with 2-(3,6-dihydro-2H-pyran-4-yl)-4,4,5,5-tetramethyl-1,3,2-dioxaborolane or the corresponding boronic acid. ¹H NMR (400 MHz, DMSO-*d₆*) δ 13.10 (s, 1H), 12.40 (s, 1H), 8.48 (d, 1H), 8.38 (s, 1H), 7.95 (dd, 1H), 7.61 (s, 1H), 7.37 (ddd, 1H), 7.26 (t, 1H), 6.75 (dd, 1H), 5.54 (p, 1H), 4.37 - 4.20 (m, 2H), 4.11 (d, 2H), 3.88 (s, 3H), 3.74 (t, 2H), 3.72 - 3.63 (m, 2H), 3.27 (s, 3H), 2.24 (s, 3H), 2.16 (s, 2H). MS (EI) for C₃₀H₂₉FN₄O₇, found 577 (MH+).

**5-[(E)-2-Cyclopentylethenyl]-N-[3-fluoro-4-[[6-methoxy-7-(2-methoxyethoxy)-1,5-naphthyridin-4-yl]oxy]phenyl]-4-hydroxy-6-methylpyridine-3-carboxamide (36):** In Step 1, Compound **A1-10** was replaced with Compound **A2-7** and Compound **D1-7** was replaced with Compound **D1-5.** In Step 2, 2-(cyclopent-1-en-1-yl)-4,4,5,5-tetramethyl-1,3,2-dioxaborolane was replaced with (E)-(2-cyclopentylvinyl)boronic acid or the corresponding boronic acid pinacol ester. ¹H NMR (400 MHz, DMSO-*d₆*) δ 13.20 (s, 1H), 12.40 (s, 1H), 8.48 (d, 1H), 8.32 (s, 1H), 7.96 (dd, 1H), 7.61 (s, 1H), 7.37 (ddd, 1H), 7.27 (t, 1H), 6.76 (dd, 1H), 6.61 (dd, 1H), 6.24 (dd, 1H), 4.37 - 4.18 (m, 2H), 3.89 (s, 3H), 3.80 - 3.61 (m, 2H), 3.27 (s, 3H), 2.52 (q, 1H), 2.35 (s, 3H), 1.83 - 1.70 (m, 2H), 1.70 - 1.44 (m, 4H), 1.41 - 1.25 (m, 2H). MS (EI) for C₃₂H₃₃FN₄O₆, found 589 (MH+).

**5-[(E)-2-Cyclopropylethenyl]-N-[3-fluoro-4-[[6-methoxy-7-(2-methoxyethoxy)-1,5-naphthyridin-4-yl]oxy]phenyl]-4-hydroxy-6-methylpyridine-3-carboxamide (37):** In Step 1, Compound **A1-10** was replaced with Compound **A2-7** and Compound **D1-7** was replaced with Compound **D1-5.** In Step 2, 2-(cyclopent-1-en-1-yl)-4,4,5,5-tetramethyl-1,3,2-dioxaborolane was replaced with (E)-(2-cyclopropylvinyl)boronic acid or the corresponding boronic acid pinacol ester. ¹H NMR (400 MHz, DMSO-*d₆*) δ 13.21 (s, 1H), 12.38 (s, 1H), 8.47 (s, 1H), 8.30 (s, 1H), 7.96 (dd, 1H), 7.61 (s, 1H), 7.35 (ddd, 1H), 7.26 (t, 1H), 6.76 (dd, 1H), 6.33 (d, 2H), 4.31 - 4.21 (m, 2H), 3.89 (s, 3H), 3.74 - 3.65 (m, 2H), 3.27 (s, 3H), 2.35 (s, 3H), 1.51 (tt, 1H), 0.74 (dd, 2H), 0.43 (dd, 2H). MS (EI) for C₃₀H₂₉FN₄O₆, found 561.0 (MH+).

**5-[(E)-3,3-Dimethylbut-1-enyl]-N-[3-fluoro-4-[[6-methoxy-7-(2-methoxyethoxy)-1,5-naphthyridin-4-yl]oxy]phenyl]-4-hydroxy-6-methylpyridine-3-carboxamide (40):** In Step 1, Compound **A1-10** was replaced with Compound **A2-7** and Compound **D1-7** was replaced with Compound **D1-5.** In Step 2, 2-(cyclopent-1-en-1-yl)-4,4,5,5-tetramethyl-1,3,2-dioxaborolane was replaced with (E)-(3,3-dimethylbut-l-en-1-yl)boronic acid or the corresponding boronic acid pinacol ester. ¹H NMR (400 MHz, DMSO-*d₆*) δ 13.20 (s, 1H), 12.42 (s, 1H), 8.48 (d, 1H), 8.33 (s, 1H), 59927.97 (dd, 1H), 7.61 (s, 1H), 7.44 - 7.33 (m, 1H), 7.26 (t, 1H), 6.76 (dd, 1H), 6.51 (d, 1H), 6.16 (d, 1H), 4.35 - 4.18 (m, 2H), 3.89 (s, 3H), 3.78 - 3.62 (m, 2H), 3.27 (s, 3H), 2.35 (s, 3H), 1.04 (s, 9H). MS (EI) for C₅₁H₃₃FN₄O₆, found 577 (MH+).

**5-(Cyclopenten-1-yl)-N-[3-fluoro-4-[[6-methoxy-7-(2-methoxyethoxy)-1,5-naphthyridin-4-yl]oxy]phenyl]-4-hydroxy-2,6-dimethylpyridine-3-carboxamide (41):** In Step 1, Compound **A1-10** was replaced with Compound **A2-7.** ¹H NMR (400 MHz, DMSO-*d₆*) δ 13.43 (s, 1H), 11.71 (s, 1H), 8.47 (d, 1H), 7.93 (dd, 1H), 7.61 (s, 1H), 7.34 - 7.29 (m, 1H), 7.23 (t, 1H), 6.72 (dd, 1H), 5.68 - 5.44 (m, 1H), 4.37 - 4.16 (m, 2H), 3.90 (s, 3H), 3.75 - 3.55 (m, 2H), 3.27 (s, 3H), 2.63 (s, 3H), 2.46 (s, 2H), 2.38 (s, 2H), 2.19 (s, 3H), 1.87 (p, 2H). MS (EI) for C₃₁H₃₁FN₄O₆, found 575 (MH+).

**5-(Cyclopenten-1-yl)-N-[3-fluoro-4-[[6-methoxy-7-(2-methoxyethoxy)-1,5-naphthyridin-4-yl]oxy]phenyl]-1,2,6-trimethyl-4-oxopyridine-3-carboxamide (42):** In Step 1, Compound **A1-10** was replaced with Compound **A2-7** and Compound **D1-7** was replaced with Compound **9-3.** ¹H NMR (400 MHz, DMSO-*d₆*) δ 11.47 (s, 1H), 8.45 (d, 1H), 7.88 (dd, 1H), 7.61 (s, 1H), 7.47 - 7.33 (m, 1H), 7.26 (t, 1H), 6.70 (dd, 1H), 5.48 - 5.36 (m, 1H), 4.33 - 4.19 (m, 2H), 3.91 (s, 3H), 3.77 - 3.57 (m, 2H), 3.53 (s, 3H), 3.27 (s, 3H), 2.45 (s, 3H), 2.38 (s, 4H), 2.29 (s, 3H), 1.87 (q, 2H). MS (EI) for C₃₂H₃₃FN₄O₆, found 589 (MH+).

**5-(3,6-Dihydro-2H-pyran-4-yl)-N-[3-fluoro-4-[[6-methoxy-7-(2-methoxyethoxy)-1,5-naphthyridin-4-yl]oxy]phenyl]-4-hydroxy-2,6-dimethylpyridine-3-carboxamide (43):** In Step 1, Compound **A1-10** was replaced with Compound **A2-7.** In Step 2, 2-(cyclopent-1-en-1-yl)-4,4,5,5-tetramethyl-1,3,2-dioxaborolane was replaced with 2-(3,6-dihydro-2H-pyran-4-yl)-4,4,5,5-tetramethyl-1,3,2-dioxaborolane or the corresponding boronic acid. ¹H NMR (400 MHz, DMSO-*d₆*) δ 13.38 (s, 1H), 11.73 (s, 1H), 8.47 (d, 1H), 7.93 (dd, 1H), 7.61 (s, 1H), 7.32 (ddd, 1H), 7.23 (t, 1H), 6.72 (dd, 1H), 5.49 (dd, 1H), 4.34 - 4.19 (m, 2H), 4.10 (q, 2H), 3.90 (s, 3H), 3.72 (t, 2H), 3.70 - 3.66 (m, 2H), 3.27 (s, 3H), 2.64 (s, 3H), 2.21 (s, 3H), 2.15 (s, 2H). MS (EI) for C₃₁H₃₁FN₄O₇, found 591 (MH+).

**5-(3,6-Dihydro-2H-pyran-4-yl)-N-[3-fluoro-4-[[6-methoxy-7-(2-methoxyethoxy)-1,5-naphthyridin-4-yl]oxy]phenyl]-1,2,6-trimethyl-4-oxopyridine-3-carboxamide (44):** In Step 1, Compound **A1-10** was replaced with Compound **A2-7** and Compound **D1-7** was replaced with Compound **9-3.** In Step 2, 2-(cyclopent-1-en-1-yl)-4,4,5,5-tetramethyl-1,3,2-dioxaborolane was replaced with 2-(3,6-dihydro-2H-pyran-4-yl)-4,4,5,5-tetramethyl-1,3,2-dioxaborolane or the corresponding boronic acid. ¹H NMR (400 MHz, DMSO-*d₆*) δ 11.43 (s, 1H), 8.45 (d, 1H), 7.88 (dd, 1H), 7.61 (s, 1H), 7.49 - 7.35 (m, 1H), 7.27 (t, 1H), 6.70 (dd, 1H), 5.41 (d, 1H), 4.36 - 4.17 (m, 2H), 4.09 (d, 2H), 3.91 (s, 3H), 3.83 - 3.63 (m, 4H), 3.53 (s, 3H), 3.27 (s, 3H), 2.46 (s, 3H), 2.31 (s, 3H), 2.10 (s, 2H). MS (EI) for C₃₂H₃₃FN₄O₇, found 605 (MH+).

**5-[(E)-3,3-Dimethylbut-1-enyl]-N-[3-fluoro-4-[[6-methoxy-7-(2-methoxyethoxy)-1,5-naphthyridin-4-yl]oxy]phenyl]-1,2,6-trimethyl-4-oxopyridine-3-carboxamide (45):** In Step 1, Compound **A1-10** was replaced with Compound **A2-7** and Compound **D1-7** was replaced with Compound **9-3.** In Step 2, 2-(cyclopent-1-en-1-yl)-4,4,5,5-tetramethyl-1,3,2-dioxaborolane was replaced with (E)-(3,3-dimethylbut-1-en-1-yl)boronic acid or the corresponding boronic acid pinacol ester. ¹H NMR (400 MHz, DMSO-*d₆*) δ 11.19 (s, 1H), 8.45 (d, 1H), 7.89 (dd, 1H), 7.61 (s, 1H), 7.40 (ddd, 1H), 7.27 (t, 1H), 6.70 (dd, 1H), 6.12 (d, 2H), 4.30 - 4.19 (m, 2H), 3.92 (s, 3H), 3.76 - 3.64 (m, 2H), 3.54 (s, 3H), 3.27 (s, 3H), 2.44 (s, 3H), 2.40 (s, 3H), 1.02 (s, 9H). MS (EI) for C₃₃H₃₇FN₄O₆, found 605 (MH+).

**N-[4-[(6,7-Dimethoxy-1,5-naphthyridin-4-yl)oxy]-3-fluorophenyl]-5-[(E)-3,3-dimethylbut-1-enyl]-1,2,6-trimethyl-4-oxopyridine-3-carboxamide (49):** In Step 1, Compound **D1-7** was replaced with Compound **9-3.** In Step 2, 2-(cyclopent-1-en-1-yl)-4,4,5,5-tetramethyl-1,3,2-dioxaborolane was replaced with (E)-(3,3-dimethylbut-l-en-1-yl)boronic acid or the corresponding boronic acid pinacol ester. ¹H NMR (400 MHz, DMSO-*d₆*) δ 11.27 (s, 1H), 8.54 (d, 1H), 7.96 (dd, 1H), 7.66 (s, 1H), 7.50 - 7.43 (m, 1H), 7.34 (t, 1H), 6.78 (dd, 1H), 6.20 (s, 2H), 3.98 (d, 6H), 3.61 (s, 3H), 2.52 (s, 3H), 2.48 (s, 3H), 1.09 (s, 9H). MS (EI) for C₃₁H₃₃FN₄O₅, found 561 (MH+).

**5-[(E)-2-Cyclopropylethenyl]-N-[4-[(6,7-dimethoxy-1,5-naphthyridin-4-yl)oxy]-3-fluorophenyl]-1,2,6-trimethyl-4-oxopyridine-3-carboxamide (50):** In Step 1, Compound **D1-7** was replaced with Compound **9-3.** In Step 2, 2-(cyclopent-1-en-1-yl)-4,4,5,5-tetramethyl-1,3,2-dioxaborolane was replaced (E)-(2-cyclopropylvinyl)boronic acid or the corresponding boronic acid pinacol ester. ¹H NMR (400 MHz, DMSO-*d₆*) δ 11.11 (s, 1H), 8.46 (d, 1H), 7.88 (dd, 1H), 7.58 (s, 1H), 7.39 (ddd, 1H), 7.27 (t, 1H), 6.70 (dd, 1H), 6.29 (d, 1H), 5.91 (dd, 1H), 3.91 (d, 6H), 3.53 (s, 3H), 2.44 (s, 3H), 2.39 (s, 3H), 1.55 - 1.40 (m, 1H), 0.71 (dd, 2H), 0.36 (dd, 2H). MS (EI) for C₃₀H₂₉FN₄O₅, found 545 (MH+).

**5-[(E)-2-Cyclopropylethenyl]-N-[3-fluoro-4-[[6-methoxy-7-(2-methoxyethoxy)-1,5-naphthyridin-4-yl]oxy]phenyl]-1,2,6-trimethyl-4-oxopyridine-3-carboxamide (51):** In Step 1, Compound **A1-10** was replaced with Compound **A2-7** and Compound **D1-7** was replaced with Compound **9-3.** In Step 2, 2-(cyclopent-1-en-1-yl)-4,4,5,5-tetramethyl-1,3,2-dioxaborolane was replaced with (E)-(2-cyclopropylvinyl)boronic acid or the corresponding boronic acid pinacol ester. ¹H NMR (400 MHz, DMSO-*d₆*) δ 11.11 (s, 1H), 8.45 (d, 1H), 7.88 (dd, 1H), 7.61 (s, 1H), 7.39 (ddd, 1H), 7.27 (t, 1H), 6.69 (dd, 1H), 6.29 (d, 1H), 5.91 (dd, 1H), 4.31 - 4.18 (m, 2H), 3.92 (s, 3H), 3.71 - 3.61 (m, 2H), 3.53 (s, 3H), 3.27 (s, 3H), 2.44 (s, 3H), 2.39 (s, 3H), 1.49 (dt, 1H), 0.76 - 0.61 (m, 2H), 0.41 - 0.28 (m, 2H). MS (EI) for C₃₂H₃₃FN₄O₆, found 589 (MH+).

**5-(3,6-Dihydro-2H-pyran-4-yl)-N-[4-[(6,7-dimethoxy-1,5-naphthyridin-4-yl)oxy]-3-fluorophenyl]-1,2,6-trimethyl-4-oxopyridine-3-carboxamide (53):** In Step 1, Compound **D1-7** was replaced with Compound **9-3.** In Step 2, 2-(cyclopent-1-en-1-yl)-4,4,5,5-tetramethyl-1,3,2-dioxaborolane was replaced with 2-(3,6-dihydro-2H-pyran-4-yl)-4,4,5,5-tetramethyl-1,3,2-dioxaborolane or the corresponding boronic acid. ¹H NMR (400 MHz, DMSO-*d₆*) δ 11.43 (s, 1H), 8.46 (d, 1H), 7.88 (dd, 1H), 7.58 (s, 1H), 7.39 (ddd, 1H), 7.26 (t, 1H), 6.71 (dd, 1H), 5.41 (t, 1H), 4.19 - 3.99 (m, 2H), 3.90 (d, 6H), 3.71 (t, 2H), 3.53 (s, 3H), 2.46 (s, 3H), 2.31 (s, 3H), 2.10 (s, 2H). MS (EI) for C₃₀H₂₉FN₄O₆, found 561 (MH+).

**5-[(E)-2-Cyclopentylethenyl]-N-[3-fluoro-4-[[6-methoxy-7-(2-methoxyethoxy)-1,5-naphthyridin-4-yl]oxy]phenyl]-1,2,6-trimethyl-4-oxopyridine-3-carboxamide (54):** In Step 1, Compound **A1-10** was replaced with Compound **A2-7** and Compound **D1-7** was replaced with Compound **9-3.** In Step 2, 2-(cyclopent-1-en-1-yl)-4,4,5,5-tetramethyl-1,3,2-dioxaborolane was replaced with (E)-(2-cyclopentylvinyl)boronic acid or the corresponding boronic acid pinacol ester. ¹H NMR (400 MHz, DMSO-*d₆*) δ 11.14 (s, 1H), 8.45 (d, 1H), 7.88 (dd, 1H), 7.61 (s, 1H), 7.45 - 7.33 (m, 1H), 7.27 (t, 1H), 6.70 (dd, 1H), 6.19 (d, 2H), 4.31 - 4.22 (m, 2H), 3.92 (s, 3H), 3.69 (dd, 2H), 3.53 (s, 3H), 3.27 (s, 3H), 2.53 - 2.46 (m, 1H), 2.44 (s, 3H), 2.40 (d, 3H), 1.80 - 1.68 (m, 2H), 1.68 - 1.55 (m, 2H), 1.55 - 1.47 (m, 2H), 1.28 (t, 2H). MS (EI) for C₃₄H₃₇FN₄O₆, found 617.25 (MH+).

**5-(Cyclohexen-1-yl)-N-[3-fluoro-4-[[6-methoxy-7-(2-methoxyethoxy)-1,5-naphthyridin-4-yl]oxy]phenyl]-1,2,6-trimethyl-4-oxopyridine-3-carboxamide (58):** In Step 1, Compound **A1-10** was replaced with Compound **A2-7** and Compound **D1-7** was replaced with Compound **9-3.** In Step 2, 2-(cyclopent-1-en-1-yl)-4,4,5,5-tetramethyl-1,3,2-dioxaborolane was replaced with 2-(cyclohex-1-en-1-yl)-4,4,5,5-tetramethyl-1,3,2-dioxaborolane or the corresponding boronic acid. ¹H NMR (400 MHz, DMSO-*d₆*) δ 11.60 (s, 1H), 8.46 (d, 1H), 7.88 (dd, 1H), 7.61 (s, 1H), 7.39 (ddd, 1H), 7.26 (t, 1H), 6.70 (dd, 1H), 5.32 (t, 1H), 4.24 (d, 2H), 3.91 (s, 3H), 3.84 - 3.63 (m, 2H), 3.52 (s, 3H), 3.27 (s, 3H), 2.47 (s, 3H), 2.29 (s, 3H), 2.03 (s, 2H), 1.65 (d, 6H).MS (EI) for C₃₃H₃₅FN₄O₆, found 603 (MH+).

**5-(Cyclopenten-1-yl)-N-[3-fluoro-4-[[7-(2-methoxyethoxy)-1,5-naphthyridin-4-yl]oxy]phenyl]-1,2,6-trimethyl-4-oxopyridine-3-carboxamide (65):** In Step 1, Compound **A1-10** was replaced with Compound **A2-9** and Compound **D1-7** was replaced with Compound **9-3.** ¹H NMR (400 MHz, DMSO-*d₆*) δ 11.46 (s, 1H), 8.69 (d, 1H), 8.62 (d, 1H), 7.92 (dd, 1H), 7.76 (d, 1H), 7.48 - 7.39 (m, 1H), 7.34 (t, 1H), 6.67 (dd, 1H), 5.42 (t, 1H), 4.40 - 4.20 (m, 2H), 3.77 - 3.66 (m, 2H), 3.53 (s, 3H), 3.29 (s, 3H), 2.45 (s, 3H), 2.42 - 2.34 (m, 4H), 2.29 (s, 3H), 1.87 (p, 2H). MS (EI) for C₃₁H₃₁FN₄O₅, found 559 (MH+).

**5-(Cyclohexen-1-yl)-N-[3-fluoro-4-[[7-(2-methoxyethoxy)-1,5-naphthyridin-4-yl]oxy]phenyl]-1,2,6-trimethyl-4-oxopyridine-3-carboxamide (66):** In Step 1, Compound **A1-10** was replaced with Compound **A2-9** and Compound **D1-7** was replaced with Compound **9-3.** In Step 2, 2-(cyclopent-1-en-1-yl)-4,4,5,5-tetramethyl-1,3,2-dioxaborolane was replaced with 2-(cyclohex-1-en-1-yl)-4,4,5,5-tetramethyl-1,3,2-dioxaborolane or the corresponding boronic acid. ¹H NMR (400 MHz, DMSO-*d₆*) δ 11.59 (s, 1H), 8.70 (d, 1H), 8.63 (d, 1H), 7.92 (dd, 1H), 7.76 (d, 1H), 7.43 (ddd, 1H), 7.33 (t, 1H), 6.67 (dd, 1H), 5.32 (s, 1H), 4.38 - 4.23 (m, 2H), 3.78 - 3.64 (m, 2H), 3.52 (s, 3H), 3.29 (s, 3H), 2.47 (s, 3H), 2.29 (s, 3H), 2.04 (s, 2H), 1.66 (d, 6H). MS (EI) for C₃₂H₃₃FN₄O₅, found 573 (MH+).

**5-(3,6-Dihydro-2H-pyran-4-yl)-N-[3-fluoro-4-[[7-(2-methoxyethoxy)-1,5-naphthyridin-4-yl]oxy]phenyl]-1,2,6-trimethyl-4-oxopyridine-3-carboxamide (67):** In Step 1, Compound **A1-10** was replaced with Compound **A2-9** and Compound **D1-7** was replaced with Compound **9-3.** In Step 2, 2-(cyclopent-1-en-1-yl)-4,4,5,5-tetramethyl-1,3,2-dioxaborolane was replaced with 2-(3,6-dihydro-2H-pyran-4-yl)-4,4,5,5-tetramethyl-1,3,2-dioxaborolane or the corresponding boronic acid. ¹H NMR (400 MHz, DMSO-*d₆*) δ 11.50 (s, 1H), 8.77 (d, 1H), 8.70 (d, 1H), 7.99 (dd, 1H), 7.83 (d, 1H), 7.51 (ddd, 1H), 7.41 (t, 1H), 6.74 (dd, 1H), 5.49 (d, 1H), 4.47 - 4.29 (m, 2H), 4.17 (d, 2H), 3.85 - 3.71 (m, 4H), 3.60 (s, 3H), 3.36 (s, 3H), 2.52 (s, 3H), 2.39 (s, 3H), 2.20 (d, 2H). MS (EI) for C₅₁H₃₁FN₄O₆, found 575 (MH+).

**5-(Cyclopenten-1-yl)-N-[3-fluoro-4-[[7-(2-methoxyethoxy)-1,5-naphthyridin-4-yl]oxy]phenyl]-4-hydroxy-2,6-dimethylpyridine-3-carboxamide (101):** In Step 1, Compound **A1-10** was replaced with Compound **A2-9.** ¹H NMR (400 MHz, DMSO-*d₆*) δ 13.53 (s, 1H), 11.80 (s, 1H), 8.77 (d, 1H), 8.71 (d, 1H), 8.04 (dd, 1H), 7.83 (d, 1H), 7.46 - 7.26 (m, 2H), 6.74 (dd, 1H), 5.60 (s, 1H), 4.45 - 4.29 (m, 2H), 3.86 - 3.68 (m, 2H), 3.36 (s, 3H), 2.71 (s, 3H), 2.6 - 2.5 (bm, 2H), 2.47 - 2.42 (m, 2H), 2.27 (s, 3H), 2.05 - 1.85 (m, 2H); MS (EI) for C₃₀H₂₉FN₄O₅, found 545 (MH+).

### Example 2: 5-(Cyclohexen-1-yl)-N-[4-[(6,7-dimethoxy-1,5-naphthyridin-4-yl)oxy]-3-fluorophenyl]-4-hydroxy-2,6-dimethylpyridine-3-carboxamide (2-3):

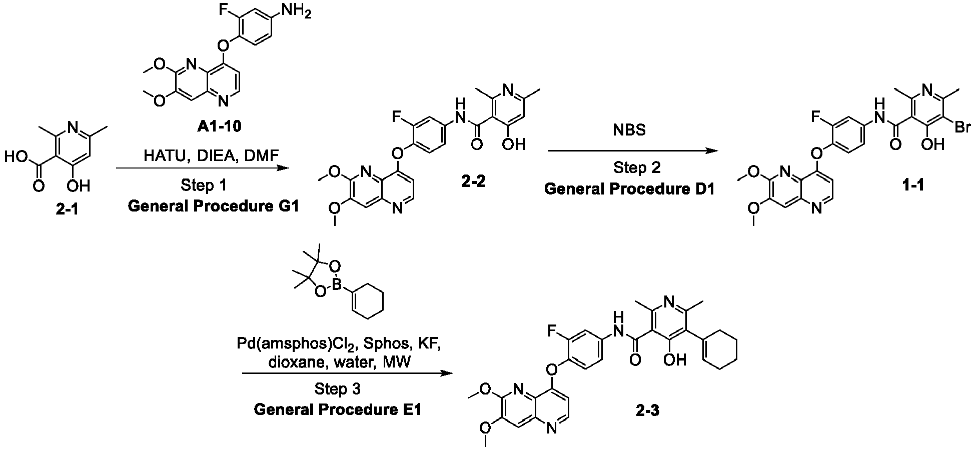

**Step 1: N-(4-((6,7-Dimethoxy-1,5-naphthyridin-4-yl)oxy)-3-fluorophenyl)-4-hydroxy-2,6-dimethylnicotinamide (2-2):** Compound **2-2** was synthesized from Compound **2-1** and Compound **A1-10** using **General Procedure G1.** MS for C₂₄H₂₁FN₄O₅: *m*/*z* 465.1 (MH+). See **Example 4** for the synthesis of Compound **2-1.**

**Step 2: 5-Bromo-N-(4-((6,7-dimethoxy-1,5-naphthyridin-4-yl)oxy)-3-fluorophenyl)-4-hydroxy-2,6-dimethylnicotinamide (1-1):** Compound **1-1** was synthesized from Compound **2-2** using **General Procedure D1.** MS for C₂₄H₂₀BrFN₄O₅: *m*/*z* 544.7 (MH+).

**Step 3: 5-(Cyclohexen-1-yl)-N-[4-[(6,7-dimethoxy-1,5-naphthyridin-4-yl)oxy]-3-fluorophenyl]-4-hydroxy-2,6-dimethylpyridine-3-carboxamide (2-3):** Compound **2-3** was synthesized from Compound **1-1** using **General Procedure E1.** ¹H NMR (400 MHz, DMSO-*d₆*) δ 13.14 (br s, 1H), 12.80 (br s, 1H), 8.90 - 8.79 (m, 1H), 8.18 - 8.04 (m, 1H), 7.90 (s, 1H), 7.56 - 7.47 (m, 2H), 7.21 - 7.15 (m, 1H), 5.54 (br s, 1H), 4.13 - 4.05 (m, 6H), 2.70 (s, 3H), 2.35 (s, 3H), 2.12 (br s, 4H), 1.68 (br d, 4H); MS for C₃₀H₂₀FN₄O₅: *m*/*z* 545.1 (MH+).

### Example 3: Methyl 8-oxo-1,3,4,8-tetrahydropyrido[2,1-c][1,4]oxazine-9-carboxylate (D1-3)

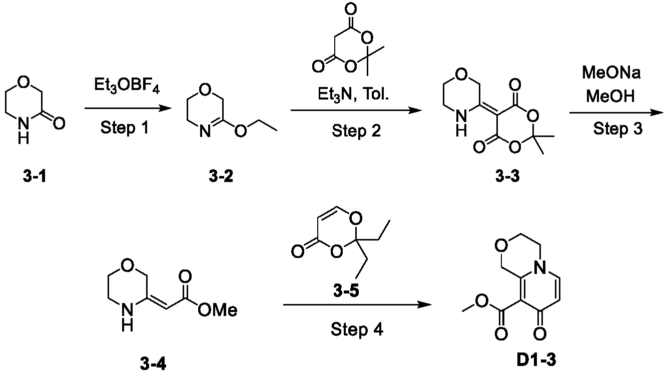

**Step 1: 5-Ethoxy-3,6-dihydro-2H-1,4-oxazine (3-2):** To a solution of Compound **3-1** (5 g, 49.4 mmol, 1 eq) in DCM (80 mL) was added Et₃OBF₄ (10.3 g, 54.4 mmol, 1.1 eq) at 20 °C and the resulting reaction mixture was stirred at 20 °C for 12 h . The reaction mixture was quenched by the addition of aq saturated Na₂CO₃ to pH=8. The organic layer was separated, dried over anhyd. Na₂SO₄ and concentrated *in vacuo* to give crude Compound **3-2** as a yellow oil (6.3 g, 99% yield) which was used without further purification. ¹H NMR (400 MHz, CDCl₃) δ 4.09 (m, 2H), 4.03 (s, 2H), 3.62-3.68 (m, 2H), 3.49-3.55 (m, 2H), 1.26 (m, 3H).

**Step 2: 2,2-Dimethyl-5-(morpholin-3-ylidene)-1,3-dioxane-4,6-dione (3-3):** A solution of Compound **3-2** (5 g, 39 mmol, 1 eq), 2,2-dimethyl-1,3-dioxane-4,6-dione (5.6 g, 39 mmol, 1 eq) and Et₃N (1.08 mL, 7.74 mmol, 0.2 eq) in toluene (50 mL) was stirred at 105 °C for 3 h. The mixture was cooled to room temperature and the solvent evaporated *in vacuo.* The resulting residue was purified by flash silica gel chromatography (0~50% EtOAc /petroleum ether) to give Compound **3-3** as a yellow solid (1.4 g, 16% yield). ¹H NMR (400 MHz, CDCl₃) δ 11.48 (br s, 1H), 5.05 (s, 2H), 3.94 (m, 2H), 3.55-3.63 (m, 2H), 1.68 (s, 6H).

**Step 3: Methyl (E)-2-(morpholin-3-ylidene)acetate (3-4):** A solution of Compound **3-3** (1.3 g, 5.7 mmol, 1 eq) and NaOMe (371 mg, 6.9 mmol, 1.2 eq) in MeOH (30 mL) was stirred at 80 °C for 12 h. The mixture was cooled to room temperature and then concentrated *in vacuo.* The resulting residue was dissolved in aq saturated NH₄Cl (100 mL) and extracted with EtOAc (3 x 50 mL). The combined organic extracts were dried over anhyd. Na₂SO₄ and concentrated *in vacuo* to give Compound **3-4** as a yellow solid (617 mg, 69% yield) which was used without further purification. ¹H NMR (400 MHz, CDCl₃) δ 8.56 (br s, 1H), 4.34 (s, 1H), 4.28 (s, 2H), 3.88-3.94 (m, 2H), 3.64 (s, 3H), 3.36 (m, 2H); MS for C₇H₁₁NO₃: *m*/*z* 157.9 (MH+).

**Step 4: 5 Methyl 8-oxo-1,3,4,8-tetrahydropyrido[2,1-c][1,4]oxazine-9-carboxylate (D1-3):** A mixture of Compound **3-4** (300 mg, 1.9 mmol, 1 eq) and Compound **3-5** (596 mg, 3.8 mmol, 2 eq) was stirred at 130 °C for 1.5 h with Dean-Stark trap removal of water. The reaction mixture was concentrated under vacuum. The resulting residue was purified by flash silica gel chromatography (0~10% MeOH in DCM) to give Compound **D1-3** as a yellow solid (220 mg, 55% yield). ¹H NMR (400 MHz, CDCl₃) δ 7.17 (d, 1H), 6.48 (d, 1H), 4.83 (s, 2H), 4.08 - 4.05 (t, 2H), 3.95 - 3.92 (t, 2H), 3.90 (s, 3H); MS for C₁₀H₁₁NO₄: *m*/*z* 209.9 (MH+).

### Example 4: 4-Hydroxy-2,6-dimethylnicotinic acid (2-1)

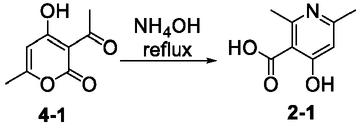

**4-Hydroxy-2,6-dimethylnicotinic acid (2-1):** To a solution of commercially available Compound **4-1** (10 g, 59.88 mmol) in water (60 mL) was added ammonium hydroxide (60 mL, 30% in water). The resulting mixture was heated to reflux overnight. The mixture was partially concentrated and then acidified to pH 2 with aq 6 M HCl. The resulting precipitate was filtered and allowed to dry in the open air to afford Compound 2-1 as a white solid (2.5 g, 25% yield). MS for C₈H₉NO₃: *m*/*z* 168 (MH+).

### Example 5: 5-(Cyclopenten-1-yl)-N-[4-[(6,7-dimethoxy-1,5-naphthyridin-4-yl)oxy]-3-fluorophenyl]-1,6-dimethyl-4-oxopyridine-3-carboxamide (1)

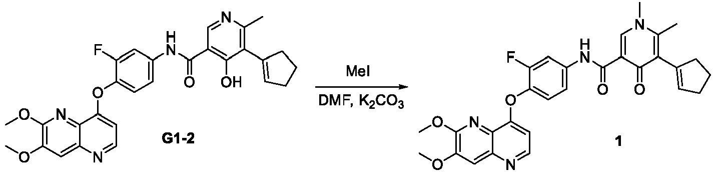

**5-(Cyclopenten-1-yl)-N-[4-[(6,7-dimethoxy-1,5-naphthyridin-4-yl)oxy]-3-fluorophenyl]-1,6-dimethyl-4-oxopyridine-3-carboxamide (1):** A mixture of Compound **G1-2** (49 mg, 0.095 mmol), DMF (0.70 mL), K₂CO₃ (50 mg, 0.36 mmol) and iodomethane (100 mg, 0.70 mmol) was stirred at ambient temperature. After 6 h, the reaction was diluted with water (5 mL). The resulting precipitate was collected and purified over silica (24 g, 0% to 10% MeOH in DCM) to give Compound **1** as a white solid (24 mg, 48% yield). ¹H NMR (400 MHz, CDCl₃) δ 12.84 (s, 1H), 8.47 - 8.39 (m, 2H), 7.89 (dd, 1H), 7.49 (s, 1H), 7.31 (ddd, 1H), 7.09 (t, 1H), 6.67 (dd, 1H), 5.61 - 5.55 (p, 1H), 4.05 (s, 3H), 3.96 (s, 3H), 3.72 (s, 3H), 2.51 (t, 4H), 2.31 (s, 3H), 2.01 (m, 2H); MS for C₂₉H₂₇FN₄O₅: *m*/*z* 531.1 (MH+).

**The following compounds were synthesized using similar alkylation methods to that** exemplified in **Example 5** in the conversion of Compound **G1-2** to Compound **1:**

**5-(Cyclohexen-1-yl)-N-[4-[(6,7-dimethoxy-1,5-naphthyridin-4-yl)oxy]-3-fluorophenyl]-1-(2-fluoroethyl)-6-methyl-4-oxopyridine-3-carboxamide (8):** Compound **G1-2** was replaced with Compound **G1-4,** iodomethane was replaced by 1-fluoro-2-iodo-ethane. ¹H NMR (400 MHz, CD₃OD) δ 8.67 (d, 1H), 8.49 (dd, 1H), 7.99 (ddd, 1H), 7.55 - 7.49 (m, 1H), 7.42 - 7.35 (m, 1H), 7.29 (td, 1H), 6.84 (dd, 1H), 5.60 (dq, 1H), 4.88 (t, 1H), 4.76 (t, 1H), 4.60 (t, 1H), 4.54 (t, 1H), 4.08 - 4.04 (m, 3H), 4.03 (d, 3H), 2.51 - 2.46 (m, 3H), 2.42 - 2.34 (m, 1H), 2.25 - 2.21 (m, 2H), 1.99 - 1.63 (m, 5H); MS for C₃₁H₃₀F₂N₄O₅: *m*/*z* 577.1 (MH+).

**5-(Cyclopenten-1-yl)-N-[4-[(6,7-dimethoxy-1,5-naphthyridin-4-yl)oxy]-3-fluorophenyl]-1-(2-fluoroethyl)-6-methyl-4-oxopyridine-3-carboxamide (11):** Iodomethane was replaced by 1-fluoro-2-iodo-ethane. MS for C₃₀H₂₈F₂N₄O₅: *m*/*z* 563.2 (MH+).

### Example 6: 5-Cyclopentyl-N-[4-[(6,7-dimethoxy-1,5-naphthyridin-4-yl)oxy]-3-fluorophenyl]-1,6-dimethyl-4-oxopyridine-3-carboxamide (2)

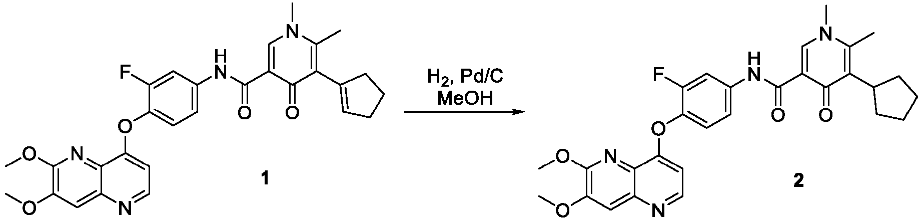

**5-Cyclopentyl-N-[4-[(6,7-dimethoxy-1,5-naphthyridin-4-yl)oxy]-3-fluorophenyl]-1,6-dimethyl-4-oxopyridine-3-carboxamide (2).** A mixture of Compound **1** (17 mg, 0.032 mmol), MeOH (0.5 mL) ammonium formate (100 mg, 1.59 mmol) and palladium hydroxide (10 mg, 0.071 mmol) was heated to reflux in a sealed 2-dram vial fit with a pressure release cap. After 4 h, the mixture was diluted with DCM and filtered through Celite. The filtrate was concentrated to dryness to give Compound 2 as a colorless solid (11.3 mg, 66% yield). ¹H NMR (400 MHz, DMSO-*d₆*) δ 13.06 (s, 1H), 8.52 (d, 1H), 8.49 (s, 1H), 7.96 (dd, 1H), 7.51 (s, 1H), 7.42 (dt, 1H), 7.18 (t, 1H), 6.73 (d, 1H), 4.13 (s, 3H), 4.03 (s, 3H), 3.77 (s, 3H), 3.49 (t, 1H), 2.44 (s, 3H), 2.03 - 1.89 (m, 4H), 1.85 - 1.77 (m, 2H), 1.70 (s, 2H); MS for C₂₀H₂₀FN₄O₅: *m*/*z* 533.2 (MH+).

### Example 7: N-[3-Fluoro-4-[(7-methoxy-1,5-naphthyridin-4-yl)oxy]phenyl]-4-hydroxy-2,6-dimethyl-5-prop-1-en-2-ylpyridine-3-carboxamide (3)

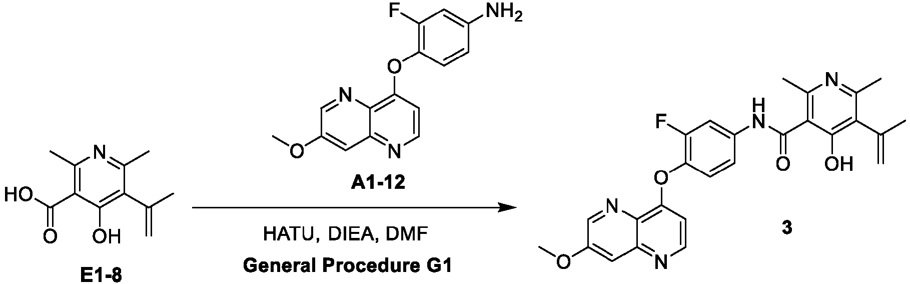

**N-[3-Fluoro-4-[(7-methoxy-1,5-naphthyridin-4-yl)oxy] phenyl]-4-hydroxy-2,6-dimethyl-5-prop-1-en-2-ylpyridine-3-carboxamide (3):** Compound 3 was made from Compound **E1-8** and Compound **A1-12** using **General Procedure G1.** ¹H NMR (400 MHz, DMSO-*d₆*) δ 13.51 (s, 1H), 11.81 (s, 1H), 8.76 (d, 1H), 8.71 (d, 1H), 8.04 (dd, 1H), 7.81 (d, 1H), 7.43 (dd, 1H), 7.40 (d, 1H), 6.74 (dd, 1H), 5.27 - 5.21 (m, 1H), 4.79 (d, 1H), 4.01 (s, 3H), 2.71 (s, 3H), 2.28 (s, 3H), 1.94 (s, 3H). MS for C₂₆H₂₃FN₄O₄: *m*/*z* 475 (MH+).

The following compounds were made using **General Procedure G1** in the same manner that Compound **3** was made from Compound **A1-12** and Compound **E1-8** in **Example 7:**

**N-[4-[(6,7-Dimethoxy-1,5-naphthyridin-4-yl)oxy]phenyl]-4-hydroxy-2,6-dimethyl-5-prop-1-en-2-ylpyridine-3-carboxamide (4):** Compound **4** was made from Compound **E1-8** and Compound **A1-11** using **General Procedure G1.** ¹H NMR (400 MHz, DMSO-*d*₆) δ 13.25 (s, 1H), 11.74 (s, 1H), 8.53 (d, 1H), 7.80 - 7.72 (m, 2H), 7.64 (s, 1H), 7.21 - 7.12 (m, 2H), 6.77 (d, 1H), 5.26 - 5.20 (m, 1H), 4.78 (t, 1H), 3.96 (d, 6H), 2.70 (s, 3H), 2.27 (s, 3H), 1.94 (s, 3H). MS for C₂₇H₂₆N₄O₅: *m*/*z* 487 (MH+).

**N-[4-[(6,7-Dimethoxy-1,5-naphthyridin-4-yl)oxy]-3-fluorophenyl]-4-hydroxy-2,6-dimethyl-5-prop-1-en-2-ylpyridine-3-carboxamide (5):** Compound **5** was made from Compound **E1-8** and Compound **A1-10** using **General Procedure G1.** ¹H NMR (400 MHz, DMSO-*d₆*) δ 13.43 (s, 1H), 11.73 (s, 1H), 8.47 (d, 1H), 7.93 (dd, 1H), 7.58 (s, 1H), 7.31 (dd, 1H), 7.24 (t, 1H), 6.72 (d, 1H), 5.19 - 5.13 (m, 1H), 4.71 (d, 1H), 3.90 (d, 6H), 2.63 (s, 3H), 2.20 (s, 3H), 1.86 (s, 3H). MS for C₂₇H₂₅FN₄O₅: *m*/*z* 505 (MH+).

**N-[4-[(6,7-Dimethoxy-1,5-naphthyridin-4-yl)oxy]-2,5-difluorophenyl]-4-hydroxy-2,6-dimethyl-5-prop-1-en-2-ylpyridine-3-carboxamide (6):** Compound **6** was made from Compound **E1-8** and Compound **A1-15** using **General Procedure G1.** ¹H NMR (400 MHz, DMSO-*d₆*) δ 14.18 (s, 1H), 11.91 (s, 1H), 8.60 - 8.51 (m, 2H), 7.67 (s, 1H), 7.52 (dd, 1H), 6.92 (d, 1H), 5.25 (s, 1H), 4.79 (s, 1H), 3.96 (d, 6H), 2.77 (s, 3H), 2.29 (s, 3H), 1.93 (s, 3H). MS for C₂₇H₂₄F₂N₄O₅: *m*/*z* 523 (MH+).

**1-(Cyclopenten-1-yl)-N-[4-[(6,7-dimethoxy-1,5-naphthyridin-4-yl)oxy]-3-fluorophenyl]-4,6-dimethyl-2-oxopyridine-3-carboxamide (14):** Compound 14 was made from Compound 12-3 and Compound A1-10 using General Procedure G1. MS for C₂₉H₂₇FN₄O₅ *m*/*z* 531.1 (MH+).

**1- 1-Cyclopentyl-N-[4-[(6,7-dimethoxy-1,5-naphthyridin-4-yl)oxy]-3-fluorophenyl]-4,6-dimethyl-2-oxopyridine-3-carboxamide (15):** Compound **15** was made from Compound **F2-2** and Compound **A1-10** using **General Procedure G1.** ¹H NMR (400 MHz, DMSO-*d₆*) δ 10.88 (s, 1H), 8.46 (d, 1H), 7.87 (dd, 1H), 7.58 (s, 1H), 7.39 (ddd, 1H), 7.27 (t, 1H), 6.71 (dd, 1H), 6.09 (s, 1H), 4.65 (p, 1H), 3.91 (s, 3H), 3.90 (s, 3H), 2.38 (s, 3H), 2.18 (s, 2H), 2.13 (s, 3H), 1.92 - 1.82 (m, 2H), 1.79 - 1.72 (m, 2H), 1.54 - 1.45 (m, 2H). MS for C₂₉H₂₉FN₄O₅: *m*/*z* 533.2 (MH+).

**N-[4-[(6,7-Dimethoxy-1,5-naphthyridin-4-yl)oxy]-3-fluorophenyl]-4,6-dimethyl-2-oxo-1-prop-1-en-2-ylpyridine-3-carboxamide (20):** Compound **20** was made from Compound **12-4** and Compound **A1-10** using **General Procedure G1.** ¹H NMR (400 MHz, DMSO-*d₆*) δ 11.18 (s, 1H), 8.53 (d, 1H), 7.95 (dd, 1H), 7.65 (s, 1H), 7.46 (ddd, 1H), 7.34 (dd, 1H), 6.79 (dd, 1H), 6.28 (s, 1H), 5.47 (d, 1H), 5.04 (s, 1H), 3.99 - 3.95 (m, 6H), 2.30 (s, 3H), 2.30 (s, 3H), 2.02 (s, 3H). MS for C₂₇H₂₅FN₄O₅: *m*/*z* 505.1 (MH+).

**N-[3-Fluoro-4-[(7-methoxy-1,5-naphthyridin-4-yl)oxy]phenyl]-4,6-dimethyl-2-oxo-1-prop-1-en-2-ylpyridine-3-carboxamide (21):** Compound **21** was made from Compound **12-4** and Compound **A1-12** using **General Procedure G1.** ¹H NMR (400 MHz, DMSO-*d₆*) δ 11.20 (s, 1H), 8.76 (d, 1H), 8.71 (d, 1H), 7.99 (dd, 1H), 7.81 (d, 1H), 7.51 (ddd, 1H), 7.41 (dd, 1H), 6.76 (dd, 1H), 6.29 (s, 1H), 5.48 (d, 1H), 5.05 (s, 1H), 4.01 (s, 3H), 2.31 (s, 3H), 2.31 (s, 3H), 2.04 (s, 3H). MS for C₂₆H₂₃FN₄O₄: *m*/*z* 475.1 (MH+).

**N-[4-[(6,7-Dimethoxy-1,5-naphthyridin-4-yl)oxy]-3-fluorophenyl]-4-hydroxy-2,6-dimethyl-5-propan-2-ylpyridine-3-carboxamide (31):** Compound **31** was made from Compound **F2-1** and Compound **A1-10** using **General Procedure G1.** ¹H NMR (400 MHz, DMSO-*d*₆) δ 13.58 (s, 1H), 11.47 - 11.43 (m, 1H), 8.47 (d, 1H), 7.93 (dd, 1H), 7.58 (s, 1H), 7.30 (dd, 1H), 7.23 (t, 1H), 6.73 (d, 1H), 3.90 (d, 6H), 3.12 - 3.04 (m, 1H), 2.59 (s, 3H), 2.27 (s, 3H), 1.21 (d, 6H). MS for C₂₇H₂₇FN₄O₅: *m*/*z* 507 (MH+).

**N-[3-Fluoro-4-[(7-methoxy-1,5-naphthyridin-4-yl)oxy]phenyl]-4-hydroxy-2,6-dimethyl-5-propan-2-ylpyridine-3-carboxamide (32):** Compound **32** was made from Compound **F2-1** and Compound **A1-12** using **General Procedure G1.** ¹H NMR (400 MHz, DMSO-*d*₆) δ 13.60 (s, 1H), 11.45 (s, 1H), 8.68 (d, 1H), 8.64 (d, 1H), 7.96 (dd, 1H), 7.73 (d, 1H), 7.35 (dd, 1H), 7.32 (d, 1H), 6.67 (dd, 1H), 3.94 (s, 3H), 3.08 (p, 1H), 2.60 (s, 3H), 2.28 (s, 3H), 1.22 (d, 6H). MS for C₂₆H₂₅FN₄O₄: *m*/*z* 477 (MH+).

**N-[4-[(6,7-Dimethoxy-1,5-naphthyridin-4-yl)oxy]-3-fluorophenyl]-4-methyl-2-oxo-1-propan-2-ylpyridine-3-carboxamide (38):** Compound **38** was made from Compound **12-5** and Compound **A1-10** using **General Procedure G1, purified by preparative HPLC and recovered as the partial formic acid salt.** ¹H NMR (400 MHz, DMSO-*d*₆) δ 10.97 (s, 1H), 8.62 (d, 1H), 8.04 (dd, 1H), 7.88 (d, 1H), 7.74 (s, 1H), 7.57 (ddd, 1H), 7.44 (t, 1H), 6.88 (dd, 1H), 6.40 (d, 1H), 5.18 (hept, 1H), 4.07 (s, 3H), 4.06 (s, 3H), 2.33 (s, 3H), 1.41 (d, 6H). MS for C₂₆H₂₅FN₄O₅: *m*/*z* 493.1 (MH+).

**N-[4-[(6,7-Dimethoxy-1,5-naphthyridin-4-yl)oxy]phenyl]-4-methyl-2-oxo-1-propan-2-ylpyridine-3-carboxamide (39):** Compound **39** was made from Compound **12-5** and Compound **A1-11** using **General Procedure G1, purified by preparative HPLC and recovered as the partial formic acid salt.** ¹H NMR (400 MHz, DMSO-*d*₆) δ 10.62 (s, 1H), 8.45 (d, 1H), 7.77 - 7.67 (m, 3H), 7.57 (s, 1H), 7.16 - 7.08 (m, 2H), 6.68 (d, 1H), 6.23 (d, 1H), 5.01 (h, 1H), 3.90 (s, 3H), 3.90 (s, 3H), 2.17 (s, 3H), 1.25 (d, 6H); MS for C₂₆H₂₆N₄O₅: *m*/*z* 475.1 (MH+).

**5-Cyclopropyl-N-[4-[(6,7-dimethoxy-1,5-naphthyridin-4-yl)oxy]-3-fluorophenyl]-1,2,6-trimethyl-4-oxopyridine-3-carboxamide (46):** Compound **46** was made from Compound **14-1** and Compound **A1-10** using **General Procedure G1.** ¹H NMR (400 MHz, DMSO-*d*₆) δ 11.59 (s, 1H), 8.53 (d, 1H), 7.96 (dd, 1H), 7.66 (s, 1H), 7.49 - 7.42 (m, 1H), 7.34 (t, 1H), 6.78 (dd, 1H), 3.98 (d, 6H), 3.57 (s, 3H), 2.54 (s, 6H), 1.49 - 1.37 (m, 1H), 0.95 - 0.85 (m, 2H), 0.55 - 0.47 (m, 2H). MS for C₂₈H₂₇FN₄O₅: *m*/*z* 519 (MH+).

**5-Cyclopropyl-N-[4-[(6,7-dimethoxy-1,5-naphthyridin-4-yl)oxy]-2,5-difluorophenyl]-1,2,6-trimethyl-4-oxopyridine-3-carboxamide (47):** Compound **47** was made from Compound **14-1** and Compound **A1-15** using **General Procedure G1.** ¹H NMR (400 MHz, DMSO-*d*₆) δ 13.80 (s, 1H), 8.57 (d, 1H), 8.48 (dd, 1H), 7.67 (s, 1H), 7.52 (dd, 1H), 6.93 (d, 1H), 3.96 (d, 6H), 3.64 (s, 3H), 2.81 (s, 3H), 2.58 (s, 3H), 1.55 - 1.45 (m, 1H), 1.01 - 0.91 (m, 2H), 0.51 - 0.42 (m, 2H). MS for C₂₈H₂₆F₂N₄O₅: *m*/*z* 537 (MH+).

**5-Cyclopropyl-N-[4-[(6,7-dimethoxy-1,5-naphthyridin-4-yl)oxy]phenyl]-1,2,6-trimethyl-4-oxopyridine-3-carboxamide (48):** Compound **48** was made from Compound **14-1** and Compound **A1-11** using **General Procedure G1**. ¹H NMR (400 MHz, DMSO-*d*₆) δ 11.37 (s, 1H), 8.52 (d, 1H), 7.79 (d, 2H), 7.65 (s, 1H), 7.18 (d, 2H), 6.75 (d, 1H), 3.98 (d, 6H), 3.57 (s, 3H), 2.54 (s, 6H), 1.43 (s, 1H), 0.94 - 0.85 (m, 2H), 0.55 - 0.46 (m, 2H); MS for C₂₈H₂₈N₄O₅: *m*/*z* 501 (MH+).

**N-[4-[(6,7-Dimethoxy-1,5-naphthyridin-4-yl)oxy]-3-fluorophenyl]-1,2,6-trimethyl-4-oxo-5-prop-1-en-2-ylpyridine-3-carboxamide (52):** Compound **52** was made from Compound **E1-9** and Compound **A1-10** using **General Procedure G1**. ¹H NMR (400 MHz, DMSO-*d*₆) δ 11.42 (s, 1H), 8.46 (d, 1H), 7.89 (dd, 1H), 7.58 (s, 1H), 7.38 (d, 1H), 7.27 (t, 1H), 6.71 (d, 1H), 5.14 (t, 1H), 4.64 (d, 1H), 3.91 (d, 6H), 3.52 (s, 3H), 2.44 (s, 3H), 2.31 (s, 3H), 1.83 (s, 3H); MS for C₂₈H₂₇FN₄O₅: *m*/*z* 519 (MH+).

**N-[4-[(6,7-Dimethoxy-1,5-naphthyridin-4-yl)oxy]phenyl]-1,2,6-trimethyl-4-oxo-5-prop-1-en-2-ylpyridine-3-carboxamide (55):** Compound **55** was made from Compound **E1-9** and Compound **A1-11** using **General Procedure G1**. ¹H NMR (400 MHz, DMSO-*d*₆) δ 11.27 (s, 1H), 8.52 (d, 1H), 7.83 - 7.75 (m, 2H), 7.64 (s, 1H), 7.23 - 7.14 (m, 2H), 6.75 (d, 1H), 5.21 (dd, 1H), 4.71 (dd, 1H), 3.98 (d, 6H), 3.59 (s, 3H), 2.52 (s, 3H), 2.38 (s, 3H), 1.91 (s, 3H); MS for C₂₈H₂₈N₄O₅: *m*/*z* 501 (MH+).

**N-[4-[(6,7-Dimethoxy-1,5-naphthyridin-4-yl)oxy]-3-fluorophenyl]-1,2,6-trimethyl-4-oxo-5-propan-2-ylpyridine-3-carboxamide (56):** Compound **56** was made from Compound **F2-3** and Compound **A1-10** using **General Procedure G1**. ¹H NMR (400 MHz, DMSO-*d*₆) δ 11.39 (s, 1H), 8.53 (d, 1H), 7.97 (dd, 1H), 7.66 (s, 1H), 7.47 (ddd, 1H), 7.34 (t, 1H), 6.78 (dd, 1H), 3.98 (d, 6H), 3.57 (s, 3H), 3.40 (d, 1H), 2.46 (s, 3H), 2.41 (s, 3H), 1.26 (d, 6H); MS for C₂₈H₂₉FN₄O₅: *m*/*z* 521 (MH+).

**N-[4-[(6,7-Dimethoxy-1,5-naphthyridin-4-yl)oxy]phenyl]-1,2,6-trimethyl-4-oxo-5-propan-2-ylpyridine-3-carboxamide (57):** Compound **57** was made from Compound **F2-3** and Compound **A1-11** using **General Procedure G1**. ¹H NMR (400 MHz, DMSO-*d*₆) δ 11.18 (s, 1H), 8.52 (d, 1H), 7.80 (d, 2H), 7.65 (s, 1H), 7.18 (d, 2H), 6.75 (d, 1H), 3.98 (d, 6H), 3.56 (s, 3H), 3.40 (m, 1H), 2.46 (s, 3H), 2.41 (s, 3H), 1.26 (d, 6H); MS for C₂₈H₃₀N₄O₅: *m*/*z* 503 (MH+).

**4-Hydroxy-5-methoxy-N-[4-[(7-methoxy-1,5-naphthyridin-4-yl)oxy]phenyl]-2,6-dimethylpyridine-3-carboxamide (59):** Compound **59** was made from Compound **16-1** and Compound **A1-13** using **General Procedure G1**. ¹H NMR (400 MHz, DMSO-*d₆*) δ 13.08 (s, 1H), 11.73 (s, 1H), 8.69 - 8.60 (m, 2H), 7.77 - 7.68 (m, 3H), 7.20 - 7.11 (m, 2H), 6.66 (d, 1H), 3.93 (s, 3H), 3.70 (s, 3H), 2.60 (s, 3H), 2.21 (s, 3H); MS for C₂₄H₂₂N₄O₅: *m*/*z* 447 (MH+).

**N-[3-Fluoro-4-[(7-methoxy-1,5-naphthyridin-4-yl)oxy]phenyl]-4-hydroxy-5-methoxy-2,6-dimethylpyridine-3-carboxamide (60):** Compound **60** was made from Compound **16-1** and Compound **A1-12** using **General Procedure G1**. ¹H NMR (400 MHz, DMSO-*d*₆) δ 13.31 (s, 1H), 11.79 (s, 1H), 8.68 (d, 1H), 8.64 (d, 1H), 7.96 (dd, 1H), 7.73 (d, 1H), 7.39 - 7.28 (m, 2H), 6.67 (dd, 1H), 3.94 (s, 3H), 3.70 (s, 3H), 2.60 (s, 3H), 2.21 (s, 3H); MS for C₂₄H₂₁FN₄O₅: *m*/*z* 465 (MH+).

**N-[4-[(6,7-Dimethoxy-1,5-naphthyridin-4-yl)oxy]phenyl]-4-hydroxy-5-methoxy-2,6-dimethylpyridine-3-carboxamide (61):** Compound **61** was made from Compound **16-1** and Compound **A1-11** using **General Procedure G1**. ¹H NMR (400 MHz, DMSO-*d*₆) δ 13.06 (s, 1H), 11.73 (s, 1H), 8.46 (d, 1H), 7.73 - 7.65 (m, 2H), 7.57 (s, 1H), 7.14 - 7.07 (m, 2H), 6.71 (d, 1H), 3.89 (d, 6H), 3.69 (s, 3H), 2.60 (s, 3H), 2.20 (s, 3H); MS for C₂₅H₂₄N₄O₆: *m*/*z* 477 (MH+).

**N-[4-[(6,7-Dimethoxy-1,5-naphthyridin-4-yl)oxy]-3-fluorophenyl]-4-hydroxy-5-methoxy-2,6-dimethylpyridine-3-carboxamide (62):** Compound **62** was made from Compound **16-1** and Compound **A1-10** using **General Procedure G1**. ¹H NMR (400 MHz, DMSO-*d*₆) δ 13.25 (s, 1H), 11.78 (s, 1H), 8.47 (d, 1H), 7.93 (dd, 1H), 7.58 (s, 1H), 7.35 - 7.29 (m, 1H), 7.25 (t, 1H), 6.73 (dd, 1H), 3.90 (d, 6H), 3.69 (s, 3H), 2.60 (s, 3H), 2.21 (s, 3H); MS for C₂₅H₂₃FN₄O₆: *m*/*z* 495 (MH+).

**N-[4-[(6,7-Dimethoxy-1,5-naphthyridin-4-yl)oxy]-3-fluorophenyl]-4-hydroxy-5-methoxy-2,6-dimethylpyridine-3-carboxamide (68):** Compound **68** was made from Compound **16-1** and Compound **A1-15** using **General Procedure G1**. ¹H NMR (400 MHz, DMSO-*d*₆) δ 13.97 (s, 1H), 11.92 (s, 1H), 8.53 - 8.43 (m, 2H), 7.60 (s, 1H), 7.45 (dd, 1H), 6.85 (d, 1H), 3.89 (d, 6H), 3.69 (s, 3H), 2.67 (s, 3H), 2.23 (s, 3H); MS for C₂₅H₂₂F₂N₄O₆: *m*/*z* 513 (MH+).

**7-(Cyclopenten-1-yl)-N-[4-[(6,7-dimethoxy-1,5-naphthyridin-4-yl)oxy]-3-fluorophenyl]-6-methyl-8-oxo-3,4-dihydro-1H-pyrido[2,1-c][1,4]oxazine-9-carboxamide (69):** Compound **69** was made from Compound **F1-4** and Compound **A1-10** using **General Procedure G1.** ¹H NMR (400 MHz, CDCl₃) δ 13.55 (s, 1H), 8.51 (d, 1H), 7.89 (dd, 1H), 7.52 (s, 1H), 7.36 (d, 1H), 7.15 (t, 1H), 6.72 (d, 1H), 5.67 (s, 1H), 5.52 (s, 2H), 4.17 - 4.06 (m, 5H), 4.03 (s, 5H), 2.60 (t, 4H), 2.43 (s, 3H), 2.11 - 2.01 (m, 2H); MS for C₃₁H₂₀FN₄O₆: *m*/*z* 573.3 (MH+).

**N-[3-Fluoro-4-[[6-methoxy-7-(2-methoxyethoxy)-1,5-naphthyridin-4-yl]oxy]phenyl]-4-hydroxy-5-methoxy-2,6-dimethylpyridine-3-carboxamide (70):** Compound **70** was made from Compound **16-1** and Compound **A2-7** using **General Procedure G1**. ¹H NMR (400 MHz, DMSO-*d*₆) δ 13.34 (s, 1H), 11.87 (s, 1H), 8.54 (d, 1H), 8.01 (dd, 1H), 7.68 (s, 1H), 7.42 - 7.29 (m, 2H), 6.80 (dd, 1H), 4.36 - 4.29 (m, 2H), 3.98 (s, 3H), 3.79 - 3.72 (m, 5H), 2.67 (s, 3H), 2.50 (s, 3H), 2.28 (s, 3H); MS for C₂₇H₂₇FN₄O₇: *m*/*z* 539 (MH+).

**4-Hydroxy-5-methoxy-N-[4-[[6-methoxy-7-(2-methoxyethoxy)-1,5-naphthyridin-4-yl]oxy]phenyl]-2,6-dimethylpyridine-3-carboxamide (71):** Compound **71** was made from Compound **16-1** and Compound **A2-8** using **General Procedure G1**. ¹H NMR (400 MHz, DMSO-*d*₆) δ 13.13 (s, 1H), 11.81 (s, 1H), 8.53 (d, 1H), 7.81 - 7.73 (m, 2H), 7.67 (s, 1H), 7.22 - 7.14 (m, 2H), 6.77 (d, 1H), 4.35 - 4.28 (m, 2H), 3.97 (s, 3H), 3.76 (s, 3H),3.80 - 3.60 (m, 2H), 2.67 (s, 3H), 2.50 (s, 3H), 2.28 (s, 3H); MS for C₂₇H₂₈N₄O₇: *m*/*z* 521 (MH+).

**N-[3-Fluoro-4-[6-methoxy-7-(2-methoxyethoxy)pyrido[3,2-d]pyrimidin-4-yl]oxyphenyl]-4-hydroxy-5-methoxy-2,6-dimethylpyridine-3-carboxamide (72):** Compound **72** was made from Compound **16-1** and Compound **C1-9** using **General Procedure G1**. ¹H NMR (400 MHz, DMSO-*d*₆) δ 13.28 (s, 1H), 11.78 (s, 1H), 8.53 (s, 1H), 7.93 - 7.85 (m, 1H), 7.62 (s, 1H), 7.39 - 7.28 (m, 2H), 4.35 - 4.28 (m, 2H), 4.03 (s, 3H), 3.70 (s, 5H), 3.28 (s, 3H), 2.60 (s, 3H), 2.21 (s, 3H); MS for C₂₆H₂₆FN₅O₇: *m*/*z* 540 (MH+).

**7-(3,6-Dihydro-2H-pyran-4-yl)-N-[4-[(6,7-dimethoxy-1,5-naphthyridin-4-yl)oxy]phenyl]-6-methyl-8-oxo-3,4-dihydro-1H-pyrido[2,1-c][1,4]oxazine-9-carboxamide (73):** Compound **73** was made from Compound **E1-12** and Compound **A1-11** using **General Procedure G1**. ¹H NMR (400 MHz, CDCl₃) δ 13.24 (s, 1H), 8.50 (d, 1H), 7.76 (d, 2H), 7.52 (s, 1H), 7.13 (d, 2H), 6.74 (d, 1H), 5.71 - 5.48 (m, 3H), 4.42 - 4.20 (m, 2H), 4.17 - 4.12 (m, 3H), 4.10 (d, 2H), 4.08 - 3.95 (m, 7H), 2.48 (s, 3H), 2.01 (s, 2H); MS for C₃₁H₃₀N₄O₇: *m*/*z* 571.3 (MH+).

**N-[3-Fluoro-4-[(7-methoxy-1,5-naphthyridin-4-yl)oxy]phenyl]-5-methoxy-1,2,6-trimethyl-4-oxopyridine-3-carboxamide (75):** Compound **75** was made from Compound **17-1** and Compound **A1-12** using **General Procedure G1**. ¹H NMR (400 MHz, DMSO-*d*₆) δ 11.24 (s, 1H), 8.69 (d, 1H), 8.63 (d, 1H), 7.92 (dd, 1H), 7.74 (d, 1H), 7.43 (dd, 1H), 7.35 (t, 1H), 6.68 (dd, 1H), 3.94 (s, 3H), 3.67 (s, 3H), 3.52 (s, 3H), 2.40 (s, 3H), 2.32 (s, 3H); MS for C₂₅H₂₃FN₄O₅: *m*/*z* 479 (MH+).

**N-[4-[(6,7-Dimethoxy-1,5-naphthyridin-4-yl)oxy]phenyl]-5-methoxy-1,2,6-trimethyl-4-oxopyridine-3-carboxamide (76):** Compound **76** was made from Compound **17-1** and Compound **A1-11** using **General Procedure G1**. ¹H NMR (400 MHz, DMSO-*d*₆) δ 11.01 (s, 1H), 8.45 (d, 1H), 7.77 - 7.68 (m, 2H), 7.57 (s, 1H), 7.16 - 7.08 (m, 2H), 6.67 (d, 1H), 3.91 (s, 3H), 3.90 (s, 3H), 3.66 (s, 3H), 3.51 (s, 3H), 2.40 (s, 3H), 2.31 (s, 3H); MS for C₂₆H₂₆N₄O₆: *m*/*z* 491 (MH+).

**N-[4-[(6,7-Dimethoxy-1,5-naphthyridin-4-yl)oxy]-3-fluorophenyl]-5-methoxy-1,2,6-trimethyl-4-oxopyridine-3-carboxamide (77):** Compound **77** was made from Compound **17-1** and Compound **A1-10** using **General Procedure G1**. ¹H NMR (400 MHz, DMSO-*d*₆) δ 11.22 (s, 1H), 8.46 (d, 1H), 7.89 (dd, 1H), 7.59 (s, 1H), 7.39 (dd, 1H), 7.28 (t, 1H), 6.71 (d, 1H), 3.91 (d, 6H), 3.66 (s, 3H), 3.51 (s, 3H), 2.40 (s, 3H), 2.31 (s, 3H); MS for C₂₆H₂₅FN₄O₆: *m*/*z* 509 (MH+).

**5-Cyano-N-[4-[(6,7-dimethoxy-1,5-naphthyridin-4-yl)oxy]-3-fluorophenyl]-1,2,6-trimethyl-4-oxopyridine-3-carboxamide (78):** Compound **78** was made from Compound **18-1** and Compound **A1-10** using **General Procedure G1**. ¹H NMR (400 MHz, DMSO-*d*₆) δ 10.65 (s, 1H), 8.46 (d, 1H), 7.86 (dd, 1H), 7.59 (s, 1H), 7.39 (dd, 1H), 7.30 (t, 1H), 6.72 (d, 1H), 3.91 (d, 6H), 3.56 (s, 3H), 2.59 (s, 3H), 2.36 (s, 3H); MS for C₂₆H₂₂FN₅O₅: *m*^{/}*z* 504 (MH+).

**7-(3,6-Dihydro-2H-pyran-4-yl)-N-[4-[(6,7-dimethoxy-1,5-naphthyridin-4-yl)oxy]-3-fluorophenyl]-6-methyl-8-oxo-3,4-dihydro-1H-pyrido[2,1-c][1,4]oxazine-9-carboxamide (79):** Compound **79** was made from Compound **E1-12** and Compound **A1-10** using **General Procedure G1.** ¹H NMR (400 MHz, CDCl₃) δ 13.46 (s, 1H), 8.51 (d, 1H), 7.90 (dd, 1H), 7.60 - 7.52 (m, 1H), 7.42 - 7.33 (m, 1H), 7.17 (t, 1H), 6.74 (d, 1H), 5.66 (s, 1H), 5.54 (s, 2H), 4.40 - 4.32 (m, 2H), 4.14 (s, 3H), 4.11 (d, 2H), 4.07 - 3.99 (m, 7H), 2.53 - 2.18 (m, 5H); MS for C₃₁H₂₉FN₄O₇: *m*/*z* 589.3 (MH+).

**7-(Cyclohexen-1-yl)-N-[4-[(6,7-dimethoxy-1,5-naphthyridin-4-yl)oxy]-3-fluorophenyl]-6-methyl-8-oxo-3,4-dihydro-1H-pyrido[2,1-c][1,4]oxazine-9-carboxamide (80):** Compound **80** was made from Compound **F1-5** and Compound **A1-10** using **General Procedure G1.** ¹H NMR (400 MHz, CDCl₃) δ 13.57 (s, 1H), 8.51 (d, 1H), 7.90 (dd, 1H), 7.54 (s, 1H), 7.41 - 7.36 (m, 1H), 7.16 (t, 1H), 6.73 (d, 1H), 5.60 (s, 1H), 5.53 (d, 2H), 4.15 - 4.02 (m, 10H), 2.45 (s, 3H), 2.34 - 2.13 (m, 2H), 1.90 - 1.78 (m, 3H), 1.64 - 1.54 (m, 3H); MS for C₃₂H₃₁FN₄O₆: *m*/*z* 587.3 (MH+).

**5-Cyano-N-[3-fluoro-4-[(7-methoxy-1,5-naphthyridin-4-yl)oxy]phenyl]-1,2,6-trimethyl-4-oxopyridine-3-carboxamide (81):** Compound 81 was made from Compound **18-1** and Compound **A1-12** using **General Procedure G1**. ¹H NMR (400 MHz, DMSO-*d*₆) δ 10.69 (s, 1H), 8.70 (dd, 1H), 8.65 (dd, 1H), 7.89 (dd, 1H), 7.74 (d, 1H), 7.43 (dd, 1H), 7.37 (t, 1H), 6.76 - 6.70 (m, 1H), 3.94 (s, 3H), 3.56 (s, 3H), 2.59 (s, 3H), 2.36 (s, 3H); MS for C₂₅H₂₀FN₅O₄: *m*/*z* 474 (MH+).

**5-Cyano-N-[4-[(6,7-dimethoxy-1,5-naphthyridin-4-yl)oxy]phenyl]-1,2,6-trimethyl-4-oxopyridine-3-carboxamide (82):** Compound **82** was made from Compound **18-1** and Compound **A1-11** using **General Procedure G1**. ¹H NMR (400 MHz, DMSO-*d*₆) δ 10.45 (s, 1H), 8.45 (d, 1H), 7.75 - 7.67 (m, 2H), 7.58 (s, 1H), 7.18 - 7.10 (m, 2H), 6.69 (d, 1H), 3.90 (d, 6H), 3.55 (s, 3H), 2.58 (s, 3H), 2.35 (s, 3H); MS for C₂₆H₂₃N₅O₅: *m*/*z* 486 (MH+).

**5-Cyano-N-[2,5-difluoro-4-[[6-methoxy-7-(2-methoxyethoxy)-1,5-naphthyridin-4-yl]oxy]phenyl]-1,2,6-trimethyl-4-oxopyridine-3-carboxamide (83):** Compound **83** was made from Compound **18-1** and Compound **A2-10** using **General Procedure G1**. MS for C₂₈H₂₅F₂N₅O₆: *m*/*z* 566 (MH+).

**3-N-[4-[(6,7-Dimethoxy-1,5-naphthyridin-4-yl)oxy]-3-fluorophenyl]-4-hydroxy-2,6-dimethylpyridine-3,5-dicarboxamide (84):** Compound **84** was made from Compound **19-2** and Compound **A1-10** using **General Procedure G1**. ¹H NMR (400 MHz, DMSO-*d*₆) δ 12.15 (s, 1H), 11.83 (s, 1H), 8.58 (s, 1H), 8.47 (d, 1H), 7.91 (dd, 1H), 7.59 (s, 1H), 7.39 - 7.31 (m, 1H), 7.27 (t, 2H), 6.72 (dd, 1H), 3.90 (d, 6H), 2.48 (s, 3H), 2.42 (s, 3H); MS for C₂₅H₂₂FN₅O₆: *m*/*z* 508 (MH+).

**3-N-[4-[(6,7-Dimethoxy-1,5-naphthyridin-4-yl)oxy]phenyl]-4-hydroxy-2,6-dimethylpyridine-3,5-dicarboxamide (85):** Compound **85** was made from Compound **19-2** and Compound **A1-11** using **General Procedure G1**. ¹H NMR (400 MHz, DMSO-*d*₆) δ 11.92 (s, 1H), 11.78 (s, 1H), 8.65 (s, 1H), 8.46 (d, 1H), 7.74 - 7.66 (m, 2H), 7.57 (s, 1H), 7.28 (s, 1H), 7.16 - 7.07 (m, 2H), 6.69 (d, 1H), 3.90 (d, 6H), 2.48 (s, 3H), 2.42 (s, 3H); MS for C₂₅H₂₃N₅O₆: *m*/*z* 490 (MH+).

**3-N-[2,5-Difluoro-4-[[6-methoxy-7-(2-methoxyethoxy)-1,5-naphthyridin-4-yl]oxy]phenyl]-4-hydroxy-2,6-dimethylpyridine-3,5-dicarboxamide (86):** Compound **86** was made from Compound **19-2** and Compound **A2-10** using **General Procedure G1**. ¹H NMR (400 MHz, DMSO-*d*₆) δ 13.64 (s, 1H), 11.98 (s, 1H), 8.52 - 8.41 (m, 2H), 8.02 (s, 1H), 7.62 (s, 1H), 7.47 (dd, 1H), 7.38 (s, 1H), 6.85 (d, 1H), 4.25 (dd, 2H), 3.87 (s, 3H), 3.71 - 3.65 (m, 2H), 2.68 (s, 3H), 2.42 (s, 3H), 2.33 (s, 3H); MS for C₂₇H₂₅F₂N₅O₇: *m*/*z* 570 (MH+).

**5-Cyano-N-[4-[(6,7-dimethoxy-1,5-naphthyridin-4-yl)oxy]-3-fluorophenyl]-4-hydroxy-2,6-dimethylpyridine-3-carboxamide (87):** Compound **87** was made from 5-cyano-4-hydroxy-2,6-dimethylnicotinic acid (made from the ester hydrolysis of Compound **19-1)** and Compound **A1-10** using **General Procedure G1**. ¹H NMR (400 MHz, DMSO-*d*₆) δ 12.47 (s, 1H), 8.46 (d, 1H), 7.90 (dd, 1H), 7.59 (s, 1H), 7.30 (dt, 2H), 6.72 (dd, 1H), 6.48 (s, 1H), 3.90 (s, 6H), 2.47 (s, 3H), 2.39 (s, 3H); MS for C₂₅H₂₀FN₅O₅: *m*/*z* 490.1 (MH+).

**3-N-[3-Fluoro-4-[(7-methoxy-1,5-naphthyridin-4-yl)oxy]phenyl]-4-hydroxy-2,6-dimethylpyridine-3,5-dicarboxamide (91):** Compound **91** was made from Compound **19-2** and Compound **A1-12** using **General Procedure G1**. ¹H NMR (400 MHz, DMSO-*d*₆) δ 12.15 (s, 1H), 11.84 (s, 1H), 8.78 - 8.68 (m, 2H), 8.56 (s, 1H), 7.96 (dd, 1H), 7.74 (d, 1H), 7.45 - 7.34 (m, 2H), 7.32 (s, 1H), 6.79 (q, 1H), 3.96 (s, 3H), 2.49 (s, 3H), 2.42 (s, 3H); MS for C₂₄H₂₀FN₅O₅: *m*/*z* 478 (MH+).

**7-(Cyclopenten-1-yl)-N-[4-[(6,7-dimethoxy-1,5-naphthyridin-4-yl)oxy]phenyl]-6-methyl-8-oxo-3,4-dihydro-1H-pyrido[2,1-c][1,4]oxazine-9-carboxamide (99):** Compound **99** was made from Compound **F1-4** and Compound **A1-11** using **General Procedure G1**. ¹H NMR (400 MHz, CDCl₃) δ 13.37 (s, 1H), 8.49 (d, 1H), 7.77 (d, 2H), 7.57 (s, 1H), 7.13 (d, 2H), 6.75 (d, 1H), 5.67 (s, 1H), 5.54 (s, 2H), 4.18 - 4.00 (m, 10H), 2.60 (t, 4H), 2.43 (s, 3H), 2.10 (s, 2H); MS for C₃₁H₃₀N₄O₆: *m*/*z* 555.3 (MH+).

**7-(Cyclohexen-1-yl)-N-[4-[(6,7-dimethoxy-1,5-naphthyridin-4-yl)oxy]phenyl]-6-methyl-8-oxo-3,4-dihydro-1H-pyrido[2,1-c][1,4]oxazine-9-carboxamide (100):** Compound **100** was made from Compound **F1-5** and Compound **A1-11** using **General Procedure G1**. ¹H NMR (400 MHz, CDCl₃) δ 13.36 (s, 1H), 8.50 (d, 1H), 7.76 (d, 2H), 7.51 (s, 1H), 7.13 (d, 2H), 6.74 (d, 1H), 5.60 (br s, 1H), 5.46-5.57 (m, 2H), 4.14 (s, 3H), 4.10 (br d, 2H), 4.03 (s, 5H), 2.44 (s, 3H), 2.07-2.36 (m, 2H), 1.72-1.89 (m, 4H), 1.61-1.71 (m, 2H); MS for C₃₂H₃₂N₄O₆: *m*/*z* 569.4 (MH+).

### Example 8: 8-(4-Amino-2-fluorophenoxy)-2-methoxy-1,5-naphthyridin-3-ol (8-7)

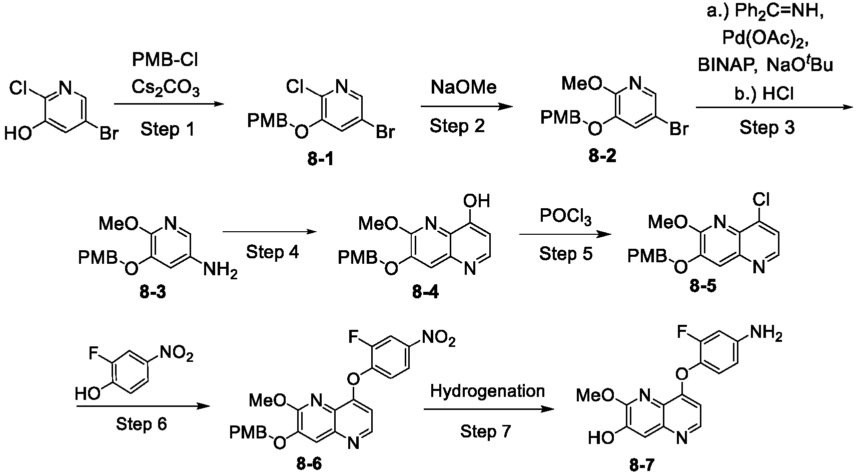

**8-(4-Amino-2-fluorophenoxy)-2-methoxy-1,5-naphthyridin-3-ol (8-7):** Compound **8-7** was made in 7 steps from 5-bromo-2-chloropyridin-3-ol following the procedure used in Bannen, L., et. al. WO2021062245. MS for C₁₅H₁₂FN₃O₃, found 302 (MH+).

### Example 9: 5-Bromo-1,2,6-trimethyl-4-oxo-1,4-dihydropyridine-3-carboxylic acid (9-3)

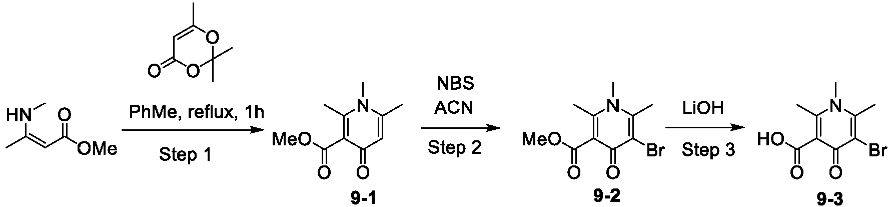

**5-Bromo-1,2,6-trimethyl-4-oxo-1,4-dihydropyridine-3-carboxylic acid (9-3):** Compound **9-3** was made in 3 steps from methyl 3-(methylamino)but-2-enoate and 2,2,6-trimethyl-4H-1,3-dioxin-4-one following the procedure used in Bannen, L., et. al. WO2021173591. MS for C₉H₁₀BrNO₃: *m*/*z* 260/262 (MH+).

### Example 10: N-[4-[[7-(2-Cyclobutylethoxy)-6-methoxy-1,5-naphthyridin-4-yl]oxy]-3-fluorophenyl]-5-(cyclopenten-1-yl)-1,2,6-trimethyl-4-oxopyridine-3-carboxamide (74)

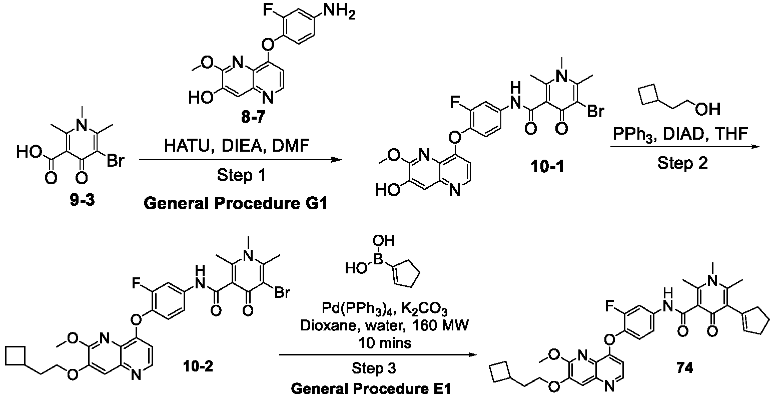

Step 1: 5-Bromo-N-(3-fluoro-4-((7-hydroxy-6-methoxy-1,5-naphthyridin-4-yl)oxy)phenyl)-1,2,6-trimethyl-4-oxo-1,4-dihydropyridine-3-carboxamide (10-1): Compound 10-1 was synthesized from Compound 8-7 and Compound 9-3 using General Procedure G1. MS for C₂₄H₂₀BrFN₄O₅: *m*/*z* 543 (MH+).

Step 2: 5-Bromo-N-(4-((7-(2-cyclobutylethoxy)-6-methoxy-1,5-naphthyridin-4-yl)oxy)-3-fluorophenyl)-1,2,6-trimethyl-4-oxo-1,4-dihydropyridine-3-carboxamide (10-2): A mixture of Compound 10-1 (54 mg, 0.1 mmol, 1 eq), triphenylphosphine (78 mg, 0.30 mmol, 3.0 eq) and 2-cyclobutylethanol (11 mg, 0.11 mmol, 1.1eq) in THF (0.5 mL, 0.2M) was cooled to 0 °C in an ice bath followed by the addition of DIAD (61 mg, 0.3 mmol, 3.0 eq). The resulting mixture was allowed to warm to room temperature overnight with stirring. The reaction mixture was then concentrated under reduced pressure. EtOAc was added to the resulting residue and the solution with washed with water, dried over anhyd. Na₂SO₄ and concentrated under reduced pressure. The resulting residue was purified by flash column chromatography over silica gel (DCM/MeOH) to give Compound 10-2 as an oil (40 mg, 64% yield). ¹H NMR (400MHz, DMSO-*d*₆) δ 10.81 (s, 1H), 8.58 (d, 1H), 7.96 (dd, 1H), 7.55 - 7.43 (m, 2H), 7.40 (t, 1H), 6.86 (d, 1H), 4.14 (t, 2H), 4.01 (s, 3H), 3.67 (s, 3H), 2.70 (s, 3H), 2.46 (m, 1H), 2.42 (s, 3H), 2.08 (dt, 2H), 1.93 (q, 2H), 1.89 - 1.78 (m, 2H), 1.78 - 1.61 (m, 2H); MS for C₃₀H₃₀BrFN₄O₅: *m*/*z* 627 (MH+).

Step 3: N-[4-[[7-(2-Cyclobutylethoxy)-6-methoxy-1,5-naphthyridin-4-yl]oxy]-3-fluorophenyl]-5-(cyclopenten-1-yl)-1,2,6-trimethyl-4-oxopyridine-3-carboxamide (74): Compound 74 was made from Compound 10-2 and cyclopent-1-en-1-ylboronic acid using General Procedure E1.¹H NMR (400MHz, DMSO-*d*₆) δ 11.44 (s, 1H), 8.45 (d, 1H), 8.31 (s, 1H), 7.55 (s, 1H), 7.39 (ddd, 1H), 7.26 (t, 1H), 6.69 (dd, 1H), 5.44 - 5.36 (m, 1H), 4.04 (t, 2H), 3.90 (s, 3H), 3.52 (s, 3H), 2.44 (s, 3H), 2.41 - 2.36 (m, 4H), 2.29 (s, 3H), 2.00 (dddd, 2H), 1.91 - 1.80 (m, 5H), 1.80 - 1.70 (m, 2H), 1.70 - 1.52 (m, 2H); MS for C₃₅H₃₇FN₄O₅*: m*/*z* 613 (MH+).

### Example 11: N-[4-[(6,7-Dimethoxy-1,5-naphthyridin-4-yl)oxy]phenyl]-4-hydroxy-2,6-dimethyl-5-propan-2-ylpyridine-3-carboxamide (9)

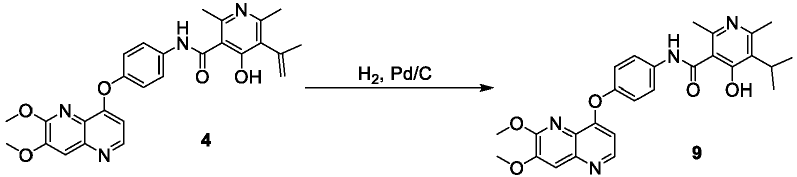

**N-[4-[(6,7-Dimethoxy-1,5-naphthyridin-4-yl)oxy]phenyl]-4-hydroxy-2,6-dimethyl-5-propan-2-ylpyridine-3-carboxamide (9):** Compound **9** was prepared from Compound **4** using standard hydrogenation techniques (hydrogen gas, 1 atm, Pd/C, MeOH) followed by purification by flash chromatography on silica gel. ¹H NMR (400 MHz, DMSO-*d*₆) δ 13.35 (s, 1H), 11.45 (s, 1H), 8.46 (d, 1H), 7.73 - 7.65 (m, 2H), 7.57 (s, 1H), 7.14 - 7.05 (m, 2H), 6.70 (d, 1H), 3.90 (d, 6H), 3.09 - 3.01 (m, 1H), 2.59 (s, 3H), 2.27 (s, 3H), 1.21 (d, 6H). MS for C₂₇H₂₈N₄O₅: *m*/*z* 489 (MH+).

The following compounds were made using the same method used to convert Compound 4 to Compound **9** in **Example 11:**

**N-[4-[(6,7-Dimethoxy-1,5-naphthyridin-4-yl)oxy]-2,5-difluorophenyl]-4-hydroxy-2,6-dimethyl-5-propan-2-ylpyridine-3-carboxamide (10):** Compound **4** was replaced with Compound **6** and the resulting product was purified by preparative HPLC. ¹H NMR (400 MHz, DMSO-*d*₆) δ 14.36 (s, 1H), 11.6 (s, 1H), 8.54 - 8.41 (m, 2H), 7.60 (s, 1H), 7.43 (dd, 1H), 6.84 (d, 1H), 3.91 (s 3H), 3.87 (s, 3H), 3.13 - 3.05 (m, 1H), 2.65 (s, 3H), 2.28 (s, 3H), 1.21 (d, 6H). MS for C₂₇H₂₆F₂N₄O₅: *m*/*z* 525 (MH+).

**5-Cyclopentyl-N-[4-[(6,7-dimethoxy-1,5-naphthyridin-4-yl)oxy]-3-fluorophenyl]-1-(2-fluoroethyl)-6-methyl-4-oxopyridine-3-carboxamide (13):** Compound **4** was replaced with Compound 11. MS for C₃₀H₃₀F₂N₄O₅: *m*/*z* 565.2 (MH+).

### Example 12: 1-(Cyclopent-1-en-1-yl)-4,6-dimethyl-2-oxo-1,2-dihydropyridine-3-carboxylic acid (12-3)

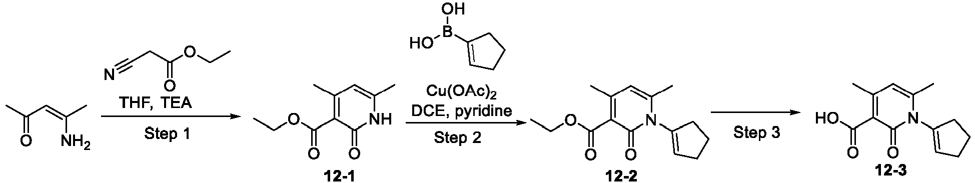

**Step 1: Ethyl 4,6-dimethyl-2-oxo-1,2-dihydropyridine-3-carboxylate (12-1):** (Z)-4-aminopent-3-en-2-one (5.0 g, 50 mmol, 1 eq) and ethyl 2-cyanoacetate (5.8 g, 51 mmol, 1.02 eq) were added to a stirring solution of THF (56 mL) and TEA (2.0 g, 20 mmol, 0.4 eq). After heating for 48 h, the reaction was cooled to ambient temperature and allowed to sit for 4 days. The resulting mixture was filtered, and the solids were washed with EtOAc to give Compound 12-1 (2.98 g, 30% yield). MS for C₁₀H₁₃NO₃: *m*/*z* 196 (MH+).

**Step 2: Ethyl 1-(cyclopent-1-en-1-yl)-4,6-dimethyl-2-oxo-1,2-dihydropyridine-3-carboxylate (12-2):** Compound **12-1** (500 mg, 2.5612 mmol), cupric acetate (700 mg, 3.5061 mmol), cyclopentylboronic acid (600 mg, 5.2655 mmol), 1,2-dichloroethane (3.0 mL) and pyridine (1.0 mL) were heated to 45 °C under an atmosphere of oxygen (1 atm). After overnight reaction, the contents were cooled, diluted with EtOAc and filtered through a short plug of silica gel. The crude product was concentrated onto Celite and purified over silica (220 g, 0% to 2% MeOH in DCM) to give Compound **12-2** as a tan solid (227 mg, 0.87 mmol, 34% yield). ¹H NMR (400 MHz, CDCl₃) δ 5.94 (s, 1H), 5.71 (t, 1H), 4.37 (q, 2H), 2.55 (ddd, 2H), 2.20 (d, 3H), 2.20 (s, 3H), 2.10 (s, 2H), 2.06 - 1.92 (m, 2H), 1.36 (t, 3H); MS for C₁₅H₁₉NO₃: *m*/*z* 262.1 (MH+).

**Step 3: 1-(Cyclopent-1-en-1-yl)-4,6-dimethyl-2-oxo-1,2-dihydropyridine-3-carboxylic acid (12-3):** Compound **12-3** was synthesized from Compound **12-2** using standard ester hydrolysis conditions such as that employed in **General Procedure F1** to convert Compound **E1-3** to Compound **F1-1.** MS for C₁₃H₁₅NO₃: *m*/*z* 234 (MH+).

The following compounds were made using the same three step procedure to make Compound **12-3** in **Example 12:**

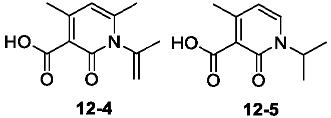

**1-Isopropenyl-4,6-dimethyl-2-oxo-pyridine-3-carboxylic acid (12-4):** Cyclopentylboronic acid was replaced with isopropenylboronic acid. MS for C₁₁H₁₃NO₃: m/z 208 (MH+).

**1-isopropyl-4-methyl-2-oxo-1,2-dihydropyridine-3-carboxylic acid (12-5):** Cyclopentylboronic acid was replaced with isopropylboronic acid. MS, for the corresponding methyl ester, C₁₁H₁₃NO₃: m/z 208 (MH+).

### Example 13: 5-Bromo-1-cyclopentyl-N-[4-[(6,7-dimethoxy-1,5-naphthyridin-4-yl)oxy]-3-fluorophenyl]-4,6-dimethyl-2-oxopyridine-3-carboxamide (22)

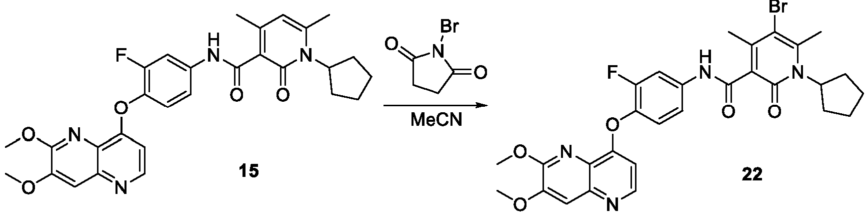

**5-Bromo-1-cyclopentyl-N-[4-[(6,7-dimethoxy-1,5-naphthyridin-4-yl)oxy]-3-fluorophenyl]-4,6-dimethyl-2-oxopyridine-3-carboxamide (22):** To a stirring solution of Compound 15 (105 mg, 0.20 mmol) and ACN (2 mL) was added 1-bromopyrrolidine-2,5-dione (90 mg, 0.50 mmol). The resulting solution was stirred for 1 h at ambient temperature then diluted with 10 mL DCM and an equal volume of dilute aq NaOH. The aqueous layer was washed with an additional amount of DCM, and the combined organic layers were washed with conc aq Na₂S₂O₃, then water. The organic phase was concentrated and purified by prep HPLC (10% to 100% ACN in water (+0.1% FA) to give the formic acid salt of Compound **22** as a colorless solid (26.3 mg, 21% yield). ¹H NMR (400MHz, DMSO-*d*₆) δ 10.54 (s, 1H), 8.45 (d, 1H), 7.83 (dd, 1H), 7.59 (s, 1H), 7.38 (dt, 1H), 7.30 (t, 1H), 6.71 (dd, 1H), 4.86 - 4.78 (m, 1H), 3.92 (s, 3H), 3.90 (s, 3H), 2.65 (s, 3H), 2.16 (s, 3H), 2.14 - 2.05 (m, 2H), 1.90 - 1.72 (m, 4H), 1.54 - 1.42 (m, 2H). MS for C₂₉H₂₈BrFN₄O₅: *m*/*z* 611.1 (MH+).

### Example 14: 5-Cyclopropyl-1,2,6-trimethyl-4-oxo-1,4-dihydropyridine-3-carboxylic acid (14-1)

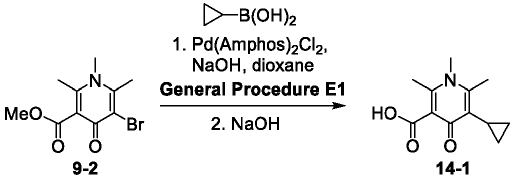

**5-Cyclopropyl-1,2,6-trimethyl-4-oxo-1,4-dihydropyridine-3-carboxylic acid (14-1):** Compound **14-1** was synthesized using **General Procedure E-1,** followed by ester hydrolysis via standard hydrolysis conditions using NaOH. Specifically, in this case, Compound **9-2** (500 mg, 2.0 mmol), cyclopropylboronic acid (750 mg, 8.7 mmol), 4-ditert-butylphosphanyl-N,N-dimethyl-aniline-dichloropalladium (144 mg, 0.2 mmol) and NaOH (300 mg, 7.5 mmol) were combined in dioxane (6 mL) and the resulting mixture was stirred under nitrogen at 100 °C overnight, after which was added more NaOH (400 mg), MeOH (6 mL), and water (5 mL). The resulting mixture was stirred at 90 °C overnight, concentrated to remove organic solvent, and washed with EtOAc (2x). The aqueous phase was filtered to remove solid, acidified with aq 6 M HCl to pH 3, and extracted with EtOAc (2x). The combined EtOAc extracts were concentrated to dryness and the resulting residue was purified by silica gel chromatography (0-5% MeOH in EtOAc), to give Compound **14-1** as a white solid (100 mg, 23% yield). MS for C₁₂H₁₅NO₃: *m*/*z* 222 (MH+).

### Example 15: 5-Bromo-N-[4-[(6,7-dimethoxy-1,5-naphthyridin-4-yl)oxy]-2,5-difluorophenyl]-6-ethyl-1-methyl-4-oxopyridine-3-carboxamide (63)

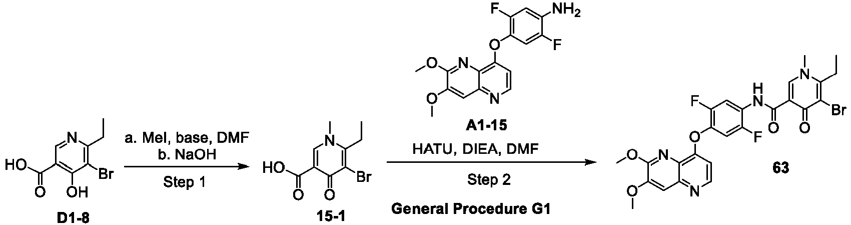

**Step 1: 5-Bromo-6-ethyl-1-methyl-4-oxo-1,4-dihydropyridine-3-carboxylic acid (15-1):** Compound **15-1** was made from Compound **D1-8** in two steps. In the first step, Compound **D1-8** was alkylated using the same type of procedure used in **Example 5** to form methyl 5-bromo-6-ethyl-1-methyl-4-oxo-1,4-dihydropyridine-3-carboxylate (MS for C₁₀H₁₂BrNO₃: *m*/*z* 275.9 (MH+). In the second step, the methyl ester of methyl 5-bromo-6-ethyl-1-methyl-4-oxo-1,4-dihydropyridine-3-carboxylate was hydrolyzed using standard NaOH hydrolysis methods to form Compound **15-1.** MS for C₉H₁₀BrNO₃: *m*/*z* 260 (MH+).

**Step 2: 5-Bromo-N-[4-[(6,7-dimethoxy-1,5-naphthyridin-4-yl)oxy]-2,5-difluorophenyl]-6-ethyl-1-methyl-4-oxopyridine-3-carboxamide (63):** Compound **63** was synthesized from Compound **15-1** and Compound **A1-15** using **General Procedure G1**. ¹H NMR (400MHz, DMSO-*d*₆) δ 13.10 (d, 1H), 8.78 (s, 1H), 8.60 - 8.48 (m, 2H), 7.67 (s, 1H), 7.66 - 7.55 (m, 1H), 6.96 (d, 1H), 4.00 (s, 3H), 3.98 (s, 3H), 3.94 (s, 3H), 3.02 (q, 2H), 1.26 - 1.16 (m, 3H); MS for C₂₅H₂₁BrF₂N₄O₅: *m*/*z* 577 (MH+).

The following compound was made using the same two step procedure to make Compound 63 in **Example 15:**
**5-Bromo-6-ethyl-N-[4-[[6-methoxy-7-(2-methoxyethoxy)-1,5-naphthyridin-4-yl]oxy]phenyl]-1-methyl-4-oxopyridine-3-carboxamide (64):** Compound **A1-15** was replaced with Compound **A2-8** in Step 2. ¹H NMR (400MHz, DMSO-*d*₆) δ 12.65 (s, 1H), 8.74 (s, 1H), 8.54 (d, 1H), 7.85 - 7.76 (m, 2H), 7.67 (s, 1H), 7.27 - 7.19 (m, 2H), 6.80 (d, 1H), 4.35 - 4.28 (m, 2H), 3.98 (s, 3H), 3.96 (s, 3H), 3.79 - 3.72 (m, 2H), 3.35 (s, 3H), 3.02 (q, 2H), 1.24 - 1.16 (m, 3H); MS for C₂₇H₂₇BrN₄O₆: *m*/*z* 585.1 (MH+).

### Example 16: 4-Hydroxy-5-methoxy-2,6-dimethylnicotinic acid (16-1)

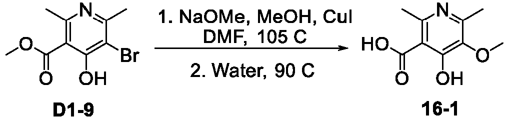

**4-Hydroxy-5-methoxy-2,6-dimethylnicotinic acid (16-1):** To a solution of Compound **D1-9** (1.3 g, 5 mmol) and copper(I) iodide (0.5 g, 2.6 mmol) in DMF (15 mL), were added NaOMe (2.5 g) and MeOH (4 mL). The reaction mixture was stirred at 105 °C for 100 min. After cooling, ice water (10 mL) was added, and the resulting mixture stirred at 90 °C until ester hydrolysis was complete. The mixture was concentrated to remove MeOH and filtered to remove solids. The aqueous filtrate was acidified to pH 3-4, and the resulting suspension was filtered, washed with water and dried to give crude Compound 16-1 (610 mg, 62%). MS for C₉H₁₁NO₄: *m*/*z* 198 (MH+).

### Example 17: 5-Methoxy-1,2,6-trimethyl-4-oxo-1,4-dihydropyridine-3-carboxylic acid (17-1)

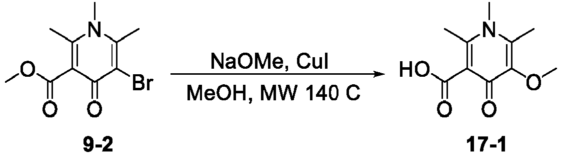

**5-Methoxy-1,2,6-trimethyl-4-oxo-1,4-dihydropyridine-3-carboxylic acid (17-1):** A mixture of Compound **9-2** (0.866 g, 3 mmol), copper(I) iodide (0.45 g, 2.4 mmol), NaOMe (1.6 g) and MeOH (15 mL) was stirred at 140 °C under microwave irradiation for 1 h, cooled to room temperature, and filtered through Celite. The filtrate was treated with 3 N NaOH at 80 °C until ester hydrolysis was complete, and the resulting mixture was washed with EtOAc (2x). The aqueous phase was acidified to pH 2-3; the resulting suspension was filtered, washed with water and dried to provide crude Compound **17-1** (88 mg). MS for C₁₀H₁₃NO₄: *m*/*z* 212 (MH+).

### Example 18: 5-Cyano-1,2,6-trimethyl-4-oxo-1,4-dihydropyridine-3-carboxylic acid (18-1)

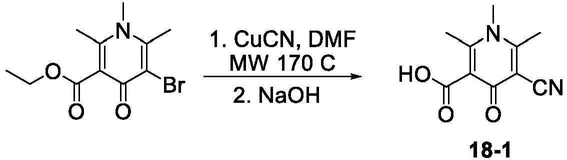

**5-Cyano-1,2,6-trimethyl-4-oxo-1,4-dihydropyridine-3-carboxylic acid (18-1):** A suspension of ethyl 5-bromo-1,2,6-trimethyl-4-oxo-1,4-dihydropyridine-3-carboxylate (1.5 g, 5.2 mmol) and CuCN (0.6 g, 7 mmol) in DMF (12 mL) was stirred at 170 °C under microwave irradiation for 2h. The resulting mixture was concentrated to dryness, the residue suspended in DCM/MeOH (4/1), the resulting suspension stirred for 20 min and then filtered through Celite; the filtrate was concentrated to dryness to give ethyl 5-cyano-1,2,6-trimethyl-4-oxo-1,4-dihydropyridine-3-carboxylate as a light brown solid (780 mg). MS: *m*/*z* 235 (MH+). This solid was treated with 2 N NaOH (8 mL) in MeOH (10 mL) at 80 °C overnight. The resulting mixture was concentrated to remove MeOH. The resulting aqueous solution was acidified with HCl (1 M) to pH 2-3; the resulting suspension was filtered, washed with water and dried to provide crude Compound **18-1** (300 mg). MS for C₁₀H₁₀N₂O₃: *m*/*z* 207 (MH+).

Note: Ethyl 5-bromo-1,2,6-trimethyl-4-oxo-1,4-dihydropyridine-3-carboxylate can be made using the same method as that used to make Compound **9-2** in **Example 9,** substituting ethyl 3-(methylamino)but-2-enoate for the methyl 3-(methylamino)but-2-enoate in Step 1.

### Example 19: 5-Carbamoyl-4-hydroxy-2,6-dimethylnicotinic acid (19-2)

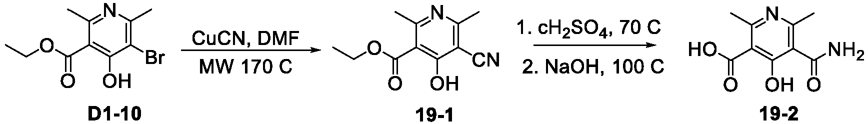

**Step 1: Ethyl 5-cyano-4-hydroxy-2,6-dimethylnicotinate (19-1):** A suspension of Compound **D1-10** (1.4 g, 5.1 mmol) and CuCN (0.6 g, 7 mmol) in DMF (12 mL) was stirred at 170 °C under microwave irradiation for 2 h. The mixture was concentrated to dryness and the residue suspended in DCM/MeOH (4/1) and the resulting suspension stirred for 20 min, then filtered through Celite. The filtrate was concentrated to give crude Compound **19-1** as a light brown solid (850 mg). MS for C₁₁H₁₂N₂O₃: *m*/*z* 221 (MH+).

**Step 2: 5-Carbamoyl-4-hydroxy-2,6-dimethylnicotinic acid (19-2):** Compound **19-1** (221 mg, 1 mmol) was treated with conc H₂SO₄ (5 mL) at 70 °C overnight. The resulting mixture was basified with aq NaOH (10%) carefully to pH >14. The resulting mixture was filtered, and the filtrate was stirred at 100 °C until ester hydrolysis was complete. The mixture was then acidified to pH 2 and concentrated to almost dryness. The resulting residue was extracted with DCM/MeOH (8/2 mL, twice) and the combined extracts were concentrated to give the crude Compound **19-2.** MS for C₉H₁₀N₂O₄: *m*/*z* 211 (MH+).

### Example 20: 4-Hydroxy-2,6-dimethylpyridine-3,5-dicarboxylic acid (20-4)

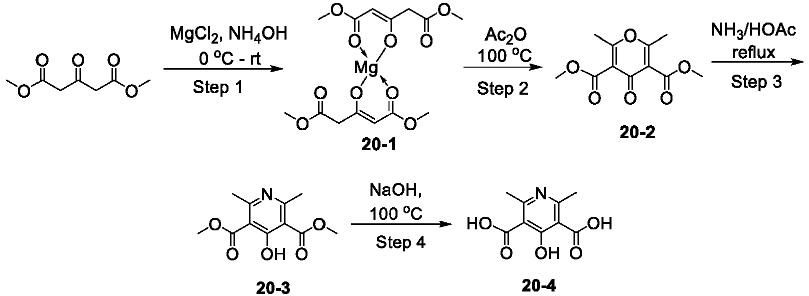

**Step 1: Bis(dimethyl-3-hydroxypent-2-enedioate) Mg Complex (20-1):** To a mixture of dimethyl 3-oxopentanedioate (17.4 g, 100 mmol) and MgCl₂ (9.6 g, 100 mmol) in 150 mL water was added ammonia (30 mL) dropwise and the resulting mixture was stirred at room temperature for 5 h. The resulting suspension was filtered, washed with water (3x) and recrystallized from MeOH to give 14.8 g of the desired Mg complex. MS for C₁₄H₁₈MgO₁₀: *m*/*z* 371 (MH+).

**Step 2: Dimethyl 2,6-dimethyl-4-oxo-4H-pyran-3,5-dicarboxylate (20-2):** Compound **20-1** (14.7 g, 39.7 mmol) in Ac₂O (15 mL) was stirred at 100 °C 30 min. The mixture was concentrated to remove most of the Ac₂O, neutralized to pH 9 and extracted with EtOAc (3x). The combined extracts were evaporated, and the resulting residue was purified over silica gel (0-60-% EtOAc in hexane), to give Compound **20-2** as a white solid (6.7 g). MS for C₁₁H₁₂O₆*: m*/*z* 241 (MH+). Monatshefte fuer Chemie (2005), 136(7), 1197-1203; Chemistry of Heterocyclic Compounds (New York, NY, United States) (2009), 45(6), 666-671.

**Step 3: Dimethyl 4-hydroxy-2,6-dimethylpyridine-3,5-dicarboxylate (20-3):** A mixture of Compound **20-2** (3 g.), NH₃ (5 mL, 7M in MeOH) and AcOH (15 mL) was refluxed for 1 h, diluted with 3 volumes of water, and neutralized with NH₄OH to give a suspension, which was filtered. The resulting solid was washed with water and dried to give Compound **20-3** (1.5 g). MS for C₁₁H₁₃NO₅: *m*/*z* 240 (MH+).

**Step 4: 4-Hydroxy-2,6-dimethylpyridine-3,5-dicarboxylic acid (20-4):** Compound **20-3** (1.0 g) was treated with NaOH (3 M in water, 10 eq) at 100 °C until ester hydrolysis was complete. The resulting solution was washed with EtOAc (2x) and the aqueous phase was acidified to pH 2 to give a suspension, which was filtered to give crude Compound **20-4** (0.5 g). MS for C₉H₉NO₅*: m*/*z* 212 (MH+).

### Example 21: 4-Hydroxy-2,6-dimethylpyridine-3,5-dicarboxylic acid (21-2)

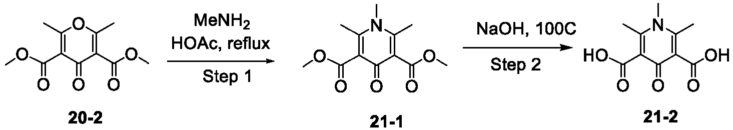

**Step 1: Dimethyl 1,2,6-trimethyl-4-oxo-1,4-dihydropyridine-3,5-dicarboxylate (21-1):** Compound **21-1** was prepared from Compound **20-2** in the same way Compound **20-3** was prepared from Compound **20-2** in Step 3 of **Example 20,** using MeNH₂ in place of NH₃. MS for C₁₂H₁₅NO₅: *m*/*z* 254 (MH+).

**Step 2: 4-Hydroxy-2,6-dimethylpyridine-3,5-dicarboxylic acid (21-2):** Compound **21-2** was prepared from Compound **21-1** in the same way Compound **20-4** was prepared from Compound **20-3** in Step 4 of **Example 20.** MS for C₁₀H₁₁NO₅: *m*/*z* 226 (MH+).

### Example 22: 5-[[4-[(6,7-Dimethoxy-1,5-naphthyridin-4-yl)oxy]-3-fluorophenyl]carbamoyl]-1,2,6-trimethyl-4-oxopyridine-3-carboxylic acid (92)

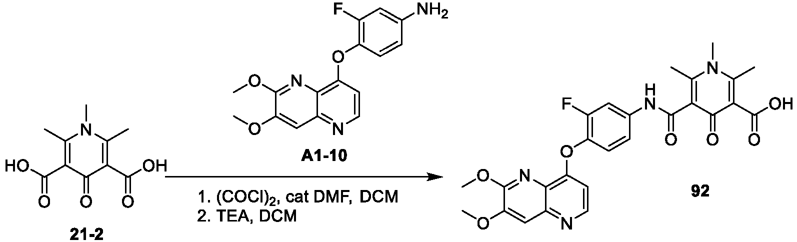

**5-[[4-[(6,7-Dimethoxy-1,5-naphthyridin-4-yl)oxy]-3-fluorophenyl]carbamoyl]-1,2,6-trimethyl-4-oxopyridine-3-carboxylic acid (92):** To a mixture of Compound **21-2** (300 mg, 1.33 mmol) in DCM (12 mL) was added (COCl)₂ (0.15 mL) and 2 drops of DMF. The mixture was stirred at room temperature for 1 h and then concentrated to dryness. To the residue was added Compound **A1-10** (120 mg, 0.38 mmol), followed by the addition of DCM (10 mL) and DIEA (140 mg, 1.08 mmol), and the resulting reaction mixture was stirred at room temperature for 1 h. The reaction mixture was concentrated and 0.1 M HCl (aq) was added to adjust the pH to ~5. The resulting suspension was filtrated, washed with water and dried to give crude Compound **92** (108 mg), a small sample of which was purified by prep HPLC to give a white solid. The remaining crude material was used in subsequent reactions without further purification. ¹H NMR (400 MHz, DMSO-*d*₆) δ 12.66 (s, 1H), 10.74 (s, 1H), 8.47 (d, 1H), 7.87 (dd, 1H), 7.59 (s, 1H), 7.41 (dd, 1H), 7.31 (t, 1H), 6.73 (d, 1H), 3.91 (d, 6H), 2.71 (s, 3H), 2.47 (s, 3H), 2.37 (s, 3H); MS for C₂₆H₂₃FN₄O₇: *m*/*z* 523 (MH+).

### Example 23: 5-((4-((6,7-Dimethoxy-1,5-naphthyridin-4-yl)oxy)-3-fluorophenyl)carbamoyl)-4-hydroxy-2,6-dimethylnicotinic acid (23-1)

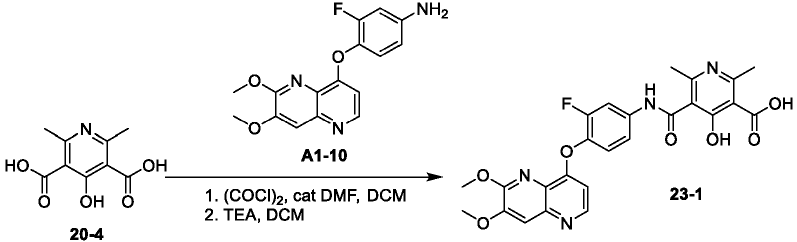

**5-((4-((6,7-Dimethoxy-1,5-naphthyridin-4-yl)oxy)-3-fluorophenyl)carbamoyl)-4-hydroxy-2,6-dimethylnicotinic acid (23-1):** Compound **23-1** was made from Compound **20-4** and Compound **A1-10** using the same procedure that was used to make Compound **92** in **Example 22.** MS for C₂₅H₂₁FN₄O₇: *m*/*z* 509 (MH+).

### Example 24: 3-N-[4-[(6,7-Dimethoxy-1,5-naphthyridin-4-yl)oxy]-3-fluorophenyl]-4-hydroxy-5-N,5-N,2,6-tetramethylpyridine-3,5-dicarboxamide (93)

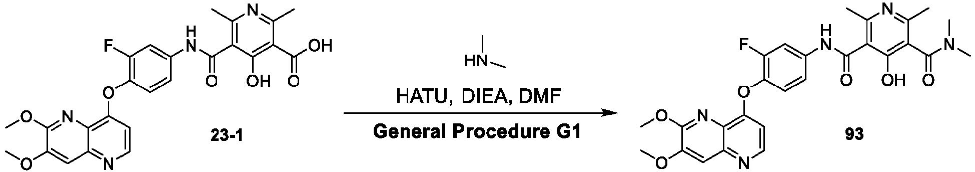

**3-N-[4-[(6,7-Dimethoxy-1,5-naphthyridin-4-yl)oxy]-3-fluorophenyl]-4-hydroxy-5-N,5-N,2,6-tetramethylpyridine-3,5-dicarboxamide (93):** Compound **93** was made from Compound **23-1** and dimethylamine using **General Procedure G1**. ¹H NMR (400 MHz, DMSO-*d*₆) δ 13.05 (s, 1H), 11.97 (s, 1H), 8.50 (d, 1H), 7.93 (dd, 1H), 7.59 (s, 1H), 7.34 (dd, 1H), 7.26 (t, 1H), 6.76 (d, 1H), 3.91 (s, 3H), 3.90 (s, 3H), 2.90 (s, 3H), 2.77 (s, 3H), 2.64 (s, 3H), 2.13 (s, 3H); MS for C₂₇H₂₆FN₅O₆: *m*/*z* 536 (MH+).

The following compounds were made using **General Procedure G1** in the same manner that Compound **93** was made in **Example 24:**

**3-N-[4-[(6,7-Dimethoxy-1,5-naphthyridin-4-yl)oxy]-3-fluorophenyl]-4-hydroxy-2,6-dimethyl-5-N-propan-2-ylpyridine-3,5-dicarboxamide (94):** Compound **94** was made from Compound **23-1** and isopropylamine using **General Procedure G1**. ¹H NMR (400 MHz, DMSO-*d*₆) δ 12.00 (s, 1H), 11.83 (s, 1H), 9.19 (d, 1H), 8.47 (d, 1H), 7.91 (dd, 1H), 7.59 (s, 1H), 7.40 - 7.33 (m, 1H), 7.27 (t, 1H), 6.72 (dd, 1H), 3.98 - 3.91 (m, 1H), 3.90 (s, 6H), 2.47 (s, 3H), 2.41 (s, 3H), 1.06 (d, 6H); MS for C₂₈H₂₈FN₅O₆: *m*/*z* 550 (MH+).

**5-N-[4-[(6,7-Dimethoxy-1,5-naphthyridin-4-yl)oxy]-3-fluorophenyl]-3-N,3-N,1,2,6-pentamethyl-4-oxopyridine-3,5-dicarboxamide (95):** Compound **95** was made from Compound **92** and dimethylamine using **General Procedure G1**. ¹H NMR (400 MHz, DMSO-*d*₆) δ 11.25 (s, 1H), 8.47 (d, 1H), 7.88 (dd, 1H), 7.59 (s, 1H), 7.39 (dd, 1H), 7.28 (t, 1H), 6.72 (d, 1H), 3.91 (d, 6H), 3.52 (s, 3H), 2.89 (s, 3H), 2.77 (s, 3H), 2.45 (s, 3H), 2.20 (s, 3H); MS for C₂₈H₂₈FN₅O₆: *m*/*z* 550 (MH+).

**3-N-Cyclopropyl-5-N-[4-[(6,7-dimethoxy-1,5-naphthyridin-4-yl)oxy]-3-fluorophenyl]-1,2,6-trimethyl-4-oxopyridine-3,5-dicarboxamide (96):** Compound **96** was made from Compound **92** and cyclopropylamine using **General Procedure G1**. ¹H NMR (400 MHz, DMSO-*d*₆) δ 11.12 (s, 1H), 8.61 (d, 1H), 8.47 (d, 1H), 7.88 (dd, 1H), 7.59 (s, 1H), 7.39 (dd, 1H), 7.29 (t, 1H), 6.73 (d, 1H), 3.91 (d, 6H), 3.52 (s, 3H), 2.69 (tt, 1H), 2.43 (s, 3H), 2.36 (s, 3H), 0.60 (td, 2H), 0.42 - 0.34 (m, 2H); MS for C₂₉H₂₈FN₅O₆: *m*/*z* 562 (MH+).

**3-N-[4-[(6,7-Dimethoxy-1,5-naphthyridin-4-yl)oxy]-3-fluorophenyl]-4-hydroxy-5-N,2,6-trimethylpyridine-3,5-dicarboxamide (97):** Compound **97** was made from Compound **23-1** and methylamine using **General Procedure G1**. ¹H NMR (400 MHz, DMSO-*d*₆) δ 12.5 (s, 1H), 11.9 (s, 1H), 9.12 (s, 1H), 8.47 (d, 1H), 7.91 (dd, 1H), 7.58 (s, 1H), 7.34 (dd, 1H), 7.25 (t, 1H), 6.72 (d, 1H), 3.90 (d, 6H), 2.67 (d, 3H), 2.50 (s, 3H), 2.39 (s, 3H); MS for C₂₆H₂₄FN₅O₆: *m*/*z* 522 (MH+).

**5-N-Cyclopropyl-3-N-[4-[(6,7-dimethoxy-1,5-naphthyridin-4-yl)oxy]-3-fluorophenyl]-4-hydroxy-2,6-dimethylpyridine-3,5-dicarboxamide (98):** Compound **98** was made from Compound **23-1** and cyclopropylamine using **General Procedure G1**. ¹H NMR (400 MHz, DMSO-*d*₆) δ 12.15 (s, 1H), 11.92 (s, 1H), 9.26 (s, 1H), 8.47 (d, 1H), 7.90 (dd, 1H), 7.59 (s, 1H), 7.39 - 7.32 (m, 1H), 7.26 (t, 1H), 6.72 (dd, 1H), 3.90 (d, 6H), 2.71 (tt, 1H), 2.48 (s, 3H), 2.39 (s, 3H), 0.61 (td, 2H), 0.43 - 0.35 (m, 2H); MS for C₂₈H₂₆FN₅O₆: *m*/*z* 548 (MH+).

### Example 25: N-[4-[(6,7-Dimethoxy-1,5-naphthyridin-4-yl)oxy]-3-fluorophenyl]-1,2,6-trimethyl-5-morpholin-4-yl-4-oxopyridine-3-carboxamide (88)

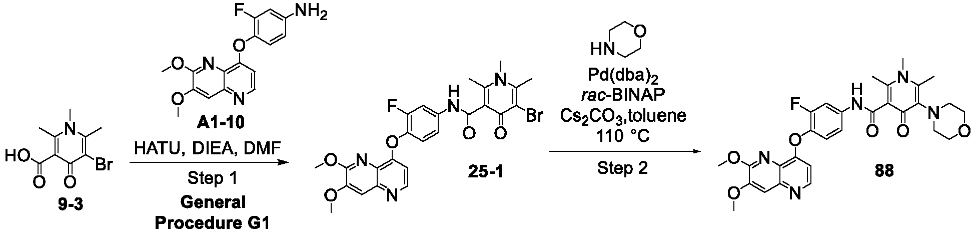

**Step 1: 5-Bromo-N-(4-((6,7-dimethoxy-1,5-naphthyridin-4-yl)oxy)-3-fluorophenyl)-1,2,6-trimethyl-4-oxo-1,4-dihydropyridine-3-carboxamide (25-1):** Compound **25-1** was made from Compound **A1-10** and Compound **9-3** using **General Procedure G1**. MS for C₂₅H₂₂BrFN₄O₅: *m*/*z* 559.1 (MH+).

**Step 2: N-[4-[(6,7-Dimethoxy-1,5-naphthyridin-4-yl)oxy]-3-fluorophenyl]-1,2,6-trimethyl-5-morpholin-4-yl-4-oxopyridine-3-carboxamide (88):** To a 4 mL vial equipped with a magnetic stir bar and a pressure relief septum was added Compound **25-1** (150 mg, 0.27 mmol), cesium carbonate (270 mg, 0.83 mmol, 3.0 equiv.), morpholine (0.035 mL, 0.41 mmol, 1.5 equiv.), Pd(dba)₂ (17 mg, 0.03 mmol, 0.11 equiv.), and *rac*-BINAP (36 mg, 0.06 mmol, 0.21 equiv.). The vial was purged with N₂, then toluene (2 mL) was added. The vial was sealed and heated at 110 °C for 10 h. The reaction was then evaporated under reduced pressure and loaded onto Celite and purified by chromatography over silica gel (0 - 5% MeOH/DCM) followed by further purification using preparative HPLC to yield Compound **88** as a white solid (3.0 mg, 2% yield). ¹H NMR (400 MHz, DMSO-*d*₆) δ 11.22 (s, 1H), 8.46 (d, 1H), 7.89 (dd, 1H), 7.59 (s, 1H), 7.40 (ddd, 1H), 7.28 (t, 1H), 6.71 (dd, 1H), 3.91 (s, 3H), 3.90 (s, 3H), 3.51 (s, 3H), 3.30 - 3.22 (m, 8H), 2.49 (s, 3H), 2.37 (s, 3H); MS for C₂₀H₃₀FN₅O₆: *m*/*z* 564.2 (MH+).

The following compounds were made using the same procedure used to make Compound **88** in **Example 25:**
N-[4-[(6,7-Dimethoxy-1,5-naphthyridin-4-yl)oxy]-3-fluorophenyl]-1,2,6-trimethyl-4-oxo-5-pyrrolidin-1-ylpyridine-3-carboxamide (89): The morpholine in Step 2 was replaced with pyrrolidine. ¹H NMR (400 MHz, DMSO-*d*₆) δ 11.27 (s, 1H), 8.46 (d, 1H), 7.89 (dd, 1H), 7.59 (s, 1H), 7.39 (ddd, 1H), 7.27 (t, 1H), 6.70 (dd, 1H), 3.91 (s, 3H), 3.90 (s, 3H), 3.52 (s, 3H), 3.03 - 2.94 (m, 4H), 2.44 (s, 3H), 2.39 (s, 3H), 1.83 - 1.75 (m, 4H); MS for C₂₉H₃₀FN₅O₅: *m*/*z* 548.2

**N-[4-[(6,7-Dimethoxy-1,5-naphthyridin-4-yl)oxy]-3-fluorophenyl]-1,2,6-trimethyl-4-oxo-5-pyrrolidin-1-ylpyridine-3-carboxamide (90):** The morpholine in Step 2 was replaced with piperidine. ¹H NMR (400 MHz, DMSO-*d*₆) δ 11.40 (s, 1H), 8.46 (d, 1H), 7.89 (dd, 1H), 7.59 (s, 1H), 7.44 - 7.35 (m, 1H), 7.27 (t, 1H), 6.71 (d, 1H), 3.91 (s, 3H), 3.90 (s, 3H), 3.51 (s, 3H), 3.47 - 3.33 (m, 2H), 2.58 - 2.48 (m, 2H), 2.46 (s, 3H), 2.39 (s, 3H), 1.71 - 1.07 (m, 6H); MS for C₃₀H₃₂FN₅O₅: *m*/*z* 562.2 (MH+).

### Example 26: Methyl 6-methyl-8-oxo-1,3,4,8-tetrahydropyrido[2,1-c][1,4]oxazine-9-carboxylate (26-2)

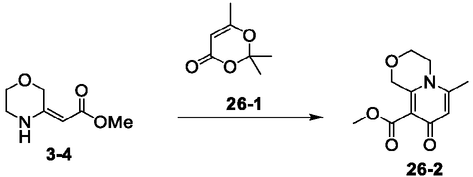

**Methyl 6-methyl-8-oxo-1,3,4,8-tetrahydropyrido[2,1-c][1,4]oxazine-9-carboxylate (26-2):** A mixture of Compound **3-4** (2.7 g, 17.18 mmol, 1 eq) and Compound **26-1** (5.83 g, 41.03 mmol, 5.40 mL, 2.39 eq) was stirred at 130 °C for 3 h with a Dean-Stark trap. The reaction mixture was concentrated under vacuum. The resulting residue was triturated with EtOAc at 20 - 25 °C for 30 min to give Compound **26-2** as a brown solid (2.3 g, 56% yield). MS for C₁₁H₁₃NO₄: *m*/*z* 224.1 (MH+).

Compounds 1-101 in Table 1 were prepared using methods similar to the general procedures described above and the synthetic procedures described in Examples 1-26 above.

### Kinase Assays

Kinase activity and compound inhibition were investigated using the ³³P-Phosphoryl transfer radiometric kinase assay, performed using the KinaseProfiler^{™} service of Eurofins Pharma Discovery Services UK Limited. Dose-response experiments were performed using nine compound concentrations in a 96-well microtiter plate. For each assay, all compounds were prepared to a 50x final assay concentration (50 µM) in 100% DMSO, then diluted in a half-log series, with the final top concentration at 1 µM. This working stock of the compound was added to the assay well as the first component in the reaction, followed by the remaining components as detailed in the following assay protocols below. The positive control wells (100% kinase activity) contain all components of the reaction including 2% DMSO (control for solvent effects), except the compound of interest. Blank wells contain all components of the reaction, with the reference inhibitor, staurosporine. This reference compound was used to abolish kinase activity and generated the 0% kinase activity base-line. IC₅₀ values were calculated by nonlinear regression analysis using the sigmoidal dose-response (variable slope) curve fit on XLFit version 5.3 (ID Business Solutions).

Kinase activity and compound inhibition were also investigated using the HTRF ^{®} KinEase assay (Cisbio Cat # 62TK0PEB) per manufacturer's instructions. In short, compounds were delivered in 300 nL volumes at 10 different concentrations in DMSO (3% final) to empty 384-well assay plates (Corning cat # 3824). A mixture of enzyme, 1 µM biotinylated peptide substrate, and buffer in 10 µL volume was added. The assay was started upon the addition of ATP (at Km). The 10 µL reaction was incubated at room temperature. The reaction was stopped upon the addition of detection buffer containing streptavidin-XL665 (5 µL) and TK antibody-Eu3+ (5 µL). After a 60 min incubation at room temperature, the fluorescence at 665 nm and 620 nm was read on an Envision microplate reader (Perkin Elmer).

Kinase activity normalized to DMSO (100% activity) and reference compound at 1 µM and (0% activity) was calculated using the fluorescence ratio 620/665 x 10,000. IC₅₀ values were calculated by nonlinear regression analysis using a 4-parameter logistic curve fit in ActivityBase XE (IDBS).

### Example A: Human AXL Kinase Assay

**Example A-1:** Human Axl (residues H473-A894 with Q764R, 161 nM) was incubated with 8 mM MOPS pH 7.0, 0.2 mM EDTA, 250 µM KKSRGDYMTMQIG (SEQ ID NO:1), 10 mM magnesium acetate and 10 µM [γ-³³P-ATP]. The reaction was initiated by the addition of the Mg/ATP mix. After incubation for 40 minutes at room temperature, the reaction was stopped by the addition of phosphoric acid to a concentration of 0.5%. A reaction aliquot of 10 µL was then spotted onto a P30 filtermat and washed four times for 4 minutes in 0.425% phosphoric acid and once in methanol prior to drying and scintillation counting. Incorporated ³³P was measured using the Wallac Microbeta scintillation counter (Perkin Elmer).

**Example A-2:** Human AXL (residues 464-885; CarnaBio, 1 ng/ well) was also incubated with enzymatic buffer (Cisbio) supplemented with 5 mM MgCl₂, 1 mM DTT, and Supplemental Enzymatic Buffer (SEB; Cisbio). The mixture was added to the pre-plated compounds. The reaction was initiated upon addition of ATP at Km (1.0 µM). The reaction was incubated at room temperature for 50 min and stopped upon the addition of SA-XL665 and TK-antibody both diluted in EDTA-containing kinase detection buffer (Cisbio). The kinase activity was calculated as stated above and the IC₅₀ values were reported.

### Example B: Human KDR Kinase Assay

**Example B-1:** Human KDR (residues K790-V1356, 55nM) was incubated with 8 mM MOPS pH 7.0, 0.2 mM EDTA, 0.33 mg/mL myelin basic protein, 10 mM magnesium acetate, and 10 µM [γ-³³P-ATP]. The reaction was initiated by the addition of the Mg/ATP mix. After incubation for 40 minutes at room temperature, the reaction was stopped by the addition of phosphoric acid to a concentration of 0.5%. A reaction aliquot of 10 µL was then spotted onto a P30 filtermat and washed four times for 4 minutes in 0.425% phosphoric acid and once in methanol prior to drying and scintillation counting. Incorporated ³³P was measured using the Wallac Microbeta scintillation counter (Perkin Elmer).

**Example B-2:** Human KDR (residues 790-1356; CarnaBio, 0.1 ng/ well) was also incubated with enzymatic buffer (Cisbio) supplemented with 5 mM MgCl₂, 1 mM MnCl₂, and 1 mM DTT. The mixture was added to the pre-plated compounds. The reaction was initiated upon addition of ATP at Km (4.0 µM). The reaction was incubated at room temperature for 40 min and stopped upon the addition of SA-XL665 and TK-antibody both diluted in EDTA-containing kinase detection buffer (Cisbio). The kinase activity was calculated as stated above and the IC₅₀ values were reported.

### Example C: Human Mer Kinase Assay

**Example C-1:** Human Mer (residues R557-E882 with H628Q and R794A, 0.7nM) was incubated with 8 mM MOPS pH 7.0, 0.2 mM EDTA, 30 mM NaCl, 250 µM GGMEDIYFEFMGGKKK (SEQ ID NO: 2), 10 mM magnesium acetate, and 10 µM [γ-³³P-ATP]. The reaction was initiated by the addition of the Mg/ATP mix. After incubation for 40 minutes at room temperature, the reaction was stopped by the addition of phosphoric acid to a concentration of 0.5%. A reaction aliquot of 10 µL was then spotted onto a P30 filtermat and washed four times for 4 minutes in 0.425% phosphoric acid and once in methanol prior to drying and scintillation counting. Incorporated ³³P was measured using the Wallac Microbeta scintillation counter (Perkin Elmer).

**Example C-2:** Human MER (residues 528-999; CarnaBio, 1 ng/ well) was also incubated with enzymatic buffer (Cisbio) supplemented with 5 mM MgCl₂ and 1 mM DTT. The mixture was added to the pre-plated compounds. The reaction was initiated upon addition of ATP at Km (40 µM). The reaction was incubated at room temperature for 60 min and stopped upon the addition of SA-XL665 and TK-antibody both diluted in EDTA-containing kinase detection buffer (Cisbio). The kinase activity was calculated as stated above and the IC₅₀ values were reported.

### Example D: Human Met Kinase Assay

**Example D-1:** Human Met (residues R974-S1390 with A1209G and V1290L, 3.4nM) was incubated with 8 mM MOPS pH 7.0, 0.2 mM EDTA, 250 µM KKKGQEEEYVFIE (SEQ ID NO:3), 1 mM sodium orthovanadate, 5 mM sodium-6-glycerophosphate, 10 mM magnesium acetate, and 10 µM [γ-³³P-ATP]. The reaction was initiated by the addition of the Mg/ATP mix. After incubation for 40 minutes at room temperature, the reaction was stopped by the addition of phosphoric acid to a concentration of 0.5%. A reaction aliquot of 10 µL was then spotted onto a P30 filtermat and washed four times for 4 minutes in 0.425% phosphoric acid and once in methanol prior to drying and scintillation counting. Incorporated ³³P was measured using the Wallac Microbeta scintillation counter (Perkin Elmer).

**Example D-2:** Human MET (residues 956-1390; CarnaBio, 0.1 ng/ well) was also incubated with enzymatic buffer (Cisbio) supplemented with 5 mM MgCl₂, 1 mM DTT and 1 mM MnCl₂. The mixture was added to the pre-plated compounds. The reaction was initiated upon addition of ATP at Km (3.0 µM). The reaction was incubated at room temperature for 40 min and stopped upon the addition of SA-XL665 and TK-antibody both diluted in EDTA-containing kinase detection buffer (Cisbio). The kinase activity was calculated as stated above and the IC₅₀ values were reported.

### Example E: AXL Autophosphorylation ELISA in A-172 Cells

A-172 glioblastoma cells (ATCC #CRL-1620) were seeded at 2.5 x 10⁵ cells/well onto 24-well plates (Greiner #662165), in DMEM (Thermo Fisher #11995-040) containing 10% FBS (Thermo Fisher #26140-079), 1% MEM NEAA (Thermo Fisher #11140-050), 1% GlutaMax (Thermo Fisher #35050-061), and 1% Penicillin Streptomycin (Thermo Fisher #15140-122). A-172 cells were incubated at 37°C, 5% CO₂ for 24 h and then starved for 24 h in serum-free medium. Test compounds were serially diluted to produce an 8-point dose curve in fresh serum-free medium to a final concentration of 0.3% DMSO (vehicle) and added to the cells and incubated for 1 h. Cells were then stimulated with 1 µg/mL recombinant human Gas6 (R&D Systems #885-GSB-500) for 15 min, washed with cold PBS, and immediately lysed with 150 µL of cold 1X lysis buffer [20 mM Tris, 137 mM sodium chloride, 2 mM EDTA, 10% glycerol, 1% NP-40 alternative, 1 mM activated sodium orthovanadate, 1 mM PefaBloc SC (Sigma-Aldrich #11429868001), protease/phosphatase inhibitor tablet (Thermo Fisher #A32959)]. Lysates were collected and 100 µL/well added into the human phospho-AXL DuoSet IC ELISA (R&D Systems #DYC2228-2). Assay was performed according to manufacturer's instructions and sample phospho-AXL concentrations were extrapolated using human phospho-AXL control (R&D Systems #841645) as a standard. Positive control wells (100% activity) contained Gas6-stimulated, DMSO-treated cell lysates. Negative control wells (0% activity) contained Gas6-stimulated, reference inhibitor-treated cell lysates. IC₅₀ values were calculated by nonlinear regression analysis using a 4-parameter logistic curve fit in ActivityBase XE (IDBS).

### Example F: Met Autophosphorylation ELISA in PC-3 Cells

PC-3 prostate cancer cells (ATCC #CRL-1435) were seeded at 4 x 10⁴ cells/well onto 24-well plates (Greiner #662165), in DMEM (Thermo Fisher #11995-040) containing 10% FBS (Thermo Fisher #26140-079), 1% MEM NEAA (Thermo Fisher #11140-050), 1% GlutaMax (Thermo Fisher #35050-061), and 1% Penicillin Streptomycin (Thermo Fisher #15140-122). PC-3 cells were incubated at 37°C, 5% CO₂ for 24 h and then starved for 3 h in serum-free medium. Test compounds were serially diluted to produce an 8-point dose curve in fresh serum-free medium to a final concentration of 0.3% DMSO (vehicle) and added to the cells and incubated for 1 h. Cells were then stimulated with 100 ng/mL recombinant human HGF (R&D Systems #294-HG-250) for 10 min, washed with cold PBS, and immediately lysed with 130 µL of cold 1X lysis buffer [20 mM Tris, 137 mM sodium chloride, 2 mM EDTA, 10% glycerol, 1% NP-40 alternative, 1 mM activated sodium orthovanadate, 1 mM PefaBloc SC (Sigma-Aldrich #11429868001), protease/phosphatase inhibitor tablet (Thermo Fisher #A32959)]. Lysates were clarified by centrifugation and 100 µL/well added into the PathScan phospho-Met (panTyr) Sandwich ELISA (Cell Signaling Technology #7333). Assay was performed according to manufacturer's instructions. Positive control wells (100% activity) contained HGF-stimulated, DMSO-treated cell lysates. Negative control wells (0% activity) contained HGF-stimulated, reference inhibitor-treated cell lysates. IC₅₀ values were calculated by nonlinear regression analysis using a 4-parameter logistic curve fit in ActivityBase XE (IDBS).

### Example G: KDR Autophosphorylation ELISA in HUVEC Cells

Human umbilical vein endothelial cells or HUVEC (Lonza #C2519A) were seeded at 2 x 10⁴ cells/well onto 96-well plates (Corning #3904), in EGM-2 growth medium (Lonza #CC-3162) containing 1% Penicillin Streptomycin (Thermo Fisher #15140-122). HUVEC cells were incubated at 37°C, 5% CO₂ for 24 h and then starved for 24 h in serum-free EBM-2 basal medium (Lonza #CC-3156) containing 1% Penicillin Streptomycin. Test compounds were serially diluted to produce an 8-point dose curve in fresh serum-free medium to a final concentration of 0.3% DMSO (vehicle) and added to the cells and incubated for 1 h. Cells were then stimulated with 100 ng/mL recombinant human VEGF165 (R&D Systems #293-VE-500) for 5 min, washed with cold PBS, and immediately lysed with 130 µL of cold 1X lysis buffer [20 mM Tris, 137 mM sodium chloride, 2 mM EDTA, 10% glycerol, 1% NP-40 alternative, 1 mM activated sodium orthovanadate, 1 mM PefaBloc SC (Sigma-Aldrich #11429868001), protease/phosphatase inhibitor tablet (Thermo Fisher #A32959)]. Lysates were collected and 100 µL/well added into the human phospho-KDR DuoSet IC ELISA (R&D Systems #DYC1766-2). Assay was performed according to manufacturer's instructions and sample phospho-KDR concentrations were extrapolated using human phospho-KDR control (R&D Systems #841421) as a standard. Positive control wells (100% activity) contained VEGF165-stimulated, DMSO-treated cell lysates. Negative control wells (0% activity) contained non-stimulated cell lysates. IC₅₀ values were calculated by nonlinear regression analysis using a 4-parameter logistic curve fit in ActivityBase XE (IDBS).

### Example H: Mer Autophosphorylation ELISA in Transient Transfected 293A Cells

293A cells (Thermo Fisher #R70507) were seeded at 1.5 x 10⁶ cells/well onto 100 mm dish (Greiner #664169), in DMEM (Thermo Fisher #11995-040) containing 10% FBS (Thermo Fisher #26140-079), 1% MEM NEAA (Thermo Fisher #11140-050), 1% GlutaMax (Thermo Fisher #35050-061), and 1% Penicillin Streptomycin (Thermo Fisher #15140-122). 293A cells were incubated at 37°C, 5% CO₂ for 24 h and then transfected with 6 µg MERTK DNA (Genecopoeia #EX-Z8208-M02) using TransIT LT1 transfection reagent (Mirus-Bio #MIR2305). After 24 h incubation, the transfected 293A cells were seeded at 1 x 10⁵ cells/well onto 96-well plates (Corning #3904) in DMEM growth medium overnight. Test compounds were serially diluted to produce an 8-point dose curve in fresh serum-free medium to a final concentration of 0.3% DMSO (vehicle) and added to the cells and incubated for 1 h. Cells were then immediately lysed with 150 µL of cold 1X lysis buffer [20 mM Tris, 137 mM sodium chloride, 2 mM EDTA, 10% glycerol, 1% NP-40 alternative, 1 mM activated sodium orthovanadate, 1 mM PefaBloc SC (Sigma-Aldrich #11429868001), protease/phosphatase inhibitor tablet (Thermo Fisher #A32959)]. Lysates were clarified by centrifugation and 50 µL/well added into the human phospho-Mer DuoSet IC ELISA (R&D Systems #DYC2579-2). Assay was performed according to manufacturer's instructions and sample phospho-Mer concentrations were extrapolated using human phospho-Mer control (R&D Systems #841793) as a standard. Positive control wells (100% activity) contained DMSO-treated cell lysates. Negative control wells (0% activity) contained reference inhibitor-treated cell lysates. IC₅₀ values were calculated by nonlinear regression analysis using a 4-parameter logistic curve fit in ActivityBase XE (IDBS).

Results of Examples A-H are summarized in Table 2. A, B, and C of Table 2 have the following meanings: A = IC₅₀ ≤ 100 nM; B = 100 < IC₅₀ ≤ 300 nM; C = IC₅₀ > 300 nM. "NT" refers to "Not Tested." The data for compounds in Table 52 were obtained using the protocols set forth in Examples A-1, B-1, C-1 D-1, A-2, B-2, C-2, and/or D-2.

**Table 2. Cellular Activities of Selected Compounds**

| **Compound No.** | **Axl IC₅₀ (nM)** | **Mer IC₅₀ (nM)** | **c-Met IC₅₀ (nM)** | **KDR IC₅₀ (nM)** |
|---|---|---|---|---|
| G1-1 | NT | A | A | B |
| G1-2 | A | A | A | A |
| G1-3 | A | A | A | A |
| G1-4 | A | A | A | A |
| G1-5 | A | A | A | C |
| G1-6 | NT | A | A | C |
| G1-7 | NT | A | A | C |
| G1-8 | A | A | C | C |
| 1-2 | A | A | A | A |
| 1-3 | A | A | A | A |
| 1-4 | A | A | A | A |
| 1-5 | A | A | C | A |
| 1-6 | A | A | A | B |
| 1-7 | A | A | A | A |
| 1-8 | NT | A | A | A |
| 1-9 | A | A | A | A |
| 2-3 | A | A | A | A |
| 1 | A | A | A | A |
| 2 | A | A | A | A |
| 3 | NT | A | A | A |
| 4 | NT | A | A | A |
| 5 | NT | A | A | A |
| 6 | A | A | A | A |
| 7 | A | A | A | A |
| 8 | A | A | A | A |
| 9 | A | A | A | A |
| 10 | A | A | A | A |
| 11 | A | A | A | A |
| 12 | A | A | A | C |
| 13 | A | A | A | A |
| 14 | A | A | A | A |
| 15 | A | A | A | A |
| 16 | A | A | A | A |
| 17 | A | A | A | A |
| 18 | A | A | A | A |
| 19 | A | A | A | A |
| 20 | A | A | A | A |
| 21 | C | A | A | B |
| 22 | A | A | A | B |
| 23 | A | A | A | A |
| 24 | A | A | A | A |
| 25 | A | A | C | A |
| 26 | A | A | A | A |
| 27 | A | A | A | A |
| 28 | A | A | C | A |
| 29 | A | A | A | A |
| 30 | A | A | A | A |
| 31 | A | A | A | A |
| 32 | A | A | A | A |
| 33 | A | A | A | A |
| 34 | A | A | A | A |
| 35 | A | A | A | A |
| 36 | A | A | A | A |
| 37 | A | A | A | A |
| 38 | B | A | C | B |
| 39 | C | A | C | C |
| 40 | A | A | A | A |
| 41 | A | A | A | A |
| 42 | A | A | A | A |
| 43 | A | A | A | A |
| 44 | A | A | A | B |
| 45 | A | A | A | A |
| 46 | B | A | C | C |
| 47 | A | A | C | C |
| 48 | A | C | C | C |
| 49 | A | A | C | A |
| 50 | A | A | C | C |
| 51 | A | A | A | C |
| 52 | A | A | C | C |
| 53 | A | A | A | C |
| 54 | A | A | B | B |
| 55 | A | A | C | C |
| 56 | NT | A | C | C |
| 57 | NT | A | C | A |
| 58 | A | A | A | A |
| 59 | NT | A | C | B |
| 60 | NT | C | C | A |
| 61 | NT | C | C | A |
| 62 | NT | A | C | A |
| 63 | NT | A | C | C |
| 64 | NT | A | C | C |
| 65 | A | A | A | B |
| 66 | A | A | A | C |
| 67 | NT | C | C | C |
| 68 | NT | C | C | A |
| 69 | NT | A | A | A |
| 70 | NT | C | C | A |
| 71 | NT | C | C | A |
| 72 | NT | C | C | A |
| 73 | A | A | A | C |
| 74 | A | A | A | A |
| 75 | NT | C | C | C |
| 76 | NT | C | C | C |
| 77 | NT | C | C | C |
| 78 | NT | C | C | C |
| 79 | A | A | A | C |
| 80 | A | A | A | A |
| 81 | C | C | C | C |
| 82 | C | C | C | C |
| 83 | B | C | C | C |
| 84 | C | C | C | C |
| 85 | C | C | C | C |
| 86 | C | C | C | C |
| 87 | C | C | C | B |
| 88 | A | A | C | C |
| 89 | A | A | A | A |
| 90 | A | A | C | B |
| 91 | C | C | C | C |
| 92 | C | C | C | B |
| 93 | A | C | A | A |
| 94 | A | A | C | A |
| 95 | B | C | C | C |
| 96 | C | C | C | C |
| 97 | A | C | C | B |
| 98 | A | C | C | A |
| 99 | A | A | A | C |
| 100 | A | A | A | C |
| 101 | A | A | NT | NT |

| | | | | |
|---|---|---|---|---|
| NT means not tested | | | | |

Unless otherwise defined, all technical and scientific terms used herein have the same meaning as commonly understood by one of ordinary skill in the art to which this invention belongs.

## Claims

1. A compound of Formula (Ia), Formula (Ib), or Formula (Ic): 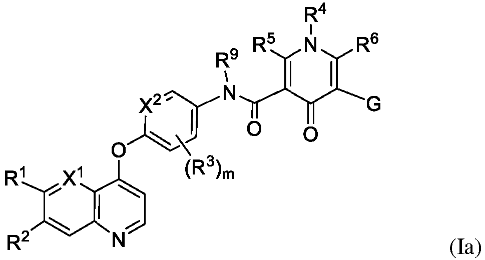 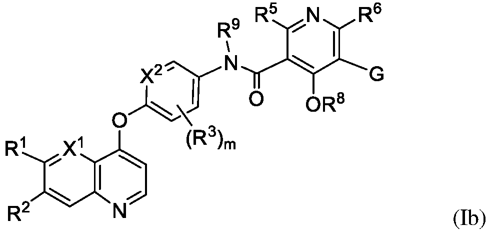 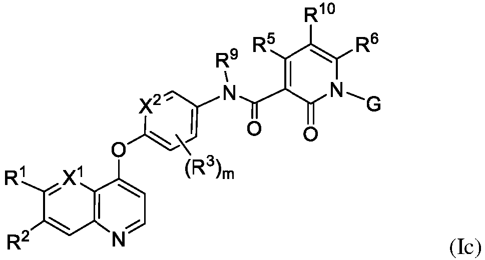 or a pharmaceutically acceptable salt, stereoisomer or tautomer thereof, wherein:
G is C₃₋₁₀ cycloalkyl, 4- to 14-membered heterocycloalkyl, C₁₋₆ alkyl, C₂₋₆ alkenyl, C₁₋₆ alkoxy, cyano, halo, C(O)OR^{a}, or C(O)NR^{a}R^{a}, wherein the C₃₋₁₀ cycloalkyl, 4- to 14-membered heterocycloalkyl, C₁₋₆ alkyl, C₂₋₆ alkenyl, and C₁₋₆ alkoxy of G are each optionally substituted with 1, 2, 3, or 4 independently selected R⁷ substituents;
X¹ is N;
X² is CH or CF;
R¹ and R² are each independently selected from H, halo, C₁₋₆ alkyl, C₂₋₆ alkenyl, C₂₋₆ alkynyl, C₁₋₆ haloalkyl, C₁₋₆ haloalkoxy, C₆₋₁₀ aryl, C₃₋₁₄ cycloalkyl, 5-14 membered heteroaryl, 4-14 membered heterocycloalkyl, C₆₋₁₀ aryl-C₁₋₄ alkylene-, C₃₋₁₄ cycloalkyl-C₁₋₄ alkylene-, (5-14 membered heteroaryl)-C₁₋₄ alkylene-, (4-14 membered heterocycloalkyl)-C₁₋₄ alkylene-, CN, NO₂, OR^{a}, SR^{a}, NHOR^{a}, C(O)R^{a}, C(O)NR^{a}R^{a}, C(O)OR^{a}, C(O)NR^{a}S(O)₂R^{a}, OC(O)R^{a}, OC(O)NR^{a}R^{a}, NHR^{a}, NR^{a}R^{a}, NR^{a}C(O)R^{a}, NR^{a}C(=NR^{a})R^{a}, NR^{a}C(O)OR^{a}, NR^{a}C(O)NR^{a}R^{a}, C(=NR^{a})R^{a}, C(=NOH)R^{a}, C(=NOH)NR^{a}, C(=NCN)NR^{a}R^{a}, NR^{a}C(=NCN)NR^{a}R^{a}, C(=NR^{a})NR^{a}R^{a}, NR^{a}C(=NR^{a})NR^{a}R^{a}, NR^{a}S(O)R^{a}, NR^{a}S(O)₂R^{a}, NR^{a}S(O)₂NR^{a}R^{a}, S(O)R^{a}, S(O)NR^{a}R^{a}, S(O)₂R^{a}, S(O)₂NR^{a}C(O)R^{a}, P(O)R^{a}R^{a}, P(O)(OR^{a})(OR^{a}), B(OH)₂, B(OR^{a})₂, and S(O)₂NR^{a}R^{a}, wherein the C₁₋₆ alkyl, C₂₋₆ alkenyl, C₂₋₆ alkynyl, C₆₋₁₀ aryl, C₃₋₁₄ cycloalkyl, 5-14 membered heteroaryl, 4-14 membered heterocycloalkyl, C₆₋₁₀ aryl-C₁₋₄ alkylene-, C₃₋₁₄ cycloalkyl-C₁₋₄ alkylene-, (5-14 membered heteroaryl)-C₁₋₄ alkylene-, and (4-14 membered heterocycloalkyl)-C₁₋₄ alkylene- of R¹ and R² are each optionally substituted with 1, 2, 3, 4 or 5 independently selected R^{b} substituents;
each R³ is independently selected from halo, OH, CN, -C(O)OH, -C(O)NH(C₁₋₆ alkyl), -SO₂(C₁₋₆ alkyl), -SO₂NH(C₁₋₆ alkyl), C₁-C₆ alkyl, C₁-C₆ alkoxy, C₁-C₆ haloalkoxy, NH₂, -NH(C₁-C₆ alkyl), -N(C₁-C₆ alkyl)₂, and C₃-C₆ cycloalkyl, wherein the C₁-C₆ alkyl, C₁-C₆ alkoxy, -NH(C₁-C₆alkyl), -N(C₁-C₆ alkyl)₂, and C₃-C₆ cycloalkyl of R³ are each optionally substituted with 1, 2, or 3 independently selected R^{g} substituents;
R⁴ is selected from H, halo, C₁₋₆ alkyl, C₂₋₆ alkenyl,
C₂₋₆ alkynyl, C₁₋₆ haloalkyl, C₁₋₆ haloalkoxy, C₆₋₁₀ aryl, C₃₋₁₄ cycloalkyl, 5-14 membered heteroaryl, 4-14 membered heterocycloalkyl, C₆₋₁₀ aryl-C₁₋₄ alkylene-, C₃₋₁₄ cycloalkyl-C₁₋₄ alkylene-, (5-14 membered heteroaryl)-C₁₋₄ alkylene-, (4-14 membered heterocycloalkyl)-C₁₋₄ alkylene-, CN, NO₂, OR^{a}, SR^{a}, NHOR^{a}, C(O)R^{a}, C(O)NR^{a}R^{a}, C(O)OR^{a}, C(O)NR^{a}S(O)₂R^{a}, OC(O)R^{a}, OC(O)NR^{a}R^{a}, NHR^{a}, NR^{a}R^{a}, NR^{a}C(O)R^{a}, N=C(NR^{a}R^{a})₂, NR^{a}C(=NR^{a})R^{a}, NR^{a}C(O)OR^{a}, NR^{a}C(O)NR^{a}R^{a}, C(=NR^{a})R^{a}, C(=NOH)R^{a}, C(=NOH)NR^{a}, C(=NCN)NR^{a}R^{a} , NR^{a}C(=NCN)NR^{a}R^{a}, C(=NR^{a})NR^{a}R^{a}, NR^{a}C(=NR^{a})NR^{a}R^{a}, NR^{a}S(O)R^{a}, NR^{a}S(O)₂R^{a}, NR^{a}S(O)₂NR^{a}R^{a}, S(O)R^{a}, S(O)NR^{a}R^{a}, S(O)₂R^{a}, S(O)₂NR^{a}C(O)R^{a}, P(O)R^{a}R^{a}, P(O)(OR^{a})(OR^{a}), B(OH)₂, B(OR^{a})₂, and S(O)₂NR^{a}R^{a}, wherein the C₁₋₆ alkyl, C₂₋₆ alkenyl, C₂₋₆ alkynyl, C₆₋₁₀ aryl, C₃₋₁₄ cycloalkyl, 5-14 membered heteroaryl, 4-14 membered heterocycloalkyl, C₆₋₁₀ aryl-C₁₋₄ alkylene-, C₃₋₁₄ cycloalkyl-C₁₋₄ alkylene-, (5-14 membered heteroaryl)-C₁₋₄ alkylene-, and (4-14 membered heterocycloalkyl)-C₁₋₄ alkylene- of R⁴ are each optionally substituted with 1, 2, 3, 4, or 5 independently selected R^{b} substituents;
R⁵, R⁶ and R¹⁰ are each independently H, halo, C₁₋₆ alkyl, C₂₋₆ alkenyl, C₁₋₆ alkoxy, C₁₋₆ alkylthio, CN, C₁₋₄ haloalkyl, C₁₋₄ haloalkoxy, OH, C₁₋₄alkyl-C(O)-, C₁₋₄alkyl-OC(O)-,
-C(O)NH(C₁₋₄ alkyl), NH₂, -NHC₁₋₄alkyl, or -N(C₁₋₄ alkyl)₂, wherein the C₁₋₆ alkyl, C₂₋₆ alkenyl, C₁₋₆ alkoxy, C₁₋₆ alkylthio, C₁₋₆ alkyl-C(O)- and C₁₋₄ alkyl of -NH(C₁₋₄alkyl), or -N(C₁₋₄ alkyl)₂ of R⁵, R⁶, and R¹⁰ are each optionally substituted with 1 or 2 independently selected R^{g} substituents;
each R⁷ is independently selected from halo, OH, C(O)OR^{a}, C(O)R^{a}, C(O)NR^{a}R^{a}, CN, NH₂, - NH(C₁₋C₆ alkyl), -N(C₁₋C₆ alkyl)₂, C₁₋C₆ alkyl, C₂₋C₆ alkenyl, C₁₋C₆ alkoxy, C₁₋C₆ haloalkyl, C₁₋C₆ haloalkoxy, C(O)NR^{a}R^{a}, NR^{a}C(O)R^{a}, NR^{a}C(O)NR^{a}R^{a}, SO₂R^{a}, NR^{a}S(O)₂R^{a}, NR^{a}S(O)₂NR^{a}R^{a}, C₃-C₆ cycloalkyl, 4- to 6-membered heterocycloalkyl, phenyl, 5- or 6-membered heteroaryl, C₃-C₆ cycloalkyl-C₁₋C₄ alkylene-, (4- to 6-membered heterocycloalkyl)-C₁₋C₄ alkylene-, phenyl-C₁₋C₂ alkylene, and (5- or 6-membered heteroaryl)-C₁₋C₄ alkylene-, wherein the C₁₋C₆ alkyl, C₁₋C₆ alkoxy, C₃-C₆ cycloalkyl, 4- to 6-membered heterocycloalkyl, phenyl, 5- or 6-membered heteroaryl, C₃-C₆ cycloalkyl-C₁₋C₄ alkylene-, (4-to 6-membered heterocycloalkyl)-C₁₋C₄ alkylene-, phenyl-C₁₋C₂ alkylene, and (5- or 6-membered heteroaryl)-C₁₋C₄ alkylene- of R⁷ are each optionally substituted with 1, 2, or 3 independently selected R^{f} substituents;
R⁸ is H, C₁₋₆ alkyl optionally substituted with 1 or 2 R^{g} substituents or a hydroxy protecting group;
R⁹ is H or C₁₋₆ alkyl optionally substituted with 1, 2, or 3 independently selected R^{g} substituents;
or R⁴ and R⁵ taken together with the atoms to which they are attached form 4- to 7-membered fused heterocycloalkyl or 5- to 6-membered fused heteroaryl, wherein the 4- to 7-membered fused heterocycloalkyl and 5- to 6-membered fused heteroaryl are each optionally substituted with 1 or 2 independently selected R^{g} substituents;
or R¹⁰ and R⁵ taken together with the atoms to which they are attached form fused C₃₋₇ cycloalkyl, 4- to 6-membered fused heterocycloalkyl, fused 5- or 6-membered heteroaryl or fused phenyl, wherein the fused C₃₋₇ cycloalkyl, 4- to 6-membered fused heterocycloalkyl, 5- or 6-membered heteroaryl, or fused phenyl is each optionally substituted with 1 or 2 independently selected R^{g} substituents, and wherein one or two ring carbon atoms of the fused C₃₋₇ cycloalkyl or fused heterocycloalkyl are optionally replaced by a carbonyl group;
or R¹² and R¹⁰ taken together with the atoms to which they are attached form 4- to 7-membered fused heterocycloalkyl or 5- to 6-membered fused heteroaryl, wherein the 4- to 7-membered fused heterocycloalkyl and 5- to 6-membered fused heteroaryl are each optionally substituted with 1 or 2 independently selected R^{g} substituents;
or R⁶ and R⁴ taken together with the atoms to which they are attached form 4- to 7-membered fused heterocycloalkyl or 5- to 6-membered fused heteroaryl, wherein the 4- to 7-membered fused heterocycloalkyl and 5- to 6-membered fused heteroaryl are each optionally substituted with 1 or 2 independently selected R^{g} substituents;
or R⁶ and R¹⁰ taken together with the atoms to which they are attached form fused C₃₋₇ cycloalkyl, 4- to 6-membered fused heterocycloalkyl, 5- to 6-membered fused heteroaryl, or fused heteroaryl, wherein the fused C₃₋₇ cycloalkyl, 4- to 6-membered fused heterocycloalkyl, 5- to 6-membered fused heteroaryl, and fused heteroaryl are each optionally substituted with 1 or 2 independently selected R^{g} substituents and wherein one or two ring carbon atoms of the fused C₃₋₇ cycloalkyl or 4- to 6-membered fused heterocycloalkyl are optionally replaced by a carbonyl;
each R^{a} is independently selected from H, CN, C₁-C₆ alkyl, C₁-C₆ haloalkyl, C₂-C₆ alkenyl, C₂-C₆ alkynyl, C₆-C₁₀ aryl, C₃-C₁₀ cycloalkyl, 5-14 membered heteroaryl, 4-14 membered heterocycloalkyl, C₆-C₁₀ aryl-C₁₋C₄ alkylene-, C₃-C₁₀ cycloalkyl-C₁₋C₄ alkylene-, (5-14 membered heteroaryl)-C₁₋C₄ alkylene-, and (4-14 membered heterocycloalkyl)-C₁₋C₄ alkylene-, wherein the C₁-C₆ alkyl, C₁-C₆ haloalkyl, C₂-C₆ alkenyl, C₂-C₆ alkynyl, C₆-C₁₀ aryl, C₃-C₁₀ cycloalkyl, 5-14 membered heteroaryl, 4-14 membered heterocycloalkyl, C₆-C₁₀ aryl-C₁₋C₄ alkylene-, C₃-C₁₀ cycloalkyl-C₁₋C₄ alkylene-, (5-14 membered heteroaryl)-C₁₋C₄ alkylene-, and (4-14 membered heterocycloalkyl)-C₁₋C₄ alkylene- of R^{a} are each optionally substituted with 1, 2, 3, 4, or 5 independently selected R^{d} substituents;
or any two R^{a} substituents together with the nitrogen atom to which they are attached form 4-, 5-, 6-, 7-, 8-, 9-, or 10-membered heterocycloalkyl, each of which is optionally substituted with 1, 2, or 3 independently selected R^{f} substituents;
each R^{b} is independently selected from halo, C₁-C₆ alkyl, C₂-C₆ alkenyl, C₂-C₆ alkynyl, C₁-C₆ haloalkyl, C₁-C₆ haloalkoxy, C₆-C₁₀ aryl, C₃-C₁₀ cycloalkyl, 5-10 membered heteroaryl, 4-10 membered heterocycloalkyl, C₆₋C₁₀ aryl-C₁₋C₄ alkylene-, C₃₋C₁₀ cycloalkyl-C₁₋C₄ alkylene-, (5-10 membered heteroaryl)-C₁₋C₄ alkylene-, (4-10 membered heterocycloalkyl)-C₁₋C₄ alkylene-, CN, OH, NH₂, NO₂, NHOR^{c}, OR^{c}, SR^{c}, C(O)R^{c}, C(O)NR^{c}R^{c}, C(O)OR^{c}, C(O)NR^{c}S(O)₂R^{c}, OC(O)R^{c}, OC(O)NR^{c}R^{c}, C(=NOH)R^{c}, C(=NOH)NR^{c}, C(=NCN)NR^{c}R^{c}, NR^{c}C(=NCN)NR^{c}R^{c}, C(=NR^{c})NR^{c}R^{c},
NR^{c}C(=NR^{c})NR^{c}R^{c}, NHR^{c}, NR^{c}R^{c}, NR^{c}C(O)R^{c}, NR^{c}C(=NR^{c})R^{c}, NR^{c}C(O)OR^{c}, NR^{c}C(O)NR^{c}R^{c}, NR^{c}S(O)R^{c}, NR^{c}S(O)₂R^{c}, NR^{c}S(O)₂NR^{c}R^{c}, S(O)R^{c}, S(O)NR^{c}R^{c}, S(O)₂R^{c}, S(O)₂NR^{c}C(O)R^{c}, Si(R^{c})₃, P(O)R^{c}R^{c}, P(O)(OR^{c})(OR^{c}), B(OH)₂, B(OR^{c})₂, and S(O)₂NR^{c}R^{c}, wherein the C₁-C₆ alkyl, C₂-C₆ alkenyl, C₂-C₆ alkynyl, C₆-C₁₀ aryl, C₃-C₁₀ cycloalkyl, 5-10 membered heteroaryl, 4-10 membered heterocycloalkyl, C₆-C₁₀ aryl-C₁₋C₄ alkylene-, C₃₋C₁₀ cycloalkyl-C₁₋C₄ alkylene-, (5-10 membered heteroaryl)-C₁₋C₄ alkylene-, and (4-10 membered heterocycloalkyl)-C₁-C₄ alkylene- of R^{b} are each further optionally substituted with 1, 2, or 3 independently selected R^{d} substituents;
each R^{c} is independently selected from H, C₁-C₆ alkyl, C₁-C₆ haloalkyl, C₂-C₆ alkenyl, C₂-C₆ alkynyl, C₆-C₁₀ aryl, C₃-C₁₀ cycloalkyl, 5-10 membered heteroaryl, 4-10 membered heterocycloalkyl, C₆-C₁₀ aryl-C₁₋C₄ alkylene-, C₃-C₁₀ cycloalkyl-C₁₋C₄ alkylene-, (5-10 membered heteroaryl)-C₁₋C₄ alkylene-, and (4-10 membered heterocycloalkyl)-C₁₋C₄ alkylene-, wherein the C₁-C₆ alkyl, C₂-C₆ alkenyl, C₂-C₆ alkynyl, C₆-C₁₀ aryl, C₃-C₁₀ cycloalkyl, 5-10 membered heteroaryl, 4-10 membered heterocycloalkyl, C₆-C₁₀ aryl-C₁₋C₄ alkylene-, C₃-C₁₀ cycloalkyl-C₁₋C₄ alkylene-, (5-10 membered heteroaryl)-C₁₋C₄ alkylene-, and (4-10 membered heterocycloalkyl)-C₁₋C₄ alkylene- of R^{c} are each optionally substituted with 1, 2, 3, 4, or 5 independently selected R^{f} substituents;
or any two R^{c} substituents together with the nitrogen atom to which they are attached form 4-, 5-, 6-, 7-, 8-, 9-, or 10-membered heterocycloalkyl, each of which is optionally substituted with 1, 2, or 3 independently selected R^{f} substituents;
each R^{d} is independently selected from C₁₋C₆ alkyl, C₁₋C₆ haloalkyl, halo, C₆₋C₁₀ aryl, 5-10 membered heteroaryl, C₃₋C₁₀ cycloalkyl, 4-10 membered heterocycloalkyl, C₆₋C₁₀ aryl-C₁₋C₄ alkylene-, C₃₋C₁₀ cycloalkyl-C₁₋C₄ alkylene-, (5-10 membered heteroaryl)-C₁₋C₄ alkylene-, (4-10 membered heterocycloalkyl)-C₁₋C₄ alkylene-, CN, NH₂, NHOR^{e}, OR^{e}, SR^{e}, C(O)R^{e}, C(O)NR^{e}R^{e}, C(O)OR^{e},
OC(O)R^{e}, OC(O)NR^{e}R^{e}, NHR^{e}, NR^{e}R^{e}, NR^{e}C(O)R^{e}, NR^{e}C(O)NR^{e}R^{e}, NR^{e}C(O)OR^{e}, C(=NR^{e})NR^{e}R^{e}, NR^{e}C(=NR^{e})NR^{e}R^{e}, NR^{e}C(=NOH)NR^{e}R^{e}, NR^{e}C(=NCN)NR^{e}R^{e}, S(O)R^{e}, S(O)NR^{e}R^{e}, S(O)₂R^{e}, NR^{e}S(O)₂R^{e}, NR^{e}S(O)₂NR^{e}R^{e}, and S(O)₂NR^{e}R^{e}, wherein the C₁₋C₆ alkyl, C₆-C₁₀ aryl, 5-10 membered heteroaryl, C₃₋C₁₀ cycloalkyl, 4-10 membered heterocycloalkyl, C₆₋C₁₀ aryl-C₁₋C₄ alkylene-, C₃₋C₁₀ cycloalkyl-C₁₋C₄ alkylene-, (5-10 membered heteroaryl)-C₁₋C₄ alkylene-, and (4-10 membered heterocycloalkyl)-C₁₋C₄ alkylene- of R^{d} are each optionally substituted with 1, 2, or 3 independently selected R^{f} substituents;
each R^{e} is independently selected from H, C₁₋C₆ alkyl, C₁₋C₆ alkoxy, C₃₋C₆ cycloalkyl, C₃₋C₆ cycloalkyl-C₁₋C₄ alkylene-, C₆₋C₁₀ aryl, C₆₋C₁₀ aryl-C₁₋C₄ alkylene-, 5- or 6-membered heteroaryl, (5- or 6-membered heteroaryl)-C₁₋C₄ alkylene-, 4-7-membered heterocycloalkyl, (4-7-membered heterocycloalkyl)-C₁₋C₄ alkylene-, C₁₋C₆ haloalkyl, C₁₋C₆ haloalkoxy, C₂₋C₄ alkenyl, and C₂₋C₄ alkynyl, wherein the C₁₋C₆ alkyl, C₁₋C₆ alkoxy, C₃₋C₆ cycloalkyl, C₆₋C₁₀ aryl, 5 or 6-membered heteroaryl, 4-7-membered heterocycloalkyl, C₆₋C₁₀ aryl-C₁₋C₄ alkylene-, (5- or 6-membered heteroaryl)-C₁₋C₄ alkylene-, (4-7-membered heterocycloalkyl)-C₁₋C₄ alkylene-, C₂₋C₄ alkenyl, and C₂₋C₄ alkynyl of R^{e} are each optionally substituted with 1, 2, or 3 R^{f} substituents;
or any two R^{e} substituents together with the nitrogen atom to which they are attached form a 4-, 5-, 6-, 7-, 8-, 9-, or 10-membered heterocycloalkyl, each of which is optionally substituted with 1, 2, or 3 independently selected R^{f} substituents;
each R^{f} is independently selected from halo, OH, CN, C(O)OH, NH₂, -NH(C₁₋C₆ alkyl), -N(C₁₋C₆ alkyl)₂, C₁₋C₆ alkyl, vinyl, C₁₋C₆ alkoxy, C₁₋C₆ alkylthio, C₁₋C₆haloalkyl, C₁₋C₆haloalkoxy, phenyl, 5-6 membered heteroaryl, 4-6 membered heterocycloalkyl, and C₃₋C₆ cycloalkyl, wherein the C₁₋C₆ alkyl, C₁₋C₆ alkoxy, C₁₋C₆ alkylthio, phenyl, C₃₋C₆ cycloalkyl, 4-6 membered heterocycloalkyl, and 5-6 membered heteroaryl of R^{f} are each optionally substituted with 1, 2, or 3 substituents selected from halo, OH, CN, - C(O)OH, -NH₂, C₁₋C₄ alkyl, C₁₋C₄ alkoxy, C₁₋C₄ haloalkyl, C₁₋C₄ haloalkoxy, phenyl, C₃₋C₁₀ cycloalkyl, 5-6 membered heteroaryl, and 4-6 membered heterocycloalkyl;
each R^{g} is independently selected from halo, OH, CN, C(O)OH, -C(O)O-C₁₋C₄ alkyl, NH₂, - NH(C₁₋C₆ alkyl), -N(C₁₋C₆ alkyl)₂, C₁₋C₆ alkyl, C₁₋C₆ alkoxy, C₁₋C₆ alkylthio, C₁₋C₆ haloalkyl, C₁₋C₆ haloalkoxy, phenyl, 5-6 membered heteroaryl, 4-6 membered heterocycloalkyl, and C₃₋C₆ cycloalkyl; and
the subscript m is 0.

2. The compound of claim 1, or a pharmaceutically acceptable salt, stereoisomer, or tautomer thereof, wherein G is:
C₃₋₆ cycloalkyl or 4- to 6-membered heterocycloalkyl;
cyclopropyl, cyclobutyl, cyclopentyl, cyclohexyl, cyclobutene-1-yl, cyclopenten-1-yl, cyclohexten-1-yl, 3,6-dihydro-2H-pyran-4-yl, piperidin-1-yl, pyrrolidine-1-yl, or morpholino;
C₁₋₆ alkyl, C₂₋₆ alkenyl, C₁₋₆alkoxy, or halo; or
C₁₋₆ alkyl, vinyl, methoxy, or halo.

3. The compound of claim 1 or claim 2, having formula (Ia-1): 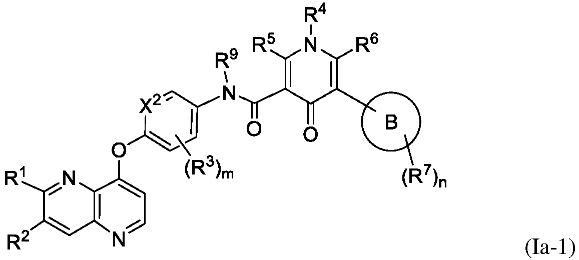 or a pharmaceutically acceptable salt, stereoisomer, or tautomer thereof, wherein ring B is cyclopentene-1-yl, cyclohexen-1-yl, or 3,6-dihydro-2H-pyran-4-yl and the subscript n is 0, 1, 2, 3, or 4.

4. The compound of claim 1 or claim 2, having formula (Ib-1): 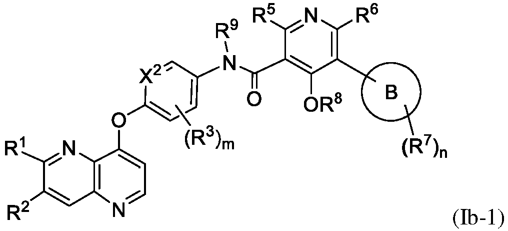 or a pharmaceutically acceptable salt, stereoisomer, or tautomer thereof, wherein ring B is cyclopentene-1-yl, cyclohexen-1-yl, or 3,6-dihydro-2H-pyran-4-yl and the subscript n is 0, 1, 2, 3, or 4.

5. The compound of claim 1 or claim 2, having formula (Ic-1): 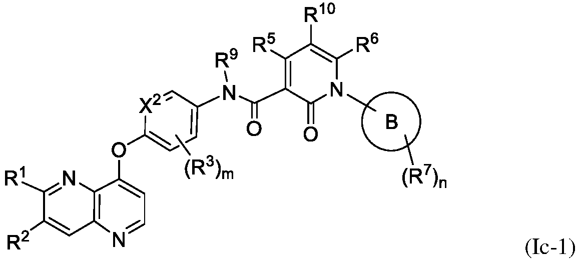 or a pharmaceutically acceptable salt, stereoisomer, or tautomer thereof, wherein ring B is cyclopentene-1-yl, cyclohexen-1-yl, or 3,6-dihydro-2H-pyran-4-yl and the subscript n is 0, 1, 2, 3 or 4.

6. The compound of any one of claims 1-5, or a pharmaceutically acceptable salt, stereoisomer, or tautomer thereof, wherein:
R' is H, C₁₋₆ alkyl, C₁₋₆ alkoxy, halo, NH₂, -NH(C₁₋₆ alkyl), -N(C₁₋₆ alkyl)₂, C₁₋₆ alkylNHC(O)-, or C₁₋₆alkylSO₂NH-;
R¹ is H or C₁₋₆ alkoxy;
R² is H, C₁₋₆ alkyl, C₁₋₆ alkoxy, halo, OH, NH₂, -NH(C₁₋₆ alkyl), -N(C₁₋₆ alkyl)₂, -C₁₋₆ alkylNHC(O)-, CF₃, C₁₋₆ alkylOC(O)-, pyridyl, C₁₋₆alkylSO₂NH-, or 1H-pyrazol-4-yl optionally substituted with R^{g}; and/or
R² is H or C₁₋₆ alkoxy optionally substituted with C₁₋₆ alkoxy.

7. The compound of any one of claims 1-6, or a pharmaceutically acceptable salt, stereoisomer, or tautomer thereof, wherein:
one of R¹ and R² is OR^{a} and R^{a} is C₁₋₆ alkyl substituted with one R^{d}; or
R² is OR^{a}; R^{a} is C₁₋₆ alkyl substituted with one R^{d}; and R^{d} is OR^{e} or C₃₋₁₀ cycloalkyl, and wherein R^{e} is C₁₋₆ alkyl.

8. The compound of any one of claims 1-7, or a pharmaceutically acceptable salt, stereoisomer, or tautomer thereof, wherein
R⁷ is H, halo, C₁₋₆ alkyl or C₁₋₆ alkoxy; and/or
R⁹ is H or methyl.

9. The compound of any one of claims 1-8, or a pharmaceutically acceptable salt, stereoisomer, or tautomer thereof, wherein:
R⁴ is selected from H, C₁₋₆ alkyl, C₁₋₆ alkoxy, OH, C₃₋₆ cycloalkyl, C₁₋₆ haloalkyl, C₃₋₆ cycloalkyl-C₁₋₄ alkylene-, 4-6 membered heterocycloalkyl, (4-6 membered heterocycloalkyl)-C₁₋₄ alkylene-, 5-6 membered heteroaryl, (5-6 membered heteroaryl)-C₁₋₄ alkylene-, and N=C[N(C₁₋₆ alkyl)(C₁₋₆ alkyl)]₂, wherein the C₁₋₆ alkyl, C₁₋₆ alkoxy,C₃₋₆ cycloalkyl, C₃₋₆ cycloalkyl-C₁₋₄ alkylene-, 4-6 membered heterocycloalkyl, (4-6 membered heterocycloalkyl)-C₁₋₄ alkylene-, 5-6 membered heteroaryl, (5-6 membered heteroaryl)-C₁₋₄ alkylene-, and N=C[N(C₁₋₆ alkyl)(C₁₋₆ alkyl)]₂ of R⁴ are each optionally substituted with 1 or 2 independently selected R^{b} or R^{g} substituents;
R⁴ is C₁₋₆ alkyl or C₁₋₆ haloalkyl; or
R⁴ and R⁵ taken together with the atoms to which they are attached form 4- to 7-membered fused heterocycloalkyl.

10. The compound of any one of claims 1-9, or a pharmaceutically acceptable salt, stereoisomer, or tautomer thereof, wherein :
R⁵ and R⁶ are each independently selected from H, CH₃, propen-2-yl, Br, Cl, CN, methoxy, 2-fluoroethyl, isopropyl, CH₃C(O)-, OH, t-butyl, ethyl, hydroxymethyl, isopropylthio, and methoxymethyl;
R¹⁰ is H, CH₃, propen-2-yl, Br, Cl, CN, methoxy, 2-fluoroethyl, isopropyl, CH₃C(O)-, OH, t-butyl, ethyl, hydroxymethyl, isopropylthio, or methoxymethyl; and/or
R⁸ is independently H or C₁₋₆ alkyl.

11. The compound of claim 1, wherein the compound is selected from:
A:
7-(cyclopenten-1-yl)-N-[4-[(6,7-dimethoxy-1,5-naphthyridin-4-yl)oxy]-3-fluorophenyl]-8-oxo-3,4-dihydro-1H-pyrido[2,1-c][1,4]oxazine-9-carboxamide;
5-(cyclopenten-1-yl)-N-[4-[(6,7-dimethoxy-1,5-naphthyridin-4-yl)oxy]-3-fluorophenyl]-4-hydroxy-6-methylpyridine-3-carboxamide;
5-(cyclohexen-1-yl)-N-[4-[(6,7-dimethoxy-1,5-naphthyridin-4-yl)oxy]-3-fluorophenyl]-4-hydroxy-6-methylpyridine-3-carboxamide;
7-(cyclohexen-1-yl)-N-[4-[(6,7-dimethoxy-1,5-naphthyridin-4-yl)oxy]-3-fluorophenyl]-8-oxo-3,4-dihydro-1H-pyrido[2,1-c][1,4]oxazine-9-carboxamide;
7-(cyclohexen-1-yl)-N-[4-[(6,7-dimethoxy-1,5-naphthyridin-4-yl)oxy]phenyl]-8-oxo-3,4-dihydro-1H-pyrido[2,1-c][1,4]oxazine-9-carboxamide;
7-(cyclopenten-1-yl)-N-[4-[(6,7-dimethoxy-1,5-naphthyridin-4-yl)oxy]phenyl]-8-oxo-3,4-dihydro-1H-pyrido[2,1-c][1,4]oxazine-9-carboxamide;
7-(3,6-dihydro-2H-pyran-4-yl)-N-[4-[(6,7-dimethoxy-1,5-naphthyridin-4-yl)oxy]-3-fluorophenyl]-8-oxo-3,4-dihydro-1H-pyrido[2,1-c][1,4]oxazine-9-carboxamide;
7-(3,6-dihydro-2H-pyran-4-yl)-N-[4-[(6,7-dimethoxy-1,5-naphthyridin-4-yl)oxy]phenyl]-8-oxo-3,4-dihydro-1H-pyrido[2,1-c][1,4]oxazine-9-carboxamide;
5-(cyclopenten-1-yl)-N-[4-[(6,7-dimethoxy-1,5-naphthyridin-4-yl)oxy]-3-fluorophenyl]-4-hydroxy-2,6-dimethylpyridine-3-carboxamide;
5-(3,6-dihydro-2H-pyran-4-yl)-N-[4-[(6,7-dimethoxy-1,5-naphthyridin-4-yl)oxy]-3-fluorophenyl]-4-hydroxy-6-methylpyridine-3-carboxamide;
5-(3,6-dihydro-2H-pyran-4-yl)-N-[3-fluoro-4-[(7-methoxy-1,5-naphthyridin-4-yl)oxy]phenyl]-4-hydroxy-6-methylpyridine-3-carboxamide;
5-(3,6-dihydro-2H-pyran-4-yl)-N-[3-fluoro-4-[(6-methoxy-1,5-naphthyridin-4-yl)oxy]phenyl]-4-hydroxy-6-methylpyridine-3-carboxamide;
5-(cyclohexen-1-yl)-N-[3-fluoro-4-[(6-methoxy-1,5-naphthyridin-4-yl)oxy]phenyl]-4-hydroxy-6-methylpyridine-3-carboxamide;
5-(cyclopenten-1-yl)-N-[3-fluoro-4-[(6-methoxy-1,5-naphthyridin-4-yl)oxy]phenyl]-4-hydroxy-6-methylpyridine-3-carboxamide;
5-(cyclohexen-1-yl)-N-[3-fluoro-4-[(7-methoxy-1,5-naphthyridin-4-yl)oxy]phenyl]-4-hydroxy-6-methylpyridine-3-carboxamide;
5-(cyclopenten-1-yl)-N-[3-fluoro-4-[(7-methoxy-1,5-naphthyridin-4-yl)oxy]phenyl]-4-hydroxy-6-methylpyridine-3-carboxamide; and
5-(cyclohexen-1-yl)-N-[4-[(6,7-dimethoxy-1,5-naphthyridin-4-yl)oxy]-3-fluorophenyl]-4-hydroxy-2,6-dimethylpyridine-3-carboxamide;
and pharmaceutically acceptable salts, stereoisomers or tautomers thereof,
or
B:
5-(cyclopenten-1-yl)-N-[4-[(6,7-dimethoxy-1,5-naphthyridin-4-yl)oxy]-3-fluorophenyl]-1,6-dimethyl-4-oxopyridine-3-carboxamide;
5-cyclopentyl-N-[4-[(6,7-dimethoxy-1,5-naphthyridin-4-yl)oxy]-3-fluorophenyl]-1,6-dimethyl-4-oxopyridine-3-carboxamide;
N-[3-fluoro-4-[(7-methoxy-1,5-naphthyridin-4-yl)oxy]phenyl]-4-hydroxy-2,6-dimethyl-5-prop-1-en-2-ylpyridine-3-carboxamide;
N-[4-[(6,7-dimethoxy-1,5-naphthyridin-4-yl)oxy]phenyl]-4-hydroxy-2,6-dimethyl-5-prop-1-en-2-ylpyridine-3-carboxamide;
N-[4-[(6,7-dimethoxy-1,5-naphthyridin-4-yl)oxy]-3-fluorophenyl]-4-hydroxy-2,6-dimethyl-5-prop-1-en-2-ylpyridine-3-carboxamide;
N-[4-[(6,7-dimethoxy-1,5-naphthyridin-4-yl)oxy]-2,5-difluorophenyl]-4-hydroxy-2,6-dimethyl-5-prop-1-en-2-ylpyridine-3-carboxamide;
5-(3,6-dihydro-2H-pyran-4-yl)-4-hydroxy-N-[4-[(7-methoxy-1,5-naphthyridin-4-yl)oxy]phenyl]-6-methylpyridine-3-carboxamide;
5-(cyclohexen-1-yl)-N-[4-[(6,7-dimethoxy-1,5-naphthyridin-4-yl)oxy]-3-fluorophenyl]-1-(2-fluoroethyl)-6-methyl-4-oxopyridine-3-carboxamide;
N-[4-[(6,7-dimethoxy-1,5-naphthyridin-4-yl)oxy]phenyl]-4-hydroxy-2,6-dimethyl-5-propan-2-ylpyridine-3-carboxamide;
N-[4-[(6,7-dimethoxy-1,5-naphthyridin-4-yl)oxy]-2,5-difluorophenyl]-4-hydroxy-2,6-dimethyl-5-propan-2-ylpyridine-3-carboxamide;
5-(cyclopenten-1-yl)-N-[4-[(6,7-dimethoxy-1,5-naphthyridin-4-yl)oxy]-3-fluorophenyl]-1-(2-fluoroethyl)-6-methyl-4-oxopyridine-3-carboxamide;
5-(3,6-dihydro-2H-pyran-4-yl)-N-[3-fluoro-4-[(7-methoxy-1,5-naphthyridin-4-yl)oxy]phenyl]-1,6-dimethyl-4-oxopyridine-3-carboxamide;
5-cyclopentyl-N-[4-[(6,7-dimethoxy-1,5-naphthyridin-4-yl)oxy]-3-fluorophenyl]-1-(2-fluoroethyl)-6-methyl-4-oxopyridine-3-carboxamide;
1-(cyclopenten-1-yl)-N-[4-[(6,7-dimethoxy-1,5-naphthyridin-4-yl)oxy]-3-fluorophenyl]-4,6-dimethyl-2-oxopyridine-3-carboxamide;
1-cyclopentyl-N-[4-[(6,7-dimethoxy-1,5-naphthyridin-4-yl)oxy]-3-fluorophenyl]-4,6-dimethyl-2-oxopyridine-3-carboxamide;
N-[4-[(6,7-dimethoxy-1,5-naphthyridin-4-yl)oxy]-3-fluorophenyl]-4,6-dimethyl-2-oxo-1-prop-1-en-2-ylpyridine-3-carboxamide;
N-[3-fluoro-4-[(7-methoxy-1,5-naphthyridin-4-yl)oxy]phenyl]-4,6-dimethyl-2-oxo-1-prop-1-en-2-ylpyridine-3-carboxamide;
5-bromo-1-cyclopentyl-N-[4-[(6,7-dimethoxy-1,5-naphthyridin-4-yl)oxy]-3-fluorophenyl]-4,6-dimethyl-2-oxopyridine-3-carboxamide;
5-[(E)-2-cyclopentylethenyl]-N-[4-[(6,7-dimethoxy-1,5-naphthyridin-4-yl)oxy]-3-fluorophenyl]-4-hydroxy-6-methylpyridine-3-carboxamide;
5-[(E)-2-cyclopropylethenyl]-N-[4-[(6,7-dimethoxy-1,5-naphthyridin-4-yl)oxy]-3-fluorophenyl]-4-hydroxy-6-methylpyridine-3-carboxamide;
N-[4-[(6,7-dimethoxy-1,5-naphthyridin-4-yl)oxy]-3-fluorophenyl]-5-[(E)-3,3-dimethylbut-1-enyl]-4-hydroxy-6-methylpyridine-3-carboxamide;
N-[4-[(6,7-dimethoxy-1,5-naphthyridin-4-yl)oxy]-3-fluorophenyl]-4-hydroxy-6-methyl-5-[(E)-4-methylpent-1-enyl]pyridine-3-carboxamide;
N-[4-[(6,7-dimethoxy-1,5-naphthyridin-4-yl)oxy]-3-fluorophenyl]-4-hydroxy-2,6-dimethyl-5-propan-2-ylpyridine-3-carboxamide;
N-[3-fluoro-4-[(7-methoxy-1,5-naphthyridin-4-yl)oxy]phenyl]-4-hydroxy-2,6-dimethyl-5-propan-2-ylpyridine-3-carboxamide;
5-(cyclopenten-1-yl)-N-[3-fluoro-4-[[6-methoxy-7-(2-methoxyethoxy)-1,5-naphthyridin-4-yl]oxy]phenyl]-4-hydroxy-6-methylpyridine-3-carboxamide;
5-(cyclohexen-1-yl)-N-[3-fluoro-4-[[6-methoxy-7-(2-methoxyethoxy)-1,5-naphthyridin-4-yl]oxy]phenyl]-4-hydroxy-6-methylpyridine-3-carboxamide;
5-(3,6-dihydro-2H-pyran-4-yl)-N-[3-fluoro-4-[[6-methoxy-7-(2-methoxyethoxy)-1,5-naphthyridin-4-yl]oxy]phenyl]-4-hydroxy-6-methylpyridine-3-carboxamide;
5-[(E)-2-cyclopentylethenyl]-N-[3-fluoro-4-[[6-methoxy-7-(2-methoxyethoxy)-1,5-naphthyridin-4-yl]oxy]phenyl]-4-hydroxy-6-methylpyridine-3-carboxamide;
5-[(E)-2-cyclopropylethenyl]-N-[3-fluoro-4-[[6-methoxy-7-(2-methoxyethoxy)-1,5-naphthyridin-4-yl]oxy]phenyl]-4-hydroxy-6-methylpyridine-3-carboxamide;
N-[4-[(6,7-dimethoxy-1,5-naphthyridin-4-yl)oxy]-3-fluorophenyl]-4-methyl-2-oxo-1-propan-2-ylpyridine-3-carboxamide;
N-[4-[(6,7-dimethoxy-1,5-naphthyridin-4-yl)oxy]phenyl]-4-methyl-2-oxo-1-propan-2-ylpyridine-3-carboxamide;
5-[(E)-3,3-dimethylbut-1-enyl]-N-[3-fluoro-4-[[6-methoxy-7-(2-methoxyethoxy)-1,5-naphthyridin-4-yl]oxy]phenyl]-4-hydroxy-6-methylpyridine-3-carboxamide;
5-(cyclopenten-1-yl)-N-[3-fluoro-4-[[6-methoxy-7-(2-methoxyethoxy)-1,5-naphthyridin-4-yl]oxy]phenyl]-4-hydroxy-2,6-dimethylpyridine-3-carboxamide;
5-(cyclopenten-1-yl)-N-[3-fluoro-4-[[6-methoxy-7-(2-methoxyethoxy)-1,5-naphthyridin-4-yl]oxy]phenyl]-1,2,6-trimethyl-4-oxopyridine-3-carboxamide;
5-(3,6-dihydro-2H-pyran-4-yl)-N-[3-fluoro-4-[[6-methoxy-7-(2-methoxyethoxy)-1,5-naphthyridin-4-yl]oxy]phenyl]-4-hydroxy-2,6-dimethylpyridine-3-carboxamide;
5-(3,6-dihydro-2H-pyran-4-yl)-N-[3-fluoro-4-[[6-methoxy-7-(2-methoxyethoxy)-1,5-naphthyridin-4-yl]oxy]phenyl]-1,2,6-trimethyl-4-oxopyridine-3-carboxamide;
5-[(E)-3,3-dimethylbut-1-enyl]-N-[3-fluoro-4-[[6-methoxy-7-(2-methoxyethoxy)-1,5-naphthyridin-4-yl]oxy]phenyl]-1,2,6-trimethyl-4-oxopyridine-3-carboxamide;
5-cyclopropyl-N-[4-[(6,7-dimethoxy-1,5-naphthyridin-4-yl)oxy]-3-fluorophenyl]-1,2,6-trimethyl-4-oxopyridine-3-carboxamide;
5-cyclopropyl-N-[4-[(6,7-dimethoxy-1,5-naphthyridin-4-yl)oxy]-2,5-difluorophenyl]-1,2,6-trimethyl-4-oxopyridine-3-carboxamide;
5-cyclopropyl-N-[4-[(6,7-dimethoxy-1,5-naphthyridin-4-yl)oxy]phenyl]-1,2,6-trimethyl-4-oxopyridine-3-carboxamide;
N-[4-[(6,7-dimethoxy-1,5-naphthyridin-4-yl)oxy]-3-fluorophenyl]-5-[(E)-3,3-dimethylbut-1-enyl]-1,2,6-trimethyl-4-oxopyridine-3-carboxamide;
5-[(E)-2-cyclopropylethenyl]-N-[4-[(6,7-dimethoxy-1,5-naphthyridin-4-yl)oxy]-3-fluorophenyl]-1,2,6-trimethyl-4-oxopyridine-3-carboxamide;
5-[(E)-2-cyclopropylethenyl]-N-[3-fluoro-4-[[6-methoxy-7-(2-methoxyethoxy)-1,5-naphthyridin-4-yl]oxy]phenyl]-1,2,6-trimethyl-4-oxopyridine-3-carboxamide;
N-[4-[(6,7-dimethoxy-1,5-naphthyridin-4-yl)oxy]-3-fluorophenyl]-1,2,6-trimethyl-4-oxo-5-prop-1-en-2-ylpyridine-3-carboxamide;
5-(3,6-dihydro-2H-pyran-4-yl)-N-[4-[(6,7-dimethoxy-1,5-naphthyridin-4-yl)oxy]-3-fluorophenyl]-1,2,6-trimethyl-4-oxopyridine-3-carboxamide;
5-[(E)-2-cyclopentylethenyl]-N-[3-fluoro-4-[[6-methoxy-7-(2-methoxyethoxy)-1,5-naphthyridin-4-yl]oxy]phenyl]-1,2,6-trimethyl-4-oxopyridine-3-carboxamide;
N-[4-[(6,7-dimethoxy-1,5-naphthyridin-4-yl)oxy]phenyl]-1,2,6-trimethyl-4-oxo-5-prop-1-en-2-ylpyridine-3-carboxamide;
N-[4-[(6,7-dimethoxy-1,5-naphthyridin-4-yl)oxy]-3-fluorophenyl]-1,2,6-trimethyl-4-oxo-5-propan-2-ylpyridine-3-carboxamide;
N-[4-[(6,7-dimethoxy-1,5-naphthyridin-4-yl)oxy]phenyl]-1,2,6-trimethyl-4-oxo-5-propan-2-ylpyridine-3-carboxamide;
5-(cyclohexen-1-yl)-N-[3-fluoro-4-[[6-methoxy-7-(2-methoxyethoxy)-1,5-naphthyridin-4-yl]oxy]phenyl]-1,2,6-trimethyl-4-oxopyridine-3-carboxamide;
4-hydroxy-5-methoxy-N-[4-[(7-methoxy-1,5-naphthyridin-4-yl)oxy]phenyl]-2,6-dimethylpyridine-3-carboxamide;
N-[3-fluoro-4-[(7-methoxy-1,5-naphthyridin-4-yl)oxy]phenyl]-4-hydroxy-5-methoxy-2,6-dimethylpyridine-3-carboxamide;
N-[4-[(6,7-dimethoxy-1,5-naphthyridin-4-yl)oxy]phenyl]-4-hydroxy-5-methoxy-2,6-dimethylpyridine-3-carboxamide;
N-[4-[(6,7-dimethoxy-1,5-naphthyridin-4-yl)oxy]-3-fluorophenyl]-4-hydroxy-5-methoxy-2,6-dimethylpyridine-3-carboxamide;
5-bromo-N-[4-[(6,7-dimethoxy-1,5-naphthyridin-4-yl)oxy]-2,5-difluorophenyl]-6-ethyl-1-methyl-4-oxopyridine-3-carboxamide;
5-bromo-6-ethyl-N-[4-[[6-methoxy-7-(2-methoxyethoxy)-1,5-naphthyridin-4-yl]oxy]phenyl]-1-methyl-4-oxopyridine-3-carboxamide;
5-(cyclopenten-1-yl)-N-[3-fluoro-4-[[7-(2-methoxyethoxy)-1,5-naphthyridin-4-yl]oxy]phenyl]-1,2,6-trimethyl-4-oxopyridine-3-carboxamide;
5-(cyclohexen-1-yl)-N-[3-fluoro-4-[[7-(2-methoxyethoxy)-1,5-naphthyridin-4-yl]oxy]phenyl]-1,2,6-trimethyl-4-oxopyridine-3-carboxamide;
5-(3,6-dihydro-2H-pyran-4-yl)-N-[3-fluoro-4-[[7-(2-methoxyethoxy)-1,5-naphthyridin-4-yl]oxy]phenyl]-1,2,6-trimethyl-4-oxopyridine-3-carboxamide;
N-[4-[(6,7-dimethoxy-1,5-naphthyridin-4-yl)oxy]-2,5-difluorophenyl]-4-hydroxy-5-methoxy-2,6-dimethylpyridine-3-carboxamide;
7-(cyclopenten-1-yl)-N-[4-[(6,7-dimethoxy-1,5-naphthyridin-4-yl)oxy]-3-fluorophenyl]-6-methyl-8-oxo-3,4-dihydro-1H-pyrido[2,1-c][1,4]oxazine-9-carboxamide;
N-[3-fluoro-4-[[6-methoxy-7-(2-methoxyethoxy)-1,5-naphthyridin-4-yl]oxy]phenyl]-4-hydroxy-5-methoxy-2,6-dimethylpyridine-3-carboxamide;
4-hydroxy-5-methoxy-N-[4-[[6-methoxy-7-(2-methoxyethoxy)-1,5-naphthyridin-4-yl]oxy]phenyl]-2,6-dimethylpyridine-3-carboxamide;
7-(3,6-dihydro-2H-pyran-4-yl)-N-[4-[(6,7-dimethoxy-1,5-naphthyridin-4-yl)oxy]phenyl]-6-methyl-8-oxo-3,4-dihydro-1H-pyrido[2,1-c][1,4]oxazine-9-carboxamide;
N-[4-[[7-(2-cyclobutylethoxy)-6-methoxy-1,5-naphthyridin-4-yl]oxy]-3-fluorophenyl]-5-(cyclopenten-1-yl)-1,2,6-trimethyl-4-oxopyridine-3-carboxamide;
N-[3-fluoro-4-[(7-methoxy-1,5-naphthyridin-4-yl)oxy]phenyl]-5-methoxy-1,2,6-trimethyl-4-oxopyridine-3-carboxamide;
N-[4-[(6,7-dimethoxy-1,5-naphthyridin-4-yl)oxy]phenyl]-5-methoxy-1,2,6-trimethyl-4-oxopyridine-3-carboxamide;
N-[4-[(6,7-dimethoxy-1,5-naphthyridin-4-yl)oxy]-3-tfluorophenyl]-5-methoxy-1,2,6-trimethyl-4-oxopyridine-3-carboxamide;
5-cyano-N-[4-[(6,7-dimethoxy-1,5-naphthyridin-4-yl)oxy]-3-fluorophenyl]-1,2,6-trimethyl-4-oxopyridine-3-carboxamide;
7-(3,6-dihydro-2H-pyran-4-yl)-N-[4-[(6,7-dimethoxy-1,5-naphthyridin-4-yl)oxy]-3-fluorophenyl]-6-methyl-8-oxo-3,4-dihydro-1H-pyrido[2,1-c][1,4]oxazine-9-carboxamide;
7-(cyclohexen-1-yl)-N-[4-[(6,7-dimethoxy-1,5-naphthyridin-4-yl)oxy]-3-fluorophenyl]-6-methyl-8-oxo-3,4-dihydro-1H-pyrido[2,1-c][1,4]oxazine-9-carboxamide;
5-cyano-N-[3-fluoro-4-[(7-methoxy-1,5-naphthyridin-4-yl)oxy]phenyl]-1,2,6-trimethyl-4-oxopyridine-3-carboxamide;
5-cyano-N-[4-[(6,7-dimethoxy-1,5-naphthyridin-4-yl)oxy]phenyl]-1,2,6-trimethyl-4-oxopyridine-3-carboxamide;
5-cyano-N-[2,5-difluoro-4-[[6-methoxy-7-(2-methoxyethoxy)-1,5-naphthyridin-4-yl]oxy]phenyl]-1,2,6-trimethyl-4-oxopyridine-3-carboxamide;
3-N-[4-[(6,7-dimethoxy-1,5-naphthyridin-4-yl)oxy]-3-fluorophenyl]-4-hydroxy-2,6-dimethylpyridine-3,5-dicarboxamide;
3-N-[4-[(6,7-dimethoxy-1,5-naphthyridin-4-yl)oxy]phenyl]-4-hydroxy-2,6-dimethylpyridine-3,5-dicarboxamide;
3-N-[2,5-difluoro-4-[[6-methoxy-7-(2-methoxyethoxy)-1,5-naphthyridin-4-yl]oxy]phenyl]-4-hydroxy-2,6-dimethylpyridine-3,5-dicarboxamide;
5-cyano-N-[4-[(6,7-dimethoxy-1,5-naphthyridin-4-yl)oxy]-3-fluorophenyl]-4-hydroxy-2,6-dimethylpyridine-3-carboxamide;
N-[4-[(6,7-dimethoxy-1,5-naphthyridin-4-yl)oxy]-3-fluorophenyl]-1,2,6-trimethyl-5-morpholin-4-yl-4-oxopyridine-3-carboxamide;
N-[4-[(6,7-dimethoxy-1,5-naphthyridin-4-yl)oxy]-3-fluorophenyl]-1,2,6-trimethyl-4-oxo-5-pyrrolidin-1-ylpyridine-3-carboxamide;
N-[4-[(6,7-dimethoxy-1,5-naphthyridin-4-yl)oxy]-3-fluorophenyl]-1,2,6-trimethyl-4-oxo-5-piperidin-1-ylpyridine-3-carboxamide;
3-N-[3-fluoro-4-[(7-methoxy-1,5-naphthyridin-4-yl)oxy]phenyl]-4-hydroxy-2,6-dimethylpyridine-3,5-dicarboxamide;
5-[[4-[(6,7-dimethoxy-1,5-naphthyridin-4-yl)oxy]-3-fluorophenyl]carbamoyl]-1,2,6-trimethyl-4-oxopyridine-3-carboxylic acid;
3-N-[4-[(6,7-dimethoxy-1,5-naphthyridin-4-yl)oxy]-3-fluorophenyl]-4-hydroxy-5-N,5-N,2,6-tetramethylpyridine-3,5-dicarboxamide;
3-N-[4-[(6,7-dimethoxy-1,5-naphthyridin-4-yl)oxy]-3-fluorophenyl]-4-hydroxy-2,6-dimethyl-5-N-propan-2-ylpyridine-3,5-dicarboxamide;
5-N-[4-[(6,7-dimethoxy-1,5-naphthyridin-4-yl)oxy]-3-fluorophenyl]-3-N,3-N,1,2,6-pentamethyl-4-oxopyridine-3,5-dicarboxamide;
3-N-cyclopropyl-5-N-[4-[(6,7-dimethoxy-1,5-naphthyridin-4-yl)oxy]-3-fluorophenyl]-1,2,6-trimethyl-4-oxopyridine-3,5-dicarboxamide;
3-N-[4-[(6,7-dimethoxy-1,5-naphthyridin-4-yl)oxy]-3-fluorophenyl]-4-hydroxy-5-N,2,6-trimethylpyridine-3,5-dicarboxamide;
5-N-cyclopropyl-3-N-[4-[(6,7-dimethoxy-1,5-naphthyridin-4-yl)oxy]-3-fluorophenyl]-4-hydroxy-2,6-dimethylpyridine-3,5-dicarboxamide;
7-(cyclopenten-1-yl)-N-[4-[(6,7-dimethoxy-1,5-naphthyridin-4-yl)oxy]phenyl]-6-methyl-8-oxo-3,4-dihydro-1H-pyrido[2,1-c][1,4]oxazine-9-carboxamide;
7-(cyclohexen-1-yl)-N-[4-[(6,7-dimethoxy-1,5-naphthyridin-4-yl)oxy]phenyl]-6-methyl-8-oxo-3,4-dihydro-1H-pyrido[2,1-c][1,4]oxazine-9-carboxamide; and
5-(cyclopenten-1-yl)-N-[3-fluoro-4-[[7-(2-methoxyethoxy)-1,5-naphthyridin-4-yl]oxy]phenyl]-4-hydroxy-2,6-dimethylpyridine-3-carboxamide;
and pharmaceutically acceptable salts, stereoisomers, or tautomers thereof.

12. A pharmaceutical composition comprising a compound of any one of claims 1-11, or a pharmaceutically acceptable salt, stereoisomer, or tautomer thereof, and a pharmaceutically acceptable carrier or excipient.

13. A compound of any one of claims 1-11, or a pharmaceutically acceptable salt, stereoisomer, or tautomer thereof, or a pharmaceutical composition of claim 12, for use in a method of treating a disease, disorder, or syndrome which is mediated by modulating in vivo activity of a protein kinase,
optionally wherein the protein kinase is AXL, KDR, Mer, or Met.

14. A process for preparing a compound of Formula (Ia), Formula (Ib), or Formula (Ic), or a pharmaceutically acceptable salt, stereoisomer or tautomer thereof, as defined in any one of claims 1 to 11, wherein:
the process comprises contacting a compound of Formula A-a, Formula A-b, or Formula A-c: 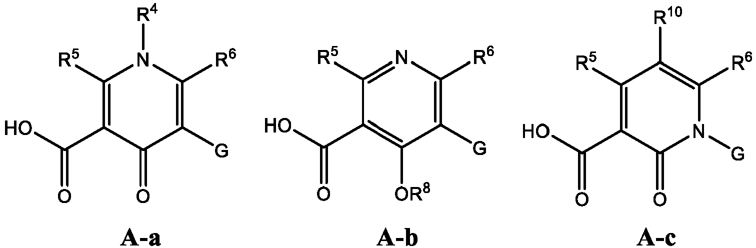
with a compound of Formula B-c: 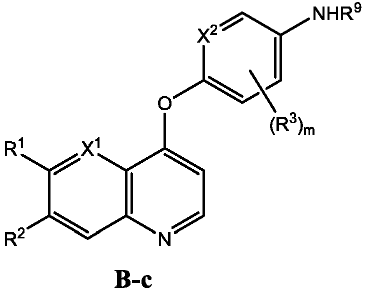
under amide bond forming conditions to provide the compound of Formula (Ia), Formula (Ib), or Formula (Ic), or a pharmaceutically acceptable salt, stereoisomer or tautomer thereof, wherein:
X¹, X², R¹, R², R³, R⁴, R⁵, R⁶, R⁸, R⁹, R¹⁰, G and m are as defined in claim 1.

15. The process of claim 14, wherein the amide bond forming conditions comprise HATU in the presence of a base in an organic solvent.

## Patentansprüche

1. Verbindung der Formel (Ia), Formel (Ib) oder Formel (Ic): 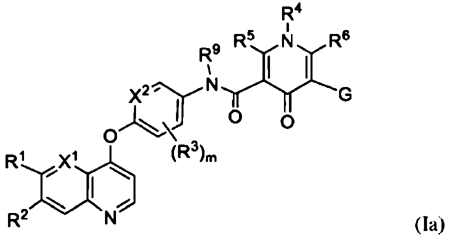 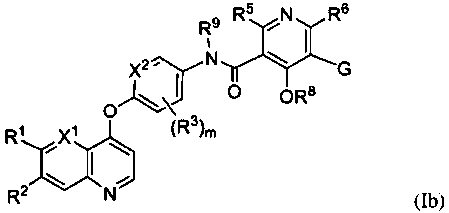 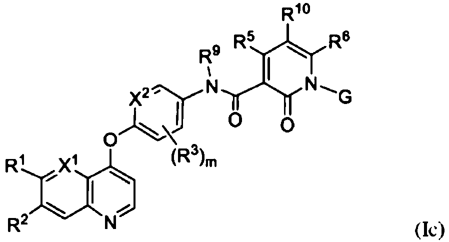 oder ein pharmazeutisch annehmbares Salz, Stereoisomer oder Tautomer davon, wobei:
G C₃₋₁₀-Cycloalkyl, 4- bis 14-gliedriges Heterocycloalkyl, C₁₋₆-Alkyl, C₂₋₆-Alkenyl, C₁₋₆-Alkoxy, Cyano, Halo, C(O)OR^{a} oder C(O)NR^{a}R^{a} ist, wobei das ₃₋₁₀-Cycloalkyl, 4- bis 14-gliedrige Heterocycloalkyl, C₁₋₆-Alkyl, C₂₋₆-Alkenyl und C₁₋₆-Alkoxy von G jeweils optional substituiert sind mit 1, 2, 3 oder 4 unabhängig ausgewählten R⁷-Substituenten;
X¹ N ist;
X² CH oder CF ist;
R¹ und R² jeweils unabhängig ausgewählt sind aus H, Halo, C₁₋₆-Alkyl, C₂₋₆-Alkenyl, C₂₋₆-Alkynyl, C₁₋₆-Haloalkyl, C₁₋₆-Haloalkoxy, C₆₋₁₀-Aryl, C₃₋₁₄-Cycloalkyl, 5-14-gliedrigem Heteroaryl, 4-14-gliedrigem Heterocycloalkyl, C₆₋₁₀-Aryl-C₁₋₄-Alkylen-, C₃₋₁₄-Cycloalkyl-C₁₋₄-Alkylen-, (5-14-gliedrigem Heteroaryl)-C₁₋₄-Alkylen-, (4-14-gliedrigem Heterocycloalkyl)-C₁₋₄-Alkylen-, CN, NO₂, OR^{a}, SR^{a}, NHOR^{a}, C(O)R^{a}, C(O)NR^{a}R^{a}, C(O)OR^{a}, C(O)NR^{a}S(O)₂R^{a}, OC(O)R^{a}, OC(O)NR^{a}R^{a}, NHR^{a}, NR^{a}R^{a}, NR^{a}C(O)R^{a}, NR^{a}C(=NR^{a})R^{a}, NR^{a}C(O)OR^{a}, NR^{a}C(O)NR^{a}R^{a}, C(=NR^{a})R^{a}, C(=NOH)R^{a}, C(=NOH)NR^{a}, C(=NCN)NR^{a}R^{a}, NR^{a}C(=NCN)NR^{a}R^{a}, C(=NR^{a})NR^{a}R^{a}, NR^{a}C(=NR^{a})NR^{a}R^{a}, NR^{a}S(O)R^{a}, NR^{a}S(O)₂R^{a}, NR^{a}S(O)₂NR^{a}R^{a}, S(O)R^{a}, S(O)NR^{a}R^{a}, S(O)₂R^{a}, S(O)₂NR^{a}C(O)R^{a}, P(O)R^{a}R^{a}, P(O)(OR^{a})(OR^{a}), B(OH)₂, B(OR^{a})₂ und S(O)₂NR^{a}R^{a}, wobei das C₁₋₆-Alkyl, C₂₋₆-Alkenyl, C₂₋₆-Alkynyl, C₆₋₁₀-Aryl, C₃₋₁₄-Cycloalkyl, 5-14-gliedrige Heteroaryl, 4-14-gliedrige Heterocycloalkyl, C₆₋₁₀-Aryl-C₁₋₄-Alkylen-, C₃₋₁₄-Cycloalkyl-C₁₋₄-Alkylen-, (5-14-gliedrige Heteroaryl)-C₁₋₄-Alkylen-und (4-14-gliedrige Heterocycloalkyl)-C₁₋₄-Alkylen- von R¹ und R² jeweils optional substituiert sind mit 1, 2, 3, 4 oder 5 unabhängig ausgewählten R^{b}-Substituenten;
jedes R³ unabhängig ausgewählt ist aus Halo, OH, CN, -C(O)OH, -C(O)NH(C₁₋₆-Alkyl), -SO₂(C₁₋₆-Alkyl), -SO₂NH(C₁₋₆-Alkyl), C₁-C₆-Alkyl, C₁-C₆-Alkoxy, C₁-C₆-Haloalkoxy, NH₂, -NH(C₁-C₆-Alkyl), -N(C₁-C₆-Alkyl)₂ und C₃-C₆-Cycloalkyl, wobei C₁-C₆-Alkyl, C₁-C₆-Alkoxy, -NH(C₁-C₆-Alkyl), -N(C₁-C₆-Alkyl)₂ und C₃-C₆-Cycloalkyl von R³ jeweils optional substituiert sind mit 1, 2 oder 3 unabhängig ausgewählten R^{g}-Substituenten;
R⁴ ausgewählt ist aus H, Halo, C₁₋₆-Alkyl, C₂₋₆-Alkenyl, C₂₋₆-Alkynyl, C₁₋₆-Haloalkyl, C₁₋₆-Haloalkoxy, C₆₋₁₀-Aryl, C₃₋₁₄-Cycloalkyl, 5-14-gliedrigem Heteroaryl, 4-14-gliedrigem Heterocycloalkyl, C₆₋₁₀-Aryl-C₁₋₄-Alkylen-, C₃₋₁₄-Cycloalkyl-C₁₋₄-Alkylen-, (5-14-gliedrigem Heteroaryl)-C₁₋₄-Alkylen-, (4-14-gliedrigem Heterocycloalkyl)-C₁₋₄-Alkylen-, CN, NO₂, OR^{a}, SR^{a}, NHOR^{a}, C(O)R^{a}, C(O)NR^{a}R^{a}, C(O)OR^{a}, C(O)NR^{a}S(O)₂R^{a}, OC(O)R^{a}, OC(O)NR^{a}R^{a}, NHR^{a}, NR^{a}R^{a}, NR^{a}C(O)R^{a}, N=C(NR^{a}R^{a})2, NR^{a}C(=NR^{a})R^{a}, NR^{a}C(O)OR^{a}, NR^{a}C(O)NR^{a}R^{a}, C(=NR^{a})R^{a}, C(=NOH)R^{a}, C(=NOH)NR^{a}, C(=NCN)NR^{a}R^{a}, NR^{a}C(=NCN)NR^{a}R^{a}, C(=NR^{a})NR^{a}R^{a}, NR^{a}C(=NR^{a})NR^{a}R^{a}, NR^{a}S(O)R^{a}, NR^{a}S(O)₂R^{a}, NR^{a}S(O)₂NR^{a}R^{a}, S(O)R^{a}, S(O)NR^{a}R^{a}, S(O)₂R^{a}, S(O)₂NR^{a}C(O)R^{a}, P(O)R^{a}R^{a}, P(O)(OR^{a})(OR^{a}), B(OH)₂, B(OR^{a})₂ und S(O)₂NR^{a}R^{a}, wobei das C₁₋₆-Alkyl, C₂₋₆-Alkenyl, C₂₋₆-Alkynyl, C₆₋₁₀-Aryl, C₃₋₁₄-Cycloalkyl, 5-14-gliedrige Heteroaryl, 4-14-gliedrige Heterocycloalkyl, C₆₋₁₀-Aryl-C₁₋₄-Alkylen-, C₃₋₁₄-Cycloalkyl-C₁₋₄-Alkylen-, (5-14-gliedrige Heteroaryl)-C₁₋₄-Alkylen- und (4-14-gliedrige Heterocycloalkyl)-C₁₋₄-Alkylen- von R⁴ jeweils optional substituiert sind mit 1, 2, 3, 4 oder 5 unabhängig ausgewählten R^{b}-Substituenten;
R⁵, R⁶ und R¹⁰ jeweils unabhängig H, Halo, C₁₋₆-Alkyl, C₂₋₆-Alkenyl, C₁₋₆-Alkoxy, C₁₋₆-Alkylthio, CN, C₁₋₄-Haloalkyl, C₁₋₄-Haloalkoxy, OH, C₁₋₄-Alkyl-C(O)-, C₁₋₄-Alkyl-OC(O)-, -C(O)NH(C₁₋₄-Alkyl), NH₂, -NHC₁₋₄-Alkyl oder -N(C₁₋₄-Alkyl)₂ sind, wobei das C₁₋₆-Alkyl, C₂₋₆-Alkenyl, C₁₋₆-Alkoxy, C₁₋₆-Alkylthio, C₁₋₆-Alkyl-C(O)- und C₁₋₄-Alkyl von -NH(C₁₋₄-Alkyl) oder -N(C₁₋₄-Alkyl)₂ von R⁵, R⁶ und R¹⁰ jeweils optional substituiert sind mit 1 oder 2 unabhängig ausgewählten R^{g}-Substituenten;
jedes R⁷ unabhängig ausgewählt ist aus Halo, OH, C(O)OR^{a}, C(O)R^{a}, C(O)NR^{a}R^{a}, CN, NH₂, -NH(C₁-C₆-Alkyl), -N(C₁-C₆-Alkyl)₂, C₁-C₆-Alkyl, C₂-C₆-Alkenyl, C₁-C₆-Alkoxy, C₁-C₆-Haloalkyl, C₁-C₆-Haloalkoxy, C(O)NR^{a}R^{a}, NR^{a}C(O)R^{a}, NR^{a}C(O)NR^{a}R^{a}, SO₂R^{a}, NR^{a}S(O)₂R^{a}, NR^{a}S(O)₂NR^{a}R^{a}, C₃-C₆-Cycloalkyl, 4- bis 6-gliedrigem Heterocycloalkyl, Phenyl, 5- oder 6-gliedrigem Heteroaryl, C₃-C₆-Cycloalkyl-C₁-C₄-Alkylen-, (4- bis 6-gliedrigem Heterocycloalkyl)-C₁-C₄-Alkylen-, Phenyl-C₁-C₂-Alkylen und (5- oder 6-gliedrigem Heteroaryl)-C₁-C₄-Alkylen-, wobei das C₁-C₆-Alkyl, C₁-C₆-Alkoxy, C₃-C₆-Cycloalkyl, 4- bis 6-gliedrige Heterocycloalkyl, Phenyl, 5- oder 6-gliedrige Heteroaryl, C₃-C₆-Cycloalkyl-C₁-C₄-Alkylen-, (4- bis 6-gliedrige Heterocycloalkyl)-C₁-C₄-Alkylen-, Phenyl-C₁-C₂-Alkylen und (5- oder 6-gliedrige Heteroaryl)-C₁-C₄-Alkylen- von R⁷ jeweils optional substituiert sind mit 1, 2 oder 3 unabhängig ausgewählten R^{f}-Substituenten;
R⁸ H, C₁₋₆-Alkyl optional substituiert mit 1 oder 2 R⁸-Substituenten oder eine Hydroxyschutzgruppe ist;
R⁹ H oder C₁₋₆-Alkyl optional substituiert mit 1, 2 oder 3 unabhängig ausgewählten R⁸-Substituenten ist;
oder R⁴ und R⁵ zusammengenommen mit den Atomen, an denen sie befestigt sind, 4-bis 7-gliedriges fusioniertes Heterocycloalkyl oder 5- bis 6-gliedriges fusioniertes Heteroaryl bilden, wobei das 4- bis 7-gliedrige fusionierte Heterocycloalkyl und 5- bis 6-gliedrige fusionierte Heteroaryl jeweils optional substituiert sind mit 1 oder 2 unabhängig ausgewählten R^{g}-Substituenten;
oder R¹⁰ und R⁵ zusammengenommen mit den Atomen, an denen sie befestigt sind, fusioniertes C₃₋₇-Cycloalkyl, 4- bis 6-gliedriges fusioniertes Heterocycloalkyl, fusioniertes 5-oder 6-gliedriges Heteroaryl oder fusioniertes Phenyl bilden, wobei das fusionierte C₃₋₇-Cycloalkyl, 4- bis 6-gliedrige fusionierte Heterocycloalkyl, 5- oder 6-gliedrige Heteroaryl oder fusionierte Phenyl jeweils optional substituiert ist mit 1 oder 2 unabhängig ausgewählten R⁸-Substituenten, und wobei ein oder zwei Ringkohlenstoffatome des fusionierten C₃₋₇-Cycloalkyls oder fusionierten Heterocycloalkyls optional durch eine Carbonylgruppe ersetzt sind;
oder R¹² und R¹⁰ zusammengenommen mit den Atomen, an denen sie befestigt sind, 4-bis 7-gliedriges fusioniertes Heterocycloalkyl oder 5- bis 6-gliedriges fusioniertes Heteroaryl bilden, wobei das 4- bis 7-gliedrige fusionierte Heterocycloalkyl und 5- bis 6-gliedrige fusionierte Heteroaryl jeweils optional substituiert sind mit 1 oder 2 unabhängig ausgewählten R^{g}-Substituenten;
oder R⁶ und R⁴ zusammengenommen mit den Atomen, an denen sie befestigt sind, 4-bis 7-gliedriges fusioniertes Heterocycloalkyl oder 5- bis 6-gliedriges fusioniertes Heteroaryl bilden, wobei das 4- bis 7-gliedrige fusionierte Heterocycloalkyl und 5- bis 6-gliedrige fusionierte Heteroaryl jeweils optional substituiert sind mit 1 oder 2 unabhängig ausgewählten R^{g}-Substituenten;
oder R⁶ und R¹⁰ zusammengenommen mit den Atomen, an denen sie befestigt sind, fusioniertes C₃₋₇-Cycloalkyl, 4- bis 6-gliedriges fusioniertes Heterocycloalkyl, 5- oder 6-gliedriges fusioniertes Heteroaryl oder fusioniertes Heteroaryl bilden, wobei das fusionierte C₃₋₇-Cycloalkyl, 4- bis 6-gliedrige fusionierte Heterocycloalkyl, 5- oder 6-gliedrige fusionierte Heteroaryl und fusionierte Heteroaryl jeweils optional substituiert ist mit 1 oder 2 unabhängig ausgewählten R⁸-Substituenten, und wobei ein oder zwei Ringkohlenstoffatome des fusionierten C₃₋₇-Cycloalkyls oder 4- bis 6-gliedrigen fusionierten Heterocycloalkyls optional durch ein Carbonyl ersetzt sind;
jedes R^{a} unabhängig ausgewählt ist aus H, CN, C₁-C₆-Alkyl, C₁-C₆-Haloalkyl, C₂-C₆-Alkenyl, C₂-C₆-Alkynyl, C₆-C₁₀-Aryl, C₃-C₁₀-Cycloalkyl, 5-14-gliedrigem Heteroaryl, 4-14-gliedrigem Heterocycloalkyl, C₆-C₁₀-Aryl-C₁-Cₐ-Alkylen-, C₃-C₁₀-Cycloalkyl-C₁-C₄-Alkylen-, (5-14-gliedrigem Heteroaryl)-C₁-C₄-Alkylen- und (4-14-gliedrigem Heterocycloalkyl)-C₁-C₄-Alkylen-, wobei das C₁-C₆-Alkyl, C₁-C₆-Haloalkyl, C₂-C₆-Alkenyl, C₂-C₆-Alkynyl, C₆-C₁₀-Aryl, C₃-C₁₀-Cycloalkyl, 5-14-gliedrige Heteroaryl, 4-14-gliedrige Heterocycloalkyl, C₆-C₁₀-Aryl-C₁-C₄-Alkylen-, C₃-C₁₀-Cycloalkyl-C₁-C₄-Alkylen-, (5-14-gliedrige Heteroaryl)-C₁-C₄-Alkylen- und (4-14-gliedrige Heterocycloalkyl)-C₁-C₄-Alkylen- von R^{a} jeweils optional substituiert sind mit 1, 2, 3, 4 oder 5 unabhängig ausgewählten R^{d}-Substituenten;
oder jegliche zwei R^{a}-Substituenten zusammen mit dem Stickstoffatom, an dem sie befestigt sind, 4-, 5-, 6-, 7-, 8-, 9- oder 10-gliedriges Heterocycloalkyl bilden, wovon jedes mit 1, 2 oder 3 unabhängig ausgewählten R^{f}-Substituenten ersetzt ist;
jedes R^{b} unabhängig ausgewählt ist aus Halo, C₁-C₆-Alkyl, C₂-C₆-Alkenyl, C₂-C₆-Alkynyl, C₁-C₆-Haloalkyl, C₁-C₆-Haloalkoxy, C₆-C₁₀-Aryl, C₃-C₁₀-Cycloalkyl, 5-10-gliedrigem Heteroaryl, 4-10-gliedrigem Heterocycloalkyl, C₆-C₁₀-Aryl-C₁-C₄-Alkylen-, C₃-C₁₀-Cycloalkyl-C₁-C₄-Alkylen-, (5-10-gliedrigem Heteroaryl)-C₁-C₄-Alkylen-, (4-10-gliedrigem Heterocycloalkyl)-C₁-C₄-Alkylen-, CN, OH, NH₂, NO₂, NHOR^{c}, OR^{c}, SR^{c}, C(O)R^{c}, C(O)NR^{c}R^{c}, C(O)OR^{c}, C(O)NR^{c}S(O)₂R^{c}, OC(O)R^{c}, OC(O)NR^{c}R^{c}, C(=NOH)R^{c}, C(=NOH)NR^{c}, C(=NCN)NR^{c}R^{c}, NR^{c}C(=NCN)NR^{c}R^{c}, C(=NR^{c})NR^{c}R^{c}, NR^{c}C(=NR^{c})NR^{c}R^{c}, NHR^{c}, NR^{c}R^{c}, NR^{c}C(O)R^{c}, NR^{c}C(=NR^{c})R^{c}, NR^{c}C(O)OR^{c}, NR^{c}C(O)NR^{c}R^{c}, NR^{c}S(O)R^{c}, NR^{c}S(O)₂R^{c}, NR^{c}S(O)₂NR^{c}R^{c}, S(O)R^{c}, S(O)NR^{c}R^{c}, S(O)₂R^{c}, S(O)₂NR^{c}C(O)R^{c}, Si(R^{c})₃, P(O)R^{c}R^{c}, P(O)(OR^{c})(OR^{c}), B(OH)₂, B(OR^{c})₂ und S(O)₂NR^{c}R^{c}, wobei das C₁-C₆-Alkyl, C₂-C₆-Alkenyl, C₂-C₆-Alkynyl, C₆-C₁₀-Aryl, C₃-C₁₀-Cycloalkyl, 5-10-gliedrige Heteroaryl, 4-10-gliedrige Heterocycloalkyl, C₆-C₁₀-Aryl-C₁-C₄-Alkylen-, C₃-C₁₀-Cycloalkyl-C₁-C₄-Alkylen-, (5-10-gliedrige Heteroaryl)-C₁-C₄-Alkylen- und (4-10-gliedrige Heterocycloalkyl)-C₁-C₄-Alkylen- von R^{b} jeweils ferner optional substituiert sind mit 1, 2 oder 3 unabhängig ausgewählten R^{d}-Substituenten;
jedes R^{c} unabhängig ausgewählt ist aus H, C₁-C₆-Alkyl, C₁-C₆-Haloalkyl, C₂-C₆-Alkenyl, C₂-C₆-Alkynyl, C₆-C₁₀-Aryl, C₃-C₁₀-Cycloalkyl, 5-10-gliedrigem Heteroaryl, 4-10-gliedrigem Heterocycloalkyl, C₆-C₁₀-Aryl-C₁-Cₐ-Alkylen-, C₃-C₁₀-Cycloalkyl-C₁-C₄-Alkylen-, (5-10-gliedrigem Heteroaryl)-C₁-C₄-Alkylen- und (4-10-gliedrigem Heterocycloalkyl-C₁-C₄-Alkylen-, wobei das C₁-C₆-Alkyl, C₂-C₆-Alkenyl, C₂-C₆-Alkynyl, C₆-C₁₀-Aryl, C₃-C₁₀-Cycloalkyl, 5-10-gliedrige Heteroaryl, 4-10-gliedrige Heterocycloalkyl, C₆-C₁₀-Aryl-C₁-C₄-Alkylen-, C₃-C₁₀-Cycloalkyl-C₁-C₄-Alkylen-, (5-10-gliedrige Heteroaryl)-C₁-C₄-Alkylen und (4-10-gliedrige Heterocycloalkyl-C₁-C₄-Alkylen- von R^{c} jeweils optional substituiert sind mit 1, 2, 3, 4 oder 5 unabhängig ausgewählten R^{f}-Substituenten;
oder jegliche zwei R^{c}-Substituenten zusammen mit dem Stickstoffatom, an dem sie befestigt sind, 4-, 5-, 6-, 7-, 8-, 9- oder 10-gliedriges Heterocycloalkyl bilden, wovon jedes optional mit 1, 2 oder 3 unabhängig ausgewählten R^{f}-Substituenten ersetzt ist;
jedes R^{d} unabhängig ausgewählt ist aus C₁-C₆-Alkyl, C₁-C₆-Haloalkyl, Halo, C₆-C₁₀-Aryl, 5-10-gliedrigem Heteroaryl, C₃-C₁₀-Cycloalkyl, 4-10-gliedrigem Heterocycloalkyl, C₆-C₁₀-Aryl-C₁-C₄-Alkylen-, C₃-C₁₀-Cycloalkyl-C₁-C₄-Alkylen-, (5-10-gliedrigem Heteroaryl)-C₁-C₄-Alkylen-, (4-10-gliedrigem Heterocycloalkyl)-C₁-C₄-Alkylen-, CN, NH₂, NHOR^{e}, OR^{e}, SR^{e}, C(O)R^{e}, C(O)NR^{e}R^{e}, C(O)OR^{e}, OC(O)R^{e}, OC(O)NR^{e}R^{e}, NHR^{e}, NR^{e}R^{e}, NR^{e}C(O)R^{e}, NR^{e}C(O)NR^{e}R^{e}, NR^{e}C(O)OR^{e}, C(=NR^{e})NR^{e}R^{e}, NR^{e}C(=NR^{e})NR^{e}R^{e}, NR^{e}C(=NOH)NR^{e}R^{e}, NR^{e}C(=NCN)NR^{e}R^{e}, S(O)R^{e}, S(O)NR^{e}R^{e}, S(O)₂R^{e}, NR^{e}S(O)₂R^{e}, NR^{e}S(O)₂NR^{e}R^{e} und S(O)₂NR^{e}R^{e}, wobei das C₁-C₆-Alkyl, C₆-C₁₀-Aryl, 5-10-gliedrige Heteroaryl, C₃-C₁₀-Cycloalkyl, 4-10-gliedrige Heterocycloalkyl, C₆-C₁₀-Aryl-C₁-Cₐ-Alkylen-, C₃-C₁₀-Cycloalkyl-C₁-C₄-Alkylen-, (5-10-gliedrige Heteroaryl)-C₁-C₄-Alkylen- und (4-10-gliedrige Heterocycloalkyl)-C₁-C₄-Alkylen- von R^{d} jeweils optional substituiert sind mit 1, 2 oder 3 unabhängig ausgewählten R^{f}-Substituenten;
jedes R^{e} unabhängig ausgewählt ist aus H, C₁-C₆-Alkyl, C₁-C₆-Alkoxy, C₃-C₆-Cycloalkyl, C₃-C₆-Cycloalkyl-C₁-C₄-Alkylen-, C₆-C₁₀-Aryl, C₆-C₁₀-Aryl-C₁-C₄-Alkylen-, 5-oder 6-gliedrigem Heteroaryl, (5- oder 6-gliedrigem Heteroaryl)-C₁-C₄-Alkylen-, 4-7-gliedrigem Heterocycloalkyl, (4-7-gliedrigem Heterocycloalkyl)-C₁-C₄-Alkylen-, C₁-C₆-Haloalkyl, C₁-C₆-Haloalkoxy, C₂-C₄-Alkenyl und C₂-C₄-Alkynyl, wobei das C₁-C₆-Alkyl, C₁-C₆-Alkoxy, C₃-C₆-Cycloalkyl, C₆-C₁₀-Aryl, 5- oder 6-gliedrige Heteroaryl, 4-7-gliedrige Heterocycloalkyl, C₆-C₁₀-Aryl-C₁-C₄-Alkylen-, (5- oder 6-gliedrige Heteroaryl)-C₁-C₄-Alkylen-, (4-7-gliedrigem Heterocycloalkyl)-C₁-C₄-Alkylen-, C₂-C₄-Alkenyl und C₂-C₄-Alkynyl von R^{e} jeweils optional substituiert sind mit 1, 2 oder 3 R^{f}-Substituenten;
oder jegliche zwei R^{e}-Substituenten zusammen mit dem Stickstoffatom, an dem sie befestigt sind, 4-, 5-, 6-, 7-, 8-, 9- oder 10-gliedriges Heterocycloalkyl bilden, wovon jedes optional mit 1, 2 oder 3 unabhängig ausgewählten R^{f}-Substituenten ersetzt ist;
jedes R^{f} unabhängig ausgewählt ist aus Halo, OH, CN, C(O)OH, NH₂, -NH(C₁-C₆-Alkyl), -N(C₁-C₆-Alkyl)₂, C₁-C₆-Alkyl, Vinyl, C₁-C₆-Alkoxy, C₁-C₆-Alkylthio, C₁-C₆-Haloalkyl, C₁-C₆-Haloalkoxy, Phenyl, 5-6-gliedrigem Heteroaryl, 4-6-gliedrigem Heterocycloalkyl und C₃-C₆-Cycloalkyl, wobei das C₁-C₆-Alkyl, C₁-C₆-Alkoxy, C₁-C₆-Alkylthio, Phenyl, C₃-C₆-Cycloalkyl, 4-6-gliedrige Heterocycloalkyl und 5-6-gliedrige Heteroaryl von R^{f} jeweils optional substituiert sind mit 1, 2 oder 3 Substituierten, ausgewählt aus Halo, OH, CN, -C(O)OH, NH₂, C₁-C₄-Alkyl, C₁-C₄-Alkoxy, C₁-C₄-Haloalkyl, C₁-C₄-Haloalkoxy, Phenyl, C₃-C₁₀-Cycloalkyl, 5-6-gliedrigem Heteroaryl und 4-6-gliedrigem Heterocycloalkyl;
jedes R^{g} unabhängig ausgewählt ist aus Halo, OH, CN, C(O)OH, -C(O)O-C₁-C₄-Alkyl, NH₂, -NH(C₁-C₆-Alkyl), -N(C₁-C₆-Alkyl)₂, C₁-C₆-Alkyl, C₁-C₆-Alkoxy, C₁-C₆-Alkylthio, C₁-C₆-Haloalkyl, C₁-C₆-Haloalkoxy, Phenyl, 5-6-gliedrigem Heteroaryl, 4-6-gliedrigem Heterocycloalkyl und C₃-C₆-Cycloalkyl; und
das Subskript m 0 ist.

2. Verbindung nach Anspruch 1 oder ein pharmazeutisch annehmbares Salz, Stereoisomer oder Tautomer davon, wobei G Folgendes ist:
C₃₋₆-Cycloalkyl oder 4- bis 6-gliedriges Heterocycloalkyl;
Cyclopropyl, Cyclobutyl, Cyclopentyl, Cyclohexyl, Cyclobuten-1-yl, Cyclopenten-1-yl, Cyclohexten-1-yl, 3,6-Dihydro-2H-pyran-4-yl, Piperidin-1-yl, Pyrrolidin-1-yl oder Morpholino;
C₁₋₆-Alkyl, C₂₋₆-Alkenyl, C₁₋₆-Alkoxy oder Halo; oder
C₁₋₆-Alkyl, Vinyl, Methoxy oder Halo.

3. Verbindung nach Anspruch 1 oder Anspruch 2, mit Formel (Ia-1): 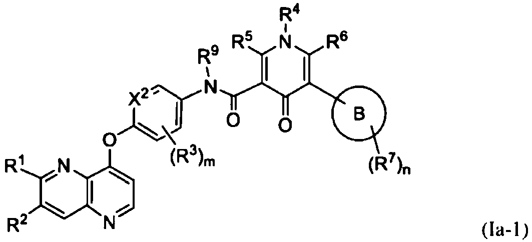 oder ein pharmazeutisch annehmbares Salz, Stereoisomer oder Tautomer davon, wobei Ring B Cyclopenten-1-yl, Cyclohexen-1-yl oder 3,6-Dihydro-2H-pyran-4-yl ist und das Subskript n 0, 1, 2, 3 oder 4 ist.

4. Verbindung nach Anspruch 1 oder Anspruch 2, mit Formel (Ib-1): 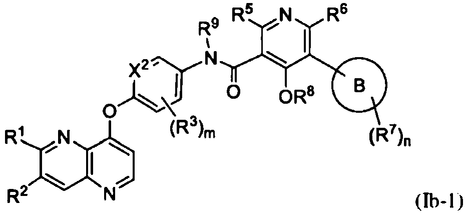 oder ein pharmazeutisch annehmbares Salz, Stereoisomer oder Tautomer davon, wobei Ring B Cyclopenten-1-yl, Cyclohexen-1-yl oder 3,6-Dihydro-2H-pyran-4-yl ist und das Subskript n 0, 1, 2, 3 oder 4 ist.

5. Verbindung nach Anspruch 1 oder Anspruch 2, mit Formel (Ic-1): 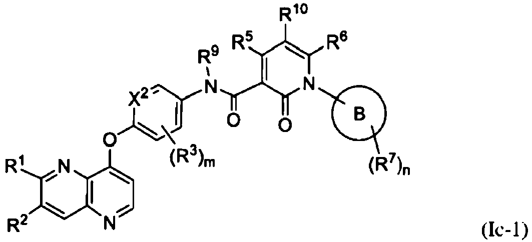 oder ein pharmazeutisch annehmbares Salz, Stereoisomer oder Tautomer davon, wobei Ring B Cyclopenten-1-yl, Cyclohexen-1-yl oder 3,6-Dihydro-2H-pyran-4-yl ist und das Subskript n 0, 1, 2, 3 oder 4 ist.

6. Verbindung nach einem der Ansprüche 1-5 oder ein pharmazeutisch annehmbares Salz, Stereoisomer oder Tautomer davon, wobei:
R¹ H, C₁₋₆-Alkyl, C₁₋₆-Alkoxy, Halo, NH₂, -NH(C₁₋₆-Alkyl), -N(C₁₋₆-Alkyl)₂, C₁₋₆-AlkylNHC(O)- oder C₁₋₆-AlkylSO₂NH- ist;
R¹ H oder C₁₋₆-Alkoxy ist;
R² H, C₁₋₆-Alkyl, C₁₋₆-Alkoxy, Halo, OH, NH₂, -NH(C₁₋₆-Alkyl), -N(C₁₋₆-Alkyl)₂, -C₁₋₆-AlkylNHC(O)-, CF₃, C₁₋₆-AlkylOC(O)-, Pyridyl, C₁₋₆-AlkylSO₂NH- oder 1H-Pyrazol-4-yl ist, optional substituiert mit R⁸; und/oder
R² H oder C₁₋₆-Alkoxy ist, optional substituiert mit C₁₋₆-Alkoxy.

7. Verbindung nach einem der Ansprüche 1-6 oder ein pharmazeutisch annehmbares Salz, Stereoisomer oder Tautomer davon, wobei:
eines aus R¹ und R² Or^{a} ist und R^{a} C₁₋₆-Alkyl ist, substituiert mit einem R^{d}; oder
R² Or^{a} ist; R^{a} C₁₋₆-Alkyl ist, substituiert mit einem R^{d}; und R^{d} OR^{e} oder C₃₋₁₀-Cycloalkyl ist, und wobei R^{e} C₁₋₆-Alkyl ist.

8. Verbindung nach einem der Ansprüche 1-7 oder ein pharmazeutisch annehmbares Salz, Stereoisomer oder Tautomer davon, wobei
R⁷ H, Halo, C₁₋₆-Alkyl oder C₁₋₆-Alkoxy ist; und/oder
R⁹ H oder Methyl ist.

9. Verbindung nach einem der Ansprüche 1-8 oder ein pharmazeutisch annehmbares Salz, Stereoisomer oder Tautomer davon, wobei:
R⁴ ausgewählt ist aus H, C₁₋₆-Alkyl, C₁₋₆-Alkoxy, OH, C₃₋₆-Cycloalkyl, C₁₋₆-Haloalkyl, C₃₋₆-Cycloalkyl-C₁₋₄-Alkylen-, 4-6-gliedrigem Heterocycloalkyl, (4-6-gliedrigem Heterocycloalkyl)-C₁₋₄-Alkylen-, 5-6-gliedrigem Heteroaryl, (5-6-gliedrigem Heteroaryl)-C₁₋₄-Alkylen- und N=C[N(C₁₋₆-Alkyl)(C₁₋₆-Alkyl)]₂, wobei das C₁₋₆-Alkyl, C₁₋₆-Alkoxy, C₃₋₆-Cycloalkyl, C₃₋₆-Cycloalkyl-C₁₋₄-Alkylen-, 4-6-gliedrige Heterocycloalkyl, (4-6-gliedrige Heterocycloalkyl)-C₁₋₄-Alkylen-, 5-6-gliedrige Heteroaryl, (5-6-gliedrige Heteroaryl)-C₁₋₄-Alkylen- und N=C[N(C₁₋₆-Alkyl)(C₁₋₆-Alkyl)]₂ von R⁴ jeweils optional substituiert sind mit 1 oder 2 unabhängig ausgewählten R^{b}- oder R^{g}-Substituenten;
R⁴ C₁₋₆-Alkyl oder C₁₋₆-Haloalkyl ist; oder
R⁴ und R⁵ zusammengenommen mit den Atomen, an denen sie befestigt sind, 4- bis 7-gliedriges fusioniertes Heterocycloalkyl bilden.

10. Verbindung nach einem der Ansprüche 1-9 oder ein pharmazeutisch annehmbares Salz, Stereoisomer oder Tautomer davon, wobei:
R⁵ und R⁶ jeweils unabhängig ausgewählt sind aus H, CH₃, Propen-2-yl, Br, Cl, CN, Methoxy, 2-Fluorethyl, Isopropyl, CH₃C(O)-, OH, t-Butyl, Ethyl, Hydroxymethyl, Isopropylthio und Methoxymethyl;
R¹⁰ H, CH₃, Propen-2-yl, Br, Cl, CN, Methoxy, 2-Fluorethyl, Isopropyl, CH₃C(O)-, OH, t-Butyl, Ethyl, Hydroxymethyl, Isopropylthio oder Methoxymethyl ist; und/oder
R⁸ unabhängig H oder C₁₋₆-Alkyl ist.

11. Verbindung nach Anspruch 1, wobei die Verbindung ausgewählt ist aus:
**A:**
7-(Cyclopenten-1-yl)-N-[4-[(6,7-dimethoxy-1,5-naphthyridin-4-yl)oxy]-3-fluorphenyl]-8-oxo-3,4-dihydro-1H-pyrido[2,1-c][1,4]oxazin-9-carboxamid;
5-(Cyclopenten-1-yl)-N-[4-[(6,7-dimethoxy-1,5-naphthyridin-4-yl)oxy]-3-fluorphenyl]-4-hydroxy-6-methylpyridin-3-carboxamide;
5-(Cyclohexen-1-yl)-N-[4-[(6,7-dimethoxy-1,5-naphthyridin-4-yl)oxy]-3-fluorphenyl]-4-hydroxy-6-methylpyridin-3-carboxamid;
7-(Cyclohexen-1-yl)-N-[4-[(6,7-dimethoxy-1,5-naphthyridin-4-yl)oxy]-3-fluorphenyl]-8-oxo-3,4-dihydro-1H-pyrido[2,1-c][1,4]oxazin-9-carboxamide;
7-(Cyclohexen-1-yl)-N-[4-[(6,7-dimethoxy-1,5-naphthyridin-4-yl)oxy]phenyl]-8-oxo-3,4-dihydro-1H-pyrido[2,1-c][1,4]oxazin-9-carboxamid;
7-(Cyclopenten-1-yl)-N-[4-[(6,7-dimethoxy-1,5-naphthyridin-4-yl)oxy]phenyl]-8-oxo-3,4-dihydro-1H-pyrido[2,1-c] [1,4]oxazin-9-carboxamid;
7-(3,6-Dihydro-2H-pyran-4-yl)-N-[4-[(6,7-dimethoxy-1,5-naphthyridin-4-yl)oxy]-3-fluorphenyl]-8-oxo-3,4-dihydro-1H-pyrido[2,1-c][1,4]oxazin-9-carboxamid;
7-(3,6-Dihydro-2H-pyran-4-yl)-N-[4-[(6,7-dimethoxy-1,5-naphthyridin-4-yl)oxy]phenyl]-8-oxo-3,4-dihydro-1H-pyrido[2,1-c][1,4]oxazin-9-carboxamid;
5-(Cyclopenten-1-yl)-N-[4-[(6,7-dimethoxy-1,5-naphthyridin-4-yl)oxy]-3-fluorphenyl]-4-hydroxy-2,6-dimethylpyridin-3-carboxamid;
5-(3,6-Dihydro-2H-pyran-4-yl)-N-[4-[(6,7-dimethoxy-1,5-naphthyridin-4-yl)oxy]-3-fluorphenyl]-4-hydroxy-6-methylpyridin-3-carboxamid;
5-(3,6-Dihydro-2H-pyran-4-yl)-N-[3-fluor-4-[(7-methoxy-1,5-naphthyridin-4-yl)oxy]phenyl]-4-hydroxy-6-methylpyridin-3-carboxamid;
5-(3,6-Dihydro-2H-pyran-4-yl)-N-[3-fluor-4-[(6-methoxy-1,5-naphthyridin-4-yl)oxy]phenyl]-4-hydroxy-6-methylpyridin-3-carboxamid;
5-(Cyclohexen-1-yl)-N-[3-fluor-4-[(6-methoxy-1,5-naphthyridin-4-yl)oxy]phenyl]-4-hydroxy-6-methylpyridin-3-carboxamid;
5-(Cyclopenten-1-yl)-N-[3-fluor-4-[(6-methoxy-1,5-naphthyridin-4-yl)oxy]phenyl]-4-hydroxy-6-methylpyridin-3-carboxamid;
5-(Cyclohexen-1-yl)-N-[3-fluor-4-[(7-methoxy-1,5-naphthyridin-4-yl)oxy]phenyl]-4-hydroxy-6-methylpyridin-3-carboxamid;
5-(Cyclopenten-1-yl)-N-[3-fluor-4-[(7-methoxy-1,5-naphthyridin-4-yl)oxy]phenyl]-4-hydroxy-6-methylpyridin-3-carboxamid; und
5-(Cyclohexen-1-yl)-N-[4-[(6,7-dimethoxy-1,5-naphthyridin-4-yl)oxy]-3-fluorphenyl]-4-hydroxy-2,6-dimethylpyridin-3-carboxamid;
und pharmazeutisch annehmbare Salze, Stereoisomere oder Tautomere davon,
oder
**B:**
5-(Cyclopenten-1-yl)-N-[4-[(6,7-dimethoxy-1,5-naphthyridin-4-yl)oxy]-3-fluorphenyl]-1,6-dimethyl-4-oxopyridin-3-carboxamid;
5-Cyclopentyl-N-[4-[(6,7-dimethoxy-1,5-naphthyridin-4-yl)oxy]-3-fluorphenyl]-1,6-dimethyl-4-oxopyridin-3-carboxamid;
N-[3-Fluor-4-[(7-methoxy-1,5-naphthyridin-4-yl)oxy]phenyl]-4-hydroxy-2,6-dimethyl-5-prop-1-en-2-ylpyridin-3-carboxamid;
N-[4-[(6,7-Dimethoxy-1,5-naphthyridin-4-yl)oxy]phenyl]-4-hydroxy-2,6-dimethyl-5-prop-1-en-2-yl-pyridin-3-carboxamid;
N-[4-[(6,7-Dimethoxy-1,5-naphthyridin-4-yl)oxy]-3-fluorphenyl]-4-hydroxy-2,6-dimethyl-5-prop-1-en-2-ylpyridin-3-carboxamid;
N-[4-[(6,7-Dimethoxy-1,5-naphthyridin-4-yl)oxy]-2,5-difluorphenyl]-4-hydroxy-2,6-dimethyl-5-prop-1-en-2-ylpyridin-3-carboxamid;
5-(3,6-Dihydro-2H-pyran-4-yl)-4-hydroxy-N-[4-[(7-methoxy-1,5-naphthyridin-4-yl)oxy]phenyl]-6-methylpyridin-3-carboxamid;
5-(Cyclohexen-1-yl)-N-[4-[(6,7-dimethoxy-1,5-naphthyridin-4-yl)oxy]-3-fluorphenyl]-1-(2-fluorethyl)-6-methyl-4-oxopyridin-3-carboxamid;
N-[4-[(6,7-Dimethoxy-1,5-naphthyridin-4-yl)oxy]phenyl]-4-hydroxy-2,6-dimethyl-5-propan-2-ylpyridin-3-carboxamid;
N-[4-[(6,7-Dimethoxy-1,5-naphthyridin-4-yl)oxy]-2,5-difluorphenyl]-4-hydroxy-2,6-dimethyl-5-propan-2-ylpyridin-3-carboxamid;
5-(Cyclopenten-1-yl)-N-[4-[(6,7-dimethoxy-1,5-naphthyridin-4-yl)oxy]-3-fluorphenyl]-1-(2-fluorethyl)-6-methyl-4-oxopyridin-3-carboxamid;
5-(3,6-Dihydro-2H-pyran-4-yl)-N-[3-fluor-4-[(7-methoxy-1,5-naphthyridin-4-yl)oxy]phenyl]-1,6-dimethyl-4-oxopyridin-3-carboxamid;
5-Cyclopentyl-N-[4-[(6,7-dimethoxy-1,5-naphthyridin-4-yl)oxy]-3-fluorphenyl]-1-(2-fluorethyl)-6-methyl-4-oxopyridin-3-carboxamid;
1-(Cyclopenten-1-yl)-N-[4-[(6,7-dimethoxy-1,5-naphthyridin-4-yl)oxy]-3-fluorphenyl]-4,6-dimethyl-2-oxopyridin-3-carboxamid;
1-Cyclopentyl-N-[4-[(6,7-dimethoxy-1,5-naphthyridin-4-yl)oxy]-3-fluorphenyl]-4,6-dimethyl-2-oxopyridin-3-carboxamid;
N-[4-[(6,7-Dimethoxy-1,5-naphthyridin-4-yl)oxy]-3-fluorphenyl]-4,6-dimethyl-2-oxo-1-prop-1-en-2-ylpyridin-3-carboxamid;
N-[3-Fluor-4-[(7-methoxy-1,5-naphthyridin-4-yl)oxy]phenyl]-4,6-dimethyl-2-oxo-1-prop-1-en-2-ylpyridin-3-carboxamid;
5-Brom-1-cyclopentyl-N-[4-[(6,7-dimethoxy-1,5-naphthyridin-4-yl)oxy]-3-fluorphenyl]-4,6-dimethyl-2-oxopyridin-3-carboxamid;
5-[(E)-2-Cyclopentylethenyl]-N-[4-[(6,7-dimethoxy-1,5-naphthyridin-4-yl)oxy]-3-fluorphenyl]-4-hydroxy-6-methylpyridin-3-carboxamid;
5-[(E)-2-Cyclopropylethenyl]-N-[4-[(6,7-dimethoxy-1,5-naphthyridin-4-yl)oxy]-3-fluorphenyl]-4-hydroxy-6-methylpyridin-3-carboxamid;
N-[4-[(6,7-Dimethoxy-1,5-naphthyridin-4-yl)oxy]-3-fluorphenyl]-5-[(E)-3,3-dimethylbut-1-enyl]-4-hydroxy-6-methylpyridin-3-carboxamid;
N-[4-[(6,7-Dimethoxy-1,5-naphthyridin-4-yl)oxy]-3-fluorphenyl]-4-hydroxy-6-methyl-5-[(E)-4-methylpent-1-enyl]pyridin-3-carboxamid;
N-[4-[(6,7-Dimethoxy-1,5-naphthyridin-4-yl)oxy]-3-fluorphenyl]-4-hydroxy-2,6-dimethyl-5-propan-2-ylpyridin-3-carboxamid;
N-[3-Fluor-4-[(7-methoxy-1,5-naphthyridin-4-yl)oxy]phenyl]-4-hydroxy-2,6-dimethyl-5-propan-2-ylpyridin-3-carboxamid;
5-(Cyclopenten-1-yl)-N-[3-fluor-4-[[6-methoxy-7-(2-methoxyethoxy)-1,5-naphthyridin-4-yl]oxy]phenyl]-4-hydroxy-6-methylpyridin-3-carboxamid;
5-(Cyclohexen-1-yl)-N-[3-fluor-4-[[6-methoxy-7-(2-methoxyethoxy)-1,5-naphthyridin-4-yl]oxy]phenyl]-4-hydroxy-6-methylpyridin-3-carboxamid;
5-(3,6-Dihydro-2H-pyran-4-yl)-N-[3-fluor-4-[[6-methoxy-7-(2-methoxyethoxy)-1,5-naphthyridin-4-yl]oxy]phenyl]-4-hydroxy-6-methylpyridin-3-carboxamid;
5-[(E)-2-Cyclopentylethenyl]-N-[3-fluor-4-[[6-methoxy-7-(2-methoxyethoxy)-1,5-naphthyridin-4-yl]oxy]phenyl]-4-hydroxy-6-methylpyridin-3-carboxamid;
5-[(E)-2-Cyclopropylethenyl]-N-[3-fluor-4-[[6-methoxy-7-(2-methoxyethoxy)-1,5-naphthyridin-4-yl]oxy]phenyl]-4-hydroxy-6-methylpyridin-3-carboxamid;
N-[4-[(6,7-Dimethoxy-1,5-naphthyridin-4-yl)oxy]-3-fluorphenyl]-4-methyl-2-oxo-1-propan-2-ylpyridin-3-carboxamid;
N-[4-[(6,7-Dimethoxy-1,5-naphthyridin-4-yl)oxy]phenyl]-4-methyl-2-oxo-1-propan-2-ylpyridin-3-carboxamid;
5-[(E)-3,3-Dimethylbut-1-enyl]-N-[3-fluor-4-[[6-methoxy-7-(2-methoxyethoxy)-1,5-naphthyridin-4-yl]oxy]phenyl]-4-hydroxy-6-methylpyridin-3-carboxamid;
5-(Cyclopenten-1-yl)-N-[3-fluor-4-[[6-methoxy-7-(2-methoxyethoxy)-1,5-naphthyridin-4-yl]oxy]phenyl]-4-hydroxy-2,6-dimethylpyridin-3-carboxamid;
5-(Cyclopenten-1-yl)-N-[3-fluor-4-[[6-methoxy-7-(2-methoxyethoxy)-1,5-naphthyridin-4-yl]oxy]phenyl]-1,2,6-trimethyl-4-oxopyridin-3-carboxamid;
5-(3,6-Dihydro-2H-pyran-4-yl)-N-[3-fluor-4-[[6-methoxy-7-(2-methoxyethoxy)-1,5-naphthyridin-4-yl]oxy]phenyl]-4-hydroxy-2,6-dimethylpyridin-3-carboxamid;
5-(3,6-Dihydro-2H-pyran-4-yl)-N-[3-fluor-4-[[6-methoxy-7-(2-methoxyethoxy)-1,5-naphthyridin-4-yl]oxy]phenyl]-1,2,6-trimethyl-4-oxopyridin-3-carboxamid;
5-[(E)-3,3-Dimethylbut-1-enyl]-N-[3-fluor-4-[[6-methoxy-7-(2-methoxyethoxy)-1,5-naphthyridin-4-yl]oxy]phenyl]-1,2,6-trimethyl-4-oxopyridin-3-carboxamid;
5-Cyclopropyl-N-[4-[(6,7-dimethoxy-1,5-naphthyridin-4-yl)oxy]-3-fluorphenyl]-1,2,6-trimethyl-4-oxopyridin-3-carboxamid;
5-Cyclopropyl-N-[4-[(6,7-dimethoxy-1,5-naphthyridin-4-yl)oxy]-2,5-difluorphenyl]-1,2,6-trimethyl-4-oxopyridin-3-carboxamid;
5-Cyclopropyl-N-[4-[(6,7-dimethoxy-1,5-naphthyridin-4-yl)oxy]phenyl]-1,2,6-trimethyl-4-oxopyridin-3-carboxamid;
N-[4-[(6,7-Dimethoxy-1,5-naphthyridin-4-yl)oxy]-3-fluorphenyl]-5-[(E)-3,3-dimethylbut-1-enyl]-1,2,6-trimethyl-4-oxopyridin-3-carboxamid;
5-[(E)-2-Cyclopropylethenyl]-N-[4-[(6,7-dimethoxy-1,5-naphthyridin-4-yl)oxy]-3-fluorphenyl]-1,2,6-trimethyl-4-oxopyridin-3-carboxamid;
5-[(E)-2-Cyclopropylethenyl]-N-[3-fluor-4-[[6-methoxy-7-(2-methoxyethoxy)-1,5-naphthyridin-4-yl]oxy]phenyl]-1,2,6-trimethyl-4-oxopyridin-3-carboxamid;
N-[4-[(6,7-Dimethoxy-1,5-naphthyridin-4-yl)oxy]-3-fluorphenyl]-1,2,6-trimethyl-4-oxo-5-prop-1-en-2-ylpyridin-3-carboxamid;
5-(3,6-Dihydro-2H-pyran-4-yl)-N-[4-[(6,7-dimethoxy-1,5-naphthyridin-4-yl)oxy]-3-fluorphenyl]-1,2,6-trimethyl-4-oxopyridin-3-carboxamid;
5-[(E)-2-Cyclopentylethenyl]-N-[3-fluor-4-[[6-methoxy-7-(2-methoxyethoxy)-1,5-naphthyridin-4-yl]oxy]phenyl]-1,2,6-trimethyl-4-oxopyridin-3-carboxamid;
N-[4-[(6,7-Dimethoxy-1,5-naphthyridin-4-yl)oxy]phenyl]-1,2,6-trimethyl-4-oxo-5-prop-1-en-2-ylpyridin-3-carboxamid;
N-[4-[(6,7-Dimethoxy-1,5-naphthyridin-4-yl)oxy]-3-fluorphenyl]-1,2,6-trimethyl-4-oxo-5-propan-2-ylpyridin-3-carboxamid;
N-[4-[(6,7-Dimethoxy-1,5-naphthyridin-4-yl)oxy]phenyl]-1,2,6-trimethyl-4-oxo-5-propan-2-ylpyridin-3-carboxamid;
5-(Cyclohexen-1-yl)-N-[3-fluor-4-[[6-methoxy-7-(2-methoxyethoxy)-1,5-naphthyridin-4-yl]oxy]phenyl]-1,2,6-trimethyl-4-oxopyridin-3-carboxamid;
4-Hydroxy-5-methoxy-N-[4-[(7-methoxy-1,5-naphthyridin-4-yl)oxy]phenyl]-2,6-dimethylpyridin-3-carboxamid;
N-[3-Fluor-4-[(7-methoxy-1,5-naphthyridin-4-yl)oxy]phenyl]-4-hydroxy-5-methoxy-2,6-dimethylpyridin-3-carboxamid;
N-[4-[(6,7-Dimethoxy-1,5-naphthyridin-4-yl)oxy]phenyl]-4-hydroxy-5-methoxy-2,6-dimethylpyridin-3-carboxamid;
N-[4-[(6,7-Dimethoxy-1,5-naphthyridin-4-yl)oxy]-3-fluorphenyl]-4-hydroxy-5-methoxy-2,6-dimethylpyridin-3-carboxamid;
5-Brom-N-[4-[(6,7-dimethoxy-1,5-naphthyridin-4-yl)oxy]-2,5-difluorphenyl]-6-ethyl-1-methyl-4-oxopyridin-3-carboxamid;
5-Brom-6-ethyl-N-[4-[[6-methoxy-7-(2-methoxyethoxy)-1,5-naphthyridin-4-yl]oxy]phenyl]-1-methyl-4-oxopyridin-3-carboxamid;
5-(Cyclopenten-1-yl)-N-[3-fluor-4-[[7-(2-methoxyethoxy)-1,5-naphthyridin-4-yl]oxy]phenyl]-1,2,6-trimethyl-4-oxopyridin-3-carboxamid;
5-(Cyclohexen-1-yl)-N-[3-fluor-4-[[7-(2-methoxyethoxy)-1,5-naphthyridin-4-yl]oxy]phenyl]-1,2,6-trimethyl-4-oxopyridin-3-carboxamid;
5-(3,6-Dihydro-2H-pyran-4-yl)-N-[3-fluor-4-[[7-(2-methoxyethoxy)-1,5-naphthyridin-4-yl]oxy]phenyl]-1,2,6-trimethyl-4-oxopyridin-3-carboxamid;
N-[4-[(6,7-Dimethoxy-1,5-naphthyridin-4-yl)oxy]-2,5-difluorphenyl]-4-hydroxy-5-methoxy-2,6-dimethylpyridin-3-carboxamid;
7-(Cyclopenten-1-yl)-N-[4-[(6,7-dimethoxy-1,5-naphthyridin-4-yl)oxy]-3-fluorphenyl]-6-methyl-8-oxo-3,4-dihydro-1H-pyrido[2,1-c][1,4]oxazin-9-carboxamid;
N-[3-Fluor-4-[[6-methoxy-7-(2-methoxyethoxy)-1,5-naphthyridin-4-yl]oxy]phenyl]-4-hydroxy-5-methoxy-2,6-dimethylpyridin-3-carboxamid;
4-Hydroxy-5-methoxy-N-[4-[[6-methoxy-7-(2-methoxyethoxy)-1,5-naphthyridin-4-yl]oxy]phenyl]-2,6-dimethylpyridin-3-carboxamid;
7-(3,6-Dihydro-2H-pyran-4-yl)-N-[4-[(6,7-dimethoxy-1,5-naphthyridin-4-yl)oxy]phenyl]-6-methyl-8-oxo-3,4-dihydro-1H-pyrido[2,1-c][1,4]oxazin-9-carboxamid;
N-[4-[[7-(2-Cyclobutylethoxy)-6-methoxy-1,5-naphthyridin-4-yl]oxy]-3-fluorphenyl]-5-(cyclopenten-1-yl)-1,2,6-trimethyl-4-oxopyridin-3-carboxamid;
N-[3-Fluor-4-[(7-methoxy-1,5-naphthyridin-4-yl)oxy]phenyl]-5-methoxy-1,2,6-trimethyl-4-oxopyridin-3-carboxamid;
N-[4-[(6,7-Dimethoxy-1,5-naphthyridin-4-yl)oxy]phenyl]-5-methoxy-1,2,6-trimethyl-4-oxopyridin-3-carboxamid;
N-[4-[(6,7-Dimethoxy-1,5-naphthyridin-4-yl)oxy]-3-fluorphenyl]-5-methoxy-1,2,6-trimethyl-4-oxopyridin-3-carboxamid;
5-Cyano-N-[4-[(6,7-dimethoxy-1,5-naphthyridin-4-yl)oxy]-3-fluorphenyl]-1,2,6-trimethyl-4-oxopyridin-3-carboxamid;
7-(3,6-Dihydro-2H-pyran-4-yl)-N-[4-[(6,7-dimethoxy-1,5-naphthyridin-4-yl)oxy]-3-fluorphenyl]-6-methyl-8-oxo-3,4-dihydro-1H-pyrido[2,1-c][1,4]oxazin-9-carboxamid;
7-(Cyclohexen-1-yl)-N-[4-[(6,7-dimethoxy-1,5-naphthyridin-4-yl)oxy]-3-fluorphenyl]-6-methyl-8-oxo-3,4-dihydro-1H-pyrido[2,1-c][1,4]oxazin-9-carboxamid;
5-Cyano-N-[3-fluor-4-[(7-methoxy-1,5-naphthyridin-4-yl)oxy]phenyl]-1,2,6-trimethyl-4-oxopyridin-3-carboxamid;
5-Cyano-N-[4-[(6,7-dimethoxy-1,5-naphthyridin-4-yl)oxy]phenyl]-1,2,6-trimethyl-4-oxopyridin-3-carboxamid;
5-Cyano-N-[2,5-difluor-4-[[6-methoxy-7-(2-methoxyethoxy)-1,5-naphthyridin-4-yl]oxy]phenyl]-1,2,6-trimethyl-4-oxopyridin-3-carboxamid;
3-N-[4-[(6,7-Dimethoxy-1,5-naphthyridin-4-yl)oxy]-3-fluorphenyl]-4-hydroxy-2,6-dimethylpyridin-3,5-dicarboxamid;
3-N-[4-[(6,7-Dimethoxy-1,5-naphthyridin-4-yl)oxy]phenyl]-4-hydroxy-2,6-dimethylpyridin-3,5-dicarboxamid;
3-N-[2,5-Difluor-4-[[6-methoxy-7-(2-methoxyethoxy)-1,5-naphthyridin-4-yl]oxy]phenyl]-4-hydroxy-2,6-dimethylpyridin-3,5-dicarboxamid;
5-Cyano-N-[4-[(6,7-dimethoxy-1,5-naphthyridin-4-yl)oxy]-3-fluorphenyl]-4-hydroxy-2,6-dimethylpyridin-3-carboxamid;
N-[4-[(6,7-Dimethoxy-1,5-naphthyridin-4-yl)oxy]-3-fluorphenyl]-1,2,6-trimethyl-5-morpholin-4-yl-4-oxopyridin-3-carboxamid;
N-[4-[(6,7-Dimethoxy-1,5-naphthyridin-4-yl)oxy]-3-fluorphenyl]-1,2,6-trimethyl-4-oxo-5-pyrrolidin-1-ylpyridin-3-carboxamid;
N-[4-[(6,7-Dimethoxy-1,5-naphthyridin-4-yl)oxy]-3-fluorphenyl]-1,2,6-trimethyl-4-oxo-5-piperidin-lylpyridin-3-carboxamid;
3-N-[3-Fluor-4-[(7-methoxy-1,5-naphthyridin-4-yl)oxy]phenyl]-4-hydroxy-2,6-dimethylpyridin-3,5-dicarboxamid;
5-[[4-[(6,7-Dimethoxy-1,5-naphthyridin-4-yl)oxy]-3-fluorphenyl]carbamoyl]-1,2,6-trimethyl-4-oxopyridin-3-carbonsäure;
3-N-[4-[(6,7-Dimethoxy-1,5-naphthyridin-4-yl)oxy]-3-fluorphenyl]-4-hydroxy-5-N,5-N,2,6-tetramethylpyridin-3,5-dicarboxamid;
3-N-[4-[(6,7-Dimethoxy-1,5-naphthyridin-4-yl)oxy]-3-fluorphenyl]-4-hydroxy-2,6-dimethyl-5-N-propan-2-ylpyridin-3,5-dicarboxamid;
5-N-[4-[(6,7-Dimethoxy-1,5-naphthyridin-4-yl)oxy]-3-fluorphenyl]-3-N,3-N,1,2,6-pentamethyl-4-oxopyridin-3,5-dicarboxamid;
3-N-Cyclopropyl-5-N-[4-[(6,7-dimethoxy-1,5-naphthyridin-4-yl)oxy]-3-fluorphenyl]-1,2,6-trimethyl-4-oxopyridin-3,5-dicarboxamid;
3-N-[4-[(6,7-Dimethoxy-1,5-naphthyridin-4-yl)oxy]-3-fluorphenyl]-4-hydroxy-5-N,2,6-trimethylpyridin-3,5-dicarboxamid;
5-N-Cyclopropyl-3-N-[4-[(6,7-dimethoxy-1,5-naphthyridin-4-yl)oxy]-3-fluorphenyl]-4-hydroxy-2,6-dimethylpyridin-3,5-dicarboxamid;
7-(Cyclopenten-1-yl)-N-[4-[(6,7-dimethoxy-1,5-naphthyridin-4-yl)oxy]phenyl]-6-methyl-8-oxo-3,4-dihydro-1H-pyrido[2,1-c][1,4]oxazin-9-carboxamid;
7-(Cyclohexen-1-yl)-N-[4-[(6,7-dimethoxy-1,5-naphthyridin-4-yl)oxy]phenyl]-6-methyl-8-oxo-3,4-dihydro-1H-pyrido[2,1-c][1,4]oxazin-9-carboxamid; und
5-(Cyclopenten-1-yl)-N-[3-fluor-4-[[7-(2-methoxyethoxy)-1,5-naphthyridin-4-yl]oxy]phenyl]-4-hydroxy-2,6-dimethylpyridin-3-carboxamid;
und pharmazeutisch annehmbare Salze, Stereoisomere oder Tautomere davon.

12. Pharmazeutische Zusammensetzung, umfassend eine Verbindung nach einem der Ansprüche 1-11 oder ein pharmazeutisch annehmbares Salz, Stereoisomer oder Tautomer davon, und einen pharmazeutisch annehmbaren Träger oder Hilfsstoff.

13. Verbindung nach einem der Ansprüche 1-11 oder ein pharmazeutisch annehmbares Salz, Stereoisomer oder Tautomer davon, oder eine pharmazeutische Zusammensetzung nach Anspruch 12, zur Verwendung in einem Verfahren des Behandelns einer Krankheit, einer Störung oder eines Syndroms, die/das durch Modulieren von In-vivo-Aktivität einer Proteinkinase vermittelt wird,
wobei optional die Proteinkinase AXL, KDR, Mer oder Met ist.

14. Prozess zum Herstellen einer Verbindung der Formel (Ia), Formel (Ib) oder Formel (Ic) oder eines pharmazeutisch annehmbaren Salzes, Stereoisomers oder Tautomers davon, nach einem der Ansprüche 1 bis 11, wobei:
der Prozess das Kontaktieren einer Verbindung der Formel A-a, Formel A-b oder Formel A-c: 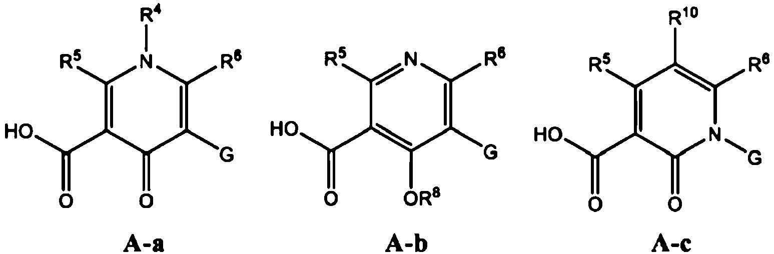 mit einer Verbindung der Formel B-c umfasst: 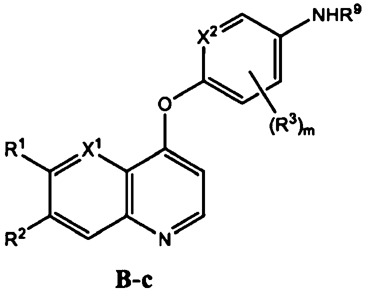
unter Amidbindungs-Bildungsbedingungen, um die Verbindung der Formel (Ia), Formel (Ib) oder Formel (Ic) bereitzustellen, oder ein pharmazeutisch annehmbares Salz, Stereoisomer oder Tautomer davon, wobei:
X¹, X², R¹, R², R³, R⁴, R⁵, R⁶, R⁷, R⁸, R⁹, R¹⁰, G und m wie in Anspruch 1 definiert sind.

15. Prozess nach Anspruch 14, wobei die Amidbindungs-Bildungsbedingungen HATU in Anwesenheit einer Base in einem organischen Lösungsmittel umfassen.

## Revendications

1. Composé de la formule (Ia), de la formule (Ib), ou de la formule (Ic) : 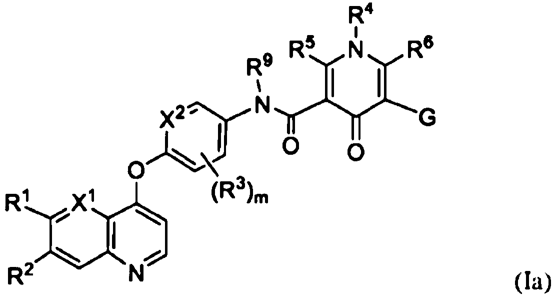 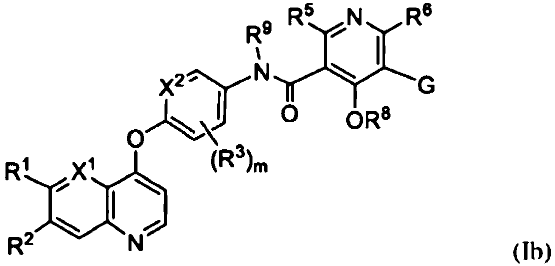 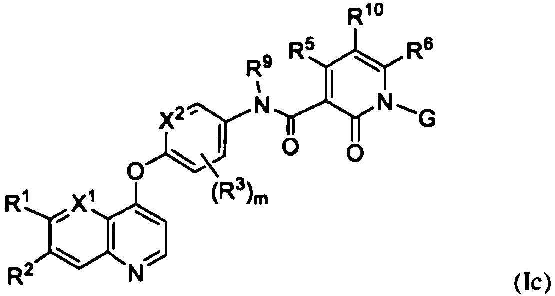 ou sel pharmaceutiquement acceptable, stéréoisomère ou tautomère de celui-ci, dans lequel :
G est cycloalkyle en C₃₋₁₀, hétérocycloalkyle à 4 à 14 membres, alkyle en C₁₋₆, alcényle en C₂₋₆, alkoxy en C₁₋₆, cyano, halo, C(O)OR^{a}, ou C(O)NR^{a}R^{a}, dans lequel le cycloalkyle en C₃₋₁₀, hétérocycloalkyle à 4 à 14 membres, alkyle en C₁₋₆, alcényle en C₂₋₆, et alkoxy en C₁₋₆ de G sont chacun facultativement substitué avec 1, 2, 3, ou 4 substituants R⁷ indépendamment sélectionnés ;
X¹ est N ;
X² est CH ou CF ;
R¹ et R² sont chacun indépendamment sélectionnés parmi H, halo, alkyle en C₁₋₆, alcényle en C₂₋₆, alcynyle en C₂₋₆, haloalkyle en C₁₋₆, haloalkoxy en C₁₋₆, aryle en C₆₋₁₀, cycloalkyle en C₃₋₁₄, hétéroaryle à 5 à 14 membres, hétérocycloalkyle à 4 à 14 membres, aryle en C₆₋₁₀-alkylène en C₁₋₄-, cycloalkyle en C₃₋₁₄-alkylène en C₁₋₄-, (hétéroaryle à 5 à 14 membres)-alkylène en C₁₋₄-, (hétérocycloalkyle à 4 à 14 membres)-alkylène en C₁₋₄-, CN, NO₂, OR^{a}, SR^{a}, NHOR^{a}, C(O)R^{a}, C(O)NR^{a}R^{a}, C(O)OR^{a}, C(O)NR^{a}S(O)₂R^{a}, OC(O)R^{a}, OC(O)NR^{a}R^{a}, NHR^{a}, NR^{a}R^{a}, NR^{a}C(O)R^{a}, NR^{a}C(=NR^{a})R^{a}, NR^{a}C(O)OR^{a}, NR^{a}C(O)NR^{a}R^{a}, C(=NR^{a})R^{a}, C(=NOH)R^{a}, C(=NOH)NR^{a}, C(=NCN)NR^{a}R^{a}, NR^{a}C(=NCN)NR^{a}R^{a}, C(=NR^{a})NR^{a}R^{a}, NR^{a}C(=NR^{a})NR^{a}R^{a}, NR^{a}S(O)R^{a}, NR^{a}S(O)₂R^{a}, NR^{a}S(O)₂NR^{a}R^{a}, S(O)R^{a}, S(O)NR^{a}R^{a}, S(O)₂R^{a}, S(O)₂NR^{a}C(O)R^{a}, P(O)R^{a}R^{a}, P(O)(OR^{a})(OR^{a}), B(OH)₂, B(OR^{a})₂, et S(O)₂NR^{a}R^{a}, dans lequel l'alkyle en C₁₋₆, alcényle en C₂₋₆, alkynyle en C₂₋₆, aryle en C₆₋₁₀, cycloalkyle en C₃₋₁₄, hétéroaryle à 5 à 14 membres, hétérocycloalkyle à 4 à 14 membres, aryle en C₆₋₁₀-alkylène en C₁₋₄-, cycloalkyle en C₃₋₁₄-alkylène en C₁₋₄-, (hétéroaryle à 5 à 14 membres)-alkylène en C₁₋₄-, et (hétérocycloalkyle à 4 à 14 membres)-alkylène en C₁₋₄- de R¹ et de R² sont chacun facultativement substitués avec 1, 2, 3, 4, ou 5 substituants R^{b} indépendamment sélectionnés ;
chaque R³ est indépendamment sélectionnés parmi halo, OH, CN, -C(O)OH, -C(O)NH(alkyle en C₁₋₆), -SO₂(alkyle en C₁₋₆), -SO₂NH(alkyle en C₁₋₆), alkyle en C₁-C₆, alkoxy en C₁-C₆, haloalkoxy en C₁-C₆, NH₂, -NH(alkyle en C₁-C₆), -N(alkyle en C₁-C₆)₂, et cycloalkyle en C₃-C₆, dans lequel l'alkyle en C₁-C₆, alkoxy en C₁-C₆, -NH(alkyle en C₁-C₆), -N(alkyle en C₁-C₆), et cycloalkyle en C₃-C₆ de R³ sont chacun facultativement substitués avec 1, 2, ou 3 substituants R^{g} indépendamment sélectionnés ;
R⁴ est sélectionné parmi H, halo, alkyle en C₁₋₆, alcényle en C₂₋₆, alkynyle en C₂₋₆, haloalkyle en C₁₋₆, haloalkoxy en C₁₋₆, aryle en C₆₋₁₀, cycloalkyle en C₃₋₁₄, hétéroaryle à 5 à 14 membres, hétérocycloalkyle à 4 à 14 membres, aryle en C₆₋₁₀-alkylène en C₁₋₄-, cycloalkyle en C₃₋₁₄ -alkylène en C₁₋₄-, (hétéroaryle à 5 à 14 membres)-alkylène en C₁₋₄-, (hétérocycloalkyle à 4 à 14 membres)-alkylène en C₁₋₄-, CN, NO₂, OR^{a}, SR^{a}, NHOR^{a}, C(O)R^{a}, C(O)NR^{a}R^{a}, C(O)OR^{a}, C(O)NR^{a}S(O)₂R^{a}, OC(O)R^{a}, OC(O)NR^{a}R^{a}, NHR^{a}, NR^{a}R^{a}, NR^{a}C(O)R^{a}, N=C(NR^{a}R^{a})₂, NR^{a}C(=NR^{a})R^{a}, NR^{a}C(O)OR^{a}, NR^{a}C(O)NR^{a}R^{a}, C(=NR^{a})R^{a}, C(=NOH)R^{a}, C(=NOH)NR^{a}, C(=NCN)NR^{a}R^{a}, NR^{a}C(=NCN)NR^{a}R^{a}, C(=NR^{a})NR^{a}R^{a}, NR^{a}C(=NR^{a})NR^{a}R^{a}, NR^{a}S(O)R^{a}, NR^{a}S(O)₂R^{a}, NR^{a}S(O)ₐNR^{a}R^{a}, S(O)R^{a}, S(O)NR^{a}R^{a}, S(O)₂R^{a}, S(O)₂NR^{a}C(O)R^{a}, P(O)R^{a}R^{a}, P(O)(OR^{a})(OR^{a}), B(OH)₂, B(OR^{a})₂, et S(O)₂NR^{a}R^{a}, dans lequel l'alkyle en C₁₋₆, alcényle en C₂₋₆, alkynyle en C₂₋₆, aryle en C₆₋₁₀, cycloalkyle en C₃₋₁₄, hétéroaryle à 5 à 14 membres, hétérocycloalkyle à 4 à 14 membres, aryle en C₆₋₁₀-alkylène en C₁₋₄-, cycloalkyle en C₃₋₁₄ -alkylène en C₁₋₄-, (hétéroaryle à 5 à 14 membres)-alkylène en C₁₋₄-, et (hétérocycloalkyle à 4 à 14 membres)-alkylène en C₁₋₄- de R⁴ sont chacun facultativement substitués avec 1, 2, 3, 4, ou 5 substituants R^{b} indépendamment sélectionnés ;
R⁵, R⁶ et R¹⁰ sont chacun indépendamment H, halo, alkyle en C₁₋₆, alcényle en C₂₋₆, alkoxy en C₁₋₆, alkylthio en C₁₋₆, CN, haloalkyle en C₁₋₄, haloalkoxy en C₁₋₄, OH, alkyle en C₁₋₄-C(O)-, alkyle en C₁₋₄-OC(O)-, -C(O)NH(alkyle en C₁₋₄), NH₂, -NH-alkyle en C₁₋₄, ou -N(alkyle en C₁₋₄)₂, dans lequel l'alkyle en C₁₋₆, alcényle en C₂₋₆, alkoxy en C₁₋₆, alkylthio en C₁₋₆, alkyle en C₁₋₆-C(O)-, et alkyle en C₁₋₄ de -NH(alkyle en C₁₋₄), ou de -N(alkyle en C₁₋₄)₂ de R⁵, de R⁶, et de R¹⁰ sont chacun facultativement substitués avec 1 ou 2 substituants R^{g} indépendamment sélectionnés ;
chaque R⁷ est indépendamment sélectionné parmi halo, OH, C(O)OR^{a}, C(O)R^{a}, C(O)NR^{a}R^{a}, CN, NH₂, -NH(alkyle en C₁-C₆), -N(alkyle en C₁-C₆)₂, alkyle en C₁-C₆, alcényle en C₂-C₆, alkoxy en C₁-C₆, haloalkyle en C₁-C₆, haloalkoxy en C₁-C₆, C(O)NR^{a}R^{a}, NR^{a}C(O)R^{a}, NR^{a}C(O)NR^{a}R^{a}, SO₂R^{a}, NR^{a}S(O)₂R^{a}, NR^{a}S(O)₂NR^{a}R^{a}, cycloalkyle en C₃-C₆, hétérocycloalkyle à 4 à 6 membres, phényle, hétéroaryle à 5 ou 6 membres, cycloalkyle en C₃-C₆-alkylène en C₁-C₄-, (hétérocycloalkyle à 4 à 6 membres)-alkylène en C₁-C₄-, phényle-alkylène en C₁-C₂, et (hétéroaryle à 5 ou 6 membres)-alkylène en C₁-C₄-, dans lequel l'alkyle en C₁-C₆, alkoxy en C₁-C₆, cycloalkyle en C₃-C₆, hétérocycloalkyle à 4 à 6 membres, phényle, hétéroaryle à 5 ou 6 membres, cycloalkyle en C₃-C₆-alkylène en C₁-C₄-, (hétérocycloalkyle à 4 à 6 membres)-alkylène en C₁-C₄-, phényle-alkylène en C₁-C₂, et (hétéroaryle à 5 ou 6 membre)-alkylène en C₁-C₄- de R⁷ sont chacun facultativement substitués avec 1, 2, ou 3 substituants R^{f} indépendamment sélectionnés ;
R⁸ est H, alkyle en C₁₋₆ facultativement substitué avec 1 ou 2 substituants R^{g} ou un groupe protecteur hydroxy ;
R⁹ est H ou alkyle en C₁₋₆ facultativement substitué avec 1, 2, ou 3 substituants R^{g} indépendamment sélectionnés ;
ou R⁴ et R⁵ considérés conjointement avec les atomes auxquels ils sont attachés forment hétérocycloalkyle fusionné à 4 à 7 membres ou hétéroaryle fusionné à 5 à 6 membres, dans lequel l'hétérocycloalkyle fusionné à 4 à 7 membres et hétéroaryle fusionné à 5 à 6 membres sont chacun facultativement substitués avec 1 ou 2 substituants R^{g} indépendamment sélectionnés ;
ou R¹⁰ et R⁵ considérés conjointement avec les atomes auxquels ils sont attachés forment cycloalkyle en C₃₋₇ fusionné, hétérocycloalkyle fusionné à 4 à 6 membres, hétéroaryle fusionné à 5 ou 6 membres ou phényle fusionné, dans lequel le cycloalkyle en C₃₋₇ fusionné, hétérocycloalkyle fusionné à 4 à 6 membres, hétéroaryle à 5 ou 6 membres, ou phényle fusionné sont chacun facultativement substitués avec 1 ou 2 substituants R^{g} indépendamment sélectionnés, et dans lequel un ou deux atomes de carbone en anneau du cycloalkyle en C₃₋₇ fusionné ou de l'hétérocycloalkyle fusionné sont facultativement remplacés par un groupe carbonyle ;
ou R¹² et R¹⁰ considérés conjointement avec les atomes auxquels ils sont attachés forment hétérocycloalkyle fusionné à 4 à 7 membres ou hétéroaryle fusionné à 5 à 6 membres, dans lequel l'hétérocycloalkyle fusionné à 4 à 7 membres et hétéroaryle fusionné à 5 à 6 membres sont chacun facultativement substitués avec 1 ou 2 substituants R^{g} indépendamment sélectionnés ;
ou R⁶ et R⁴ considérés conjointement avec les atomes auxquels ils sont attachés forment hétérocycloalkyle fusionné à 4 à 7 membres ou hétéroaryle fusionné à 5 à 6 membres, dans lequel l'hétérocycloalkyle fusionné à 4 à 7 membres et hétéroaryle fusionné à 5 à 6 membres sont chacun facultativement substitués avec 1 ou 2 substituants R^{g} indépendamment sélectionnés ;
ou R⁶ et R¹⁰ considérés conjointement avec les atomes auxquels ils sont attachés forment cycloalkyle en C₃₋₇ fusionné, hétérocycloalkyle fusionné à 4 à 6 membres, hétéroaryle fusionné à 5 à 6 membres, ou hétéroaryle fusionné, dans lequel le cycloalkyle en C₃₋₇ fusionné, hétérocycloalkyle fusionné à 4 à 6 membres, hétéroaryle fusionné à 5 à 6 membres, et hétéroaryle fusionné sont chacun facultativement substitués avec 1 ou 2 substituants R^{g} indépendamment sélectionnés et dans lequel un ou deux atomes de carbone en anneau du cycloalkyle en C₃₋₇ fusionné ou de l'hétérocycloalkyle fusionné à 4 à 6 membres sont facultativement remplacés par un carbonyle ;
chaque R^{a} est indépendamment sélectionné parmi H, CN, alkyle en C₁-C₆, haloalkyle en C₁-C₆, alcényle en C₂-C₆, alkynyle en C₂-C₆, aryle en C₆-C₁₀, cycloalkyle en C₃-C₁₀, hétéroaryle à 5 à 14 membres, hétérocycloalkyle à 4 à 14 membres, aryle en C₆-C₁₀-alkylène en C₁-C₆-, cycloalkyle en C₃-C₁₀- alkylène en C₁-C₄-, (hétéroaryle à 5 à 14 membres)-alkylène en C₁-C₄-, et (hétérocycloalkyle à 4 à 14 membres)-alkylène en C₁-C₄-, dans lequel l'alkyle en C₁-C₆, haloalkyle en C₁-C₆, alcényle en C₂-C₆, alkynyle en C₂-C₆, aryle en C₆-C₁₀, cycloalkyle en C₃-C₁₀, hétéroaryle à 5 à 14 membres, hétérocycloalkyle à 4 à 14 membres, aryle en C₆-C₁₀-alkylène en C₁-C₄-, cycloalkyle en C₃-C₁₀-alkylène en C₁-C₄-, (hétéroaryle à 5 à 14 membres)-alkylène en C₁-C₄-, et (hétérocycloalkyle à 4 à 14 membres)-alkylène en C₁-C₄- de R^{a} sont chacun facultativement substitués avec 1, 2, 3, 4, ou 5 substituants R^{d} indépendamment sélectionnés ;
ou deux quelconques substituants R^{a} conjointement avec l'atome d'azote auquel ils sont attachés forment hétérocycloalkyle à 4, 5, 6, 7, 8, 9, ou 10 membres, dont chacun est facultativement substitué avec 1, 2, ou 3 substituants R^{f} indépendamment sélectionnés ;
chaque R^{b} est indépendamment sélectionnés parmi halo, alkyle en C₁-C₆, alcényle en C₂-C₆, alkynyle en C₂-C₆, haloalkyle en C₁-C₆, haloalkoxy en C₁-C₆, aryle en C₆-C₁₀, cycloalkyle en C₃-C₁₀, hétéroaryle à 5 à 10 membres, hétérocycloalkyle à 4 à 10 membres, aryle en C₆-C₁₀-alkylène en C₁-C₄-, cycloalkyle en C₃-C₁₀-alkylène en C₁-C₄-, (hétéroaryle à 5 à 10 membres)-alkylène en C₁-C₄-, (hétérocycloalkyle à 4 à 10 membres)-alkylène en C₁-C₄-, CN, OH, NH₂, NO₂, NHOR^{c}, OR^{c}, SR^{c}, C(O)R^{c}, C(O)NR^{c}R^{c}, C(O)OR^{c}, C(O)NR^{c}S(O)₂R^{c}, OC(O)R^{c}, OC(O)NR^{c}R^{c}, C(=NOH)R^{c}, C(=NOH)NR^{c}, C(=NCN)NR^{c}R^{c}, NR^{c}C(=NCN)NR^{c}R^{c}, C(=NR^{c})NR^{c}R^{c}, NR^{c}C(=NR^{c})NR^{c}R^{c}, NHR^{c}, NR^{c}R^{c}, NR^{c}C(O)R^{c}, NR^{c}C(=NR^{c})R^{c}, NR^{c}C(O)OR^{c}, NR^{c}C(O)NR^{c}R^{c}, NR^{c}S(O)R^{c}, NR^{c}S(O)₂R^{c}, NR^{c}S(O)₂NR^{c}R^{c}, S(O)R^{c}, S(O)₂NR^{c}R^{c}, S(O)₂R^{c}, S(O)₂NR^{c}C(O)R^{c}, Si(R^{c})₃, P(O)R^{c}R^{c}, P(O)(OR^{c})(OR^{c}), B(OH)₂, B(OR^{c})₂, et S(O)₂NR^{c}R^{c}, dans lequel l'alkyle en C₁-C₆, alcényle en C₂-C₆, alkynyle en C₂-C₆, aryle en C₆-C₁₀, cycloalkyle en C₃-C₁₀, hétéroaryle à 5 à 10 membres, hétérocycloalkyle à 4 à 10 membres, aryle en C₆-C₁₀-alkylène en C₁-C₄-, cycloalkyle en C₃-C₁₀-alkylène en C₁-C₄-, (hétéroaryle à 5 à 10 membres)-alkylène en C₁-C₄-, et (hétérocycloalkyle à 4 à 10 membres)-alkylène en C₁-C₄- de R^{b} sont chacun en outre facultativement substitués avec 1, 2, ou 3 substituants R^{d} indépendamment sélectionnés ;
chaque R^{c} est indépendamment sélectionnés parmi H, alkyle en C₁-C₆, haloalkyle en C₁-C₆, alcényle en C₂-C₆, alkynyle en C₂-C₆, aryle en C₆-C₁₀, cycloalkyle en C₃-C₁₀, hétéroaryle à 5 à 10 membres, hétérocycloalkyle à 4 à 10 membres, aryle en C₆-C₁₀-alkylène en C₁-C₄-, cycloalkyle en C₃-C₁₀-alkylène en C₁-C₄-, (hétéroaryle à 5 à 10 membres)-alkylène en C₁-C₄-, et (hétérocycloalkyle à 4 à 10 membres)-alkylène en C₁-C₄-, dans lequel l'alkyle en C₁-C₆, alcényle en C₂-C₆, alkynyle en C₂-C₆, aryle en C₆-C₁₀, cycloalkyle en C₃-C₁₀, hétéroaryle à 5 à 10 membres, hétérocycloalkyle à 4 à 10 membres, aryle en C₆-C₁₀-alkylène en C₁-C₄-, cycloalkyle en C₃-C₁₀-alkylène en C₁-C₄-, (hétéroaryle à 5 à 10 membres)-alkylène en C₁-C₄-, et (hétérocycloalkyle à 4 à 10 membres)-alkylène en C₁-C₄- de R^{c} sont chacun facultativement substitués avec 1, 2, 3, 4, ou 5 substituants R^{f} indépendamment sélectionnés ;
ou deux quelconques substituants R^{c} conjointement avec l'atome d'azote auquel ils sont attachés forment hétérocycloalkyle à 4, 5, 6, 7, 8, 9, ou 10 membres, dont chacun est facultativement substitué avec 1, 2, ou 3 substituants R^{f} indépendamment sélectionnés ;
chaque R^{d} est indépendamment sélectionné parmi alkyle en C₁-C₆, haloalkyle en C₁-C₆, halo, aryle en C₆-C₁₀, hétéroaryle à 5 à 10 membres, cycloalkyle en C₃-C₁₀, hétérocycloalkyle à 4 à 10 membres, aryle en C₆-C₁₀-alkylène en C₁-C₄-, cycloalkyle en C₃-C₁₀-alkylène en C₁-C₄-, (hétéroaryle à 5 à 10 membres)-alkylène en C₁-C₄-, (hétérocycloalkyle à 4 à 10 membres)-alkylène en C₁-C₄-, CN, NH₂, NHOR^{e}, OR^{e}, SR^{e}, C(O)R^{e}, C(O)NR^{e}R^{e}, C(O)OR^{e}, OC(O)R^{e}, OC(O)NR^{e}R^{e}, NHR^{e}, NR^{e}R^{e}, NR^{e}C(O)R^{e}, NR^{e}C(O)NR^{e}R^{e}, NR^{e}C(O)OR^{e}, C(=NR^{e})NR^{e}R^{e}, NR^{e}C(=NR^{e})NR^{e}R^{e}, NR^{e}C(=NOH)NR^{e}R^{e}, NR^{e}C(=NCN)NR^{e}R^{e}, S(O)R^{e}, S(O)NR^{e}R^{e}, S(O)₂R^{e}, NR^{e}S(O)₂R^{e}, NR^{e}S(O)₂NR^{e}R^{e}, et S(O)₂NR^{e}R^{e}, dans lequel l'alkyle en C₁-C₆, aryle en C₆-C₁₀, hétéroaryle à 5 à 10 membres, cycloalkyle en C₃-C₁₀, hétérocycloalkyle à 4 à 10 membres, aryle en C₆-C₁₀-alkylène en C₁-C₄-, cycloalkyle en C₃-C₁₀-alkylène en C₁-C₄-, (hétéroaryle à 5 à 10 membres)-alkylène en C₁-C₄-, et (hétérocycloalkyle à 4 à 10 membres)-alkylène en C₁-C₄- de R^{d} sont chacun facultativement substitués avec 1, 2, ou 3 substituants R^{f} indépendamment sélectionnés ;
chaque R^{e} est indépendamment sélectionné parmi H, alkyle en C₁-C₆, alkoxy en C₁-C₆, cycloalkyle en C₃-C₆, cycloalkyle en C₃-C₆-alkylène en C₁-C₄-, aryle en C₆-C₁₀, aryle en C₆-C₁₀-alkylène en C₁-C₄-, hétéroaryle à 5 ou 6 membres, (hétéroaryle à 5 ou 6 membres)-alkylène en C₁-C₄-, hétérocycloalkyle à 4 à 7 membres, (hétérocycloalkyle à 4 à 7 membres)-alkylène en C₁-C₄-, haloalkyle en C₁-C₆, haloalkoxy en C₁-C₆, alcényle en C₂-C₄, et alkynyle en C₂-C₄, dans lequel l'alkyle en C₁-C₆, alkoxy en C₁-C₆, cycloalkyle en C₃-C₆, aryle en C₆-C₁₀, hétéroaryle à 5 ou 6 membres, hétérocycloalkyle à 4 à 7 membres, aryle en C₆-C₁₀-alkylène en C₁-C₄-, (hétéroaryle à 5 ou 6 membres)-alkylène en C₁-C₄-, (hétérocycloalkyle à 4 à 7 membres)-alkylène en C₁-C₄-, alcényle en C₂-C₄, et alkynyle en C₂-C₄ de R^{e} sont chacun facultativement substitués avec 1, 2, ou 3 substituants R^{f} ;
ou deux quelconques substituants R^{e} conjointement avec l'atome d'azote auquel ils sont attachés forment un hétérocycloalkyle à 4, 5, 6, 7, 8, 9, ou 10 membres, dont chacun est facultativement substitué avec 1, 2, ou 3 substituants R^{f} indépendamment sélectionnés ;
chaque R^{f} est indépendamment sélectionné parmi halo, OH, CN, C(O)OH, NH₂, -NH(alkyle en C₁-C₆), -N(alkyle en C₁-C₆), alkyle en C₁-C₆, vinyle, alkoxy en C₁-C₆, alkylthio en C₁-C₆, haloalkyle en C₁-C₆, haloalkoxy en C₁-C₆, phényle, hétéroaryle à 5 à 6 membres, hétérocycloalkyle à 4 à 6 membres, et cycloalkyle en C₃-C₆, dans lequel l'alkyle en C₁-C₆, alkoxy en C₁-C₆, alkylthio en C₁-C₆, phényle, cycloalkyle en C₃-C₆, hétérocycloalkyle à 4 à 6 membres, et hétéroaryle à 5 à 6 membres de R^{f} sont chacun facultativement substitué avec 1, 2, ou 3 substituants sélectionnés parmi halo, OH, CN, -C(O)OH, -NH₂, alkyle en C₁-C₄, alkoxy en C₁-C₄, haloalkyle en C₁-C₄, haloalkoxy en C₁-C₄, phényle, cycloalkyle en C₃-C₁₀, hétéroaryle à 5 à 6 membres, et hétérocycloalkyle à 4 à 6 membres ;
chaque R^{g} est indépendamment sélectionné parmi halo, OH, CN, C(O)OH, -C(O)O-alkyle en C₁-C₄, NH₂, -NH(alkyle en C₁-C₆), -N(alkyle en C₁-C₆)₂, alkyle en C₁-C₆, alkoxy en C₁-C₆, alkylthio en C₁-C₆, haloalkyle en C₁-C₆, haloalkoxy en C₁-C₆, phényle, hétéroaryle à 5 à 6 membres, hétérocycloalkyle à 4 à 6 membres, et cycloalkyle en C₃-C₆ ; et
l'indice m est 0.

2. Composé de la revendication 1, ou sel pharmaceutiquement acceptable, stéréoisomère, ou tautomère de celui-ci, dans lequel G est :
cycloalkyle en C₃₋₆ ou hétérocycloalkyle à 4 à 6 membres ;
cyclopropyle, cyclobutyle, cyclopentyle, cyclohexyle, cyclobutène-1-yle, cyclopentèn-1-yle, cyclohextèn-1-yle, 3,6-dihydro-2H-pyran-4-yle, pipéridin-1-yle, pyrrolidine-1-yle, ou morpholino ;
alkyle en C₁₋₆, alcényle en C₂₋₆, alkoxy en C₁₋₆, ou halo ; ou
alkyle en C₁₋₆, vinyle, méthoxy, ou halo.

3. Composé de la revendication 1 ou la revendication 2, ayant la formule (Ia-1) : 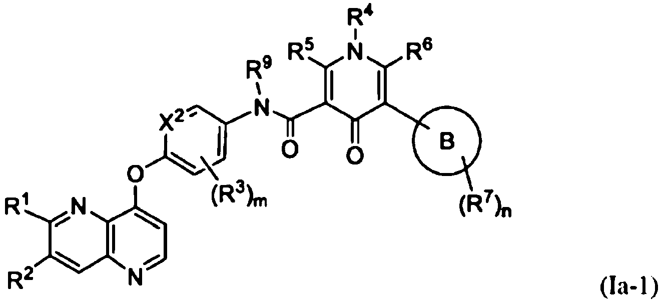 ou sel pharmaceutiquement acceptable, stéréoisomère, ou tautomère de celui-ci, dans lequel l'anneau B est cyclopentène-1-yle, cyclohexèn-1-yle, ou 3,6-dihydro-2H-pyran-4-yle, et l'indice n est 0, 1, 2, 3, ou 4.

4. Composé de la revendication 1 ou la revendication 2, ayant la formule (Ib-1) : 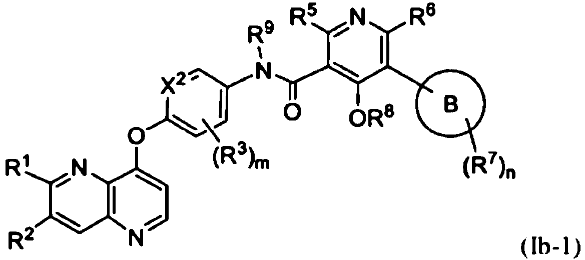 ou sel pharmaceutiquement acceptable, stéréoisomère, ou tautomère de celui-ci, dans lequel l'anneau B est cyclopentène-1-yle, cyclohexèn-1-yle, ou 3,6-dihydro-2H-pyran-4-yle, et l'indice n est 0, 1, 2, 3, ou 4.

5. Composé de la revendication 1 ou la revendication 2, ayant la formule (Ic-1) : 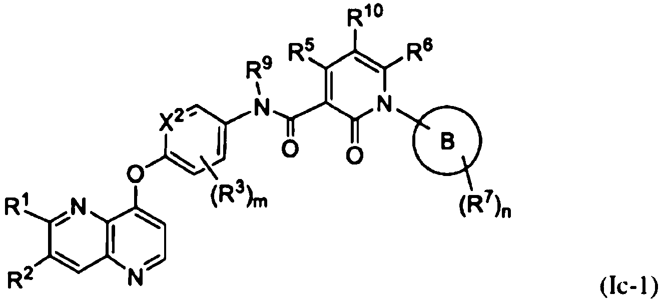 ou sel pharmaceutiquement acceptable, stéréoisomère, ou tautomère de celui-ci, dans lequel l'anneau B est cyclopentène-1-yle, cyclohexèn-1-yle, ou 3,6-dihydro-2H-pyran-4-yle, et l'indice n est 0, 1, 2, 3 ou 4.

6. Composé de l'une quelconque des revendications 1 à 5, ou sel pharmaceutiquement acceptable, stéréoisomère, ou tautomère de celui-ci, dans lequel :
R¹ est H, alkyle en C₁₋₆, alkoxy en C₁₋₆, halo, NH₂, -NH(alkyle en C₁₋₆), -N(alkyle en C₁₋₆)₂, alkyle en C₁₋₆NHC(O)-, ou alkyle en C₁₋₆SO₂NH- ;
R¹ est H ou alkoxy en C₁₋₆ ;
R² est H, alkyle en C₁₋₆, alkoxy en C₁₋₆, halo, OH, NH₂, -NH(alkyle en C₁₋₆), - N(alkyle en C₁₋₆)₂, -alkyle en C₁₋₆NHC(O)-, CF₃, alkyle en C₁₋₆OC(O)-, pyridyle, alkyle en C₁₋₆SO₂NH-, ou 1H-pyrazol-4-yle facultativement substitué avec R^{g} ; et/ou
R² est H ou alkoxy en C₁₋₆ facultativement substitué avec l'alkoxy en C₁₋₆.

7. Composé de l'une quelconque des revendications 1 à 6, ou sel pharmaceutiquement acceptable, stéréoisomère, ou tautomère de celui-ci, dans lequel :
un de R¹ et de R² est OR^{a} et R^{a} est alkyle en C₁₋₆ substitué avec un R^{d} ; ou
R² est OR^{a} ; R^{a} est alkyle en C₁₋₆ substitué avec un R^{d} ; et R^{d} est OR^{e} ou cycloalkyle en C₃₋₁₀, et dans lequel R^{e} est alkyle en C₁₋₆.

8. Composé de l'une quelconque des revendications 1 à 7, ou sel pharmaceutiquement acceptable, stéréoisomère, ou tautomère de celui-ci, dans lequel
R⁷ est H, halo, alkyle en C₁₋₆ ou alkoxy en C₁₋₆ ; et/ou
R⁹ est H ou méthyle.

9. Composé de l'une quelconque des revendications 1 à 8, ou sel pharmaceutiquement acceptable, stéréoisomère, ou tautomère de celui-ci, dans lequel :
R⁴ est sélectionné parmi H, alkyle en C₁₋₆, alkoxy en C₁₋₆, OH, cycloalkyle en C₃₋₆, haloalkyle en C₁₋₆, cycloalkyle en C₃₋₆-alkylène en C₁₋₄-, hétérocycloalkyle à 4 à 6 membres, (hétérocycloalkyle à 4 à 6 membres)-alkylène en C₁₋₄-, hétéroaryle à *5* à 6 membres, (hétéroaryle à *5* à 6 membres)-alkylène en C₁₋₄-, et N=C[N(alkyle en C₁₋₆)(alkyle en C₁₋₆)]₂, dans lequel l'alkyle en C₁₋₆, alkoxy en C₁₋₆, cycloalkyle en C₃₋₆, cycloalkyle en C₃₋₆-alkylène en C₁₋₄-, hétérocycloalkyle à 4 à 6 membres, (hétérocycloalkyle à 4 à 6 membres)-alkylène en C₁₋₄-, hétéroaryle à *5* à 6 membres, (hétéroaryle à *5* à 6 membres)-alkylène en C₁₋₄-, et N=C[N(alkyle en C₁₋₆)(alkyle en C₁₋₆)]₂ de R⁴ sont chacun facultativement substitués avec 1 ou 2 substituants R^{b} ou R^{g} indépendamment sélectionnés ;
R⁴ est alkyle en C₁₋₆ ou haloalkyle en C₁₋₆ ; ou
R⁴ et R⁵ considérés conjointement avec les atomes auxquels ils sont attachés forment hétérocycloalkyle fusionné à 4 à 7 membres.

10. Composé de l'une quelconque des revendications 1 à 9, ou sel pharmaceutiquement acceptable, stéréoisomère, ou tautomère de celui-ci, dans lequel :
R⁵ et R⁶ sont chacun indépendamment sélectionnés parmi H, CH₃, propèn-2-yle, Br, Cl, CN, méthoxy, 2- fluoroéthyle, isopropyle, CH₃C(O)-, OH, t-butyle, éthyle, hydroxyméthyle, isopropylthio, et méthoxyméthyle ;
R¹⁰ est H, CH₃, propèn-2-yl, Br, Cl, CN, méthoxy, 2-fluoroéthyle, isopropyle, CH₃C(O)-, OH, t-butyle, éthyle, hydroxyméthyle, isopropylthio, ou méthoxyméthyle ; et/ou
R⁸ est indépendamment H ou alkyle en C₁₋₆.

11. Composé de la revendication 1, dans lequel le composé est sélectionné parmi :
A :
7-(cyclopentèn-1-yl)-N-[4-[(6,7-diméthoxy-1,5-naphtyridin-4-yl)oxy]-3-fluorophényl]-8-oxo-3,4-dihydro-1H-pyrido[2,1-c][1,4]oxazine-9-carboxamide ;
5-(cyclopentèn-1-yl)-N-[4-[(6,7-diméthoxy-1,5-naphtyridin-4-yl)oxy]-3-fluorophényl]-4-hydroxy-6-méthylpyridine-3-carboxamide ;
5-(cyclohexèn-1-yl)-N-[4-[(6,7-diméthoxy-1,5-naphtyridin-4-yl)oxy]-3-fluorophényl]-4-hydroxy-6-méthylpyridine-3-carboxamide ;
7-(cyclohexèn-1-yl)-N-[4-[(6,7-diméthoxy-1,5-naphtyridin-4-yl)oxy]-3-fluorophényl]-8-oxo-3,4-dihydro-1H-pyrido[2,1-c][1,4]oxazine-9-carboxamide ;
7-(cyclohexèn-1-yl)-N-[4-[(6,7-diméthoxy-1,5-naphtyridin-4-yl)oxy]phényl]-8-oxo-3,4-dihydro-1H-pyrido[2,1-c][1,4]oxazine-9-carboxamide ;
7-(cyclopentèn-1-yl)-N-[4-[(6,7-diméthoxy-1,5-naphtyridin-4-yl)oxy]phényl]-8-oxo-3,4-dihydro-1H-pyrido[2,1-c][1,4]oxazine-9-carboxamide ;
7-(3,6-dihydro-2H-pyran-4-yl)-N-[4-[(6,7-diméthoxy-1,5-naphtyridin-4-yl)oxy]-3-fluorophényl]-8-oxo-3,4-dihydro-1H-pyrido[2,1-c][1,4]oxazine-9-carboxamide ;
7-(3,6-dihydro-2H-pyran-4-yl)-N-[4-[(6,7-diméthoxy-1,5-naphtyridin-4-yl)oxy]phényl]-8-oxo-3,4-dihydro-1H-pyrido[2,1-c][1,4]oxazine-9-carboxamide ;
5-(cyclopentèn-1-yl)-N-[4-[(6,7-diméthoxy-1,5-naphtyridin-4-yl)oxy]-3-fluorophényl]-4-hydroxy-2,6-diméthylpyridine-3-carboxamide ;
5-(3,6-dihydro-2H-pyran-4-yl)-N-[4-[(6,7-diméthoxy-1,5-naphtyridin-4-yl)oxy]-3-fluorophényl]-4-hydroxy-6-méthylpyridine-3-carboxamide ;
5-(3,6-dihydro-2H-pyran-4-yl)-N-[3-fluoro-4-[(7-méthoxy-1,5-naphtyridin-4-yl)oxy]phényl]-4-hydroxy-6-méthylpyridine-3-carboxamide ;
5-(3,6-dihydro-2H-pyran-4-yl)-N-[3-fluoro-4-[(6-méthoxy-1,5-naphtyridin-4-yl)oxy]phényl]-4-hydroxy-6-méthylpyridine-3-carboxamide ;
5-(cyclohexèn-1-yl)-N-[3-fluoro-4-[(6-méthoxy-1,5-naphtyridin-4-yl)oxy]phényl]-4-hydroxy-6-méthylpyridine-3-carboxamide ;
5-(cyclopentèn-1-yl)-N-[3-fluoro-4-[(6-méthoxy-1,5-naphtyridin-4-yl)oxy]phényl]-4-hydroxy-6-méthylpyridine-3-carboxamide ;
5-(cyclohexèn-1-yl)-N-[3-fluoro-4-[(7-méthoxy-1,5-naphtyridin-4-yl)oxy]phényl]-4-hydroxy-6-méthylpyridine-3-carboxamide ;
5-(cyclopentèn-1-yl)-N-[3-fluoro-4-[(7-méthoxy-1,5-naphtyridin-4-yl)oxy]phényl]-4-hydroxy-6-méthylpyridine-3-carboxamide ; et
5-(cyclohexèn-1-yl)-N-[4-[(6,7-diméthoxy-1,5-naphtyridin-4-yl)oxy]-3-fluorophényl]-4-hydroxy-2,6-diméthylpyridine-3-carboxamide ;
et sel pharmaceutiquement acceptables, stéréoisomères ou tautomères de celui-ci, ou
B :
5-(cyclopentèn-1-yl)-N-[4-[(6,7-diméthoxy-1,5-naphtyridin-4-yl)oxy]-3-fluorophényl]-1,6-diméthyl-4-oxopyridine-3-carboxamide ;
5-cyclopentyl-N-[4-[(6,7-diméthoxy-1,5-naphtyridin-4-yl)oxy]-3-fluorophényl]-1,6-diméthyl-4-oxopyridine-3-carboxamide ;
N-[3-fluoro-4-[(7-méthoxy-1,5-naphtyridin-4-yl)oxy]phényl]-4-hydroxy-2,6-diméthyl-5-prop-1-èn-2-ylpyridine-3-carboxamide ;
N-[4-[(6,7-diméthoxy-1,5-naphtyridin-4-yl)oxy]phényl]-4-hydroxy-2,6-diméthyl-5-prop-1-èn-2-ylpyridine-3-carboxamide ;
N-[4-[(6,7-diméthoxy-1,5-naphtyridin-4-yl)oxy]-3-fluorophényl]-4-hydroxy-2,6-diméthyl-5-prop-1-èn-2-ylpyridine-3-carboxamide ;
N-[4-[(6,7-diméthoxy-1,5-naphtyridin-4-yl)oxy]-2,5-difluorophényl]-4-hydroxy-2,6-diméthyl-5-prop-1-èn-2-ylpyridine-3-carboxamide ;
5-(3,6-dihydro-2H-pyran-4-yl)-4-hydroxy-N-[4-[(7-méthoxy-1,5-naphtyridin-4-yl)oxy]phényl]-6-méthylpyridine-3-carboxamide ;
5-(cyclohexèn-1-yl)-N-[4-[(6,7-diméthoxy-1,5-naphtyridin-4-yl)oxy]-3-fluorophényl]-1-(2-fluoroéthyl)-6-méthyl-4-oxopyridine-3-carboxamide ;
N-[4-[(6,7-diméthoxy-1,5-naphtyridin-4-yl)oxy]phényl]-4-hydroxy-2,6-diméthyl-5-propan-2-ylpyridine-3-carboxamide ;
N-[4-[(6,7-diméthoxy-1,5-naphtyridin-4-yl)oxy]-2,5-difluorophényl]-4-hydroxy-2,6-diméthyl-5-propan-2-ylpyridine-3-carboxamide ;
5-(cyclopentèn-1-yl)-N-[4-[(6,7-diméthoxy-1,5-naphtyridin-4-yl)oxy]-3-fluorophényl]-1-(2-fluoroéthyl)-6-méthyl-4-oxopyridine-3-carboxamide ;
5-(3,6-dihydro-2H-pyran-4-yl)-N-[3-fluoro-4-[(7-méthoxy-1,5-naphtyridin-4-yl)oxy]phényl]-1,6-diméthyl-4-oxopyridine-3-carboxamide ;
5-cyclopentyl-N-[4-[(6,7-diméthoxy-1,5-naphtyridin-4-yl)oxy]-3-fluorophényl]-1-(2-fluoroéthyl)-6-méthyl-4-oxopyridine-3-carboxamide ;
1-(cyclopentèn-1-yl)-N-[4-[(6,7-diméthoxy-1,5-naphtyridin-4-yl)oxy]-3-fluorophényl]-4,6-diméthyl-2-oxopyridine-3-carboxamide ;
1-cyclopentyl-N-[4-[(6,7-diméthoxy-1,5-naphtyridin-4-yl)oxy]-3-fluorophényl]-4,6-diméthyl-2-oxopyridine-3-carboxamide ;
N-[4-[(6,7-diméthoxy-1,5-naphtyridin-4-yl)oxy]-3-fluorophényl]-4,6-diméthyl-2-oxo-1-prop-1-èn-2-ylpyridine-3-carboxamide ;
N-[3-fluoro-4-[(7-méthoxy-1,5-naphtyridin-4-yl)oxy]phényl]-4,6-diméthyl-2-oxo-1-prop-1-èn-2-ylpyridine-3-carboxamide ;
5-bromo-1-cyclopentyl-N-[4-[(6,7-diméthoxy-1,5-naphtyridin-4-yl)oxy]-3-fluorophényl]-4,6-diméthyl-2-oxopyridine-3-carboxamide ;
5-[(E)-2-cyclopentylethényl]-N-[4-[(6,7-diméthoxy-1,5-naphtyridin-4-yl)oxy]-3-fluorophényl]-4-hydroxy-6-méthylpyridine-3-carboxamide ;
5-[(E)-2-cyclopropylethényl]-N-[4-[(6,7-diméthoxy-1,5-naphtyridin-4-yl)oxy]-3-fluorophényl]-4-hydroxy-6-méthylpyridine-3-carboxamide ;
N-[4-[(6,7-diméthoxy-1,5-naphtyridin-4-yl)oxy]-3-fluorophényl]-5-[(E)-3,3-diméthylbut-1-ényl]-4-hydroxy-6-méthylpyridine-3-carboxamide ;
N-[4-[(6,7-diméthoxy-1,5-naphtyridin-4-yl)oxy]-3-fluorophényl]-4-hydroxy-6-méthyl-5-[(E)-4-méthylpent-1-ényl]pyridine-3-carboxamide ;
N-[4-[(6,7-diméthoxy-1,5-naphtyridin-4-yl)oxy]-3-fluorophényl]-4-hydroxy-2,6-diméthyl-5-propan-2-ylpyridine-3-carboxamide ;
N-[3-fluoro-4-[(7-méthoxy-1,5-naphtyridin-4-yl)oxy]phényl]-4-hydroxy-2,6-diméthyl-5-propan-2-ylpyridine-3-carboxamide ;
5-(cyclopentèn-1-yl)-N-[3-fluoro-4-[[6-méthoxy-7-(2-méthoxyéthoxy)-1,5-naphtyridin-4-yl]oxy]phényl]-4-hydroxy-6-méthylpyridine-3-carboxamide ;
5-(cyclohexèn-1-yl)-N-[3-fluoro-4-[[6-méthoxy-7-(2-méthoxyéthoxy)-1,5-naphtyridin-4-yl]oxy]phényl]-4-hydroxy-6-méthylpyridine-3-carboxamide ;
5-(3,6-dihydro-2H-pyran-4-yl)-N-[3-fluoro-4-[[6-méthoxy-7-(2-méthoxyéthoxy)-1,5-naphtyridin-4-yl]oxy]phényl]-4-hydroxy-6-méthylpyridine-3-carboxamide ;
5-[(E)-2-cyclopentylethényl]-N-[3-fluoro-4-[[6-méthoxy-7-(2-méthoxyéthoxy)-1,5-naphtyridin-4-yl]oxy]phényl]-4-hydroxy-6-méthylpyridine-3-carboxamide ;
5-[(E)-2-cyclopropylethényl]-N-[3-fluoro-4-[[6-méthoxy-7-(2-méthoxyéthoxy)-1,5-naphtyridin-4-yl]oxy]phényl]-4-hydroxy-6-méthylpyridine-3-carboxamide ;
N-[4-[(6,7-diméthoxy-1,5-naphtyridin-4-yl)oxy]-3-fluorophényl]-4-méthyl-2-oxo-1-propan-2-ylpyridine-3-carboxamide ;
N-[4-[(6,7-diméthoxy-1,5-naphtyridin-4-yl)oxy]phényl]-4-méthyl-2-oxo-1-propan-2-ylpyridine-3-carboxamide ;
5-[(E)-3,3-diméthylbut-1-ényl]-N-[3-fluoro-4-[[6-méthoxy-7-(2-méthoxyéthoxy)-1,5-naphtyridin-4-yl]oxy]phényl]-4-hydroxy-6-méthylpyridine-3-carboxamide ;
5-(cyclopentèn-1-yl)-N-[3-fluoro-4-[[6-méthoxy-7-(2-méthoxyéthoxy)-1,5-naphtyridin-4-yl]oxy]phényl]-4-hydroxy-2,6-diméthylpyridine-3-carboxamide ;
5-(cyclopentèn-1-yl)-N-[3-fluoro-4-[[6-méthoxy-7-(2-méthoxyéthoxy)-1,5-naphtyridin-4-yl]oxy]phényl]-1,2,6-triméthyl-4-oxopyridine-3-carboxamide ;
5-(3,6-dihydro-2H-pyran-4-yl)-N-[3-fluoro-4-[[6-méthoxy-7-(2-méthoxyéthoxy)-1,5-naphtyridin-4-yl]oxy]phényl]-4-hydroxy-2,6-diméthylpyridine-3-carboxamide ;
5-(3,6-dihydro-2H-pyran-4-yl)-N-[3-fluoro-4-[[6-méthoxy-7-(2-méthoxyéthoxy)-1,5-naphtyridin-4-yl]oxy]phényl]-1,2,6-triméthyl-4-oxopyridine-3-carboxamide ;
5-[(E)-3,3-diméthylbut-1-ényl]-N-[3-fluoro-4-[[6-méthoxy-7-(2-méthoxyéthoxy)-1,5-naphtyridin-4-yl]oxy]phényl]-1,2,6-triméthyl-4-oxopyridine-3-carboxamide ;
5-cyclopropyl-N-[4-[(6,7-diméthoxy-1,5-naphtyridin-4-yl)oxy]-3-fluorophényl]-1,2,6-triméthyl-4-oxopyridine-3-carboxamide ;
5-cyclopropyl-N-[4-[(6,7-diméthoxy-1,5-naphtyridin-4-yl)oxy]-2,5-difluorophényl]-1,2,6-triméthyl-4-oxopyridine-3-carboxamide ;
5-cyclopropyl-N-[4-[(6,7-diméthoxy-1,5-naphtyridin-4-yl)oxy]phényl]-1,2,6-triméthyl-4-oxopyridine-3-carboxamide ;
N-[4-[(6,7-diméthoxy-1,5-naphtyridin-4-yl)oxy]-3-fluorophényl]-5-[(E)-3,3-diméthylbut-1-ényl]-1,2,6-triméthyl-4-oxopyridine-3-carboxamide ;
5-[(E)-2-cyclopropylethényl]-N-[4-[(6,7-diméthoxy-1,5-naphtyridin-4-yl)oxy]-3-fluorophényl]-1,2,6-triméthyl-4-oxopyridine-3-carboxamide ;
5-[(E)-2-cyclopropylethényl]-N-[3-fluoro-4-[[6-méthoxy-7-(2-méthoxyéthoxy)-1,5-naphtyridin-4-yl]oxy]phényl]-1,2,6-triméthyl-4-oxopyridine-3-carboxamide ;
N-[4-[(6,7-diméthoxy-1,5-naphtyridin-4-yl)oxy]-3-fluorophényl]-1,2,6-triméthyl-4-oxo-5-prop-1-èn-2-ylpyridine-3-carboxamide ;
5-(3,6-dihydro-2H-pyran-4-yl)-N-[4-[(6,7-diméthoxy-1,5-naphtyridin-4-yl)oxy]-3-fluorophényl]-1,2,6-triméthyl-4-oxopyridine-3-carboxamide ;
5-[(E)-2-cyclopentylethényl]-N-[3-fluoro-4-[[6-méthoxy-7-(2-méthoxyéthoxy)-1,5-naphtyridin-4-yl]oxy]phényl]-1,2,6-triméthyl-4-oxopyridine-3-carboxamide ;
N-[4-[(6,7-diméthoxy-1,5-naphtyridin-4-yl)oxy]phényl]-1,2,6-triméthyl-4-oxo-5-prop-1-èn-2-ylpyridine-3-carboxamide ;
N-[4-[(6,7-diméthoxy-1,5-naphtyridin-4-yl)oxy]-3-fluorophényl]-1,2,6-triméthyl-4-oxo-5-propan-2-ylpyridine-3-carboxamide ;
N-[4-[(6,7-diméthoxy-1,5-naphtyridin-4-yl)oxy]phényl]-1,2,6-triméthyl-4-oxo-5-propan-2-ylpyridine-3-carboxamide ;
5-(cyclohexèn-1-yl)-N-[3-fluoro-4-[[6-méthoxy-7-(2-méthoxyéthoxy)-1,5-naphtyridin-4-yl]oxy]phényl]-1,2,6-triméthyl-4-oxopyridine-3-carboxamide ;
4-hydroxy-5-méthoxy-N-[4-[(7-méthoxy-1,5-naphtyridin-4-yl)oxy]phényl]-2,6-diméthylpyridine-3-carboxamide ;
N-[3-fluoro-4-[(7-méthoxy-1,5-naphtyridin-4-yl)oxy]phényl]-4-hydroxy-5-méthoxy-2,6-diméthylpyridine-3-carboxamide ;
N-[4-[(6,7-diméthoxy-1,5-naphtyridin-4-yl)oxy]phényl]-4-hydroxy-5-méthoxy-2,6-diméthylpyridine-3-carboxamide ;
N-[4-[(6,7-diméthoxy-1,5-naphtyridin-4-yl)oxy]-3-fluorophényl]-4-hydroxy-5-méthoxy-2,6-diméthylpyridine-3-carboxamide ;
5-bromo-N-[4-[(6,7-diméthoxy-1,5-naphtyridin-4-yl)oxy]-2,5-difluorophényl]-6-éthyl-1-méthyl-4-oxopyridine-3-carboxamide ;
5-bromo-6-éthyl-N-[4-[[6-méthoxy-7-(2-méthoxyéthoxy)-1,5-naphtyridin-4-yl]oxy]phényl]-1-méthyl-4-oxopyridine-3-carboxamide ;
5-(cyclopentèn-1-yl)-N-[3-fluoro-4-[[7-(2-méthoxyéthoxy)-1,5-naphtyridin-4-yl]oxy]phényl]-1,2,6-triméthyl-4-oxopyridine-3-carboxamide ;
5-(cyclohexèn-1-yl)-N-[3-fluoro-4-[[7-(2-méthoxyéthoxy)-1,5-naphtyridin-4-yl]oxy]phényl]-1,2,6-triméthyl-4-oxopyridine-3-carboxamide ;
5-(3,6-dihydro-2H-pyran-4-yl)-N-[3-fluoro-4-[[7-(2-méthoxyéthoxy)-1,5-naphtyridin-4-yl]oxy]phényl]-1,2,6-triméthyl-4-oxopyridine-3-carboxamide ;
N-[4-[(6,7-diméthoxy-1,5-naphtyridin-4-yl)oxy]-2,5-difluorophényl]-4-hydroxy-5-méthoxy-2,6-diméthylpyridine-3-carboxamide ;
7-(cyclopentèn-1-yl)-N-[4-[(6,7-diméthoxy-1,5-naphtyridin-4-yl)oxy]-3-fluorophényl]-6-méthyl-8-oxo-3,4-dihydro-1H-pyrido[2,1-c][1,4]oxazine-9-carboxamide ;
N-[3-fluoro-4-[[6-méthoxy-7-(2-méthoxyéthoxy)-1,5-naphtyridin-4-yl]oxy]phényl]-4-hydroxy-5-méthoxy-2,6-diméthylpyridine-3-carboxamide ;
4-hydroxy-5-méthoxy-N-[4-[[6-méthoxy-7-(2-méthoxyéthoxy)-1,5-naphtyridin-4-yl]oxy]phényl]-2,6-diméthylpyridine-3-carboxamide ;
7-(3,6-dihydro-2H-pyran-4-yl)-N-[4-[(6,7-diméthoxy-1,5-naphtyridin-4-yl)oxy]phényl]-6-méthyl-8-oxo-3,4-dihydro-1H-pyrido[2,1-c][1,4]oxazine-9-carboxamide ;
N-[4-[[7-(2-cyclobutyléthoxy)-6-méthoxy-1,5-naphtyridin-4-yl]oxy]-3-fluorophényl]-5-(cyclopentèn-1-yl)-1,2,6-triméthyl-4-oxopyridine-3-carboxamide ;
N-[3-fluoro-4-[(7-méthoxy-1,5-naphtyridin-4-yl)oxy]phényl]-5-méthoxy-1,2,6-triméthyl-4-oxopyridine-3-carboxamide ;
N-[4-[(6,7-diméthoxy-1,5-naphtyridin-4-yl)oxy]phényl]-5-méthoxy-1,2,6-triméthyl-4-oxopyridine-3-carboxamide ;
N-[4-[(6,7-diméthoxy-1,5-naphtyridin-4-yl)oxy]-3-fluorophényl]-5-méthoxy-1,2,6-triméthyl-4-oxopyridine-3-carboxamide ;
5-cyano-N-[4-[(6,7-diméthoxy-1,5-naphtyridin-4-yl)oxy]-3-fluorophényl]-1,2,6-triméthyl-4-oxopyridine-3-carboxamide ;
7-(3,6-dihydro-2H-pyran-4-yl)-N-[4-[(6,7-diméthoxy-1,5-naphtyridin-4-yl)oxy]-3-fluorophényl]-6-méthyl-8-oxo-3,4-dihydro-1H-pyrido[2,1-c][1,4]oxazine-9-carboxamide ;
7-(cyclohexèn-1-yl)-N-[4-[(6,7-diméthoxy-1,5-naphtyridin-4-yl)oxy]-3-fluorophényl]-6-méthyl-8-oxo-3,4-dihydro-1H-pyrido[2,1-c][1,4]oxazine-9-carboxamide ;
5-cyano-N-[3-fluoro-4-[(7-méthoxy-1,5-naphtyridin-4-yl)oxy]phényl]-1,2,6-triméthyl-4-oxopyridine-3-carboxamide ;
5-cyano-N-[4-[(6,7-diméthoxy-1,5-naphtyridin-4-yl)oxy]phényl]-1,2,6-triméthyl-4-oxopyridine-3-carboxamide ;
5-cyano-N-[2,5-difluoro-4-[[6-méthoxy-7-(2-méthoxyéthoxy)-1,5-naphtyridin-4-yl]oxy]phényl]-1,2,6-triméthyl-4-oxopyridine-3-carboxamide ;
3-N-[4-[(6,7-diméthoxy-1,5-naphtyridin-4-yl)oxy]-3-fluorophényl]-4-hydroxy-2,6-diméthylpyridine-3,5-dicarboxamide ;
3-N-[4-[(6,7-diméthoxy-1,5-naphtyridin-4-yl)oxy]phényl]-4-hydroxy-2,6-diméthylpyridine-3,5-dicarboxamide ;
3-N-[2,5-difluoro-4-[[6-méthoxy-7-(2-méthoxyéthoxy)-1,5-naphtyridin-4-yl]oxy]phényl]-4-hydroxy-2,6-diméthylpyridine-3,5-dicarboxamide ;
5-cyano-N-[4-[(6,7-diméthoxy-1,5-naphtyridin-4-yl)oxy]-3-fluorophényl]-4-hydroxy-2,6-diméthylpyridine-3-carboxamide ;
N-[4-[(6,7-diméthoxy-1,5-naphtyridin-4-yl)oxy]-3-fluorophényl]-1,2,6-triméthyl-5-morpholin-4-yl-4-oxopyridine-3-carboxamide ;
N-[4-[(6,7-diméthoxy-1,5-naphtyridin-4-yl)oxy]-3-fluorophényl]-1,2,6-triméthyl-4-oxo-5-pyrrolidin-1-ylpyridine-3-carboxamide ;
N-[4-[(6,7-diméthoxy-1,5-naphtyridin-4-yl)oxy]-3-fluorophényl]-1,2,6-triméthyl-4-oxo-5-pipéridin-1-ylpyridine-3-carboxamide ;
3-N-[3-fluoro-4-[(7-méthoxy-1,5-naphtyridin-4-yl)oxy]phényl]-4-hydroxy-2,6-diméthylpyridine-3,5-dicarboxamide ;
acide 5-[[4-[(6,7-diméthoxy-1,5-naphtyridin-4-yl)oxy]-3-fluorophényl]carbamoyl]-1,2,6-triméthyl-4-oxopyridine-3-carboxylique ;
3-N-[4-[(6,7-diméthoxy-1,5-naphtyridin-4-yl)oxy]-3-fluorophényl]-4-hydroxy-5-N,5-N,2,6-tetraméthylpyridine-3,5-dicarboxamide ;
3-N-[4-[(6,7-diméthoxy-1,5-naphtyridin-4-yl)oxy]-3-fluorophényl]-4-hydroxy-2,6-diméthyl-5-N-propan-2-ylpyridine-3,5-dicarboxamide ;
5-N-[4-[(6,7-diméthoxy-1,5-naphtyridin-4-yl)oxy]-3-fluorophényl]-3-N,3-N, 1,2,6-pentaméthyl-4-oxopyridine-3,5-dicarboxamide ;
3-N-cyclopropyl-5-N-[4-[(6,7-diméthoxy-1,5-naphtyridin-4-yl)oxy]-3-fluorophényl]-1,2,6-triméthyl-4-oxopyridine-3,5-dicarboxamide ;
3-N-[4-[(6,7-diméthoxy-1,5-naphtyridin-4-yl)oxy]-3-fluorophényl]-4-hydroxy-5-N,2,6-triméthylpyridine-3,5-dicarboxamide ;
5-N-cyclopropyl-3-N-[4-[(6,7-diméthoxy-1,5-naphtyridin-4-yl)oxy]-3-fluorophényl]-4-hydroxy-2,6-diméthylpyridine-3,5-dicarboxamide ;
7-(cyclopentèn-1-yl)-N-[4-[(6,7-diméthoxy-1,5-naphtyridin-4-yl)oxy]phényl]-6-méthyl-8-oxo-3,4-dihydro-1H-pyrido[2,1-c][1,4]oxazine-9-carboxamide ;
7-(cyclohexèn-1-yl)-N-[4-[(6,7-diméthoxy-1,5-naphtyridin-4-yl)oxy]phényl]-6-méthyl-8-oxo-3,4-dihydro-1H-pyrido[2,1-c][1,4]oxazine-9-carboxamide ; et
5-(cyclopentèn-1-yl)-N-[3-fluoro-4-[[7-(2-méthoxyéthoxy)-1,5-naphtyridin-4-yl]oxy]phényl]-4-hydroxy-2,6-diméthylpyridine-3-carboxamide ;
et sel pharmaceutiquement acceptables, stéréoisomères, ou tautomères de celui-ci.

12. Composition pharmaceutique comprenant un composé de l'une quelconque des revendications 1 à 11, ou un sel pharmaceutiquement acceptable, stéréoisomère, ou tautomère de celui-ci, et un vecteur ou excipient pharmaceutiquement acceptable.

13. Composé de l'une quelconque des revendications 1 à 11, ou sel pharmaceutiquement acceptable, stéréoisomère, ou tautomère de celui-ci, ou composition pharmaceutique de la revendication 12, pour utilisation dans une méthode de traitement d'une maladie, d'un trouble, ou d'un syndrome dont la médiation est effectuée en modulant l'activité in vivo d'une protéine kinase,
facultativement dans lequel la protéine kinase est AXL, KDR, Mer, ou Met.

14. Procédé de préparation d'un composé de la formule (Ia), de la formule (Ib), ou de la formule (Ic), ou d'un sel pharmaceutiquement acceptable, stéréoisomère ou tautomère de celui-ci, tel que défini dans l'une quelconque des revendications 1 à 11, dans lequel :
le procédé comprend la mise en contact d'un composé de la formule A-a, de la formule A-b, ou de la formule A-c : 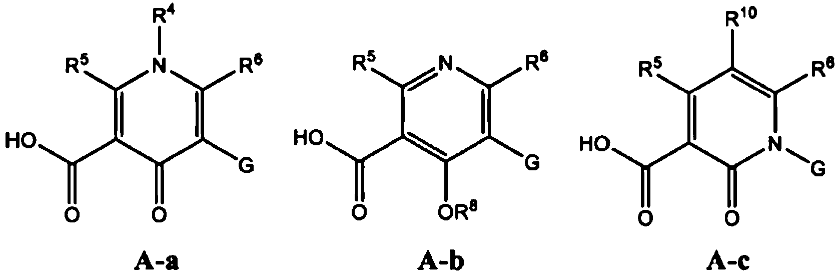
avec un composé de la formule B-c : 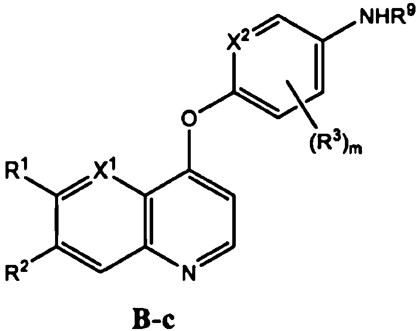
dans des conditions de formation de liaison amide pour fournir le composé de la formule (Ia), de la formule (Ib), ou de la formule (Ic), ou un sel pharmaceutiquement acceptable, stéréoisomère ou tautomère de celui-ci, dans lequel :
X¹, X², R¹, R², R³, R⁴, R⁵, R⁶, R⁸, R⁹, R¹⁰, G et m sont tels que définis dans la revendication 1.

15. Procédé de la revendication 14, dans lequel les conditions de formation de liaison amide comprennent HATU en présence d'une base dans un solvant organique.
